# EUROPEAN PATENT SPECIFICATION

(11) **EP 2 488 481 B1**
(45) Date of publication and mention of the grant of the patent: **13.12.2017**
(21) Application number: 10768839.2
(22) Date of filing: 13.10.2010
(51) Int. Cl.: C07C 69/90, C09K 11/06

(54) **CHEMICAL LIGHT PRODUCING FORMULATIONS CONTAINING BRANCHED OXALATE ESTERS**
CHEMILUMINESZENTE FORMULIERUNGEN MIT VERZWEIGTEN OXALATESTERN
FORMULATIONS CHIMILUMINESCENTS CONTENANT DES ESTERS DE TYPE OXALATES RAMIFIÉS

(30) Priority: 13.10.2009 US 250915 P
(43) Date of publication of application: 22.08.2012
(73) Proprietor: Cyalume Technologies, Inc, West Springfield, MA 01089 (US)
(72) Inventor: CRANOR, Earl, Longmeadow MA 01106 (US); JACOB, Linda, Woodbridge CT 06525 (US)
(74) Representative: HGF Limited
(86) International application number: PCT/US2010/052479
(87) International publication number: WO 2011/047026

(56) References cited:
- US-A- 5 194 666
- C. D. DOWD, D. B. PAUL: "Synthesis and Evaluation of Diaryl Oxalate Esters for Low-Intensity Chemiluminescent Illumination", AUSTRALIAN JOURNAL OF CHEMISTRY, vol. 37, 1 January 1984 (1984-01-01), pages 73-86, XP008134316,
- DATABASE CA [Online] CHEMICAL ABSTRACTS SERVICE, COLUMBUS, OHIO, US LISOWSKI, WIESLAW ET AL: 'Chemiluminescent compositions' Retrieved from STN Database accession no. 1994:90354 & PL 157 634 B1 (POLSKA AKADEMIA NAUK, CENTRUM BADAN MOLEKULARNYCH I MAKROMOLEKULARNYCH) 30 June 1992 (1992-06-30)

## Description

### FIELD OF THE INVENTION

This invention relates to chemical light producing devices and the chemiluminescent oxalate esters contained therein, which exhibit reduced hydrolysis owing to the utilization of non-straight chain oxalate ester (i.e. a ring structure oxalate ester), and thereby are characterized by an inherently long shelf-life and commercial viability.

### BACKGROUND OF THE INVENTION

Chemical light devices are useful for providing a source of non-electric light and have become exceedingly popular especially for emergency and outdoor use. Typical chemical light devices contain two liquids that produce light upon mixing. Typically, one of these liquids was referred to as an oxalate solution. It typically contained a carrier solvent, an oxalate ester, and a fluorescer. The second liquid was referred to as an activator solution. It typically contained a carrier solvent, hydrogen peroxide, and a catalyst.

The oxalate ester is the component of the system which is most deleteriously affected by moisture, although other components such as yellow and blue fluorescers and salicylate catalysts may also be affected. The instant invention provides oxalate esters and chemical light systems comprising an activator solution and a branched chain oxalic acid ester.

A typical commercially available chemiluminescent device of the dual container variety contains a diluent solution of oxalate ester and fluorescer in the outer container and a diluent solution of hydrogen peroxide and catalyst in the inner container. Typical devices are disclosed in one or more of the following U.S. Pat. Nos. 3,511,612; 3,539,794; 3,576,987; 3,584,211; 3,654,525; 3,749,620; 3,752,406; 3,800,132; 3,808,414; 3,940,604; 3,974,368; 4,064,428.

The chemiluminescent light is obtained by the reaction of the hydrogen peroxide of the activator solution with the chemiluminescent composition which comprises the oxalate ester, fluorescer, and catalyst. Typical oxalate esters have heretofore included compounds such as bis(2-nitrophenyl)oxalate, bis(2,4-dinitrophenyl)oxalate, bis(2,6-dichloro-4-nitrophenyl)oxalate, bis(2,4,6-trichlorophenyl)oxalate, bis(3-trifluoromethyl-4-nitrophenyl)oxalate, bis(2-methyl-4,6-dinitrophenyl)oxalate, bis(1,2-dimethyl-4,6-dinitrophenyl)oxalate, bis(2,4-dichlorophenyl)oxalate, bis(2,5-dinitrophenyl)oxalate, bis(2-formyl-4-nitrophenyl)oxalate, bis(pentachlorophenyloxalate, bis(1,2-dihydro-2-oxo-1-pyridyl)glyoxal, bis-N-phthalmidyl oxalate. The preferred oxalate, is bis{3,4,6-trichloro-2-[(pentoxy)carbonyl] phenyl} oxalate.

The extreme sensitivity of these oxalates to water hydrolysis has created the need to package them carefully and store under refrigerated conditions. Water can and will migrate, at varying rates, through most non-metal and non-glass materials of construction. If the prior art oxalate esters were not packaged within a polymer lined metal (foil) pouch that is then stored in an inert (dry) gas containing metal drum that is stored under refrigerated conditions, the oxalate ester would hydrolyze and would fail to retain commercially viable shelf life. C. D. Dowd et al., Aust. J. Chem., 1984, 37, 73-86, describes the synthesis and evaluation of diaryl oxalate esters for low-intensity chemiluminescent illumination. The present invention discloses a novel family of branched chain oxalate esters which are useful in chemical lighting systems while being substantially non-hydrolyzing, thus eliminating the need for such careful and expensive handling and packaging requirements. This satisfies a long felt need in the art.

### SUMMARY OF THE INVENTION

The present invention is defined by the scope of the claims and any information that does not fall within the claims is provided for information only.

In order to overcome the hydrolysis issues experienced by prior art oxalate esters in chemical light systems, the present inventors have created a novel family of branched chain oxalate esters having a specific requisite construction, a process for their synthesis, and novel chemical light systems which include the novel branched chain oxalate esters.

The present invention relates to a branched chain oxalic acid ester selected from the group consisting of bis{3,4,6-trichloro-2-[(cyclopentylmethoxy)carbonyl]phenyl} oxalate, bis{3,4,6-trichloro-2-[(cyclohexylmethoxy)carbonyl]phenyl} oxalate, bis {3,4,6-trichloro-2-[(phenylmethoxy)carbonyl]phenyl} oxalate, bis {3,4,6-trichloro-2-[(2-phenylethoxy)carbonyl]phenyl} oxalate, bis(3,4,6-trichloro-2-{[(2-methylphenyl)methoxy]carbonyl}phenyl) oxalate, bis(3,4,6-trichloro-2-{[(3-methylphenyl)methoxy]carbonyl}phenyl) oxalate, bis(3,4,6-trichloro-2-{[(4-methylphenyl)methoxy]carbonyl}phenyl) oxalate, bis(3,4,6-trichloro-2-{[(2,3-dimethylphenyl)methoxy]carbonyl}phenyl) oxalate, bis(3,4,6-trichloro-2-{[(2,4-dimethylphenyl)methoxy]carbonyl}phenyl) oxalate, bis(3,4,6-trichloro-2-{[3,4-dimethylphenyl)methoxy]carbonyl}phenyl) oxalate, bis(3,4,6-trichloro-2-{[(3,5-dimethylphenyl)methoxy]carbonyl}phenyl) oxalate, bis(3,4,6-trichloro-2-{[(2,6-dimethylphenyl)methoxy]carbonyl}phenyl) oxalate, bis(3,4,6-trichloro-2-{[(2-ethylphenyl)methoxy]carbonyl}phenyl) oxalate, bis(3,4,6-trichloro-2-{[(3-ethylphenyl)methoxy]carbonyl}phenyl) oxalate, bis(3,4,6-trichloro-2-{[(4-ethylphenyl)methoxy]carbonyl}phenyl) oxalate, bis(3,4,6-trichloro-2-{[2-(2-methylphenyl)ethoxy]carbonyl}phenyl) oxalate, bis(3,4,6-trichloro-2-{[2-(3-methylphenyl)ethoxy]carbonyl}phenyl) oxalate, bis(3,4,6-trichloro-2-{[2-(4-methylphenyl)ethoxy]carbonyl}phenyl) oxalate, bis{3,4,6-trichloro-2-[(2-phenylpropoxy)carbonyl]phenyl} oxalate, bis{3,4,6-trichloro-2-[(3-phenylpropoxy)carbonyl]phenyl} oxalate, bis{3,4,6-trichloro-2-[1-naphthalenylmethoxy)carbonyl]phenyl} oxalate, bis{3,4,6-trichloro-2-[2-naphthalenylmethoxy)carbonyl]phenyl} oxalate, bis{3,4,6-trichloro-2-[(2,2-diphenylethoxy)carbonyl]phenyl} oxalate, bis {3,4,6-trichloro-2-[(9-fluorenylmethoxy)carbonyl]phenyl} oxalate, and bis {3,4,6-trichloro-2-[(9-anthracenylmethoxy)carbonyl]phenyl} oxalate.

The present invention also relates to a chemical light system comprising an activator solution and a branched chain oxalic acid ester in accordance with the above list.

The approach adopted involved using derivatives of primary alcohols, to facilitate the synthesis of the initial salicylate ester. Additionally, it was hypothesized that the introduction of steric hindrance by including ring structure(s) on the remainder of the alcohol would retard hydrolysis issues typically experienced by prior art oxalate esters, while providing compounds which would be eminently useful in a chemical light system.

This construction of the disclosure is characterized by the following structure:

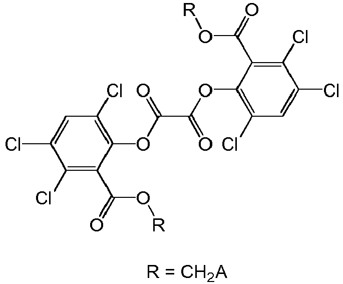

According to the disclosure, the group designated as R contains from 4-15 carbons. Furthermore, the carbon of R attached to the oxygen is a primary carbon and includes a substructure labeled A. The substructure A is composed of substituents selected from the group including alkyl chains, alkyl rings, and aromatic rings or combinations thereof such that R is nonlinear.

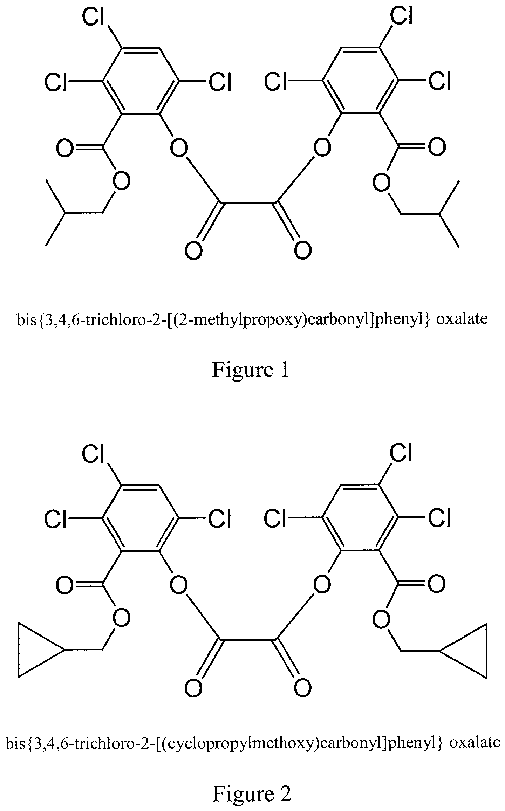

Figures 1-662 are illustrative of branched chain oxalate esters useful in chemical lighting systems.

It has been unexpectedly discovered that replacing the typical oxalate ester (bis{3,4,6-trichloro-2-[(pentoxy)carbonyl] phenyl} oxalate) with a non-straight chain, substituted methyl oxalate ester (i.e. a branched chain or ring structure oxalate ester made from a primary alcohol) retards water hydrolysis of the oxalate ester. This retardation of the hydrolysis changes significantly the storage and transportation constraints for the oxalate ester. The need for metal pouches or moisture barrier packaging is obviated without sacrificing commercially viable shelf-life.

Accordingly, it is a primary objective of the instant invention to teach non-straight chain oxalate esters, inclusive of ring structure oxalate esters, for use in conjunction with chemiluminescent formulations and chemical light producing devices.

### BRIEF DESCRIPTION OF THE FIGURES

As follows, Figures 1-662 illustrate oxalates useful in chemical light systems for the production of chemiluminescent light as herein after described.
Figure 1 illustrates a bis{3,4,6-trichloro-2-[(2-methylpropoxy)carbonyl]phenyl} oxalate;
Figure 2 illustrates a bis{3,4,6-trichloro-2-[(cyclopropylmethoxy)carbonyl]phenyl} oxalate;
Figure 3 illustrates a bis{3,4,6-trichloro-2-[(2-methylbutoxy)carbonyl]phenyl} oxalate;
Figure 4 illustrates a bis{3,4,6-trichloro-2-[(3-methylbutoxy)carbonyl]phenyl} oxalate;
Figure 5 illustrates a bis{3,4,6-trichloro-2-[(2,2-dimethylpropoxy)carbonyl]phenyl} oxalate;
Figure 6 illustrates a bis{3,4,6-trichloro-2-[(cyclobutylmethoxy)carbonyl]phenyl} oxalate;
Figure 7 illustrates a bis(3,4,6-trichloro-2-{[(1-methylcyclopropyl)methoxy]carbonyl}phenyl) oxalate;
Figure 8 illustrates a bis(3,4,6-trichloro-2-{[(2-methylcyclopropyl)methoxy]carbonyl}phenyl) oxalate;
Figure 9 illustrates a bis{3,4,6-trichloro-2-[(2-cyclopropylethoxy)carbonyl]phenyl} oxalate;
Figure 10 illustrates a bis {3,4,6-trichloro-2-[(2-methylpentyloxy)carbonyl]phenyl} oxalate;
Figure 11 illustrates a bis {3,4,6-trichloro-2-[(3-methylpentyloxy)carbonyl]phenyl} oxalate;
Figure 12 illustrates a bis{3,4,6-trichloro-2-[(4-methylpentyloxy)carbonyl]phenyl} oxalate;
Figure 13 illustrates a bis {3,4,6-trichloro-2-[(2,2-dimethylbutoxy)carbonyl]phenyl} oxalate;
Figure 14 illustrates a bis{3,4,6-trichloro-2-[(2,3-dimethylbutoxy)carbonyl]phenyl} oxalate;
Figure 15 illustrates a bis{3,4,6-trichloro-2-[(3,3-dimethylbutoxy)carbonyl]phenyl} oxalate;
Figure 16 illustrates a bis{3,4,6-trichloro-2-[(2-ethylbutoxy)carbonyl]phenyl} oxalate;
Figure 17 illustrates a bis{3,4,6-trichloro-2-[(cyclopentylmethoxy)carbonyl]phenyl} oxalate;
Figure 18 illustrates a bis(3,4,6-trichloro-2-{[(1-methylcyclobutyl)methoxy]carbonyl}phenyl) oxalate;
Figure 19 illustrates a bis(3,4,6-trichloro-2-{[(2-methylcyclobutyl)methoxy]carbonyl}phenyl) oxalate;
Figure 20 illustrates a bis(3,4,6-trichloro-2-{[(3-methylcyclobutyl)methoxy]carbonyl}phenyl) oxalate;
Figure 21 illustrates a bis(3,4,6-trichloro-2-{[(1-ethylcyclopropyl)methoxy]carbonyl}phenyl) oxalate;
Figure 22 illustrates a bis(3,4,6-trichloro-2-{[(1,2-dimethylcyclopropyl)methoxy]carbonyl}phenyl) oxalate;
Figure 23 illustrates a bis(3,4,6-trichloro-2-{[(2,3-dimethylcyclopropyl)methoxy]carbonyl}phenyl) oxalate;
Figure 24 illustrates a bis(3,4,6-trichloro-2-{[(2-ethylcyclopropyl)methoxy]carbonyl}phenyl) oxalate;
Figure 25 illustrates a bis(3,4,6-trichloro-2-{[(2,2-dimethylcyclopropyl)methoxy]carbonyl}phenyl) oxalate;
Figure 26 illustrates a bis(3,4,6-trichloro-2-{[2-(1-methylcyclopropyl)ethoxy]carbonyl}phenyl) oxalate;
Figure 27 illustrates a bis(3,4,6-trichloro-2-{[2-(2-methylcyclopropyl)ethoxy]carbonyl}phenyl) oxalate;
Figure 28 illustrates a bis{3,4,6-trichloro-2-[(2-cyclopropylpropoxy)carbonyl]phenyl} oxalate;
Figure 29 illustrates a bis{3,4,6-trichloro-2-[(3-cyclopropylpropoxy)carbonyl]phenyl} oxalate;
Figure 30 illustrates a bis{2-[(bicyclo[1.1.1]pentan-5-ylmethoxy)carbonyl]-3,4,6-trichlorophenyl} oxalate;
Figure 31 illustrates a bis {3,4,6-trichloro-2-[(2-methylhexyloxy)carbonyl]phenyl} oxalate;
Figure 32 illustrates a bis {3,4,6-trichloro-2-[(3-methylhexyloxy)carbonyl]phenyl} oxalate;
Figure 33 illustrates a bis{3,4,6-trichloro-2-[(4-methylhexyloxy)carbonyl]phenyl} oxalate;
Figure 34 illustrates a bis{3,4,6-trichloro-2-[(5-methylhexyloxy)carbonyl]phenyl} oxalate;
Figure 35 illustrates a bis{3,4,6-trichloro-2-[(2,2-dimethylpentyloxy)carbonyl]phenyl} oxalate;
Figure 36 illustrates a bis {3,4,6-trichloro-2-[(2,3-dimethylpentyloxy)carbonyl]phenyl} oxalate;
Figure 37 illustrates a bis{3,4,6-trichloro-2-[(2,4-dimethylpentyloxy)carbonyl]phenyl} oxalate;
Figure 38 illustrates a bis{3,4,6-trichloro-2-[(3,3-dimethylpentyloxy)carbonyl]phenyl} oxalate;
Figure 39 illustrates a bis{3,4,6-trichloro-2-[(3,4-dimethylpentyloxy)carbonyl]phenyl} oxalate;
Figure 40 illustrates a bis {3,4,6-trichloro-2-[(4,4-dimethylpentyloxy)carbonyl]phenyl} oxalate;
Figure 41 illustrates a bis{3,4,6-trichloro-2-[(2-ethyl-3-methylbutoxy)carbonyl]phenyl} oxalate;
Figure 42 illustrates a bis {3,4,6-trichloro-2-[(2,2,3-trimethylbutoxy)carbonyl]phenyl} oxalate;
Figure 43 illustrates a bis {3,4,6-trichloro-2-[(2,3,3-trimethylbutoxy)carbonyl]phenyl} oxalate;
Figure 44 illustrates a bis {3,4,6-trichloro-2-[(2-ethylpentyloxy)carbonyl]phenyl} oxalate;
Figure 45 illustrates a bis {3,4,6-trichloro-2-[(3-ethylpentyloxy)carbonyl]phenyl} oxalate;
Figure 46 illustrates a bis{3,4,6-trichloro-2-[(2-ethyl-2-methylbutoxy)carbonyl]phenyl} oxalate;
Figure 47 illustrates a bis {3,4,6-trichloro-2-[(cyclohexylmethoxy)carbonyl]phenyl} oxalate;
Figure 48 illustrates a bis(3,4,6-trichloro-2-{[(1-methylcyclopentyl)methoxy]carbonyl}phenyl) oxalate;
Figure 49 illustrates a bis(3,4,6-trichloro-2-{[(2-methylcyclopentyl)methoxy]carbonyl}phenyl) oxalate;
Figure 50 illustrates a bis(3,4,6-trichloro-2-{[(3-methylcyclopentyl)methoxy]carbonyl}phenyl) oxalate;
Figure 51 illustrates a bis {3,4,6-trichloro-2-[(2-cyclopentylethoxy)carbonyl]phenyl} oxalate;
Figure 52 illustrates a bis(3,4,6-trichloro-2-{[(1-ethylcyclobutyl)methoxy]carbonyl}phenyl) oxalate;
Figure 53 illustrates a bis(3,4,6-trichloro-2-{[(2-ethylcyclobutyl)methoxy]carbonyl}phenyl) oxalate;
Figure 54 illustrates a bis(3,4,6-trichloro-2-{[(3-ethylcyclobutyl)methoxy]carbonyl}phenyl) oxalate;
Figure 55 illustrates a bis(3,4,6-trichloro-2-{[(1,2-dimethylcyclobutyl)methoxy]carbonyl}phenyl) oxalate;
Figure 56 illustrates a bis(3,4,6-trichloro-2-{[(1,3-dimethylcyclobutyl)methoxy]carbonyl}phenyl) oxalate;
Figure 57 illustrates a bis(3,4,6-trichloro-2-{[(2,2-dimethylcyclobutyl)methoxy]carbonyl}phenyl) oxalate;
Figure 58 illustrates a bis(3,4,6-trichloro-2-{[(2,3-dimethylcyclobutyl)methoxy]carbonyl}phenyl) oxalate;
Figure 59 illustrates a bis(3,4,6-trichloro-2-{[(3,3-dimethylcyclobutyl)methoxy]carbonyl}phenyl) oxalate;
Figure 60 illustrates a bis(3,4,6-trichloro-2-{[2-(1-methylcyclobutyl)ethoxy]carbonyl}phenyl) oxalate;
Figure 61 illustrates a bis(3,4,6-trichloro-2-{[2-(2-methylcyclobutyl)ethoxy]carbonyl}phenyl) oxalate;
Figure 62 illustrates a bis(3,4,6-trichloro-2-{[2-(3-methylcyclobutyl)ethoxy]carbonyl}phenyl) oxalate;
Figure 63 illustrates a bis{3,4,6-trichloro-2-[(2-cyclobutylpropoxy)carbonyl]phenyl} oxalate;
Figure 64 illustrates a bis{3,4,6-trichloro-2-[(3-cyclobutylpropoxy)carbonyl]phenyl} oxalate;
Figure 65 illustrates a bis(3,4,6-trichloro-2-{[(1,2,2-trimethylcyclopropyl)methoxy]carbonyl}phenyl) oxalate;
Figure 66 illustrates a bis(3,4,6-trichloro-2-{[(1,2,3-trimethylcyclopropyl)methoxy]carbonyl}phenyl) oxalate;
Figure 67 illustrates a bis(3,4,6-trichloro-2-{[(1-ethyl-2-methylcyclopropyl)methoxy]carbonyl}phenyl) oxalate;
Figure 68 illustrates a bis(3,4,6-trichloro-2-{[(2-ethyl-1-methylcyclopropyl)methoxy]carbonyl}phenyl) oxalate;
Figure 69 illustrates a bis[3,4,6-trichloro-2-({[1-(1-methylethyl)cyclopropyl]methoxy}carbonyl)phenyl] oxalate;
Figure 70 illustrates a bis[3,4,6-trichloro-2-({[2-(1-methylethyl)cyclopropyl]methoxy} carbonyl)phenyl] oxalate;
Figure 71 illustrates a bis(3,4,6-trichloro-2-{[2-(1,2-dimethylcyclopropyl)ethoxy]carbonyl}phenyl) oxalate;
Figure 72 illustrates a bis(3,4,6-trichloro-2-{[2-(2,2-dimethylcyclopropyl)ethoxy]carbonyl}phenyl) oxalate;
Figure 73 illustrates a bis(3,4,6-trichloro-2-{[2-(2,3-dimethylcyclopropyl)ethoxy]carbonyl}phenyl) oxalate;
Figure 74 illustrates a bis(3,4,6-trichloro-2-{[2-(1-ethylcyclopropyl)ethoxy]carbonyl}phenyl) oxalate;
Figure 75 illustrates a bis(3,4,6-trichloro-2-{[2-(2-ethylcyclopropyl)ethoxy]carbonyl}phenyl) oxalate;
Figure 76 illustrates a bis(3,4,6-trichloro-2-{[2-(1-methylcyclopropyl)propoxy]carbonyl}phenyl) oxalate;
Figure 77 illustrates a bis(3,4,6-trichloro-2-{[2-(2 -methylcyclopropyl)propoxy]carbonyl}phenyl) oxalate;
Figure 78 illustrates a bis {3,4,6-trichloro-2-[(2-cyclopropyl-2-methylpropoxy)carbonyl]phenyl} oxalate;
Figure 79 illustrates a bis(3,4,6-trichloro-2-{[3-(1-methylcyclopropyl)propoxy]carbonyl}phenyl) oxalate;
Figure 80 illustrates a bis(3,4,6-trichloro-2-{[3-(2-methylcyclopropyl)propoxy]carbonyl}phenyl) oxalate;
Figure 81 illustrates a bis {3,4,6-trichloro-2-[(3-cyclopropyl-2-methylpropoxy)carbonyl]phenyl} oxalate;
Figure 82 illustrates a bis {3,4,6-trichloro-2-[(2-cyclopropylbutoxy)carbonyl]phenyl} oxalate;
Figure 83 illustrates a bis {3,4,6-trichloro-2-[(3-cyclopropylbutoxy)carbonyl]phenyl} oxalate;
Figure 84 illustrates a bis {3,4,6-trichloro-2-[(4-cyclopropylbutoxy)carbonyl]phenyl} oxalate;
Figure 85 illustrates a bis{2-[(bicyclo[2.1.1]hexan-2-ylmethoxy)carbonyl]-3,4,6-trichlorophenyl} oxalate;
Figure 86 illustrates a bis{3,4,6-trichloro-2-[(phenylmethoxy)carbonyl]phenyl} oxalate;
Figure 87 illustrates a bis{3,4,6-trichloro-2-[(2-methylheptyloxy)carbonyl]phenyl} oxalate;
Figure 88 illustrates a bis{3,4,6-trichloro-2-[(3-methylheptyloxy)carbonyl]phenyl} oxalate;
Figure 89 illustrates a bis {3,4,6-trichloro-2-[(4-methylheptyloxy)carbonyl]phenyl} oxalate;
Figure 90 illustrates a bis {3,4,6-trichloro-2-[(5-methylheptyloxy)carbonyl]phenyl} oxalate;
Figure 91 illustrates a bis{3,4,6-trichloro-2-[(6-methylheptyloxy)carbonyl]phenyl} oxalate;
Figure 92 illustrates a bis {3,4,6-trichloro-2-[(2,2-dimethylhexyloxy)carbonyl]phenyl} oxalate;
Figure 93 illustrates a bis{3,4,6-trichloro-2-[(2,3-dimethylhexyloxy)carbonyl]phenyl} oxalate;
Figure 94 illustrates a bis{3,4,6-trichloro-2-[(2,4-dimethylhexyloxy)carbonyl]phenyl} oxalate;
Figure 95 illustrates a bis{3,4,6-trichloro-2-[(2,5-dimethylhexyloxy)carbonyl]phenyl} oxalate;
Figure 96 illustrates a bis{3,4,6-trichloro-2-[(3,3-dimethylhexyloxy)carbonyl]phenyl} oxalate;
Figure 97 illustrates a bis{3,4,6-trichloro-2-[(3,4-dimethylhexyloxy)carbonyl]phenyl} oxalate;
Figure 98 illustrates a bis{3,4,6-trichloro-2-[(3,5-dimethylhexyloxy)carbonyl]phenyl} oxalate;
Figure 99 illustrates a bis{3,4,6-trichloro-2-[(4,4-dimethylhexyloxy)carbonyl]phenyl} oxalate;
Figure 100 illustrates a bis{3,4,6-trichloro-2-[(4,5-dimethylhexyloxy)carbonyl]phenyl} oxalate;
Figure 101 illustrates a bis{3,4,6-trichloro-2-[(5,5-dimethylhexyloxy)carbonyl]phenyl} oxalate;
Figure 102 illustrates a bis{3,4,6-trichloro-2-[(2-ethylhexyloxy)carbonyl]phenyl} oxalate;
Figure 103 illustrates a bis{3,4,6-trichloro-2-[(3-ethylhexyloxy)carbonyl]phenyl} oxalate;
Figure 104 illustrates a bis{3,4,6-trichloro-2-[(4-ethylhexyloxy)carbonyl]phenyl} oxalate;
Figure 105 illustrates a bis{3,4,6-trichloro-2-[(2,2,3-trimethylpentyloxy)carbonyl]phenyl} oxalate;
Figure 106 illustrates a bis{3,4,6-trichloro-2-[(2,3,3-trimethylpentyloxy)carbonyl]phenyl} oxalate;
Figure 107 illustrates a bis{3,4,6-trichloro-2-[(2,2,4-trimethylpentyloxy)carbonyl]phenyl} oxalate;
Figure 108 illustrates a bis{3,4,6-trichloro-2-[(2,3,4-trimethylpentyloxy)carbonyl]phenyl} oxalate;
Figure 109 illustrates a bis{3,4,6-trichloro-2-[(2,4,4-trimethylpentyloxy)carbonyl]phenyl} oxalate;
Figure 110 illustrates a bis{3,4,6-trichloro-2-[(3,3,4-trimethylpentyloxy)carbonyl]phenyl} oxalate;
Figure 111 illustrates a bis{3,4,6-trichloro-2-[(3.4,4-trimethylpentyloxy)carbonyl]phenyl} oxalate;
Figure 112 illustrates a bis{3,4,6-trichloro-2-[(2-ethyl-3-methylpentyloxy)carbonyl]phenyl} oxalate;
Figure 113 illustrates a bis{3,4,6-trichloro-2-[(2-ethyl-4-methylpentyloxy)carbonyl]phenyl} oxalate;
Figure 114 illustrates a bis{3,4,6-trichloro-2-[(3-ethyl-2-methylpentyloxy)carbonyl]phenyl} oxalate;
Figure 115 illustrates a bis{3,4,6-trichloro-2-[(3-ethyl-4-methylpentyloxy)carbonyl]phenyl} oxalate;
Figure 116 illustrates a bis{3,4,6-trichloro-2-[(2-ethyl-2-methylpentyloxy)carbonyl]phenyl} oxalate;
Figure 117 illustrates a bis{3,4,6-trichloro-2-[(3-ethyl-3-methylpentyloxy)carbonyl]phenyl} oxalate;
Figure 118 illustrates a bis{3,4,6-trichloro-2-[(2,2,3,3-tetramethylbutoxy)carbonyl]phenyl} oxalate;
Figure 119 illustrates a bis{3,4,6-trichloro-2-[(2-ethyl-2,3-dimethylbutoxy)carbonyl]phenyl} oxalate;
Figure 120 illustrates a bis{3,4,6-trichloro-2-[(2-ethyl-3,3-dimethylbutoxy)carbonyl]phenyl} oxalate;
Figure 121 illustrates a bis {3,4,6-trichloro-2-[(cycloheptylmethoxy)carbonyl]phenyl} oxalate;
Figure 122 illustrates a bis(3,4,6-trichloro-2-{[(1-methylcyclohexyl)methoxy]carbonyl}phenyl) oxalate;
Figure 123 illustrates a bis(3,4,6-trichloro-2-{[(2-methylcyclohexyl)methoxy]carbonyl}phenyl) oxalate;
Figure 124 illustrates a bis(3,4,6-trichloro-2-{[(3-methylcyclohexyl)methoxy]carbonyl}phenyl) oxalate;
Figure 125 illustrates a bis(3,4,6-trichloro-2-{[(4-methylcyclohexyl)methoxy]carbonyl}phenyl) oxalate;
Figure 126 illustrates a bis{3,4,6-trichloro-2-[(2-cyclohexylethoxy)carbonyl]phenyl} oxalate;
Figure 127 illustrates a bis(3,4,6-trichloro-2-{[(1,2-dimethylcyclopentyl)methoxy]carbonyl}phenyl) oxalate;
Figure 128 illustrates a bis(3,4,6-trichloro-2-{[(1,3-dimethylcyclopentyl)methoxy]carbonyl}phenyl) oxalate;
Figure 129 illustrates a bis(3,4,6-trichloro-2-{[(2,2-dimethylcyclopentyl)methoxy]carbonyl}phenyl) oxalate;
Figure 130 illustrates a bis(3,4,6-trichloro-2-{[(2,3-dimethylcyclopentyl)methoxy]carbonyl}phenyl) oxalate;
Figure 131 illustrates a bis(3,4,6-trichloro-2-{[(2,4-dimethylcyclopentyl)methoxy]carbonyl}phenyl) oxalate;
Figure 132 illustrates a bis(3,4,6-trichloro-2-{[(2,5-dimethylcyclopentyl)methoxy]carbonyl}phenyl) oxalate;
Figure 133 illustrates a bis(3,4,6-trichloro-2-{[(3,3-dimethylcyclopentyl)methoxy]carbonyl}phenyl) oxalate;
Figure 134 illustrates a bis(3,4,6-trichloro-2-{[(3,4-dimethylcyclopentyl)methoxy]carbonyl}phenyl) oxalate;
Figure 135 illustrates a bis(3,4,6-trichloro-2-{[(1-ethylcyclopentyl)methoxy]carbonyl}phenyl) oxalate;
Figure 136 illustrates a bis(3,4,6-trichloro-2-{[(2-ethylcyclopentyl)methoxy]carbonyl}phenyl) oxalate;
Figure 137 illustrates a bis(3,4,6-trichloro-2-{[(3-ethylcyclopentyl)methoxy]carbonyl}phenyl) oxalate;
Figure 138 illustrates a bis(3,4,6-trichloro-2-{[2-(1-methylcyclopentyl)ethoxy]carbonyl}phenyl) oxalate;
Figure 139 illustrates a bis(3,4,6-trichloro-2-{[2-(2-methylcyclopentyl)ethoxy]carbonyl}phenyl) oxalate;
Figure 140 illustrates a bis(3,4,6-trichloro-2-{[2-(3-methylcyclopentyl)ethoxy]carbonyl}phenyl) oxalate;
Figure 141 illustrates a bis{3,4,6-trichloro-2-[(2-cyclopentylpropoxy)carbonyl]phenyl} oxalate;
Figure 142 illustrates a bis{3,4,6-trichloro-2-[(3-cyclopentylpropoxy)carbonyl]phenyl} oxalate;
Figure 143 illustrates a bis(3,4,6-trichloro-2-{[(1,2,2-trimethylcyclobutyl)methoxy]carbonyl}phenyl) oxalate;
Figure 144 illustrates a bis(3,4,6-trichloro-2-{[(1,2,3-trimethylcyclobutyl)methoxy]carbonyl}phenyl) oxalate;
Figure 145 illustrates a bis(3,4,6-trichloro-2-{[(1,2,4-trimethylcyclobutyl)methoxy]carbonyl}phenyl) oxalate;
Figure 146 illustrates a bis(3,4,6-trichloro-2-{[(2,2,3-trimethylcyclobutyl)methoxy]carbonyl}phenyl) oxalate;
Figure 147 illustrates a bis(3,4,6-trichloro-2-{[(2,3,3-trimethylcyclobutyl)methoxy]carbonyl}phenyl) oxalate
Figure 148 illustrates a bis(3,4,6-trichloro-2-{[(2,3,4-trimethylcyclobutyl)methoxy]carbonyl}phenyl) oxalate;
Figure 149 illustrates a bis(3,4,6-trichloro-2-{[(2-ethyl-1-methylcyclobutyl)methoxy]carbonyl}phenyl) oxalate;
Figure 150 illustrates a bis(3,4,6-trichloro-2-{[(3-ethyl-1-methylcyclobutyl)methoxy]carbonyl}phenyl) oxalate;
Figure 151 illustrates a bis(3,4,6-trichloro-2-{[(1-ethyl-2-methylcyclobutyl)methoxy]carbonyl}phenyl) oxalate;
Figure 152 illustrates a bis(3,4,6-trichloro-2-{[(1-ethyl-3-methylcyclobutyl)methoxy]carbonyl}phenyl) oxalate;
Figure 153 illustrates a bis(3,4,6-trichloro-2-({[1-(1-methylethyl)-cyclobutyl]methoxy} carbonyl)phenyl) oxalate;
Figure 154 illustrates a bis(3,4,6-trichloro-2-({[2-(1-methylethyl)-cyclobutyl]methoxy} carbonyl)phenyl) oxalate;
Figure 155 illustrates a bis(3,4,6-trichloro-2-({[3-(1-methylethyl)-cyclobutyl]methoxy}carbonyl)phenyl) oxalate;
Figure 156 illustrates a bis(3,4,6-trichloro-2-{[2-(1,2-dimethylcyclobutyl)ethoxy]carbonyl}phenyl) oxalate;
Figure 157 illustrates a bis(3,4,6-trichloro-2-{[2-(1,3-dimethylcyclobutyl)ethoxy]carbonyl}phenyl) oxalate;
Figure 158 illustrates a bis(3,4,6-trichloro-2-{[2-(2,2-dimethylcyclobutyl)ethoxy]carbonyl}phenyl) oxalate;
Figure 159 illustrates a bis(3,4,6-trichloro-2-{[2-(2,3-dimethylcyclobutyl)ethoxy]carbonyl}phenyl) oxalate;
Figure 160 illustrates a bis(3,4,6-trichloro-2-{[2-(2,4-dimethylcyclobutyl)ethoxy]carbonyl}phenyl) oxalate;
Figure 161 illustrates a bis(3,4,6-trichloro-2-{[2-(3,3-dimethylcyclobutyl)ethoxy]carbonyl}phenyl) oxalate;
Figure 162 illustrates a bis(3,4,6-trichloro-2-{[2-(1-methylcyclobutyl)propoxy]carbonyl}phenyl) oxalate;
Figure 163 illustrates a bis(3,4,6-trichloro-2-{[2-(2-methylcyclobutyl)propoxy]carbonyl}phenyl) oxalate;
Figure 164 illustrates a bis(3,4,6-trichloro-2-{[2-(3-methylcyclobutyl)propoxy]carbonyl}phenyl) oxalate;
Figure 165 illustrates a bis{3,4,6-trichloro-2-[(2-cyclobutyl-2-methylpropoxy)carbonyl]phenyl} oxalate;
Figure 166 illustrates a bis(3,4,6-trichloro-2-{[2-(1-ethylcyclobutyl)ethoxy]carbonyl}phenyl) oxalate;
Figure 167 illustrates a bis(3,4,6-trichloro-2-{[2-(2-ethylcyclobutyl)ethoxy]carbonyl}phenyl) oxalate;
Figure 168 illustrates a bis(3,4,6-trichloro-2-{[2-(3-ethylcyclobutyl)ethoxy]carbonyl}phenyl) oxalate;
Figure 169 illustrates a bis(3,4,6-trichloro-2-{[3-(1-methylcyclobutyl)propoxy]carbonyl}phenyl) oxalate;
Figure 170 illustrates a bis(3,4,6-trichloro-2-{[3-(2-methylcyclobutyl)propoxy]carbonyl}phenyl) oxalate;
Figure 171 illustrates a bis(3,4,6-trichloro-2-{[3-(3-methylcyclobutyl)propoxy]carbonyl}phenyl) oxalate;
Figure 172 illustrates a bis {3,4,6-trichloro-2-[(3-cyclobutyl-2-methylpropoxy)carbonyl]phenyl} oxalate;
Figure 173 illustrates a bis{3,4,6-trichloro-2-[(2-cyclobutylbutoxy)carbonyl]phenyl} oxalate;
Figure 174 illustrates a bis{3,4,6-trichloro-2-[(3-cyclobutylbutoxy)carbonyl]phenyl} oxalate;
Figure 175 illustrates a bis{3,4,6-trichloro-2-[(4-cyclobutylbutoxy)carbonyl]phenyl} oxalate;
Figure 176 illustrates a bis(3,4,6-trichloro-2-{[(1,2,2,3,3-pentamethylcyclopropyl)methoxy]carbonyl}phenyl) oxalate;
Figure 177 illustrates a bis(3,4,6-trichloro-2-{[(3-ethyl-1,2,2-trimethylcyclopropyl)methoxy]carbonyl}phenyl) oxalate;
Figure 178 illustrates a bis(3,4,6-trichloro-2-{[(2,3-diethyl-1-methylcyclopropyl)methoxy]carbonyl}phenyl) oxalate;
Figure 179 illustrates a bis(3,4,6-trichloro-2-{[(2,2-diethyl-1-methylcyclopropyl)methoxy]carbonyl}phenyl) oxalate;
Figure 180 illustrates a bis(3,4,6-trichloro-2-{[(1-ethyl-2-propylcyclopropyl)methoxy]carbonyl}phenyl) oxalate;
Figure 181 illustrates a bis(3,4,6-trichloro-2-{[(2-ethyl-1-propylcyclopropyl)methoxy]carbonyl}phenyl) oxalate;
Figure 182 illustrates a bis(3,4,6-trichloro-2-{[(2-ethyl-2-propylcyclopropyl)methoxy]carbonyl}phenyl) oxalate;
Figure 183 illustrates a bis(3,4,6-trichloro-2-{[(2-ethyl-3-propylcyclopropyl)methoxy]carbonyl}phenyl) oxalate;
Figure 184 illustrates a bis(3,4,6-trichloro-2-{[(1-ethyl-2,2,3-trimethylcyclopropyl)methoxy]carbonyl}phenyl) oxalate;
Figure 185 illustrates a bis(3,4,6-trichloro-2-{[(1,2-diethyl-3-methylcyclopropyl)methoxy]carbonyl}phenyl) oxalate;
Figure 186 illustrates a bis[3,4,6-trichloro-2-({[2,2-dimethyl-1-(1-methylethyl)-cyclopropyl]methoxy}carbonyl)phenyl] oxalate;
Figure 187 illustrates a bis[3,4,6-trichloro-2-({[2,3-dimethyl-1-(1-methylethyl)-cyclopropyl]methoxy} carbonyl)phenyl] oxalate;
Figure 188 illustrates a bis[3,4,6-trichloro-2-({[2-ethyl-1-(1-methylethyl)-cyclopropyl]methoxy}carbonyl)phenyl] oxalate;
Figure 189 illustrates a bis[3,4,6-trichloro-2-({[1-ethyl-2-(1-methylethyl)-cyclopropyl]methoxy} carbonyl)phenyl] oxalate;
Figure 190 illustrates a bis[3,4,6-trichloro-2-({[2,2-dimethyl-3-(1-methylethyl)-cyclopropyl]methoxy} carbonyl)phenyl] oxalate;
Figure 191 illustrates a bis[3,4,6-trichloro-2-({[2-ethyl-3-(1-methylethyl)-cyclopropyl]methoxy} carbonyl)phenyl] oxalate;
Figure 192 illustrates a bis(3,4,6-trichloro-2-{[2-(1,2,2-trimethylcyclopropyl)propoxy]carbonyl}phenyl) oxalate;
Figure 193 illustrates a bis(3,4,6-trichloro-2-{[2-(1,2,3-trimethylcyclopropyl)propoxy]carbonyl}phenyl) oxalate;
Figure 194 illustrates a bis(3,4,6-trichloro-2-{[2-(2,2,3-trimethylcyclopropyl)propoxy]carbonyl}phenyl) oxalate;
Figure 195 illustrates a bis(3,4,6-trichloro-2-{[2-(2,2-dimethylcyclopropyl)propoxy]carbonyl}phenyl) oxalate;
Figure 196 illustrates a bis(3,4,6-trichloro-2-{[2-(2,3-dimethylcyclopropyl)propoxy]carbonyl}phenyl) oxalate;
Figure 197 illustrates a bis(3,4,6-trichloro-2-{[2-(2-ethyl-3-methylcyclopropyl)ethoxy]carbonyl}phenyl) oxalate;
Figure 198 illustrates a bis(3,4,6-trichloro-2-{[2-(2-methylcyclopropyl)butoxy]carbonyl}phenyl) oxalate;
Figure 199 illustrates a bis {3,4,6-trichloro-2-[(2-cyclopropyl-2-methylbutoxy)carbonyl]phenyl} oxalate;
Figure 200 illustrates a bis(3,4,6-trichloro-2-{[2-(1-methylcyclopropyl)butoxy]carbonyl}phenyl) oxalate;
Figure 201 illustrates a bis(3,4,6-trichloro-2-{[2-(2-ethyl-2-methylcyclopropyl)ethoxy]carbonyl}phenyl) oxalate;
Figure 202 illustrates a bis(3,4,6-trichloro-2-{[2-methyl-2-(1-methylcyclopropyl)propoxy]carbonyl}phenyl) oxalate;
Figure 203 illustrates a bis(3,4,6-trichloro-2-{[2-methyl-2-(2-methylcyclopropyl)propoxy]carbonyl}phenyl) oxalate;
Figure 204 illustrates a bis {3,4,6-trichloro-2-[(2-cyclopropyl-3-methylbutoxy)carbonyl]phenyl} oxalate;
Figure 205 illustrates a bis(3,4,6-trichloro-2-{[3-(1,2-dimethylcyclopropyl)propoxy]carbonyl}phenyl) oxalate;
Figure 206 illustrates a bis(3,4,6-trichloro-2-{[3-(2,2-dimethylcyclopropyl)propoxy]carbonyl}phenyl) oxalate;
Figure 207 illustrates a bis {3,4,6-trichloro-2-[(3-cyclopropyl-2-methylbutoxy)carbonyl]phenyl} oxalate;
Figure 208 illustrates a bis(3,4,6-trichloro-2-{[2-methyl-3-(1-methylcyclopropyl)propoxy]carbonyl}phenyl) oxalate;
Figure 209 illustrates a bis(3,4,6-trichloro-2-{[2-methyl-3-(2-methylcyclopropyl)propoxy]carbonyl}phenyl) oxalate;
Figure 210 illustrates a bis{3,4,6-trichloro-2-[(3-cyclopropyl-2,2-dimethylpropoxy)carbonyl]phenyl} oxalate;
Figure 211 illustrates a bis{3,4,6-trichloro-2-[(3-cyclopropyl-3-methylbutoxy)carbonyl]phenyl} oxalate;
Figure 212 illustrates a bis{3,4,6-trichloro-2-[(2,2dicyclopropylethoxy)carbonyl)phenyl} oxalate;
Figure 213 illustrates a bis(3,4,6-trichloro-2-{[4-(1-methylcyclopropyl)butoxy]carbonyl}phenyl) oxalate;
Figure 214 illustrates a bis(3,4,6-trichloro-2-{[4-(2-methylcyclopropyl)butoxy]carbonyl}phenyl) oxalate;
Figure 215 illustrates a bis {3,4,6-trichloro-2-[(4-cyclopropyl-2-methylbutoxy)carbonyl]phenyl} oxalate;
Figure 216 illustrates a bis{3,4,6-trichloro-2-[(4-cyclopropyl-3-methylbutoxy)carbonyl]phenyl} oxalate;
Figure 217 illustrates a bis(3,4,6-trichloro-2-{[2-(2-propylcyclopropyl)ethoxy]carbonyl}phenyl) oxalate;
Figure 218 illustrates a bis[3,4,6-trichloro-2-({2-[2-(1-methylethyl)cyclopropyl]ethoxy}carbonyl)phenyl] oxalate;
Figure 219 illustrates a bis {3,4,6-trichloro-2-[(2-cyclopropylpentyloxy)carbonyl]phenyl} oxalate;
Figure 220 illustrates a bis {3,4,6-trichloro-2-[(3-cyclopropylpentyloxy)carbonyl]phenyl} oxalate;
Figure 221 illustrates a bis{3,4,6-trichloro-2-[(4-cyclopropylpentyloxy)carbonyl]phenyl} oxalate;
Figure 222 illustrates a bis {3,4,6-trichloro-2-[(5-cyclopropylpentyloxy)carbonyl]phenyl} oxalate;
Figure 223 illustrates a bis(2-{[2-(bicyclo[1.1.1]pentan-2-yl)propoxy]carbonyl}-3,4,6-trichlorophenyl) oxalate;
Figure 224 illustrates a bis(2-{[2-(bicyclo[2.1.1]hexan-2-yl)ethoxy]carbonyl}-3,4,6-trichlorophenyl) oxalate;
Figure 225 illustrates a bis{2-[(bicyclo[2.2.1]heptan-2-ylmethoxy)carbonyl]3,4,6-trichlorophenyl} oxalate;
Figure 226 illustrates a bis{3,4,6-trichloro-2-[(2-phenylethoxy)carbonyl]phenyl} oxalate;
Figure 227 illustrates a bis(3,4,6-trichloro-2-{[(2-methylphenyl)methoxy]carbonyl}phenyl) oxalate;
Figure 228 illustrates a bis(3,4,6-trichloro-2-{[(3-methylphenyl)methoxy]carbonyl}phenyl) oxalate;
Figure 229 illustrates a bis(3,4,6-trichloro-2-{[(4-methylphenyl)methoxy]carbonyl}phenyl) oxalate;
Figure 230 illustrates a bis{3,4,6-trichloro-2-[(2-methyloctyloxy)carbonyl]phenyl} oxalate;
Figure 231 illustrates a bis{3,4,6-trichloro-2-[(3-methyloctyloxy)carbonyl]phenyl} oxalate;
Figure 232 illustrates a bis{3,4,6-trichloro-2-[(4-methyloctyloxy)carbonyl]phenyl} oxalate;
Figure 233 illustrates a bis{3,4,6-trichloro-2-[(5-methyloctyloxy)carbonyl]phenyl} oxalate;
Figure 234 illustrates a bis{3,4,6-trichloro-2-[(6-methyloctyloxy)carbonyl]phenyl} oxalate;
Figure 235 illustrates a bis{3,4,6-trichloro-2-[(7-methyloctyloxy)carbonyl]phenyl} oxalate;
Figure 236 illustrates a bis{3,4,6-trichloro-2-[(2,2-dimethylheptyloxy)carbonyl]phenyl} oxalate;
Figure 237 illustrates a bis{3,4,6-trichloro-2-[(2-ethylheptyloxy)carbonyl]phenyl} oxalate;
Figure 238 illustrates a bis{3,4,6-trichloro-2-[(2,3-dimethylheptyloxy)carbonyl]phenyl} oxalate;
Figure 239 illustrates a bis{3,4,6-trichloro-2-[(2,4-dimethylheptyloxy)carbonyl]phenyl} oxalate;
Figure 240 illustrates a bis{3,4,6-trichloro-2-[(2,5-dimethylheptyloxy)carbonyl]phenyl} oxalate;
Figure 241 illustrates a bis{3,4,6-trichloro-2-[(2,6-dimethylheptyloxy)carbonyl]phenyl} oxalate;
Figure 242 illustrates a bis{3,4,6-trichloro-2-[(3,3-dimethylheptyloxy)carbonyl]phenyl} oxalate;
Figure 243 illustrates a bis{3,4,6-trichloro-2-[(3-ethylheptyloxy)carbonyl]phenyl} oxalate;
Figure 244 illustrates a bis{3,4,6-trichloro-2-[(3,4-dimethylheptyloxy)carbonyl]phenyl} oxalate;
Figure 245 illustrates a bis{3,4,6-trichloro-2-[(3,5-dimethylheptyloxy)carbonyl]phenyl} oxalate;
Figure 246 illustrates a bis{3,4,6-trichloro-2-[(3,6-dimethylheptyloxy)carbonyl]phenyl} oxalate;
Figure 247 illustrates a bis{3,4,6-trichloro-2-[(4,4-dimethylheptyloxy)carbonyl]phenyl} oxalate;
Figure 248 illustrates a bis{3,4,6-trichloro-2-[(4-ethylheptyloxy)carbonyl]phenyl} oxalate;
Figure 249 illustrates a bis{3,4,6-trichloro-2-[(4,5-dimethylheptyloxy)carbonyl]phenyl} oxalate;
Figure 250 illustrates a bis{3,4,6-trichloro-2-[(4,6-dimethylheptyloxy)carbonyl]phenyl} oxalate;
Figure 251 illustrates a bis{3,4,6-trichloro-2-[(5,5-dimethylheptyloxy)carbonyl]phenyl} oxalate ;
Figure 252 illustrates a bis{3,4,6-trichloro-2-[(5-ethylheptyloxy)carbonyl]phenyl} oxalate;
Figure 253 illustrates a bis{3,4,6-trichloro-2-[(5,6-dimethylheptyloxy)carbonyl]phenyl} oxalate;
Figure 254 illustrates a bis{3,4,6-trichloro-2-[(6,6-dimethylheptyloxy)carbonyl]phenyl} oxalate;
Figure 255 illustrates a bis{3,4,6-trichloro-2-[(2,2,3-trimethylhexyloxy)carbonyl]phenyl} oxalate;
Figure 256 illustrates a bis{3,4,6-trichloro-2-[(2,2,4-trimethylhexyloxy)carbonyl]phenyl} oxalate;
Figure 257 illustrates a bis{3,4,6-trichloro-2-[(2,2,5-trimethylhexyloxy)carbonyl]phenyl} oxalate;
Figure 258 illustrates a bis{3,4,6-trichloro-2-[(2-ethyl-2-methylhexyloxy)carbonyl]phenyl} oxalate;
Figure 259 illustrates a bis{3,4,6-trichloro-2-[(2-ethyl-2-methylhexyloxy)carbonyl]phenyl} oxalate;
Figure 260 illustrates a bis{3,4,6-trichloro-2-[(2-ethyl-4-methylhexyloxy)carbonyl]phenyl} oxalate;
Figure 261 illustrates a bis{3,4,6-trichloro-2-[(2-ethyl-5-methylhexyloxy)carbonyl]phenyl} oxalate;
Figure 262 illustrates a bis{3,4,6-trichloro-2-[(2,3,3-trimethylhexyloxy)carbonyl]phenyl} oxalate;
Figure 263 illustrates a bis{3,4,6-trichloro-2-[(3,3,4-trimethylhexyloxy)carbonyl]phenyl} oxalate;
Figure 264 illustrates a s{3,4,6-trichloro-2-[(3,3,5-trimethylhexyloxy)carbonyl]phenyl} oxalate;
Figure 265 illustrates a bis{3,4,6-trichloro-2-[(3-ethyl-2-methylhexyloxy)carbonyl]phenyl} oxalate;
Figure 266 illustrates a bis{3,4,6-trichloro-2-[(3-ethyl-3-methylhexyloxy)carbonyl]phenyl} oxalate;
Figure 267 illustrates a bis{3,4,6-trichloro-2-[(3-ethyl-4-methylhexyloxy)carbonyl]phenyl oxalate;
Figure 268 illustrates a bis{3,4,6-trichloro-2-[(3-ethyl-5-methylhexyloxy)carbonyl]phenyl} oxalate;
Figure 269 illustrates a bis{3,4,6-trichloro-2-[2,4,4-trimethylhexyloxy)carbonyl]phenyl} oxalate;
Figure 270 illustrates a bis{3,4,6-trichloro-2-[(3,4,4-trimethylhexyloxy)carbonyl]phenyl} oxalate;
Figure 271 illustrates a bis{3,4,6-trichloro-2-[(4,4,5-trimethylhexyloxy)carbonyl]phenyl} oxalate;
Figure 272 illustrates a bis{3,4,6-trichloro-2-[(4-ethyl-2-methylhexyloxy)carbonyl]phenyl} oxalate;
Figure 273 illustrates a bis{3,4,6-trichloro-2-[(4-ethyl-3-methylhexyloxy)carbonyl]phenyl} oxalate;
Figure 274 illustrates a bis{3,4,6-trichloro-2-[(4-ethyl-4-methylhexyloxy)carbonyl]phenyl} oxalate;
Figure 275 illustrates a bis{3,4,6-trichloro-2-[(4-ethyl-5-methylhexyloxy)carbonyl]phenyl} oxalate;
Figure 276 illustrates a bis{3,4,6-trichloro-2-[(2,5,5-trimethylhexyloxy)carbonyl]phenyl} oxalate;
Figure 277 illustrates a bis{3,4,6-trichloro-2-[(3,5,5-trimethylhexyloxy)carbonyl]phenyl} oxalate;
Figure 278 illustrates a bis{3,4,6-trichloro-2-[(4,5,5-trimethylhexyloxy)carbonyl]phenyl} oxalate;
Figure 279 illustrates a bis{3,4,6-trichloro-2-[(2-propylhexyloxy)carbonyl]phenyl} oxalate;
Figure 280 illustrates a bis{3,4,6-trichloro-2-[(3-propylhexyloxy)carbonyl]phenyl} oxalate;
Figure 281 illustrates a bis(3,4,6-trichloro-2-{[2-(1-methylethyl)hexyloxy]carbonyl}phenyl) oxalate;
Figure 282 illustrates a bis(3,4,6-trichloro-2-{[3-(1-methylethyl)hexyloxy]carbonyl}phenyl) oxalate;
Figure 283 illustrates a bis{3,4,6-trichloro-2-[(2,2,3,3-tetramethylpentyloxy)carbonyl]phenyl} oxalate;
Figure 284 illustrates a bis{3,4,6-trichloro-2-[(2,2,3,4-tetramethylpentyloxy)carbonyl]phenyl} oxalate;
Figure 285 illustrates a bis{3,4,6-trichloro-2-[(2,2,4,4-tetramethylpentyloxy)carbonyl]phenyl} oxalate;
Figure 286 illustrates a bis {3,4,6-trichloro-2-[(2,3,3,4-tetramethylpentyloxy)carbonyl]phenyl} oxalate;
Figure 287 illustrates a bis {3,4,6-trichloro-2-[(2-ethyl-2,3-dimethylpentyloxy)carbonyl]phenyl} oxalate;
Figure 288 illustrates a bis{3,4,6-trichloro-2-[(2-ethyl-2,4-dimethylpentyloxy)carbonyl]phenyl} oxalate;
Figure 289 illustrates a bis{3,4,6-trichloro-2-[(2-ethyl-3,3-dimethylpentyloxy)carbonyl]phenyl} oxalate;
Figure 290 illustrates a bis{3,4,6-trichloro-2-[(2-ethyl-3,4-dimethylpentyloxy)carbonyl]phenyl} oxalate;
Figure 291 illustrates a bis {3,4,6-trichloro-2-[(2-ethyl-4,4-dimethylpentyloxy)carbonyl]phenyl} oxalate;
Figure 292 illustrates a bis {3,4,6-trichloro-2-[(2-propyl-2-methylpentyloxy)carbonyl]phenyl} oxalate;
Figure 293 illustrates a bis{3,4,6-trichloro-2-[(2-propyl-3-methylpentyloxy)carbonyl]phenyl} oxalate;
Figure 294 illustrates a bis{3,4,6-trichloro-2-[(2-propyl-4-methylpentyloxy)carbonyl]phenyl} oxalate;
Figure 295 illustrates a bis(3,4,6-trichloro-2-{[2-methyl-2-(1-methylethyl)pentyloxy]carbonyl}phenyl) oxalate;
Figure 296 illustrates a bis(3,4,6-trichloro-2-{[3-methyl-2-(1-methylethyl)pentyloxy]carbonyl}phenyl) oxalate;
Figure 297 illustrates a bis(3,4,6-trichloro-2-{[4-methyl-2-(1-methylethyl)pentyloxy]carbonyl}phenyl) oxalate;
Figure 298 illustrates a bis{3,4,6-trichloro-2-[(3-ethyl-2,4-dimethylpentyloxy)carbonyl]phenyl} oxalate;
Figure 299 illustrates a bis{3,4,6-trichloro-2-[(3-ethyl-3,4-dimethylpentyloxy)carbonyl]phenyl} oxalate;
Figure 300 illustrates a bis(3,4,6-trichloro-2-{[4-methyl-3-(1-methylethyl)pentyloxy]carbonyl}phenyl) oxalate;
Figure 301 illustrates a bis(3,4,6-trichloro-2-{[2-(1,1-dimethylethyl)pentyloxy]carbonyl}phenyl) oxalate;
Figure 302 illustrates a bis(3,4,6-trichloro-2-{[2-(1,1-dimethylethyl)pentyloxy]carbonyl}phenyl) oxalate;
Figure 303 illustrates a bis{3,4,6-trichloro-2-[(2-ethyl-2,3,3-trimethylbutoxy)carbonyl]phenyl} oxalate;
Figure 304 illustrates a bis(3,4,6-trichloro-2-{[3,3-dimethyl-2-(1-methylethyl)butoxy]carbonyl}phenyl) oxalate;
Figure 305 illustrates a bis(3,4,6-trichloro-2-{[2,3-dimethyl-2-(1-methylethyl)butoxy]carbonyl}phenyl) oxalate;
Figure 306 illustrates a bis{3,4,6-trichloro-2-[(cyclooctylmethoxy)carbonyl]phenyl} oxalate;
Figure 307 illustrates a bis(3,4,6-trichloro-2-{[(1-methylcyclooctyl)methoxy]carbonyl}phenyl) oxalate;
Figure 308 illustrates a bis(3,4,6-trichloro-2-{[(2-methylcyclooctyl)methoxy]carbonyl}phenyl) oxalate;
Figure 309 illustrates a bis(3,4,6-trichloro-2-{[(3-methylcyclooctyl)methoxy]carbonyl}phenyl) oxalate;
Figure 310 illustrates a bis(3,4,6-trichloro-2-{[(4-methylcyclooctyl)methoxy]carbonyl}phenyl) oxalate;
Figure 311 illustrates a bis {3,4,6-trichloro-2-[(2-cycloheptylethoxy)carbonyl]phenyl} oxalate;
Figure 312 illustrates a bis(3,4,6-trichloro-2-{[(1,2-dimethylcyclohexyl)methoxy]carbonyl}phenyl) oxalate;
Figure 313 illustrates a bis(3,4,6-trichloro-2-{[(1,3-dimethylcyclohexyl)methoxy]carbonyl}phenyl) oxalate;
Figure 314 illustrates a bis(3,4,6-trichloro-2-{[(1,4-dimethylcyclohexyl)methoxy]carbonyl}phenyl) oxalate;
Figure 315 illustrates a bis(3,4,6-trichloro-2-{[(2,2-dimethylcyclohexyl)methoxy]carbonyl}phenyl) oxalate;
Figure 316 illustrates a bis(3,4,6-trichloro-2-{[(2,3-dimethylcyclohexyl)methoxy]carbonyl}phenyl) oxalate;
Figure 317 illustrates a bis(3,4,6-trichloro-2-{[(2,4-dimethylcyclohexyl)methoxy)carbonyl}phenyl) oxalate;
Figure 318 illustrates a bis(3,4,6-trichloro-2-{[(2,5-dimethylcyclohexyl)methoxy]carbonyl}phenyl) oxalate;
Figure 319 illustrates a bis(3,4,6-trichloro-2-{[(2,6-dimethylcyclohexyl)methoxy]carbonyl}phenyl) oxalate;
Figure 320 illustrates a bis(3,4,6-trichloro-2-{[(3,3-dimethylcyclohexyl)methoxy]carbonyl}phenyl) oxalate;
Figure 321 illustrates a bis(3,4,6-trichloro-2-{[(3,4-dimethylcyclohexyl)methoxy]carbonyl}phenyl) oxalate;
Figure 322 illustrates a bis(3,4,6-trichloro-2-{[(3,5-dimethylcyclohexyl)methoxy]carbonyl}phenyl) oxalate;
Figure 323 illustrates a bis(3,4,6-trichloro-2-{[(4,4-dimethylcyclohexyl)methoxy]carbonyl}phenyl) oxalate;
Figure 324 illustrates a bis(3,4,6-trichloro-2-{[1-ethylcyclohexyl)methoxy]carbonyl}phenyl) oxalate;
Figure 325 illustrates a bis(3,4,6-trichloro-2-{[2-ethylcyclohexyl)methoxy]carbonyl}phenyl) oxalate;
Figure 326 illustrates a bis(3,4,6-trichloro-2-{[3-ethylcyclohexyl)methoxy]carbonyl}phenyl) oxalate;
Figure 327 illustrates a bis(3,4,6-trichloro-2-{[4-ethylcyclohexyl)methoxy]carbonyl}phenyl) oxalate;
Figure 328 illustrates a bis(3,4,6-trichloro-2-{[2-(1-methylcyclohexyl)ethoxy]carbonyl}phenyl) oxalate;
Figure 329 illustrates a bis(3,4,6-trichloro-2-{[2-(2-methylcyclohexyl)ethoxy]carbonyl}phenyl) oxalate;
Figure 330 illustrates a bis(3,4,6-trichloro-2-{[2-(3-methylcyclohexyl)ethoxy]carbonyl}phenyl) oxalate;
Figure 331 illustrates a bis(3,4,6-trichloro-2-{[2-(4-methylcyclohexyl)ethoxy]carbonyl}phenyl) oxalate;
Figure 332 illustrates a bis{3,4,6-trichloro-2-[(2-cyclohexylpropoxy)carbonyl]phenyl} oxalate;
Figure 333 illustrates a bis{3,4,6-trichloro-2-[(3-cyclohexylpropoxy)carbonyl]phenyl} oxalate;
Figure 334 illustrates a bis(3,4,6-trichloro-2-{[(1,2,2-trimethylcyclopentyl)methoxy]carbonyl}phenyl) oxalate;
Figure 335 illustrates a bis(3,4,6-trichloro-2-{[(2,2,3-trimethylcyclopentyl)methoxy]carbonyl}phenyl) oxalate;
Figure 336 illustrates a bis(3,4,6-trichloro-2-{[(2,2,4-trimethylcyclopentyl)methoxy]carbonyl}phenyl) oxalate;
Figure 337 illustrates a bis(3,4,6-trichloro-2-{[(2,2,5-trimethylcyclopentyl)methoxy]carbonyl}phenyl) oxalate;
Figure 338 illustrates a bis(3,4,6-trichloro-2-{[(1,3,3-trimethylcyclopentyl)methoxy]carbonyl}phenyl) oxalate;
Figure 339 illustrates a bis(3,4,6-trichloro-2-{[(2,3,3-trimethylcyclopentyl)methoxy]carbonyl}phenyl) oxalate;
Figure 340 illustrates a bis(3,4,6-trichloro-2-{[(3,3,4-trimethylcyclopentyl)methoxy]carbonyl}phenyl) oxalate;
Figure 341 illustrates a bis(3,4,6-trichloro-2-{[(2,4,4-trimethylcyclopentyl)methoxy]carbonyl}phenyl) oxalate;
Figure 342 illustrates a bis(3,4,6-trichloro-2-{[(1,2,3-trimethylcyclopentyl)methoxy]carbonyl}phenyl) oxalate;
Figure 343 illustrates a bis(3,4,6-trichloro-2-{[(1,2,4-trimethylcyclopentyl)methoxy]carbonyl}phenyl) oxalate;
Figure 344 illustrates a bis(3,4,6-trichloro-2-{[(1,2,5-trimethylcyclopentyl)methoxy]carbonyl}phenyl) oxalate;
Figure 345 illustrates a bis(3,4,6-trichloro-2-{[(1,3,4-trimethylcyclopentyl)methoxy]carbonyl}phenyl) oxalate;
Figure 346 illustrates a bis(3,4,6-trichloro-2-{[(2,3,4-trimethylcyclopentyl)methoxy]carbonyl}phenyl) oxalate;
Figure 347 illustrates a bis(3,4,6-trichloro-2-{[(2,3,5-trimethylcyclopentyl)methoxy]carbonyl}phenyl) oxalate;
Figure 348 illustrates a bis(3,4,6-trichloro-2-{[(2-ethyl-1-methylcyclopentyl)methoxy]carbonyl}phenyl) oxalate;
Figure 349 illustrates a bis(3,4,6-trichloro-2-{[(3-ethyl-1-methylcyclopentyl)methoxy]carbonyl}phenyl) oxalate;
Figure 350 illustrates a bis(3,4,6-trichloro-2-{[(1-ethyl-2-methylcyclopentyl)methoxy]carbonyl}phenyl) oxalate;
Figure 351 illustrates a bis(3,4,6-trichloro-2-{[(1-ethyl-3-methylcyclopentyl)methoxy]carbonyl}phenyl) oxalate;
Figure 352 illustrates a bis(3,4,6-trichloro-2-{[(3-ethyl-2-methylcyclopentyl)methoxy]carbonyl}phenyl) oxalate;
Figure 353 illustrates a bis(3,4,6-trichloro-2-{[(4-ethyl-2-methylcyclopentyl)methoxy]carbonyl}phenyl) oxalate;
Figure 354 illustrates a bis(3,4,6-trichloro-2-{[(2-ethyl-5-methylcyclopentyl)methoxy]carbonyl}phenyl) oxalate;
Figure 355 illustrates a bis(3,4,6-trichloro-2-{[(2-ethyl-3-methylcyclopentyl)methoxy]carbonyl}phenyl) oxalate;
Figure 356 illustrates a bis(3,4,6-trichloro-2-{[(2-ethyl-4-methylcyclopentyl)methoxy]carbonyl}phenyl) oxalate;
Figure 357 illustrates a bis(3,4,6-trichloro-2-{[(3-ethyl-4-methylcyclopentyl)methoxy]carbonyl}phenyl) oxalate;
Figure 358 illustrates a bis(3,4,6-trichloro-2-{[(2-ethyl-2-methylcyclopentyl)methoxy]carbonyl}phenyl) oxalate;
Figure 359 illustrates a bis(3,4,6-trichloro-2-{[(3-ethyl-3-methylcyclopentyl)methoxy]carbonyl}phenyl) oxalate;
Figure 360 illustrates a bis(3,4,6-trichloro-2-{[(1-propylcyclopentyl)methoxy]carbonyl}phenyl) oxalate;
Figure 361 illustrates a bis(3,4,6-trichloro-2-{[(2-propylcyclopentyl)methoxy]carbonyl}phenyl) oxalate;
Figure 362 illustrates a bis(3,4,6-trichloro-2-{[(3-propylcyclopentyl)methoxy]carbonyl}phenyl) oxalate;
Figure 363 illustrates a bis[3,4,6-trichloro-2-({[1-(1-methylethyl)-cyclopentyl]methoxy} carbonyl)phenyl] oxalate;
Figure 364 illustrates a bis[3,4,6-trichloro-2-({[2-(1-methylethyl)-cyclopentyl]methoxy} carbonyl)phenyl] oxalate;
Figure 365 illustrates a bis[3,4,6-trichloro-2-({[3-(1-methylethyl)-cyclopentyl]methoxy}carbonyl)phenyl] oxalate;
Figure 366 illustrates a bis(3,4,6-trichloro-2-{[2-(1,2-dimethylcyclopentyl)ethoxy]carbonyl}phenyl) oxalate;
Figure 367 illustrates a bis(3,4,6-trichloro-2-{[2-(1,3-dimethylcyclopentyl)ethoxy]carbonyl}phenyl) oxalate;
Figure 368 illustrates a bis(3,4,6-trichloro-2-{[2-(2,3-dimethylcyclopentyl)ethoxy]carbonyl}phenyl) oxalate;
Figure 369 illustrates a bis(3,4,6-trichloro-2-{[2-(2,4-dimethylcyclopentyl)ethoxy]carbonyl}phenyl) oxalate;
Figure 370 illustrates a bis(3,4,6-trichloro-2-{[2-(2,5-dimethylcyclopentyl)ethoxy]carbonyl}phenyl) oxalate;
Figure 371 illustrates a bis(3,4,6-trichloro-2-{[2-(3,4-dimethylcyclopentyl)ethoxy]carbonyl}phenyl) oxalate;
Figure 372 illustrates a bis(3,4,6-trichloro-2-{[2-(2,2-dimethylcyclopentyl)ethoxy]carbonyl}phenyl) oxalate;
Figure 373 illustrates a bis(3,4,6-trichloro-2-{[2-(3,3-dimethylcyclopentyl)ethoxy]carbonyl}phenyl) oxalate;
Figure 374 illustrates a bis(3,4,6-trichloro-2-{[2-(1-ethylcyclopentyl)ethoxy]carbonyl}phenyl) oxalate;
Figure 375 illustrates a bis(3,4,6-trichloro-2-{[2-(2-ethylcyclopentyl)ethoxy]carbonyl}phenyl) oxalate;
Figure 376 illustrates a bis(3,4,6-trichloro-2-{[2-(3-ethylcyclopentyl)ethoxy]carbonyl}phenyl) oxalate;
Figure 377 illustrates a bis{3,4,6-trichloro-2-[(2-cyclopentyl-2-methylpropoxy)carbonyl]phenyl} oxalate;
Figure 378 illustrates a bis(3,4,6-trichloro-2-{[2-(1-methylcyclopentyl)propoxy]carbonyl}phenyl) oxalate;
Figure 379 illustrates a bis(3,4,6-trichloro-2-{[2-(2-methylcyclopentyl)propoxy]carbonyl}phenyl) oxalate;
Figure 380 illustrates a bis(3,4,6-trichloro-2-{[2-(3-methylcyclopentyl)propoxy]carbonyl}phenyl) oxalate;
Figure 381 illustrates a bis{3,4,6-trichloro-2-[(3-cyclopentyl-2-methylpropoxy)carbonyl]phenyl} oxalate;
Figure 382 illustrates a bis(3,4,6-trichloro-2-{[3-(1-methylcyclopentyl)propoxy]carbonyl}phenyl) oxalate;
Figure 383 illustrates a bis(3,4,6-trichloro-2-{[3-(2-methylcyclopentyl)propoxy]carbonyl}phenyl) oxalate;
Figure 384 illustrates a bis(3,4,6-trichloro-2-{[3-(3-methylcyclopentyl)propoxy]carbonyl}phenyl) oxalate;
Figure 385 illustrates a bis{3,4,6-trichloro-2-[(2-cyclopentylbutoxy)carbonyl]phenyl} oxalate;
Figure 386 illustrates a bis{3,4,6-trichloro-2-[(3-cyclopentylbutoxy)carbonyl]phenyl} oxalate;
Figure 387 illustrates a bis{3,4,6-trichloro-2-[(4-cyclopentylbutoxy)carbonyl]phenyl} oxalate;
Figure 388 illustrates a bis(3,4,6-trichloro-2-{[(1,2,2,3-tetramethylcyclobutyl)methoxy]carbonyl}phenyl) oxalate;
Figure 389 illustrates a bis(3,4,6-trichloro-2-{[(1,2,2,4-tetramethylcyclobutyl)methoxy]carbonyl}phenyl) oxalate;
Figure 390 illustrates a bis(3,4,6-trichloro-2-{[(1,2,3,3-tetramethylcyclobutyl)methoxy]carbonyl}phenyl) oxalate;
Figure 391 illustrates a bis(3,4,6-trichloro-2-{[(1,2,3,4-tetramethylcyclobutyl)methoxy]carbonyl}phenyl) oxalate;
Figure 392 illustrates a bis(3,4,6-trichloro-2-{[(2,2,3,4-tetramethylcyclobutyl)methoxy]carbonyl}phenyl) oxalate;
Figure 393 illustrates a bis(3,4,6-trichloro-2-{[(2,3,3,4-tetramethylcyclobutyl)methoxy]carbonyl}phenyl) oxalate;
Figure 394 illustrates a bis(3,4,6-trichloro-2-{[(2,2,3,3-tetramethylcyclobutyl)methoxy]carbonyl}phenyl) oxalate;
Figure 395 illustrates a bis(3,4,6-trichloro-2-{[(2,2,4,4-tetramethylcyclobutyl)methoxy]carbonyl}phenyl) oxalate;
Figure 396 illustrates a bis(3,4,6-trichloro-2-{[(1-ethyl-2,2-dimethylcyclobutyl)methoxy]carbonyl}phenyl) oxalate;
Figure 397 illustrates a bis(3,4,6-trichloro-2-{[(1-ethyl-2,3-dimethylcyclobutyl)methoxy]carbonyl}phenyl) oxalate;
Figure 398 illustrates a bis(3,4,6-trichloro-2-{[(1-ethyl-2,4-dimethylcyclobutyl)methoxy]carbonyl}phenyl) oxalate;
Figure 399 illustrates a bis(3,4,6-trichloro-2-{[(1-ethyl-3,3-dimethylcyclobutyl)methoxy]carbonyl}phenyl) oxalate;
Figure 400 illustrates a bis(3,4,6-trichloro-2-{[(2-ethyl-1,2-dimethylcyclobutyl)methoxy]carbonyl}phenyl) oxalate;
Figure 401 illustrates a bis(3,4,6-trichloro-2-{[(2-ethyl-1,3-dimethylcyclobutyl)methoxy]carbonyl}phenyl) oxalate;
Figure 402 illustrates a bis(3,4,6-trichloro-2-{[(2-ethyl-1,4-dimethylcyclobutyl)methoxy]carbonyl}phenyl) oxalate;
Figure 403 illustrates a bis(3,4,6-trichloro-2-{[(2-ethyl-3,3-dimethylcyclobutyl)methoxy]carbonyl}phenyl) oxalate;
Figure 404 illustrates a bis(3,4,6-trichloro-2-{[(2-ethyl-3,4-dimethylcyclobutyl)methoxy]carbonyl}phenyl) oxalate;
Figure 405 illustrates a bis(3,4,6-trichloro-2-{[(2-ethyl-4,4-dimethylcyclobutyl)methoxy]carbonyl}phenyl) oxalate;
Figure 406 illustrates a bis(3,4,6-trichloro-2-{[(1,2-diethylcyclobutyl)methoxy]carbonyl}phenyl) oxalate;
Figure 407 illustrates a bis(3,4,6-trichloro-2-{[(1,3-diethylcyclobutyl)methoxy]carbonyl}phenyl) oxalate;
Figure 408 illustrates a bis(3,4,6-trichloro-2-{[(2,2-diethylcyclobutyl)methoxy]carbonyl}phenyl) oxalate;
Figure 409 illustrates a bis(3,4,6-trichloro-2-{[(3,3-diethylcyclobutyl)methoxy]carbonyl}phenyl) oxalate;
Figure 410 illustrates a bis(3,4,6-trichloro-2-{[(1-methyl-2-propylcyclobutyl)methoxy]carbonyl}phenyl) oxalate;
Figure 411 illustrates a bis(3,4,6-trichloro-2-{[(2-methyl-2-propylcyclobutyl)methoxy]carbonyl}phenyl) oxalate;
Figure 412 illustrates a bis(3,4,6-trichloro-2-{[(2-methyl-1-propylcyclobutyl)methoxy]carbonyl}phenyl) oxalate;
Figure 413 illustrates a bis(3,4,6-trichloro-2-{[(3-methyl-1-propylcyclobutyl)methoxy]carbonyl}phenyl) oxalate;
Figure 414 illustrates a bis(3,4,6-trichloro-2-{[(3-methyl-2-propylcyclobutyl)methoxy]carbonyl}phenyl) oxalate;
Figure 415 illustrates a bis(3,4,6-trichloro-2-{[(2-methyl-4-propylcyclobutyl)methoxy]carbonyl}phenyl) oxalate;
Figure 416 illustrates a bis(3,4,6-trichloro-2-{[(1-methyl-3-propylcyclobutyl)methoxy]carbonyl}phenyl) oxalate;
Figure 417 illustrates a bis(3,4,6-trichloro-2-{[(2-methyl-3-propylcyclobutyl)methoxy]carbonyl}phenyl) oxalate;
Figure 418 illustrates a bis(3,4,6-trichloro-2-{[(3-methyl-3-propylcyclobutyl)methoxy]carbonyl}phenyl) oxalate;
Figure 419 illustrates a bis[3,4,6-trichloro-2-({[2-methyl-1-(1-methylethyl)cyclobutyl]methoxy} carbonyl)phenyl] oxalate;
Figure 420 illustrates a bis[3,4,6-trichloro-2-({[3-methyl-1-(1-methylethyl)cyclobutyl]methoxy} carbonyl)phenyl] oxalate;
Figure 421 illustrates a bis[3,4,6-trichloro-2-({[1-methyl-2-(1-methylethyl)cyclobutyl]methoxy}carbonyl)phenyl] oxalate;
Figure 422 illustrates a bis[3,4,6-trichloro-2-({[2-methyl-2-(1-methylethyl)cyclobutyl]methoxy} carbonyl)phenyl] oxalate;
Figure 423 illustrates a bis[3,4,6-trichloro-2-({[3-methyl-2-(1-methylethyl)cyclobutyl]methoxy} carbonyl)phenyl] oxalate;
Figure 424 illustrates a bis[3,4,6-trichloro-2-({[2-methyl-4-(1-methylethyl)cyclobutyl]methoxy} carbonyl)phenyl] oxalate;
Figure 425 illustrates a bis[3,4,6-trichloro-2-({[3-methyl-3-(1-methylethyl)cyclobutyl]methoxy} carbonyl)phenyl] oxalate;
Figure 426 illustrates a bis(3,4,6-trichloro-2-{[(1-butylcyclobutyl)methoxy]carbonyl}phenyl) oxalate;
Figure 427 illustrates a bis(3,4,6-trichloro-2-{[(2-butylcyclobutyl)methoxy]carbonyl}phenyl) oxalate;
Figure 428 illustrates a bis(3,4,6-trichloro-2-{[(3-butylcyclobutyl)methoxy]carbonyl}phenyl) oxalate;
Figure 429 illustrates a bis[3,4,6-trichloro-2-({[1-(1-methylpropyl)cyclobutyl]methoxy} carbonyl)phenyl] oxalate;
Figure 430 illustrates a bis[3,4,6-trichloro-2-({[1-(2-methylpropyl)cyclobutyl]methoxy} carbonyl)phenyl] oxalate;
Figure 431 illustrates a bis[3,4,6-trichloro-2-({[2-(1-methylpropyl)cyclobutyl]methoxy} carbonyl)phenyl] oxalate;
Figure 432 illustrates a bis[3,4,6-trichloro-2-({[2-(2-methylpropyl)cyclobutyl]methoxy}carbonyl)phenyl] oxalate;
Figure 433 illustrates a bis[3,4,6-trichloro-2-({[3-(1-methylpropyl)cyclobutyl]methoxy}carbonyl)phenyl] oxalate;
Figure 434 illustrates a bis[3,4,6-trichloro-2-({[3-(2-methylpropyl)cyclobutyl]methoxy} carbonyl)phenyl] oxalate;
Figure 435 illustrates a bis(3,4,6-trichloro-2-{[2-(1,2,2-trimethylcyclobutyl)ethoxy]carbonyl}phenyl) oxalate;
Figure 436 illustrates a bis(3,4,6-trichloro-2-{[2-(1,2,3-trimethylcyclobutyl)ethoxy]carbonyl}phenyl) oxalate;
Figure 437 illustrates a bis(3,4,6-trichloro-2-{[2-(1,2,4-trimethylcyclobutyl)ethoxy]carbonyl}phenyl) oxalate;
Figure 438 illustrates a bis(3,4,6-trichloro-2-{[2-(1,3,3-trimethylcyclobutyl)ethoxy]carbonyl}phenyl) oxalate;
Figure 439 illustrates a bis(3,4,6-trichloro-2-{[2-(2,2,3-trimethylcyclobutyl)ethoxy]carbonyl}phenyl) oxalate;
Figure 440 illustrates a bis(3,4,6-trichloro-2-{[2-(2,2,4-trimethylcyclobutyl)ethoxy]carbonyl}phenyl) oxalate;
Figure 441 illustrates a bis(3,4,6-trichloro-2-{[2-(2,3,4-trimethylcyclobutyl)ethoxy]carbonyl}phenyl) oxalate;
Figure 442 illustrates a bis(3,4,6-trichloro-2-{[2-(2-ethyl-1-methylcyclobutyl)ethoxy]carbonyl}phenyl) oxalate;
Figure 443 illustrates a bis(3,4,6-trichloro-2-{[2-(3-ethyl-1-methylcyclobutyl)ethoxy]carbonyl}phenyl) oxalate;
Figure 444 illustrates a bis(3,4,6-trichloro-2-{[2-(1-ethyl-2-methylcyclobutyl)ethoxy]carbonyl}phenyl) oxalate;
Figure 445 illustrates a bis(3,4,6-trichloro-2-{[2-(1-ethyl-3-methylcyclobutyl)ethoxy]carbonyl}phenyl) oxalate;
Figure 446 illustrates a bis(3,4,6-trichloro-2-{[2-(2-ethyl-2-methylcyclobutyl)ethoxy]carbonyl}phenyl) oxalate;
Figure 447 illustrates a bis(3,4,6-trichloro-2-{[2-(2-ethyl-3-methylcyclobutyl)ethoxy]carbonyl}phenyl) oxalate;
Figure 448 illustrates a bis(3,4,6-trichloro-2-{[2-(2-ethyl-4-methylcyclobutyl)ethoxy]carbonyl}phenyl) oxalate;
Figure 449 illustrates a bis(3,4,6-trichloro-2-{[2-(3-ethyl-2-methylcyclobutyl)ethoxy]carbonyl}phenyl) oxalate;
Figure 450 illustrates a bis(3,4,6-trichloro-2-{[2-(3-ethyl-3-methylcyclobutyl)ethoxy]carbonyl}phenyl) oxalate;
Figure 451 illustrates a bis(3,4,6-trichloro-2-{[2-(1-propylcyclobutyl)ethoxy]carbonyl}phenyl) oxalate;
Figure 452 illustrates a bis(3,4,6-trichloro-2-{[2-(2-propylcyclobutyl)ethoxy]carbonyl}phenyl) oxalate;
Figure 453 illustrates a bis(3,4,6-trichloro-2-{[2-(3-propylcyclobutyl)ethoxy]carbonyl}phenyl) oxalate;
Figure 454 illustrates a bis[3,4,6-trichloro-2-({2-[1-(1-methylethyl)cyclobutyl]ethoxy}carbonyl)phenyl] oxalate;
Figure 455 illustrates a bis[3,4,6-trichloro-2-({2-[2-(1-methylethyl)cyclobutyl]ethoxy}carbonyl)phenyl] oxalate;
Figure 456 illustrates a bis[3,4,6-trichloro-2-({2-[3-(1-methylethyl)cyclobutyl]ethoxy}carbonyl)phenyl] oxalate;
Figure 457 illustrates a bis(3,4,6-trichloro-2-{[2-methyl-2-(1-methylcyclobutylpropoxy]carbonyl}phenyl) oxalate;
Figure 458 illustrates a bis(3,4,6-trichloro-2-{[2-methyl-2-(2-methylcyclobutylpropoxy]carbonyl}phenyl) oxalate;
Figure 459 illustrates a bis(3,4,6-trichloro-2-{[2-methyl-2-(3-methylcyclobutylpropoxy]carbonyl}phenyl) oxalate;
Figure 460 illustrates a bis(3,4,6-trichloro-2-{[2-(1,2-dimethylcyclobutylpropoxy]carbonyl}phenyl) oxalate;
Figure 461 illustrates a bis(3,4,6-trichloro-2-{[2-(1,3-dimethylcyclobutylpropoxy]carbonyl}phenyl) oxalate;
Figure 462 illustrates a bis(3,4,6-trichloro-2-{[2-(1,2,2-triimethylcyclobutylethoxy]carbonyl}phenyl) oxalate;
Figure 463 illustrates a bis(3,4,6-trichloro-2-{[2-(1,2,3-triimethylcyclobutylethoxy]carbonyl}phenyl) oxalate;
Figure 464 illustrates a bis(3,4,6-trichloro-2-{[2-(1,2,4-triimethylcyclobutylethoxy]carbonyl}phenyl) oxalate;
Figure 465 illustrates a bis(3,4,6-trichloro-2-{[2-(1,3,3-triimethylcyclobutylethoxy]carbonyl}phenyl) oxalate;
Figure 466 illustrates a bis(3,4,6-trichloro-2-{[2-(2,2,3-triimethylcyclobutylethoxy]carbonyl}phenyl) oxalate;
Figure 467 illustrates a bis(3,4,6-trichloro-2-{[2-(2,3,3-triimethylcyclobutylethoxy]carbonyl}phenyl) oxalate;
Figure 468 illustrates a bis(3,4,6-trichloro-2-{[2-(2,2,4-triimethylcyclobutylethoxy]carbonyl}phenyl) oxalate;
Figure 469 illustrates a bis(3,4,6-trichloro-2-{[2-(2,3,4-triimethylcyclobutylethoxy]carbonyl}phenyl) oxalate;
Figure 470 illustrates a bis(3,4,6-trichloro-2-{[3-(1,2-dimethylcyclobutylpropoxy]carbonyl}phenyl) oxalate;
Figure 471 illustrates a bis(3,4,6-trichloro-2-{[3-(1,3-dimethylcyclobutylpropoxy]carbonyl}phenyl) oxalate;
Figure 472 illustrates a bis(3,4,6-trichloro-2-{[3-(2,2-dimethylcyclobutylpropoxy]carbonyl}phenyl) oxalate;
Figure 473 illustrates a bis(3,4,6-trichloro-2-{[3-(2,3-dimethylcyclobutylpropoxy]carbonyl}phenyl) oxalate;
Figure 474 illustrates a bis(3,4,6-trichloro-2-{[3-(2,4-dimethylcyclobutylpropoxy]carbonyl}phenyl) oxalate;
Figure 475 illustrates a bis(3,4,6-trichloro-2-{[3-(3,3-dimethylcyclobutylpropoxy]carbonyl}phenyl) oxalate;
Figure 476 illustrates a bis(3,4,6-trichloro-2-{[3-(1-ethylcyclobutylpropoxy]carbonyl}phenyl) oxalate;
Figure 477 illustrates a bis(3,4,6-trichloro-2-{[3-(2-ethylcyclobutylpropoxy]carbonyl}phenyl) oxalate;
Figure 478 illustrates a bis(3,4,6-trichloro-2-{[3-(3-ethylcyclobutylpropoxy]carbonyl}phenyl) oxalate;
Figure 479 illustrates a bis(3,4,6-trichloro-2-{[2-(1-methylcyclobutylbutoxy]carbonyl}phenyl) oxalate;
Figure 480 illustrates a bis(3,4,6-trichloro-2-{[2-(2-methylcyclobutylbutoxy]carbonyl}phenyl) oxalate;
Figure 481 illustrates a bis(3,4,6-trichloro-2-{[2-(3-methylcyclobutylbutoxy]carbonyl}phenyl) oxalate;
Figure 482 illustrates a bis{3,4,6-trichloro-2-[(3-cyclobutyl-2-methylbutoxy)carbonyl]phenyl} oxalate;
Figure 483 illustrates a bis {3,4,6-trichloro-2-[(3-cyclobutyl-3-methylbutoxy)carbonyl]phenyl} oxalate;
Figure 484 illustrates a bis(3,4,6-trichloro-2-{[3-(1-methylcyclobutylbutoxy]carbonyl}phenyl) oxalate;
Figure 485 illustrates a bis(3,4,6-trichloro-2-{[3-(2-methylcyclobutylbutoxy]carbonyl}phenyl) oxalate;
Figure 486 illustrates a bis(3,4,6-trichloro-2-{[3-(2-methylcyclobutylbutoxy]carbonyl}phenyl) oxalate;
Figure 487 illustrates a bis {3,4,6-trichloro-2-[(4-cyclobutyl-2-methylbutoxy)carbonyl]phenyl} oxalate;
Figure 488 illustrates a bis{3,4,6-trichloro-2-[(4-cyclobutyl-3-methylbutoxy)carbonyl]phenyl} oxalate;
Figure 489 illustrates a bis(3,4,6-trichloro-2-{[4-(1-methylcyclobutylbutoxy]carbonyl}phenyl) oxalate;
Figure 490 illustrates a bis(3,4,6-trichloro-2-{[4-(2-methylcyclobutylbutoxy]carbonyl}phenyl) oxalate;
Figure 491 illustrates a bis(3,4,6-trichloro-2-{[4-(3-methylcyclobutylbutoxy]carbonyl}phenyl) oxalate;
Figure 492 illustrates a bis{3,4,6-trichloro-2-[(2-cyclobutylpentyloxy)carbonyl]phenyl} oxalate;
Figure 493 illustrates a bis{3,4,6-trichloro-2-[(3-cyclobutylpentyloxy)carbonyl]phenyl} oxalate;
Figure 494 illustrates a bis{3,4,6-trichloro-2-[(4-cyclobutylpentyloxy)carbonyl]phenyl} oxalate;
Figure 495 illustrates a bis {3,4,6-trichloro-2-[(5-cyclobutylpentyloxy)carbonyl]phenyl} oxalate;
Figure 496 illustrates a bis(3,4,6-trichloro-2-{[(1,2,2,3,3-pentamethylcyclopropyl)methoxy]carbonyl}phenyl) oxalate;
Figure 497 illustrates a bis(3,4,6-trichloro-2-{[(3-ethyl-1,2,2-trimethylcyclopropyl)methoxy]carbonyl}phenyl) oxalate;
Figure 498 illustrates a bis(3,4,6-trichloro-2-{[(2-ethyl-1,2,3-trimethylcyclopropyl)methoxy]carbonyl}phenyl) oxalate;
Figure 499 illustrates a bis(3,4,6-trichloro-2-{[(1-ethyl-2,2,3-trimethylcyclopropyl)methoxy]carbonyl}phenyl) oxalate;
Figure 500 illustrates a bis(3,4,6-trichloro-2-{[(2,2-dimethyl-3-propylcyclopropyl)methoxy]carbonyl}phenyl) oxalate;
Figure 501 illustrates a bis[3,4,6-trichloro-2-({[2,2-dimethyl-3-(1-methylethyl)cyclopropyl]methoxy}carbonyl)phenyl] oxalate;
Figure 502 illustrates a bis(3,4,6-trichloro-2-{[(2,3-dimethyl-1-propylcyclopropyl)methoxy]carbonyl}phenyl) oxalate;
Figure 503 illustrates a bis[3,4,6-trichloro-2-({[2,3-dimethyl-1-(1-methylethyl)cyclopropyl]methoxy}carbonyl)phenyl] oxalate;
Figure 504 illustrates a bis(3,4,6-trichloro-2-{[(2-ethyl-1-propylcyclopropyl)methoxy]carbonyl}phenyl) oxalate;
Figure 505 illustrates a bis(3,4,6-trichloro-2-{[(1-ethyl-2-propylcyclopropyl)methoxy]carbonyl}phenyl) oxalate;
Figure 506 illustrates a bis[3,4,6-trichloro-2-({[1-ethyl-2-(1-methylethyl)cyclopropyl]methoxy}carbonyl)phenyl] oxalate;
Figure 507 illustrates a bis(3,4,6-trichloro-2-{[(1,2-diethyl-3-methylcyclopropyl)methoxy]carbonyl}phenyl) oxalate;
Figure 508 illustrates a bis(3,4,6-trichloro-2-{[(1-butyl-2-methylcyclopropyl)methoxy]carbonyl}phenyl) oxalate;
Figure 509 illustrates a bis[3,4,6-trichloro-2-({[2-methyl-1-(2-methylpropyl)cyclopropyl]methoxy}carbonyl)phenyl] oxalate;
Figure 510 illustrates a bis[3,4,6-trichloro-2-({[2-methyl-1-(1-methylpropyl)cyclopropyl]methoxy}carbonyl)phenyl] oxalate;
Figure 511 illustrates a bis(3,4,6-trichloro-2-{[(2-butyl-1-methylcyclopropyl)methoxy]carbonyl}phenyl) oxalate;
Figure 512 illustrates a bis[3,4,6-trichloro-2-({[1-methyl-2-(1-methylpropyl)cyclopropyl]methoxy}carbonyl)phenyl] oxalate;
Figure 513 illustrates a bis[3,4,6-trichloro-2-({[1-methyl-2-(2-methylpropyl)cyclopropyl]methoxy}carbonyl)phenyl] oxalate;
Figure 514 illustrates a bis(3,4,6-trichloro-2-{[(2-butyl-3-methylcyclopropyl)methoxy]carbonyl}phenyl) oxalate;
Figure 515 illustrates a bis[3,4,6-trichloro-2-({[2-methyl-3-(1-methylpropyl)cyclopropyl]methoxy} carbonyl)phenyl] oxalate;
Figure 516 illustrates a bis[3,4,6-trichloro-2-({[2-methyl-3-(2-methylpropyl)cyclopropyl]methoxy}carbonyl)phenyl] oxalate;
Figure 517 illustrates a bis[3,4,6-trichloro-2-({[1-(1,1-dimethylpropyl)cyclopropyl]methoxy} carbonyl)phenyl] oxalate;
Figure 518 illustrates a bis[3,4,6-trichloro-2-({[1-(2,2-dimethylpropyl)cyclopropyl]methoxy}carbonyl)phenyl] oxalate;
Figure 519 illustrates a bis[3,4,6-trichloro-2-({[1-(1,2-dimethylpropyl)cyclopropyl]methoxy} carbonyl)phenyl] oxalate;
Figure 520 illustrates a bis[3,4,6-trichloro-2-({[1-(1-methylbutyl)cyclopropyl]methoxy}carbonyl)phenyl] oxalate;
Figure 521 illustrates a bis[3,4,6-trichloro-2-({[1-(2-methylbutyl)cyclopropyl]methoxy}carbonyl)phenyl] oxalate;
Figure 522 illustrates a bis[3,4,6-trichloro-2-({[1-(3-methylbutyl)cyclopropyl]methoxy} carbonyl)phenyl] oxalate;
Figure 523 illustrates a bis(3,4,6-trichloro-2-{[(1-pentylcyclopropyl)methoxy]carbonyl}phenyl) oxalate;
Figure 524 illustrates a bis[3,4,6-trichloro-2-({[2-(1,1-dimethylpropyl)cyclopropyl]methoxy}carbonyl)phenyl] oxalate;
Figure 525 illustrates a bis[3,4,6-trichloro-2-({[2-(2,2-dimethylpropyl)cyclopropyl]methoxy}carbonyl)phenyl] oxalate;
Figure 526 illustrates a bis[3,4,6-trichloro-2-({[2-(1,2-dimethylpropyl)cyclopropyl]methoxy}carbonyl)phenyl] oxalate;
Figure 527 illustrates a bis[3,4,6-trichloro-2-({[2-(1-methylbutyl)cyclopropyl]methoxy}carbonyl)phenyl] oxalate;
Figure 528 illustrates a bis[3,4,6-trichloro-2-({[2-(2-methylbutyl)cyclopropyl]methoxy}carbonyl)phenyl] oxalate;
Figure 529 illustrates a bis[3,4,6-trichloro-2-({[2-(3-methylbutyl)cyclopropyl]methoxy} carbonyl)phenyl] oxalate;
Figure 530 illustrates a bis(3,4,6-trichloro-2-{[(2-pentylcyclopropyl)methoxy]carbonyl}phenyl) oxalate;
Figure 531 illustrates a bis(3,4,6-trichloro-2-{[2-(1,2,2,3-tetramethylcyclopropyl)ethoxy]carbonyl}phenyl) oxalate;
Figure 532 illustrates a bis(3,4,6-trichloro-2-{[2-(2,2,3,3-tetramethylcyclopropyl)ethoxy]carbonyl}phenyl) oxalate;
Figure 533 illustrates a bis(3,4,6-trichloro-2-{[2-(1-ethyl-2,3-dimethylcyclopropyl)ethoxy]carbonyl}phenyl) oxalate;
Figure 534 illustrates a bis(3,4,6-trichloro-2-{[2-(1-ethyl-2,2-dimethylcyclopropyl)ethoxy]carbonyl}phenyl) oxalate;
Figure 535 illustrates a bis(3,4,6-trichloro-2-{[2-(2-ethyl-1,3-dimethylcyclopropyl)ethoxy]carbonyl}phenyl) oxalate;
Figure 536 illustrates a bis(3,4,6-trichloro-2-{[2-(3-ethyl-2,2-dimethylcyclopropyl)ethoxy]carbonyl}phenyl) oxalate;
Figure 537 illustrates a bis(3,4,6-trichloro-2-{[2-(1,2-diethylcyclopropyl)ethoxy]carbonyl}phenyl) oxalate;
Figure 538 illustrates a bis(3,4,6-trichloro-2-{[2-(2,2-diethylcyclopropyl)ethoxy]carbonyl}phenyl) oxalate;
Figure 539 illustrates a bis(3,4,6-trichloro-2-{[2-(2,3-diethylcyclopropyl)ethoxy]carbonyl}phenyl) oxalate;
Figure 540 illustrates a bis(3,4,6-trichloro-2-{[2-(1-methyl-3-propylcyclopropyl)ethoxy]carbonyl}phenyl) oxalate;
Figure 541 illustrates a bis(3,4,6-trichloro-2-{[2-(3-methyl-1-propylcyclopropyl)ethoxy]carbonyl}phenyl) oxalate;
Figure 542 illustrates a bis(3,4,6-trichloro-2-{[2-(2-methyl-3-propylcyclopropyl)ethoxy]carbonyl}phenyl) oxalate;
Figure 543 illustrates a bis(3,4,6-trichloro-2-{[2-(2-methyl-2-propylcyclopropyl)ethoxy]carbonyl}phenyl) oxalate;
Figure 544 illustrates a bis[3,4,6-trichloro-2-({2-[1-methyl-2-(1-methylethyl)cyclopropyl]ethoxy}carbonyl)phenyl] oxalate;
Figure 545 illustrates a bis[3,4,6-trichloro-2-({2-[2-methyl-1-(1-methylethyl)cyclopropyl]ethoxy}carbonyl)phenyl] oxalate;
Figure 546 illustrates a bis[3,4,6-trichloro-2-({2-[2-methyl-3-(1-methylethyl)cyclopropyl]ethoxy}carbonyl)phenyl] oxalate;
Figure 547 illustrates a bis[3,4,6-trichloro-2-({2-[2-methyl-1-(1-methylethyl)cyclopropyl]ethoxy}carbonyl)phenyl] oxalate;
Figure 548 illustrates a bis(3,4,6-trichloro-2-{[2-(1-butylcyclopropyl)ethoxy]carbonyl}phenyl) oxalate;
Figure 549 illustrates a bis[3,4,6-trichloro-2-({2-[1-(1-methylpropyl)cyclopropyl]ethoxy}carbonyl)phenyl] oxalate;
Figure 550 illustrates a bis[3,4,6-trichloro-2-({2-[1-(2-methylpropyl)cyclopropyl]ethoxy}carbonyl)phenyl] oxalate;
Figure 551 illustrates a bis(3,4,6-trichloro-2-{[2-(2-butylcyclopropyl)ethoxy]carbonyl}phenyl) oxalate;
Figure 552 illustrates a bis[3,4,6-trichloro-2-({2-[2-(1-methylpropyl)cyclopropyl]ethoxy}carbonyl)phenyl] oxalate;
Figure 553 illustrates a bis[3,4,6-trichloro-2-({2-[2-(2-methylpropyl)cyclopropyl]ethoxy}carbonyl)phenyl] oxalate;
Figure 554 illustrates a bis(3,4,6-trichloro-2-{[2-(1,2,2-trimethylcyclopropyl)propoxy]carbonyl}phenyl) oxalate;
Figure 555 illustrates a bis(3,4,6-trichloro-2-{[2-(1,2,3-trimethylcyclopropyl)propoxy]carbonyl}phenyl) oxalate;
Figure 556 illustrates a bis(3,4,6-trichloro-2-{[2-(2,2,3-trimethylcyclopropyl)propoxy]carbonyl}phenyl) oxalate;
Figure 557 illustrates a bis(3,4,6-trichloro-2-{[2-methyl-2-(2,3-dimethylcyclopropyl)propoxy]carbonyl}phenyl) oxalate;
Figure 558 illustrates a bis(3,4,6-trichloro-2-{[2-(1-ethyl-2-methylcyclopropyl)propoxy]carbonyl}phenyl) oxalate;
Figure 559 illustrates a bis(3,4,6-trichloro-2-{[2-(2-ethyl-1-methylcyclopropyl)propoxy]carbonyl}phenyl) oxalate;
Figure 560 illustrates a bis(3,4,6-trichloro-2-{[2-(2-ethyl-3-methylcyclopropyl)propoxy]carbonyl}phenyl) oxalate;
Figure 561 illustrates a bis(3,4,6-trichloro-2-{[2-(2-ethyl-2-methylcyclopropyl)propoxy]carbonyl}phenyl) oxalate;
Figure 562 illustrates a bis(3,4,6-trichloro-2-{[2-(1-propylcyclopropyl)propoxy]carbonyl}phenyl) oxalate;
Figure 563 illustrates a bis(3,4,6-trichloro-2-{[2-(2-propylcyclopropyl)propoxy]carbonyl}phenyl) oxalate;
Figure 564 illustrates a bis[3,4,6-trichloro-2-({2-[1-(1-methylethyl)cyclopropyl]propoxy}carbonyl)phenyl] oxalate;
Figure 565 illustrates a bis[3,4,6-trichloro-2-({2-[1-(1-methylethyl)cyclopropyl]propoxy} carbonyl)phenyl] oxalate;
Figure 566 illustrates a bis(3,4,6-trichloro-2-{[2-methyl-2-(1,2-dimethylcyclopropyl)propoxy]carbonyl}phenyl) oxalate;
Figure 567 illustrates a bis(3,4,6-trichloro-2-{[2-methyl-2-(2,2-dimethylcyclopropyl)propoxy]carbonyl}phenyl) oxalate;
Figure 568 illustrates a bis(3,4,6-trichloro-2-{[-2-(1-ethylcyclopropyl)-2-methylpropoxy]carbonyl}phenyl) oxalate;
Figure 569 illustrates a bis(3,4,6-trichloro-2-{[-2-(2-ethylcyclopropyl)-2-methylpropoxy]carbonyl}phenyl) oxalate;
Figure 570 illustrates a bis(3,4,6-trichloro-2-{[2,2-dimethyl-3-(1-methylcyclopropyl)propoxy]carbonyl}phenyl) oxalate;
Figure 571 illustrates a bis(3,4,6-trichloro-2-{[2,2-dimethyl-3-(2-methylcyclopropyl)propoxy]carbonyl}phenyl) oxalate;
Figure 572 illustrates a bis(3,4,6-trichloro-2-{[2,3-dimethyl-3-(1-methylcyclopropyl)propoxy]carbonyl}phenyl) oxalate;
Figure 573 illustrates a bis(3,4,6-trichloro-2-{[2,3-dimethyl-3-(2-methylcyclopropyl)propoxy]carbonyl}phenyl) oxalate;
Figure 574 illustrates a bis(3,4,6-trichloro-2-{[2-methyl-3-(1,2-dimethylcyclopropyl)propoxy]carbonyl}phenyl) oxalate;
Figure 575 illustrates a bis(3,4,6-trichloro-2-{[2-methyl-3-(2,3-dimethylcyclopropyl)propoxy]carbonyl}phenyl) oxalate;
Figure 576 illustrates a bis(3,4,6-trichloro-2-{[2-methyl-3-(1-ethylcyclopropyl)propoxy]carbonyl}phenyl) oxalate;
Figure 577 illustrates a bis(3,4,6-trichloro-2-{[2-methyl-3-(2-ethylcyclopropyl)propoxy]carbonyl}phenyl) oxalate;
Figure 578 illustrates a bis(3,4,6-trichloro-2-{[3-(1,2,2-trimethylcyclopropyl)propoxy]carbonyl}phenyl) oxalate;
Figure 579 illustrates a bis(3,4,6-trichloro-2-{[3-(1,2,3-trimethylcyclopropyl)propoxy]carbonyl}phenyl) oxalate;
Figure 580 illustrates a bis(3,4,6-trichloro-2-{[3-(2-ethyl-1-methylcyclopropyl)propoxy]carbonyl}phenyl) oxalate;
Figure 581 illustrates a bis(3,4,6-trichloro-2-{[3-(1-ethyl-2-methylcyclopropyl)propoxy]carbonyl}phenyl) oxalate;
Figure 582 illustrates a bis(3,4,6-trichloro-2-{[3-(2-ethyl-3-methylcyclopropyl)propoxy]carbonyl}phenyl) oxalate;
Figure 583 illustrates a bis(3,4,6-trichloro-2-{[3-(2-ethyl-2-methylcyclopropyl)propoxy]carbonyl}phenyl) oxalate;
Figure 584 illustrates a bis(3,4,6-trichloro-2-{[3-(1-propylcyclopropyl)propoxy]carbonyl}phenyl) oxalate;
Figure 585 illustrates a bis(3,4,6-trichloro-2-{[3-(1-propylcyclopropyl)propoxy]carbonyl}phenyl) oxalate;
Figure 586 illustrates a bis(3,4,6-trichloro-2-{[3-(2-propylcyclopropyl)propoxy]carbonyl}phenyl) oxalate;
Figure 587 illustrates a bis[3,4,6-trichloro-2-({3-[2-(1-methylethyl)cyclopropyl]propoxy}carbonyl)phenyl] oxalate;
Figure 588 illustrates a bis {3,4,6-trichloro-2-[(2-cyclopropyl-3,3-dimethylbutoxy)carbonyl]phenyl} oxalate;
Figure 589 illustrates a bis(3,4,6-trichloro-2-{[3-(1-ethylcyclopropyl)butoxy]carbonyl}phenyl) oxalate;
Figure 590 illustrates a bis(3,4,6-trichloro-2-{[2-methyl-4-(1-methylcyclopropyl)butoxy]carbonyl}phenyl) oxalate;
Figure 591 illustrates a bis(3,4,6-trichloro-2-{[3-methyl-4-(1-methylcyclopropyl)butoxy]carbonyl}phenyl) oxalate;
Figure 592 illustrates abis{3,4,6-trichloro-2-[(4-cyclopropyl-2-ethylbutoxy)carbonyl]phenyl} oxalate;
Figure 593 illustrates a bis{3,4,6-trichloro-2-[(4-cyclopropyl-3,3-dimethylbutoxy)carbonyl]phenyl oxalate;
Figure 594 illustrates a bis{3,4,6-trichloro-2-[(4-cyclopropyl-3-methylpentyloxy)carbonyl]phenyl} oxalate;
Figure 595 illustrates a bis(3,4,6-trichloro-2-{[4-(1,2-dimethylcyclopropyl)-3-methylbutoxy]carbonyl}phenyl) oxalate;
Figure 596 illustrates a bis(3,4,6-trichloro-2-{[4-(1-ethylcyclopropyl)butoxy]carbonyl}phenyl) oxalate;
Figure 597 illustrates a bis{3,4,6-trichloro-2-[(4-cyclopropyl-4-methylpentyloxy)carbonyl]phenyl} oxalate;
Figure 598 illustrates a bis(3,4,6-trichloro-2-{[4-(1-methylcyclopropyl)pentyloxy]carbonyl}phenyl) oxalate;
Figure 599 illustrates a bis(3,4,6-trichloro-2-{[4-(2-methylcyclopropyl)pentyloxy]carbonyl}phenyl) oxalate;
Figure 600 illustrates a bis{3,4,6-trichloro-2-[(3-cyclopropyl-4-methylpentyloxy)carbonyl]phenyl} oxalate;
Figure 601 illustrates a bis{3,4,6-trichloro-2-[(2-cyclopropyl-2-methylpentyloxy)carbonyl]phenyl} oxalate;
Figure 602 illustrates a bis{3,4,6-trichloro-2-[(2-cyclopropyl-3-methylpentyloxy)carbonyl]phenyl} oxalate;
Figure 603 illustrates a bis{3,4,6-trichloro-2-[(2-cyclopropyl-4-methylpentyloxy)carbonyl]phenyl} oxalate;
Figure 604 illustrates a bis{3,4,6-trichloro-2-[(3-cyclopropyl-2-methylpentyloxy)carbonyl]phenyl} oxalate;
Figure 605 illustrates a bis{3,4,6-trichloro-2-[(5-cyclopropyl-2-methylpentyloxy)carbonyl]phenyl} oxalate;
Figure 606 illustrates a bis{3,4,6-trichloro-2-[(5-cyclopropyl-3-methylpentyloxy)carbonyl]phenyl} oxalate;
Figure 607 illustrates a bis{3,4,6-trichloro-2-[(5-cyclopropyl-4-methylpentyloxy)carbonyl]phenyl} oxalate;
Figure 608 illustrates a bis(3,4,6-trichloro-2-{[3-(cyclopropylmethyl)pentyloxy]carbonyl}phenyl) oxalate;
Figure 609 illustrates a bis(3,4,6-trichloro-2-{[5-(1-methylcyclopropyl)pentyloxy]carbonyl}phenyl) oxalate;
Figure 610 illustrates a bis {3,4,6-trichloro-2-[(2-cyclopropylhexyloxy)carbonyl]phenyl} oxalate;
Figure 611 illustrates a bis{3,4,6-trichloro-2-[(3-cyclopropylhexyloxy)carbonyl]phenyl} oxalate;
Figure 612 illustrates a bis{3,4,6-trichloro-2-[(4-cyclopropylhexyloxy)carbonyl]phenyl} oxalate;
Figure 613 illustrates a bis{3,4,6-trichloro-2-[(5-cyclopropylhexyloxy)carbonyl]phenyl} oxalate;
Figure 614 illustrates a bis{3,4,6-trichloro-2-[(6-cyclopropylhexyloxy)carbonyl]phenyl} oxalate;
Figure 615 illustrates a bis{3,4,6-trichloro-2-[(2,2-dicyclopropylpropoxy)carbonyl]phenyl} oxalate;
Figure 616 illustrates a bis{3,4,6-trichloro-2-[(3,3-dicyclopropylpropoxy)carbonyl]phenyl} oxalate;
Figure 617 illustrates a bis {3,4,6-trichloro-2-[(2-cyclobutyl-2-cyclopropylethoxy)carbonyl]phenyl} oxalate;
Figure 618 illustrates a bis{2-[(bicyclo[2.2.2]octan-2-ylmethoxy)carbonyl]-3,4,6-trichlorophenyl} oxalate;
Figure 619 illustrates a bis{2-[(bicyclo[3.3.0]octan-3-ylmethoxy)carbonyl]-3,4,6-trichlorophenyl} oxalate;
Figure 620 illustrates a bis(3,4,6-trichloro-2-{[(2,3-dimethylphenyl)methoxy]carbonyl}phenyl) oxalate;
Figure 621 illustrates a bis(3,4,6-trichloro-2-{[(2,4-dimethylphenyl)methoxy]carbonyl}phenyl) oxalate;
Figure 622 illustrates a bis(3,4,6-trichloro-2-{[3,4-dimethylphenyl)methoxy]carbonyl}phenyl) oxalate;
Figure 623 illustrates a bis(3,4,6-trichloro-2-{[(3,5-dimethylphenyl)methoxy]carbonyl}phenyl) oxalate;
Figure 624 illustrates a bis(3,4,6-trichloro-2-{[(2,6-dimethylphenyl)methoxy]carbonyl}phenyl) oxalate;
Figure 625 illustrates a bis(3,4,6-trichloro-2-{[(2-ethylphenyl)methoxy]carbonyl}phenyl) oxalate;
Figure 626 illustrates a bis(3,4,6-trichloro-2-{[(3-ethylphenyl)methoxy]carbonyl}phenyl) oxalate;
Figure 627 illustrates a bis(3,4,6-trichloro-2-{[(4-ethylphenyl)methoxy]carbonyl}phenyl) oxalate;
Figure 628 illustrates a bis(3,4,6-trichloro-2-{[2-(2-methylphenyl)ethoxy]carbonyl}phenyl) oxalate;
Figure 629 illustrates a bis(3,4,6-trichloro-2-{[2-(3-methylphenyl)ethoxy]carbonyl}phenyl) oxalate;
Figure 630 illustrates a bis(3,4,6-trichloro-2-{[2-(4-methylphenyl)ethoxy]carbonyl}phenyl) oxalate;
Figure 631 illustrates a bis{3,4,6-trichloro-2-[(2-phenylpropoxy)carbonyl]phenyl} oxalate;
Figure 632 illustrates a bis{3,4,6-trichloro-2-[(3-phenylpropoxy)carbonyl]phenyl} oxalate;
Figure 633 illustrates a bis{3,4,6-trichloro-2-[(2-methylnonyloxy)carbonyl]phenyl} oxalate;
Figure 634 illustrates a bis{3,4,6-trichloro-2-[(3-methylnonyloxy)carbonyl]phenyl} oxalate;
Figure 635 illustrates a bis{3,4,6-trichloro-2-[(4-methylnonyloxy)carbonyl]phenyl} oxalate;
Figure 636 illustrates a bis{3,4,6-trichloro-2-[(5-methylnonyloxy)carbonyl]phenyl} oxalate;
Figure 637 illustrates a bis{3,4,6-trichloro-2-[(6-methylnonyloxy)carbonyl]phenyl} oxalate;
Figure 638 illustrates a bis{3,4,6-trichloro-2-[(7-methylnonyloxy)carbonyl]phenyl} oxalate;
Figure 639 illustrates a bis{3,4,6-trichloro-2-[(8-methylnonyloxy)carbonyl]phenyl} oxalate;
Figure 640 illustrates a bis{3,4,6-trichloro-2-[(3,7-dimethyloctyloxy)carbonyl]phenyl} oxalate;
Figure 641 illustrates a bis{3,4,6-trichloro-2-[(4-ethyloctyloxy)carbonyl]phenyl} oxalate;
Figure 642 illustrates a bis{3,4,6-trichloro-2-[(2-propylheptyloxy)carbonyl]phenyl} oxalate;
Figure 643 illustrates a bis {3,4,6-trichloro-2-[(2-butylhexyloxy)carbonyl]phenyl} oxalate;
Figure 644 illustrates a bis{3,4,6-trichloro-2-[(4-cyclohexylbutoxy)carbonyl]phenyl} oxalate;
Figure 645 illustrates a bis(2-{[(6,6-dimethylbicyclo[3.1.1]heptan-2-yl)methoxy]carbonyl}3,4,6-trichlorophenyl) oxalate;
Figure 646 illustrates a bis(3,4,6-trichloro-2-{[(2,4,6-trimethylphenyl)methoxy]carbonyl}phenyl) oxalate;
Figure 647 illustrates a bis(3,4,6-trichloro-2-{[(4-propylphenyl)methoxy]carbonyl}phenyl) oxalate;
Figure 648 illustrates a bis[3,4,6-trichloro-2-({[4-(1-methylethyl)phenyl]methoxy}carbonyl)phenyl] oxalate;
Figure 649 illustrates a bis{3,4,6-trichloro-2-[(2-phenylbutoxy)carbonyl]phenyl} oxalate;
Figure 650 illustrates a bis{3,4,6-trichloro-2-[(3-phenylbutoxy)carbonyl]phenyl} oxalate;
Figure 651 illustrates a bis {3,4,6-trichloro-2-[(4-phenylbutoxy)carbonyl]phenyl} oxalate;
Figure 652 illustrates a bis(3,4,6-trichloro-2-{[(2-phenylcyclopropyl)methoxy]carbonyl}phenyl) oxalate;
Figure 653 illustrates a bis{3,4,6-trichloro-2-[(1-adamantylmethoxy)carbonyl]phenyl} oxalate;
Figure 654 illustrates a bis {3,4,6-trichloro-2-[(2,2-dicyclohexylethoxy)carbonyl]phenyl} oxalate;
Figure 655 illustrates a bis(3,4,6-trichloro-2-{[(4-butylphenyl)methoxy]carbonyl}phenyl) oxalate;
Figure 656 illustrates a bis[3,4,6-trichloro-2-({[4-(1,1-dimethylethyl)phenyl]methoxy} carbonyl)phenyl] oxalate;
Figure 657 illustrates a bis{3,4,6-trichloro-2-[(5-phenylpentyloxy)carbonyl]phenyl} oxalate;
Figure 658 illustrates a bis{3,4,6-trichloro-2-[1-naphthalenylmethoxy)carbonyl]phenyl} oxalate;
Figure 659 illustrates a bis{3,4,6-trichloro-2-[2-naphthalenylmethoxy)carbonyl]phenyl} oxalate;
Figure 660 illustrates a bis {3,4,6-trichloro-2-[(2,2-diphenylethoxy)carbonyl]phenyl} oxalate;
Figure 661 illustrates a bis {3,4,6-trichloro-2-[(9-fluorenylmethoxy)carbonyl]phenyl} oxalate;
Figure 662 illustrates a bis {3,4,6-trichloro-2-[(9-anthracenylmethoxy)carbonyl]phenyl} oxalate.

### DETAILED DESCRIPTION OF THE INVENTION

### EXAMPLES

Starting materials and solvents were purchased from Aldrich, VWR, Strem, and Alfa Aesar. All of the desired compounds were analyzed via HPLC, and their spectra (NMR) recorded.

### EXAMPLE 1 (REFERENCE EXAMPLE)

### Procedure For Producing Bis{3,4,6-Trichloro-2-[(2-Methylpropoxy)Carbonyl]Phenyl} Oxalate (Figure 1)

Aliphatic 3,5,6-trichlorosalicylate esters, including 2-methylbutyl 3,5,6-trichlorosalicylate can be made via the procedures taught in U.S. Patent 4,308,395 and U.S. Patent 5,194,666. The material was analyzed by HPLC and used without further purification in the next step.

The oxalate, bis{3,4,6-trichloro-2-[(2-methylpropoxy)carbonyl]phenyl} oxalate (Compound in Figure 1) can be made using processes described in U.S. 3,749,679. The recrystallization solvent of choice is acetonitrile.

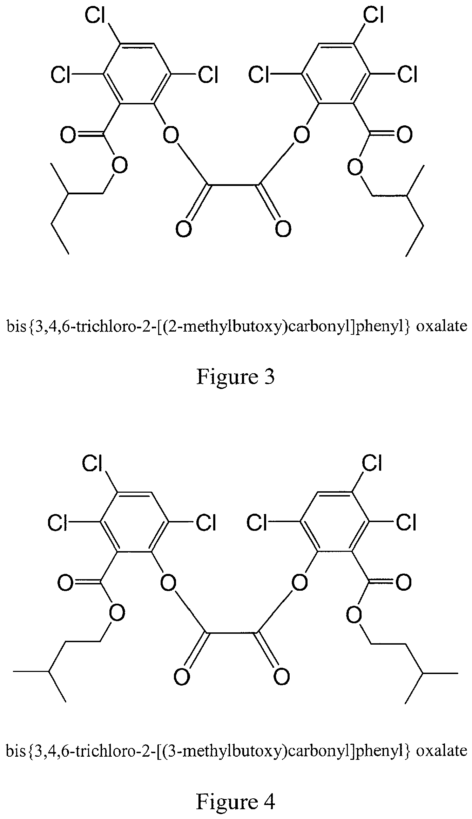

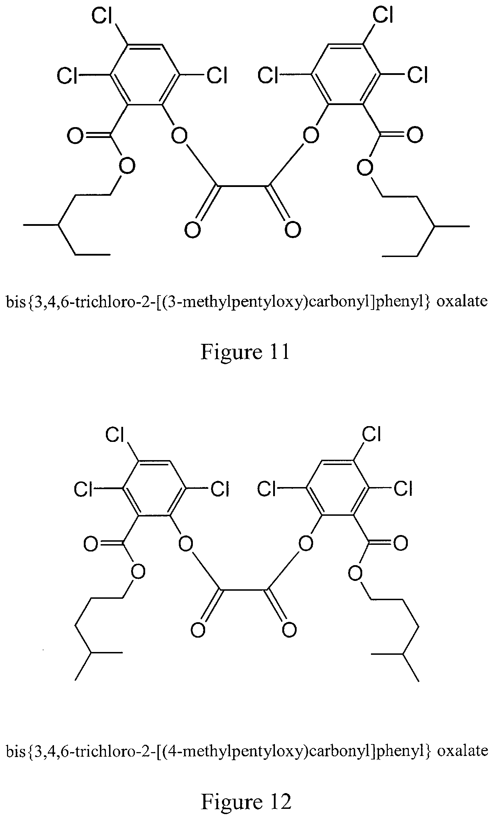

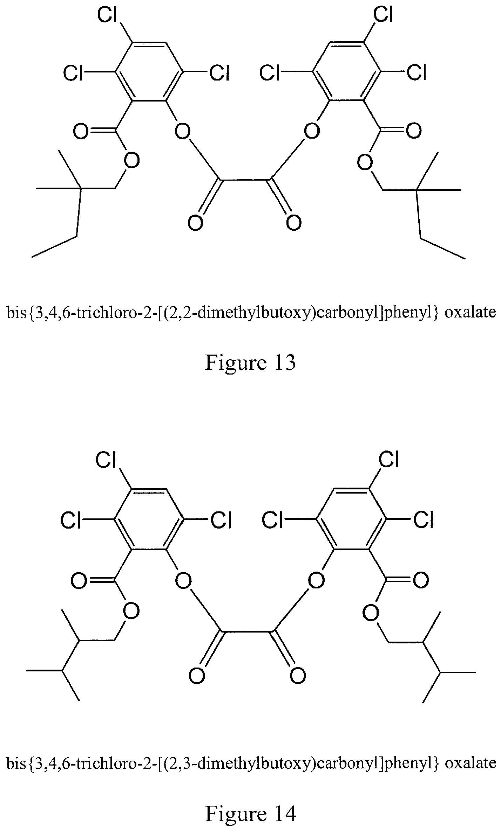

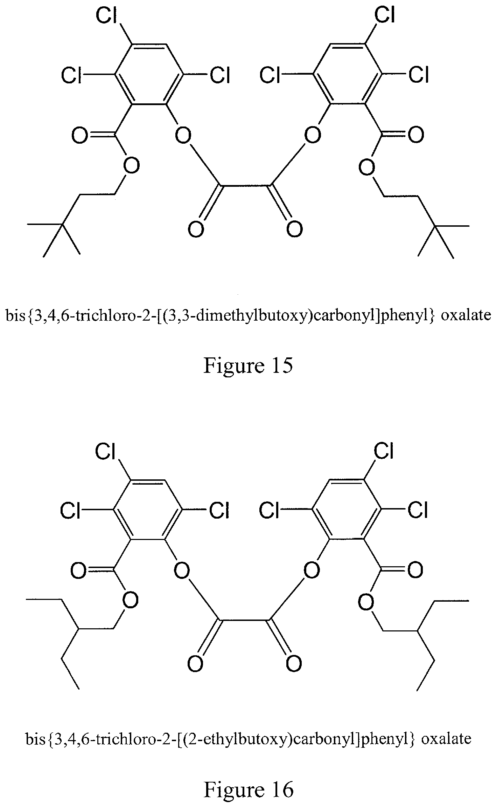

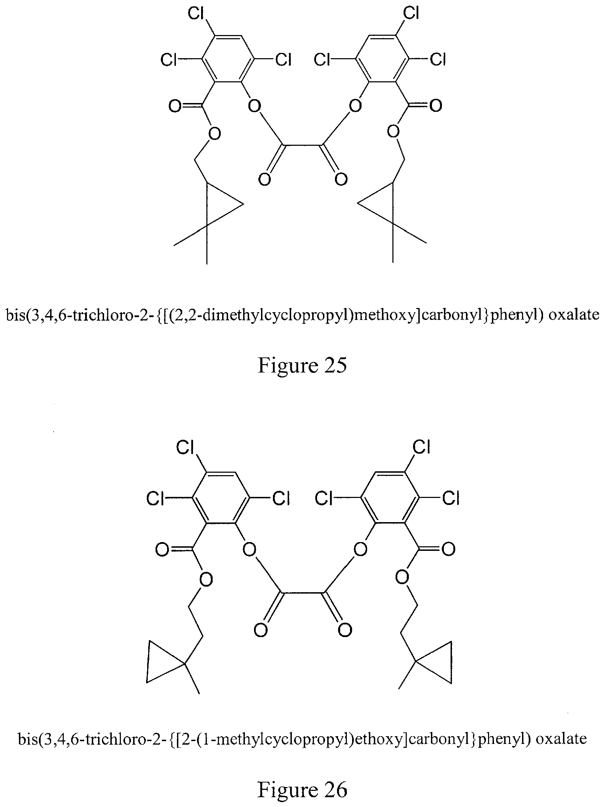

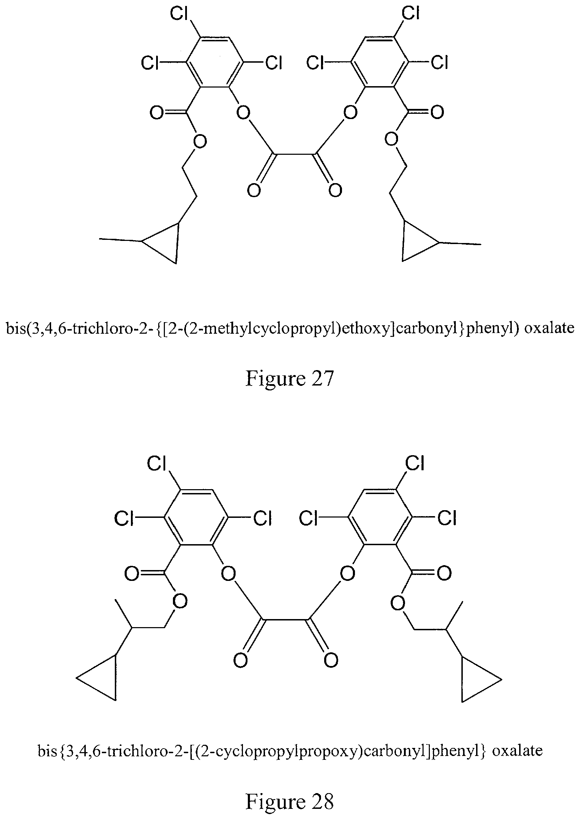

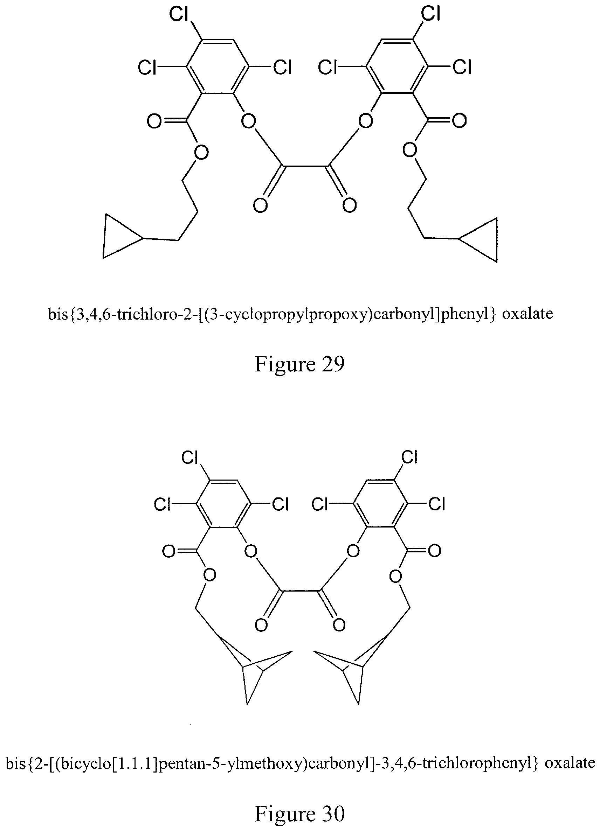

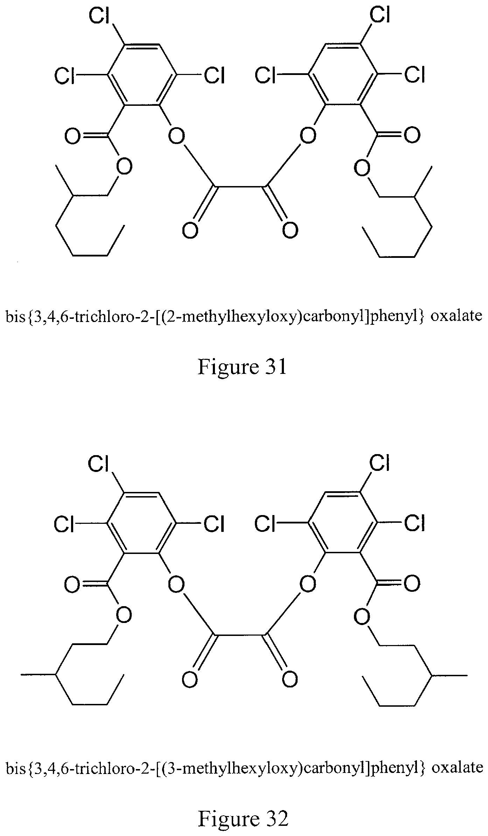

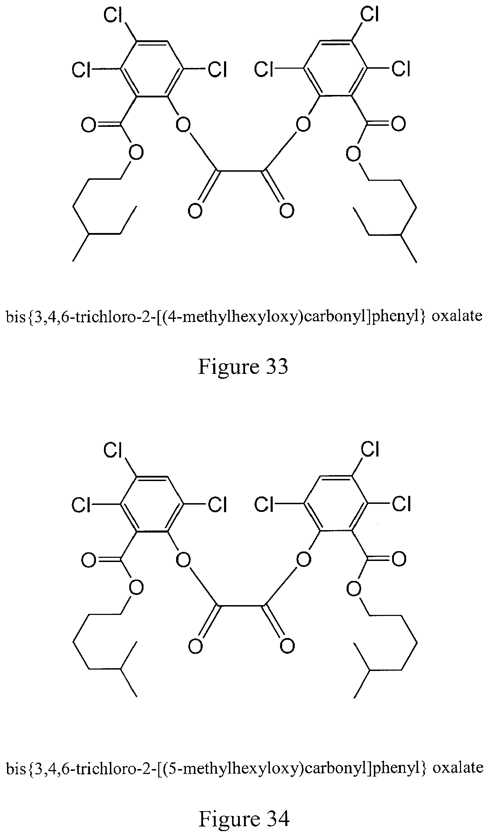

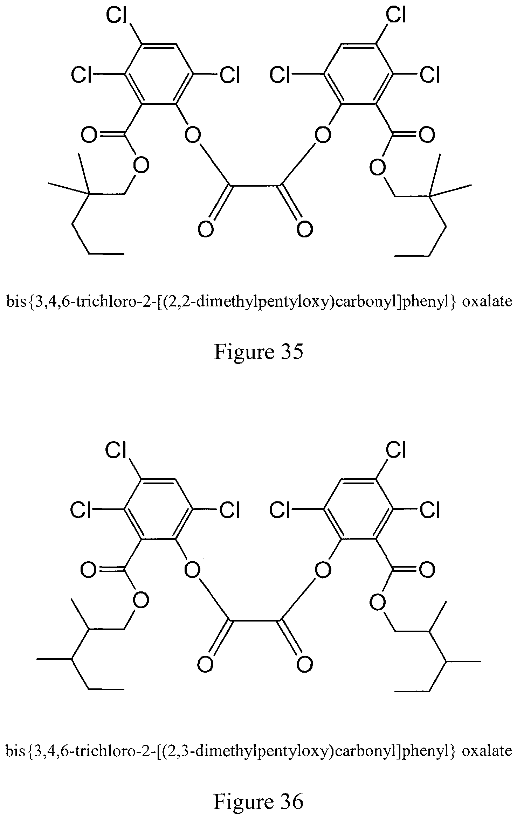

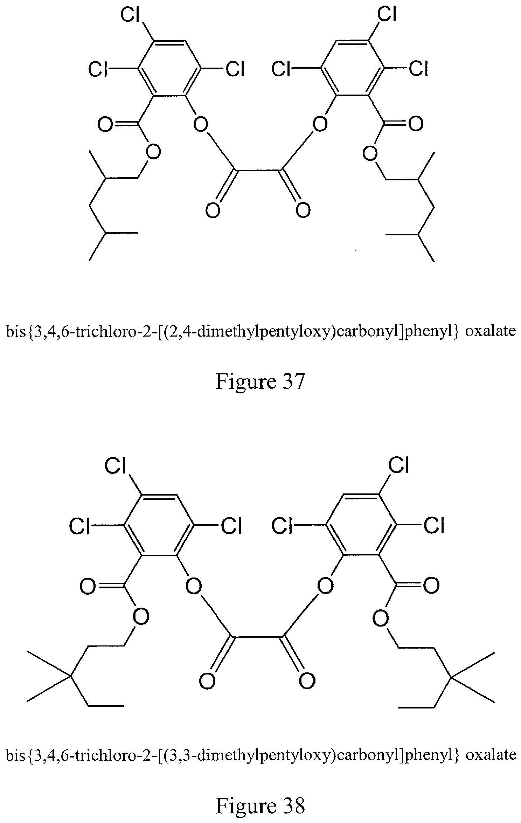

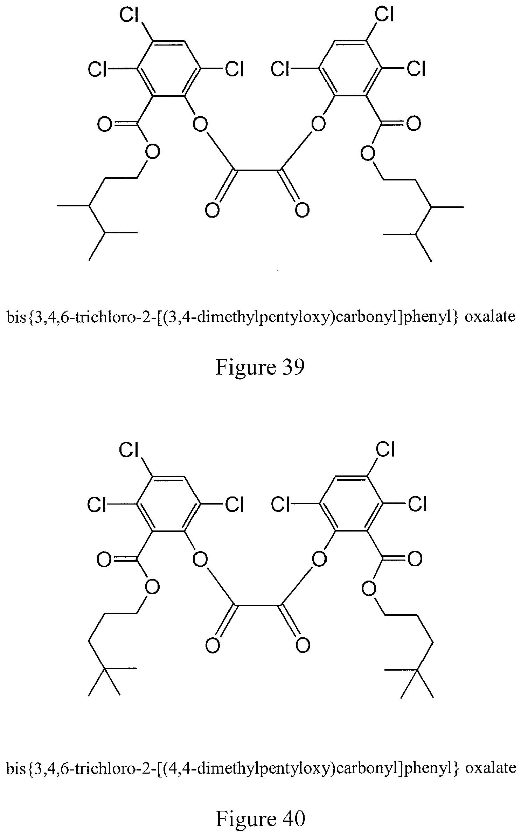

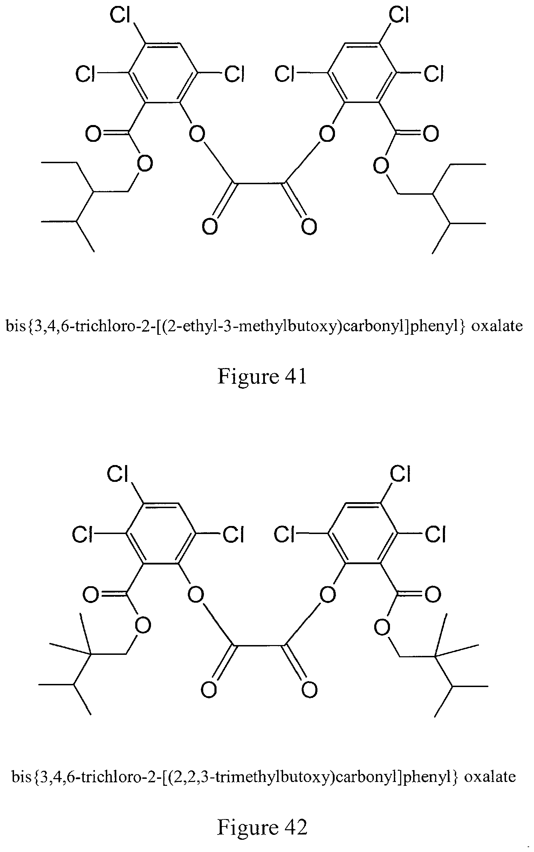

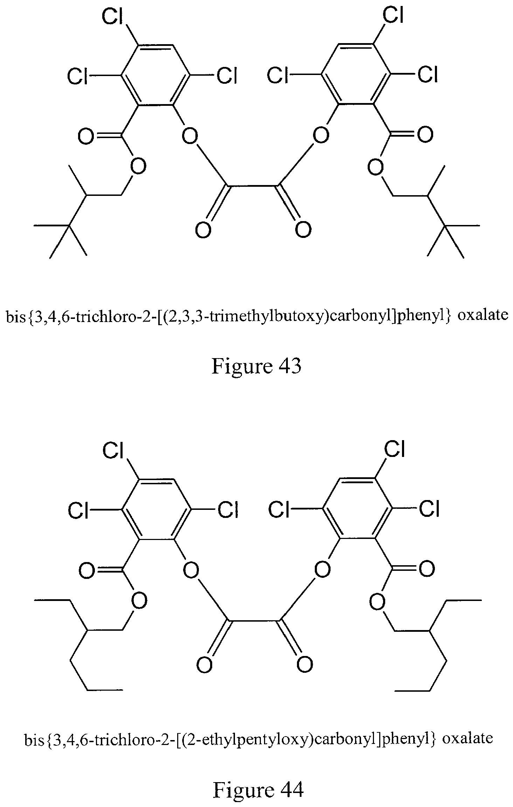

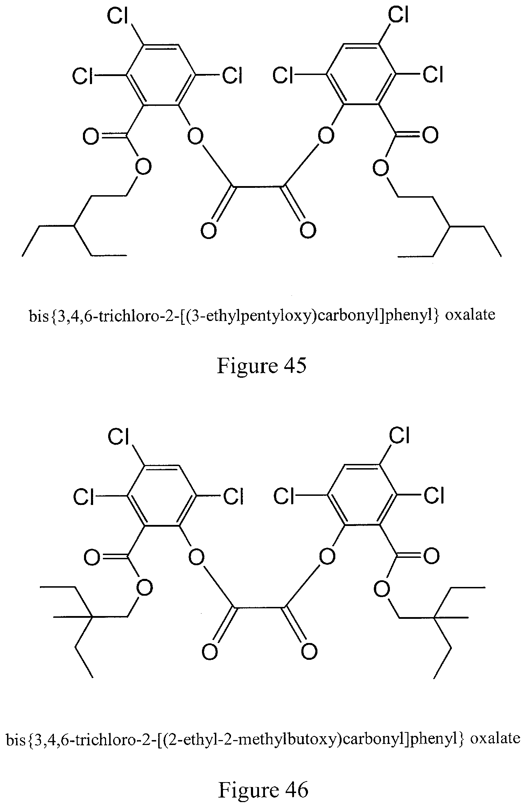

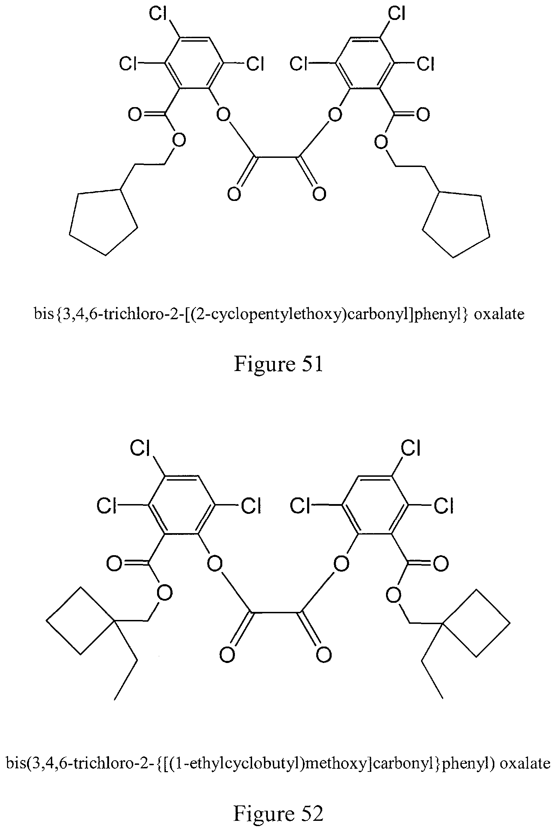

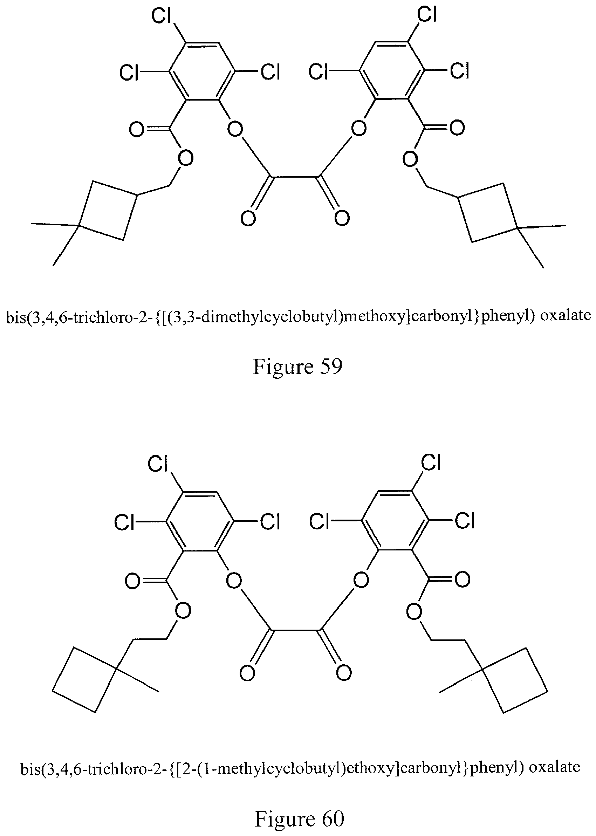

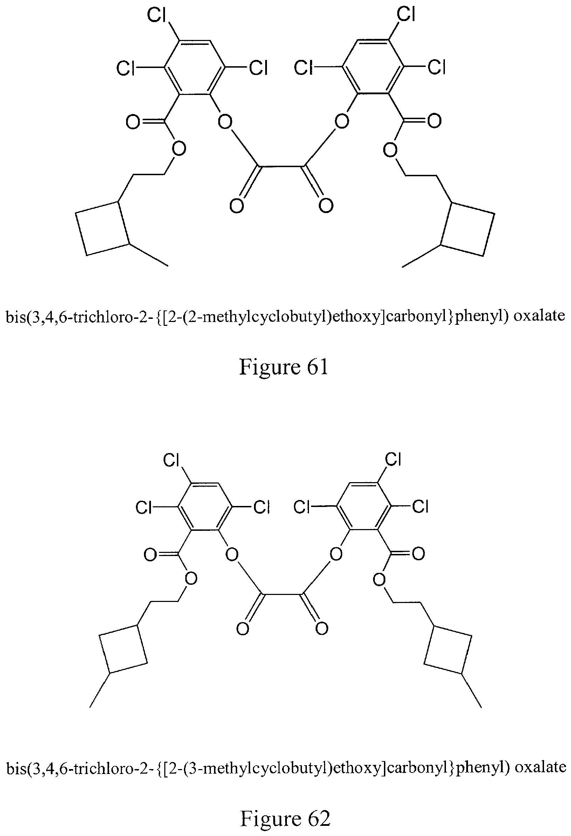

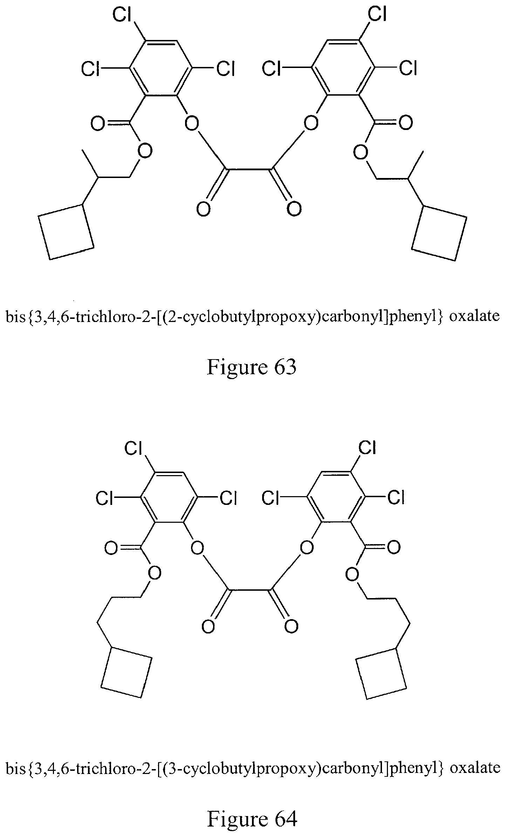

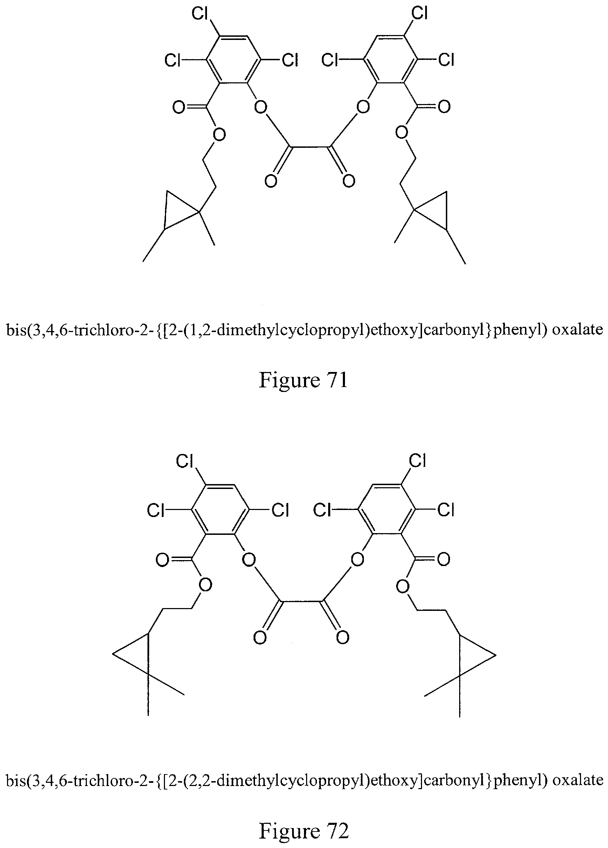

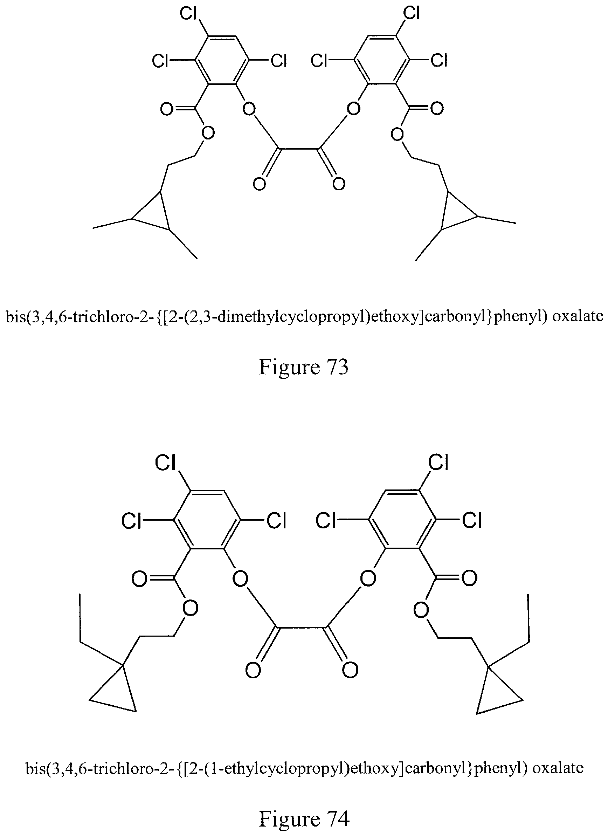

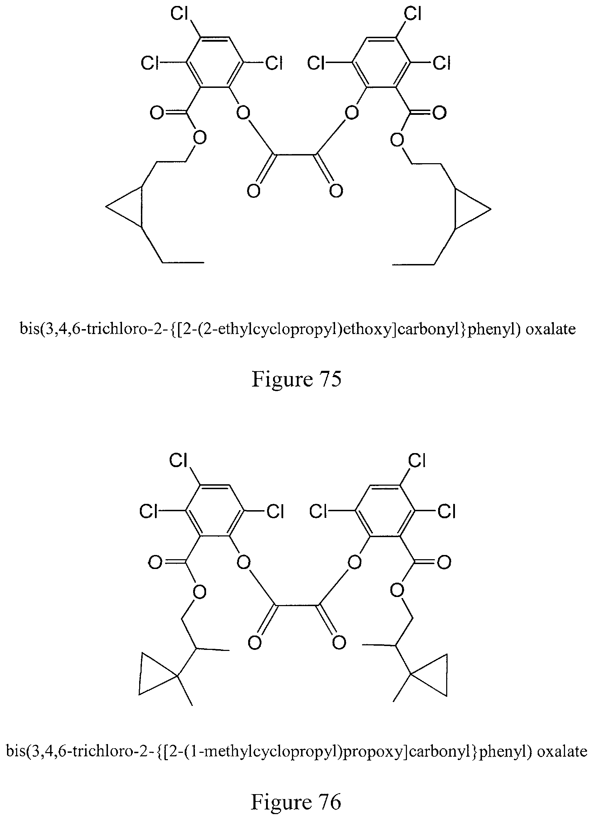

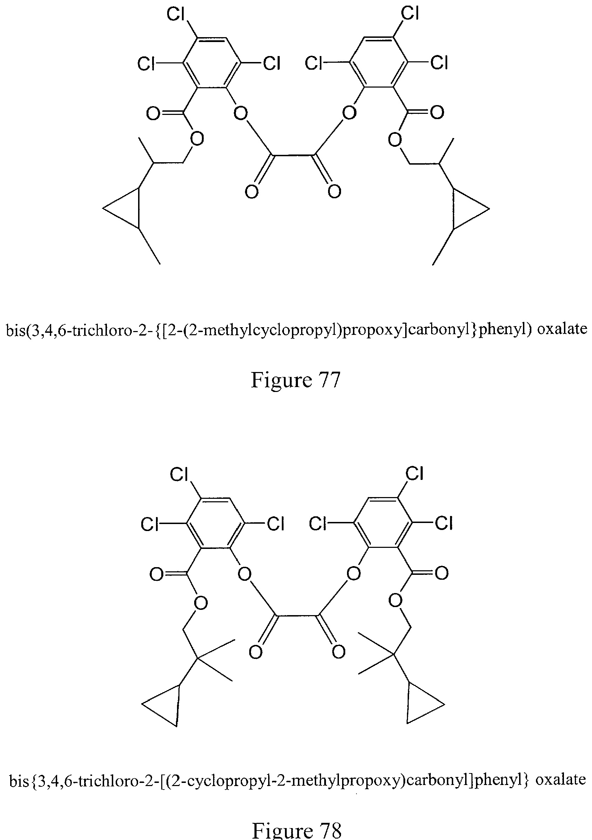

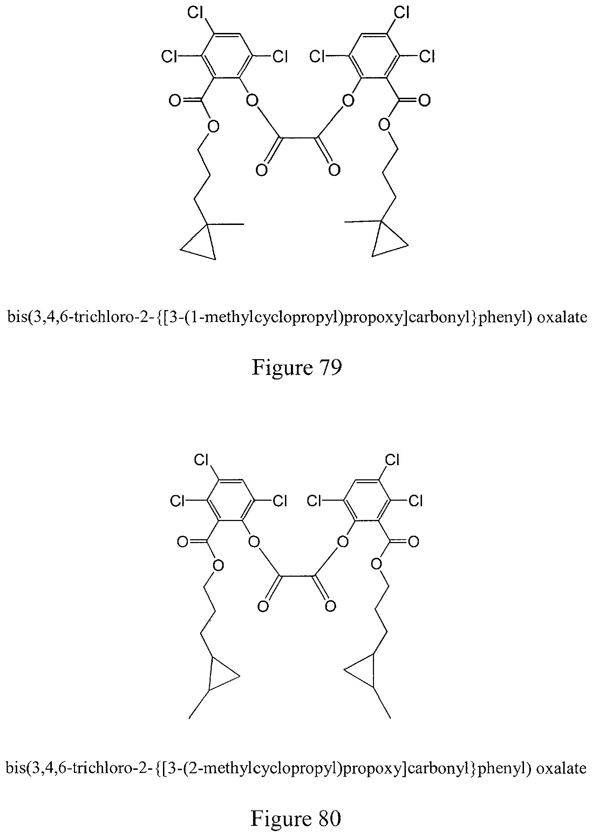

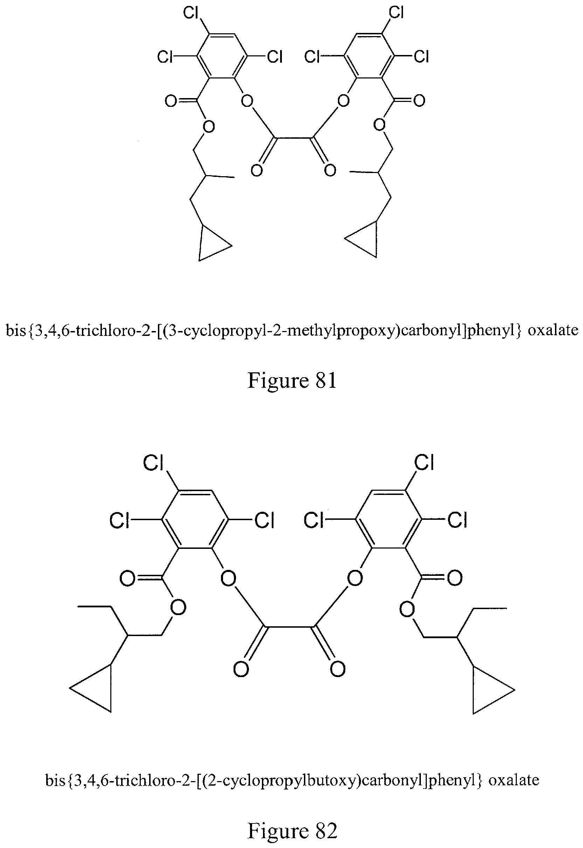

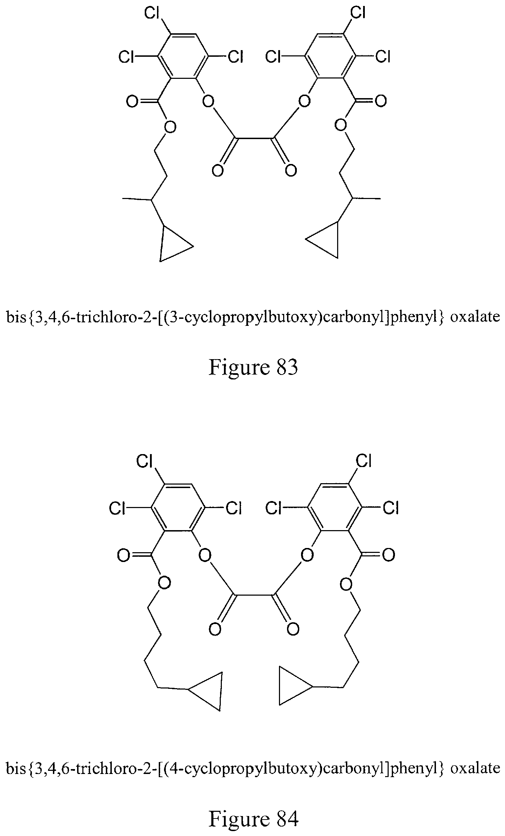

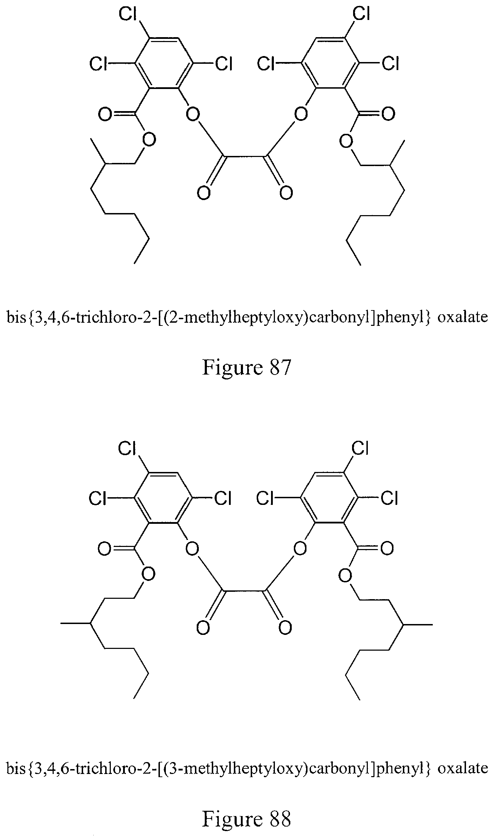

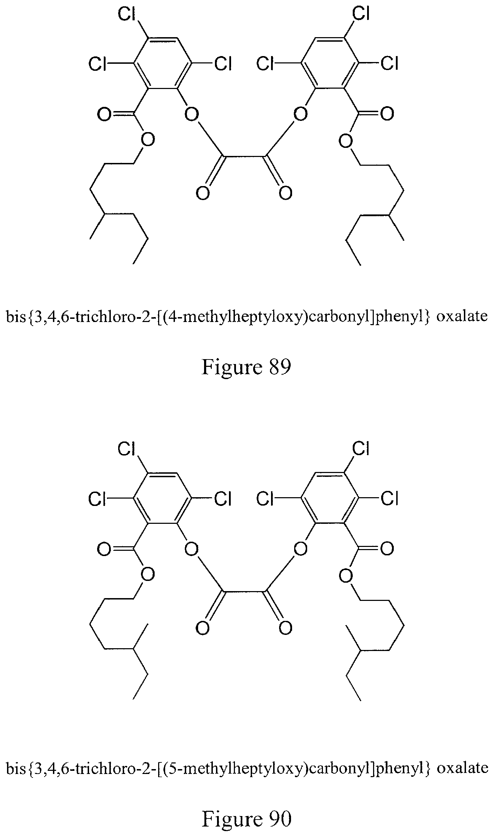

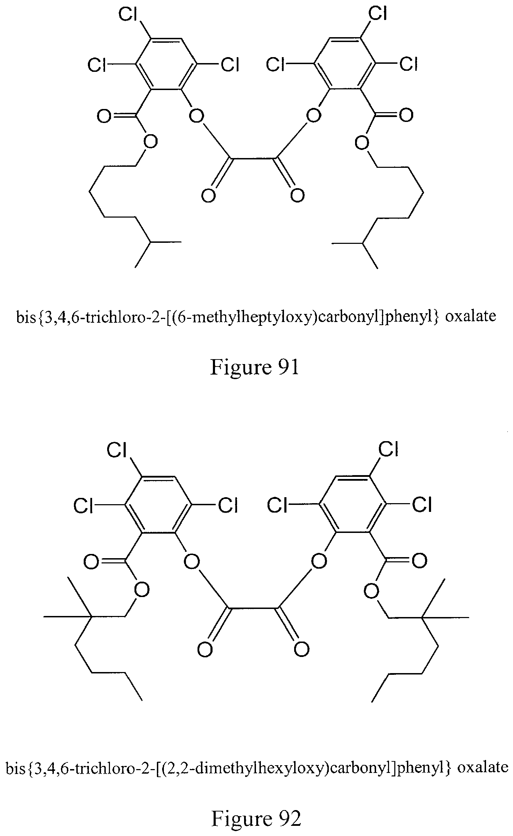

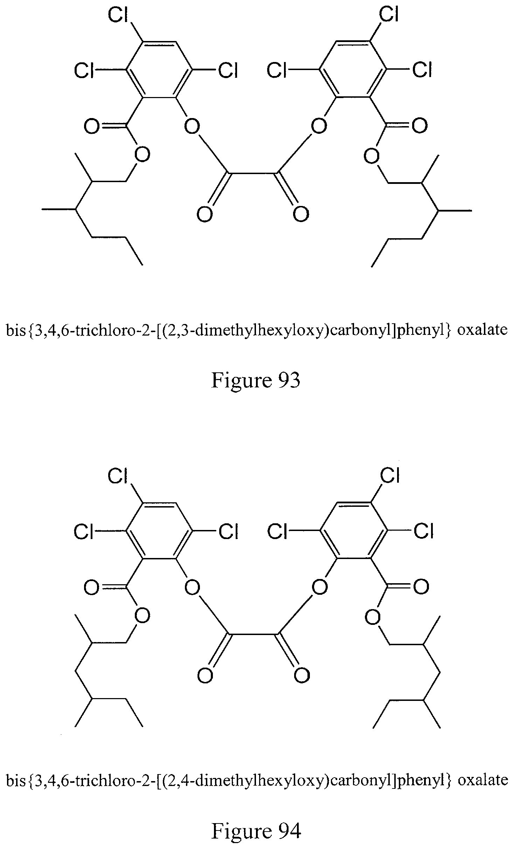

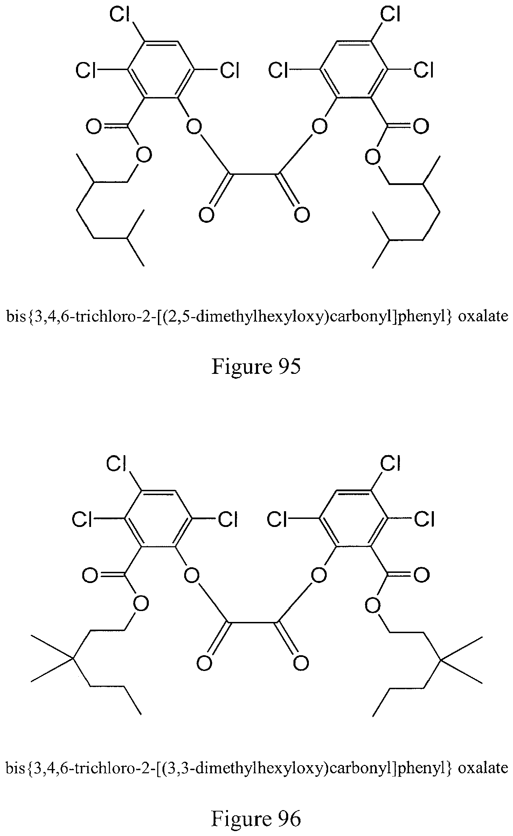

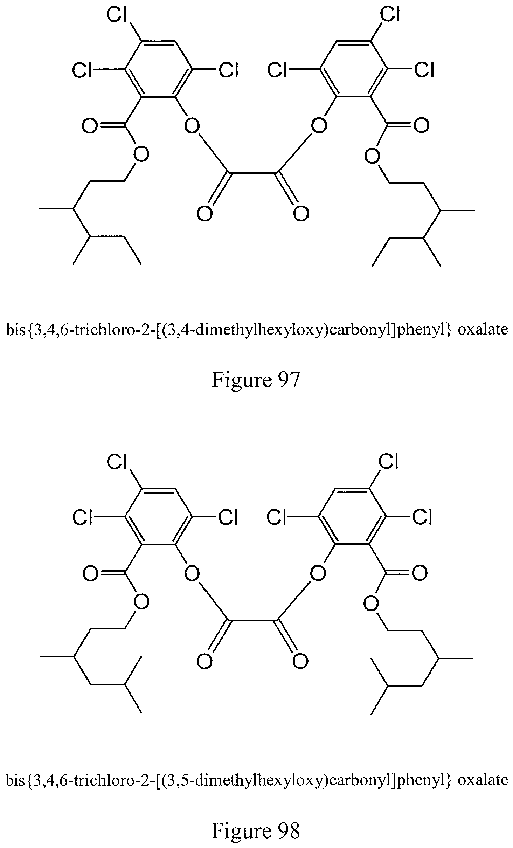

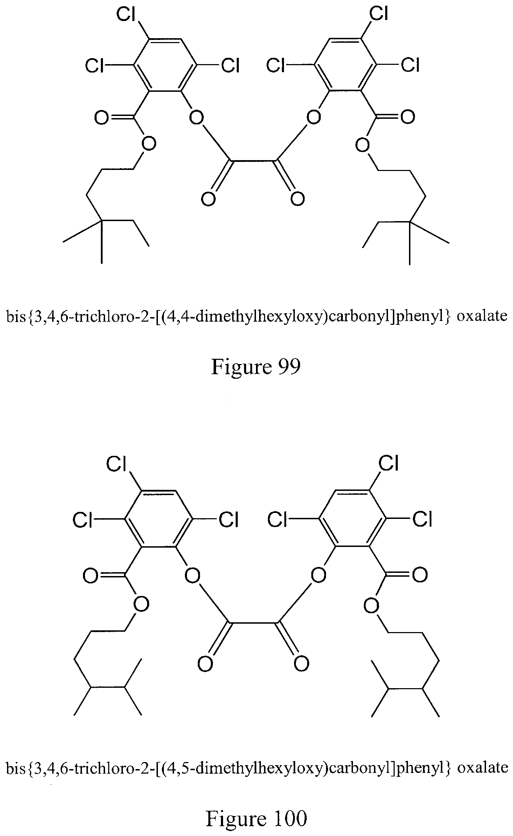

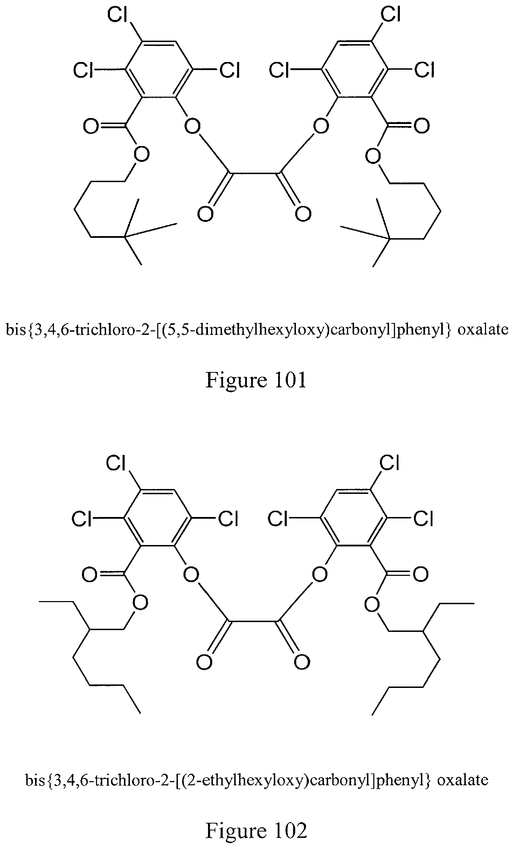

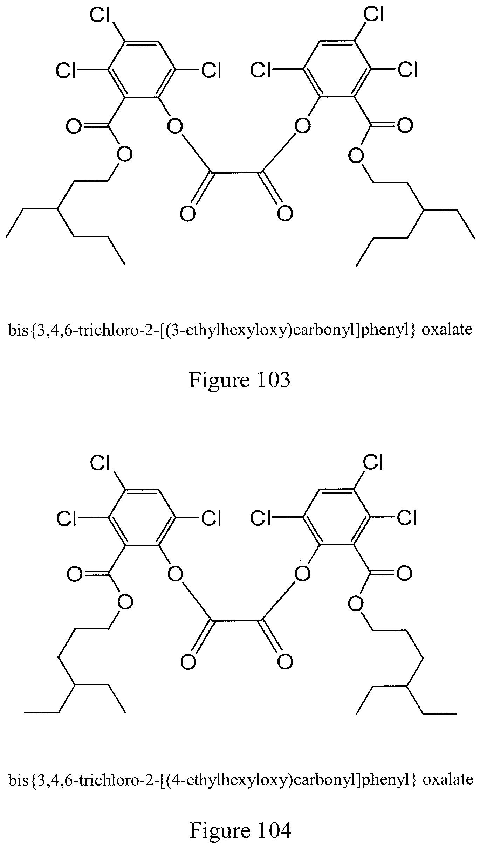

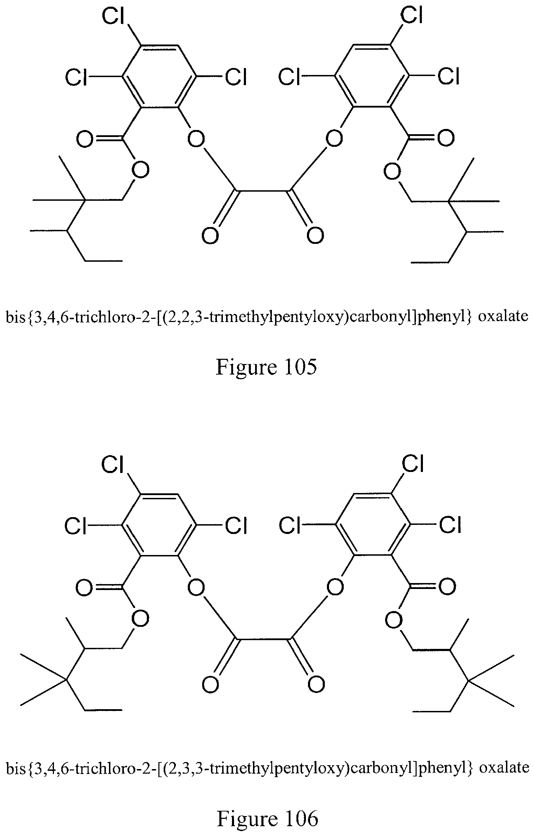

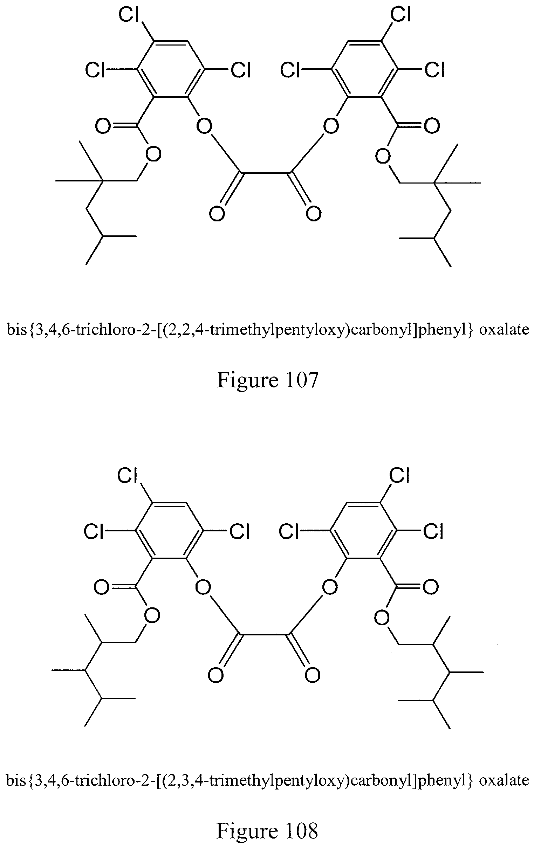

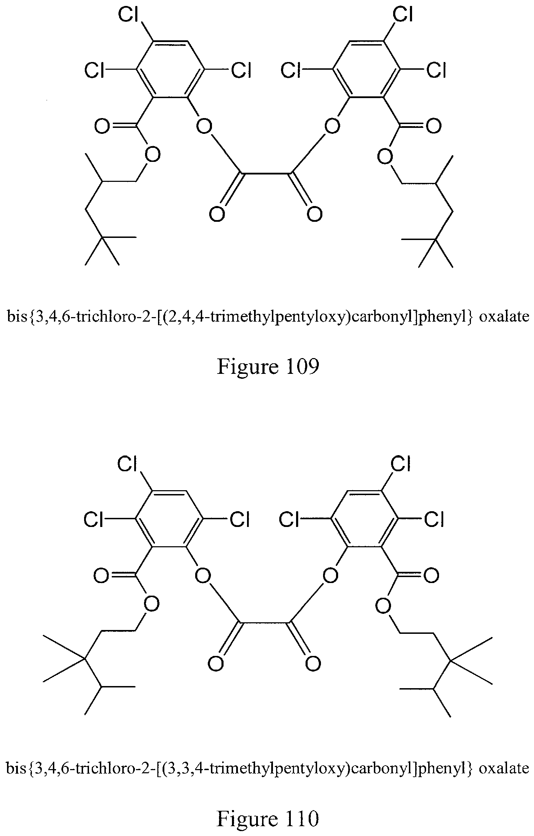

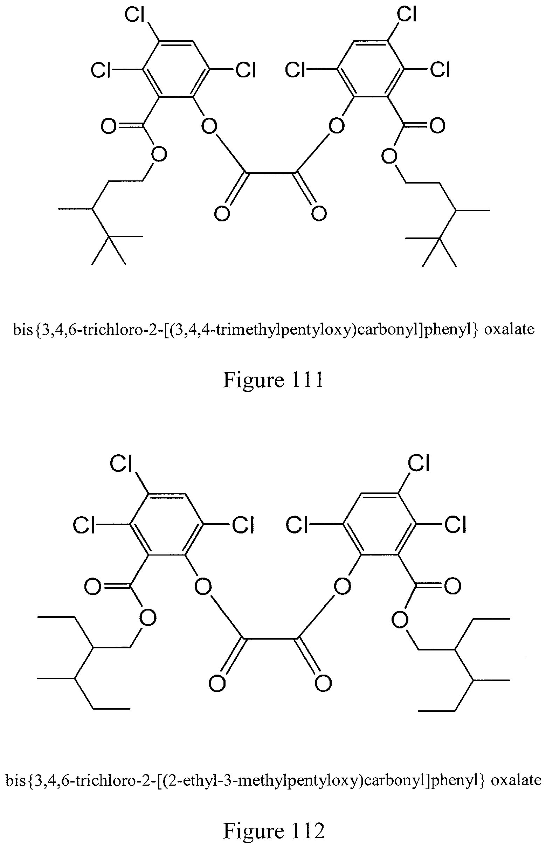

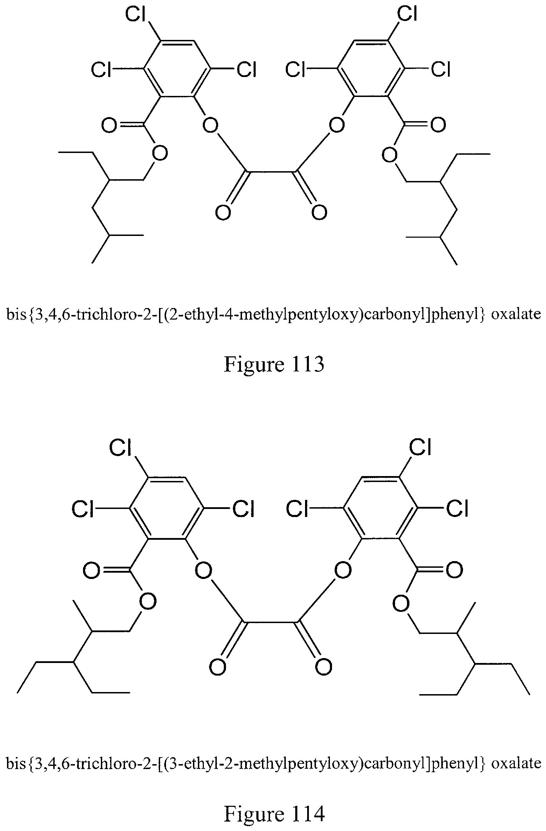

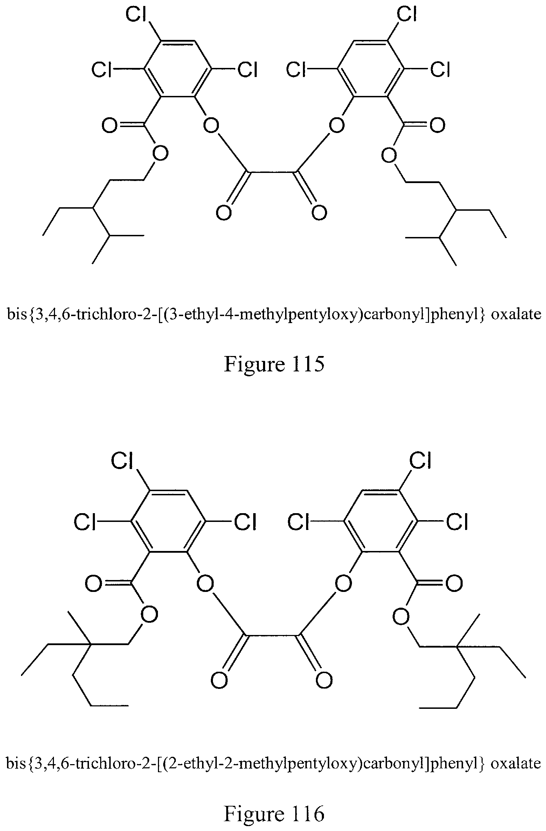

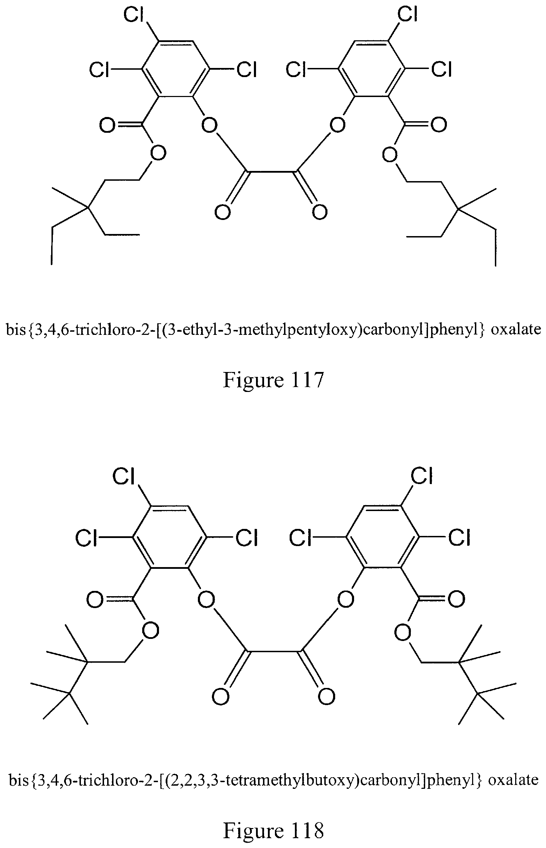

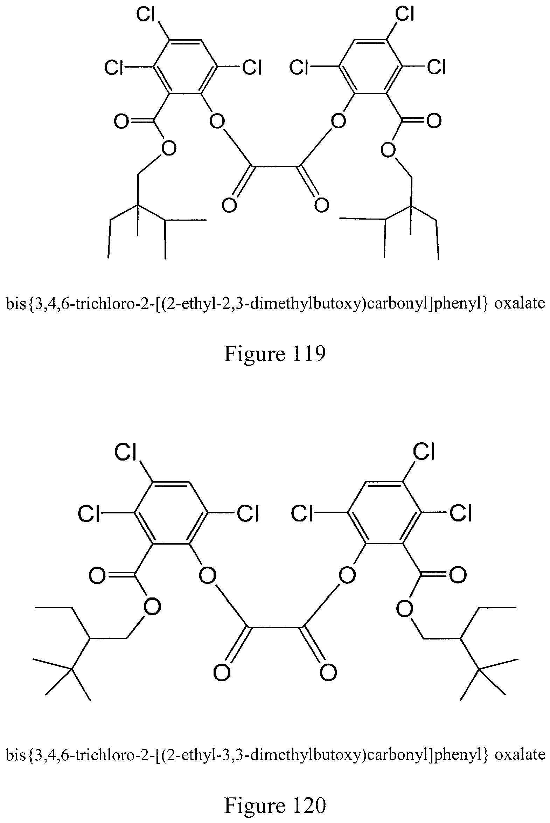

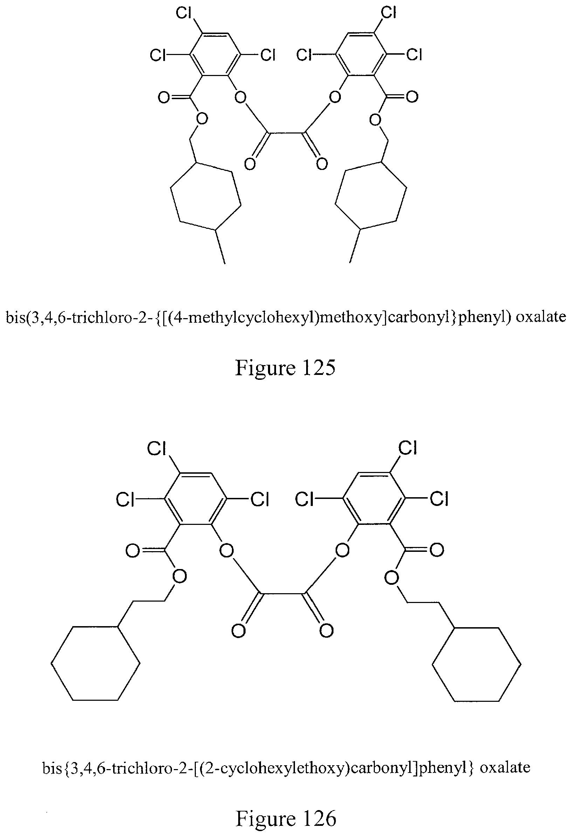

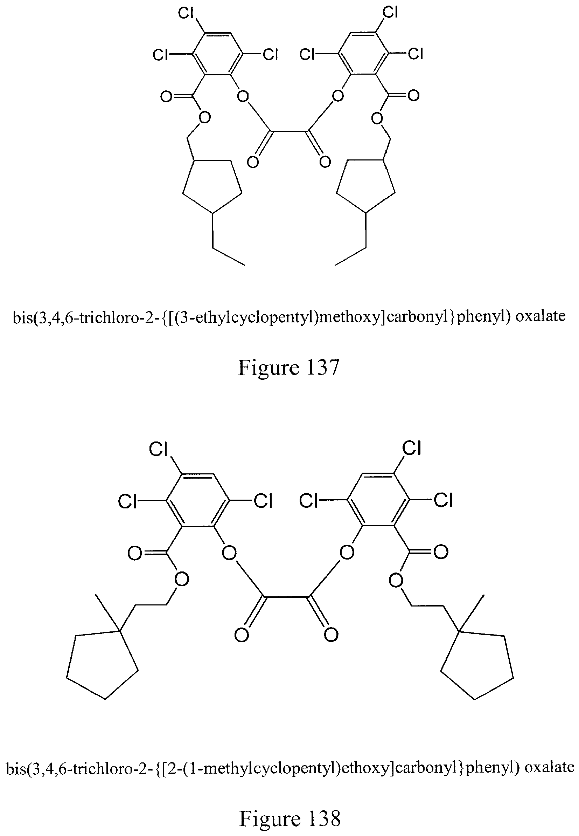

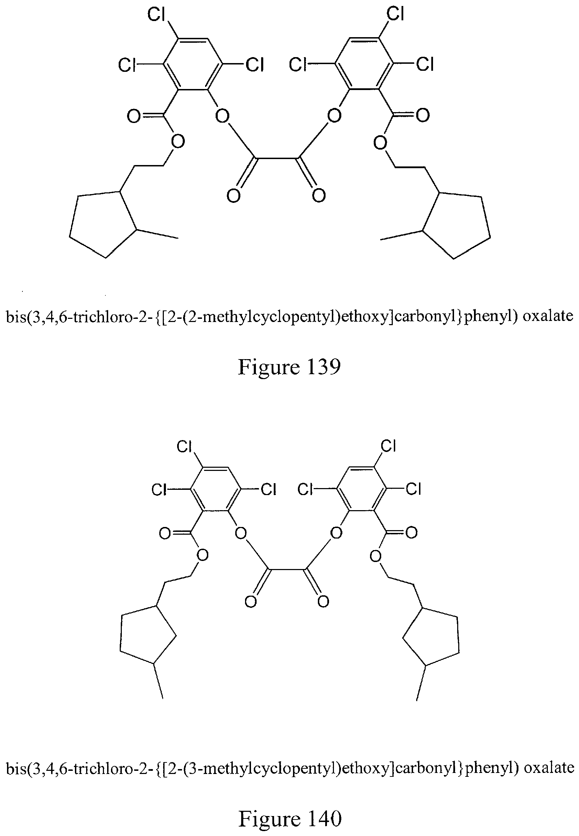

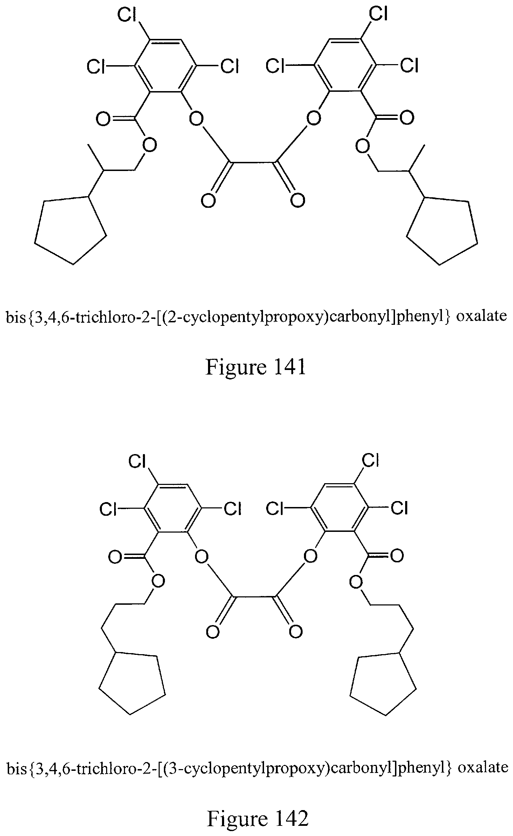

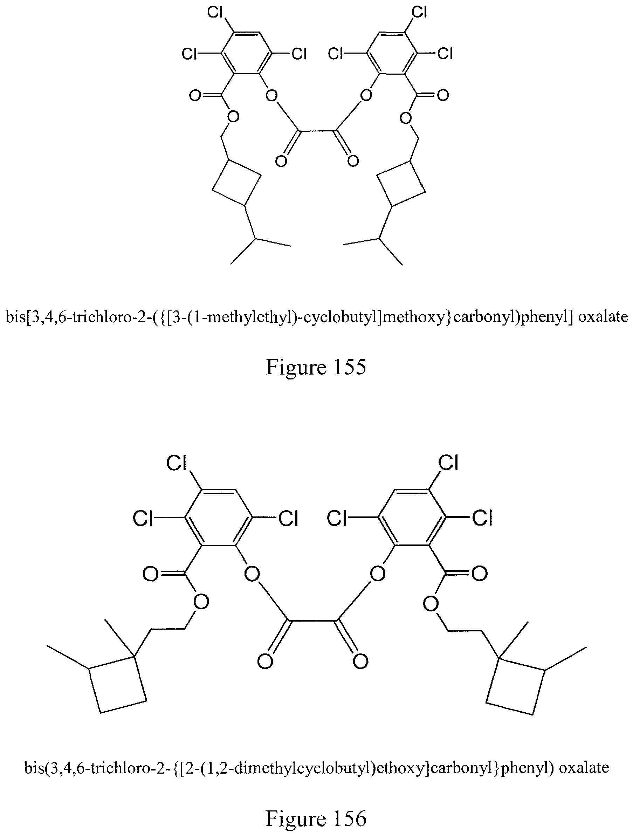

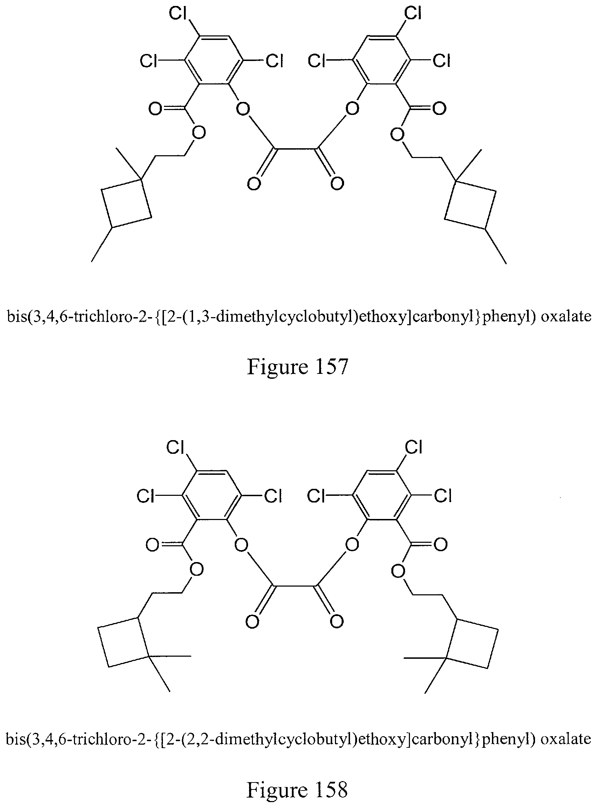

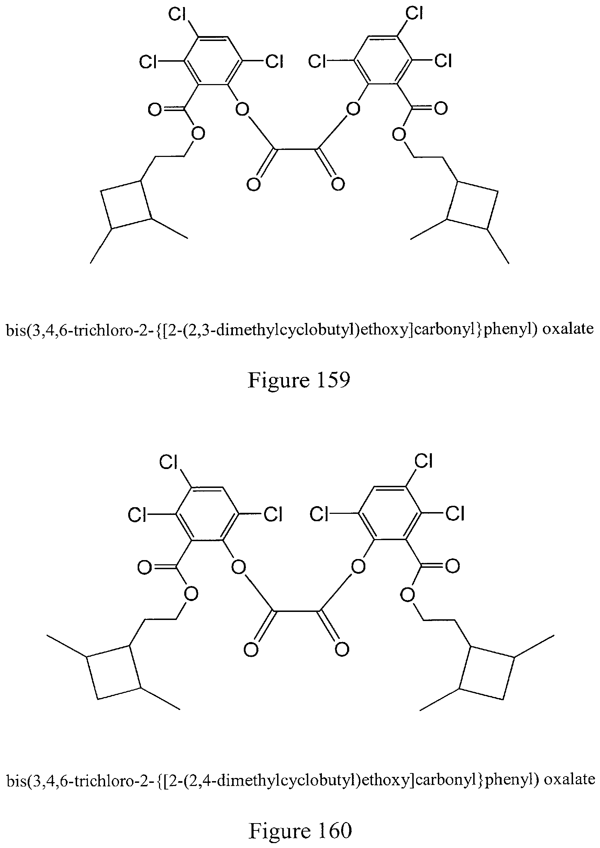

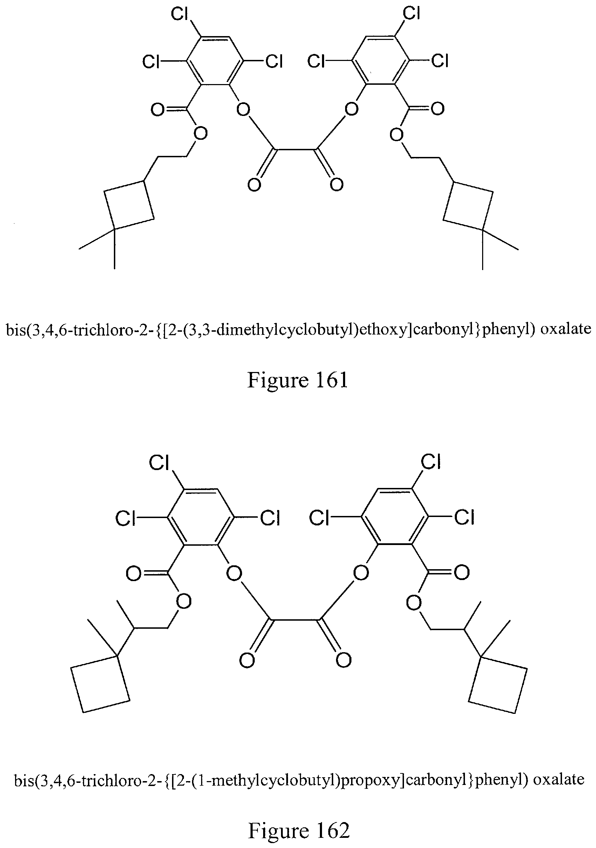

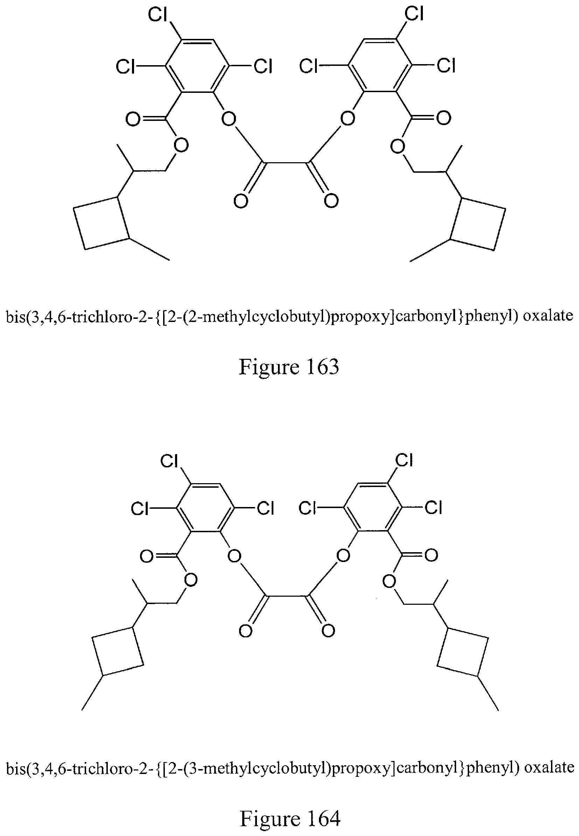

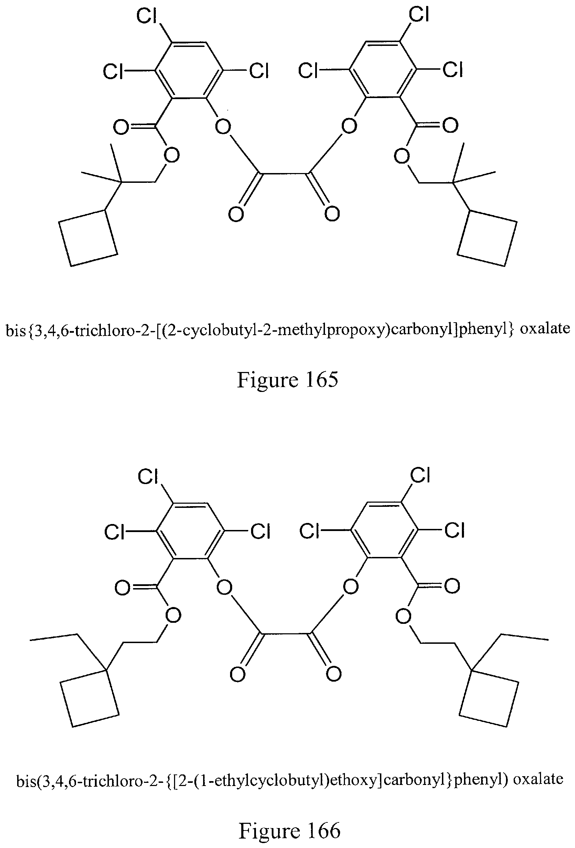

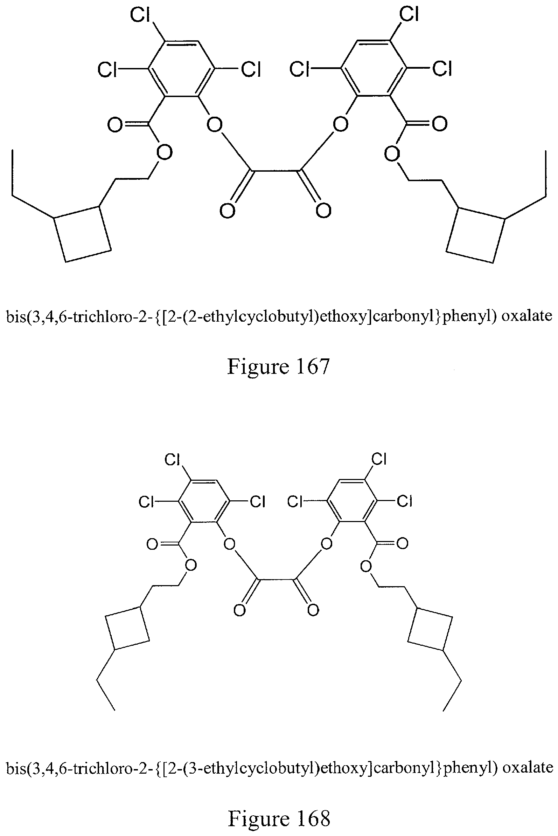

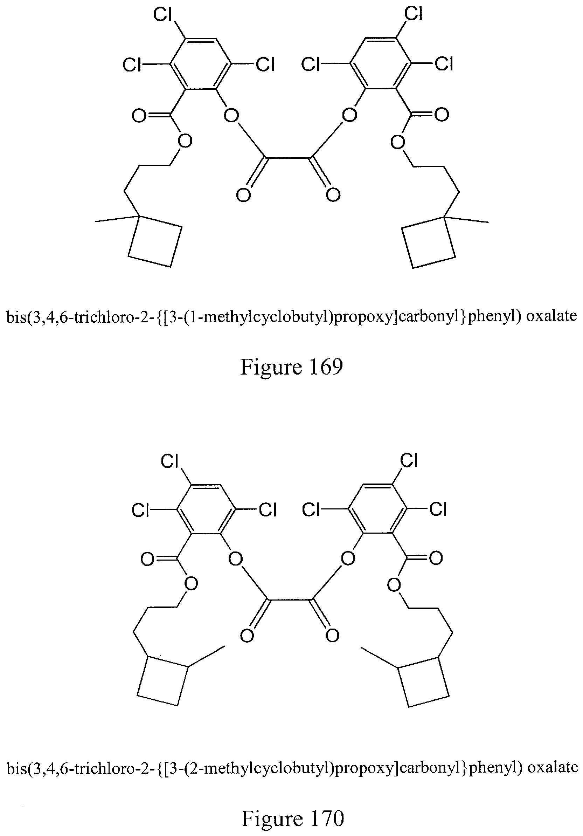

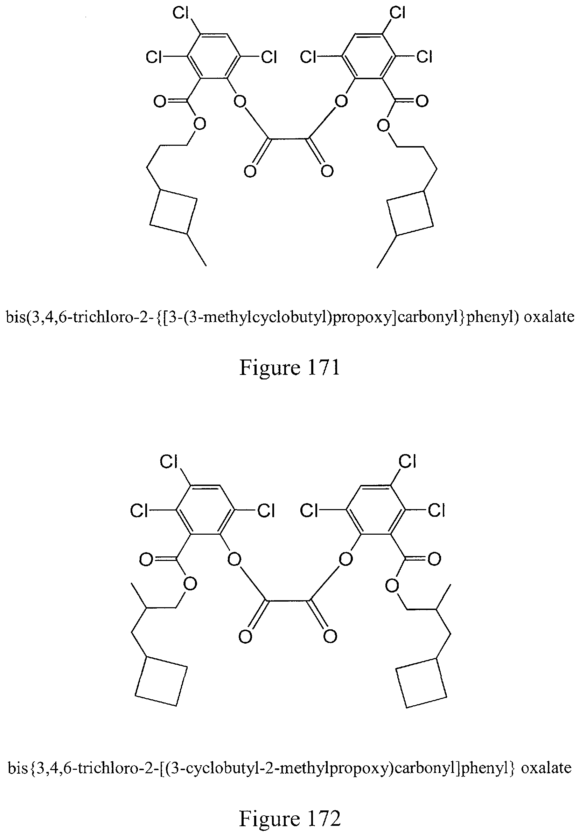

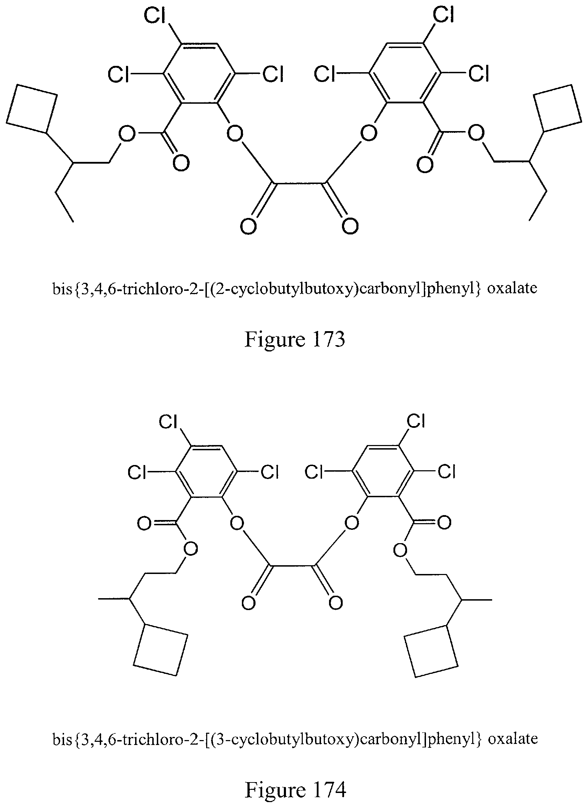

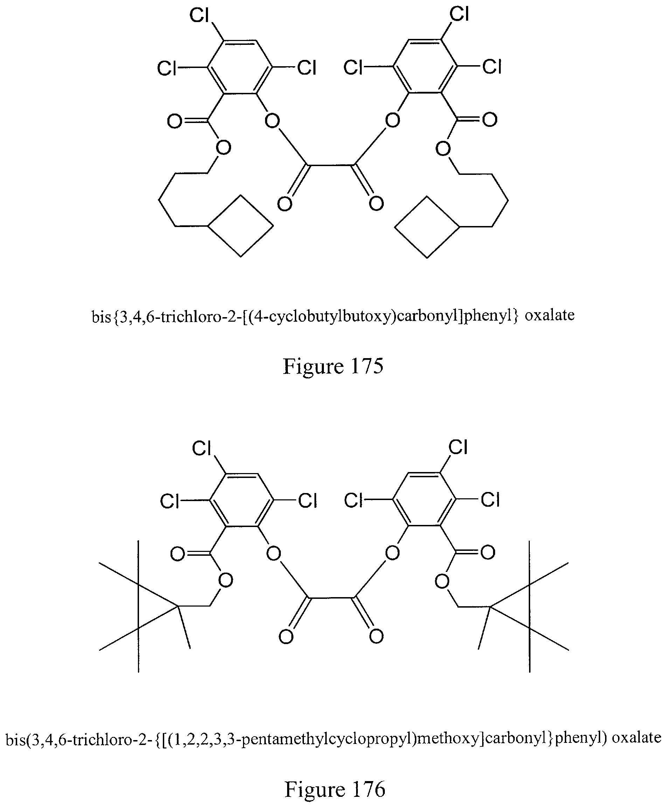

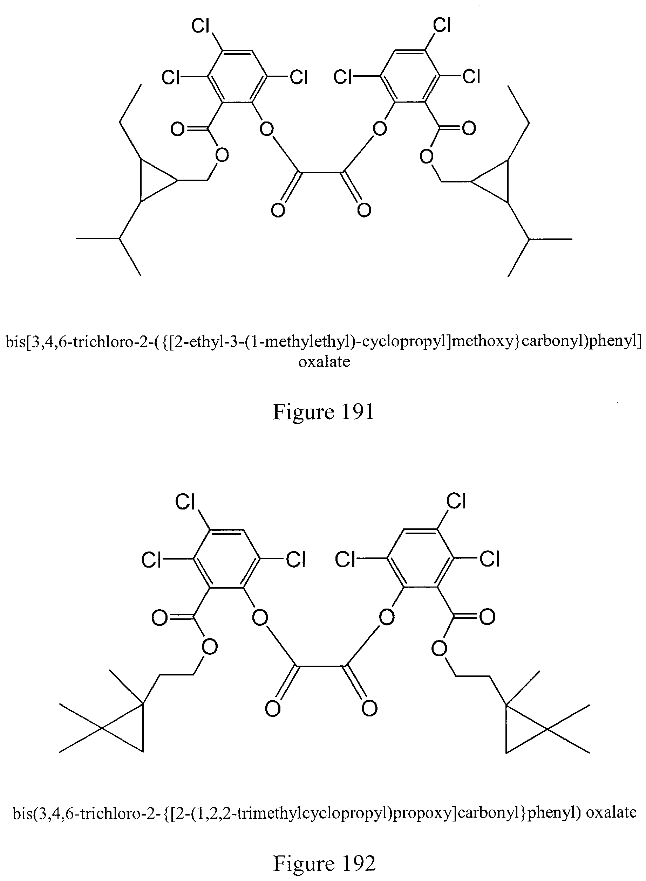

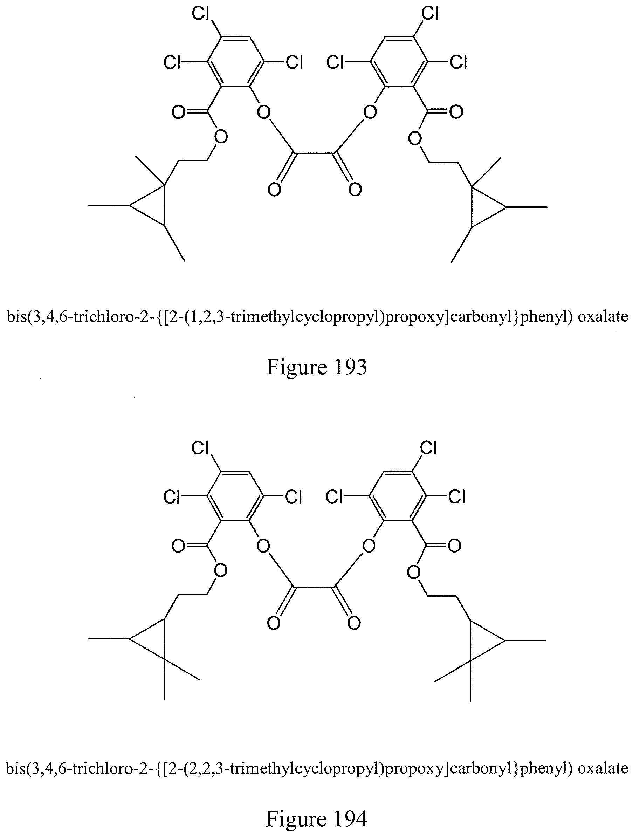

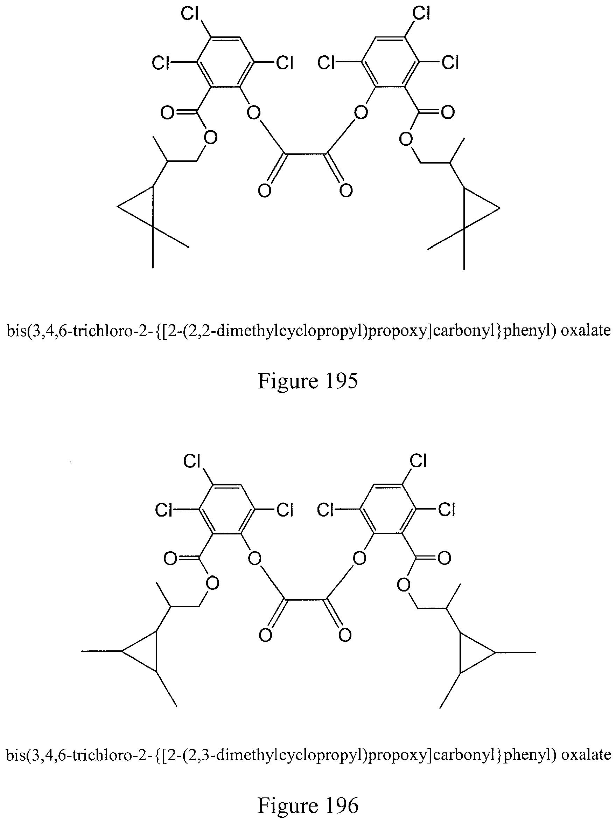

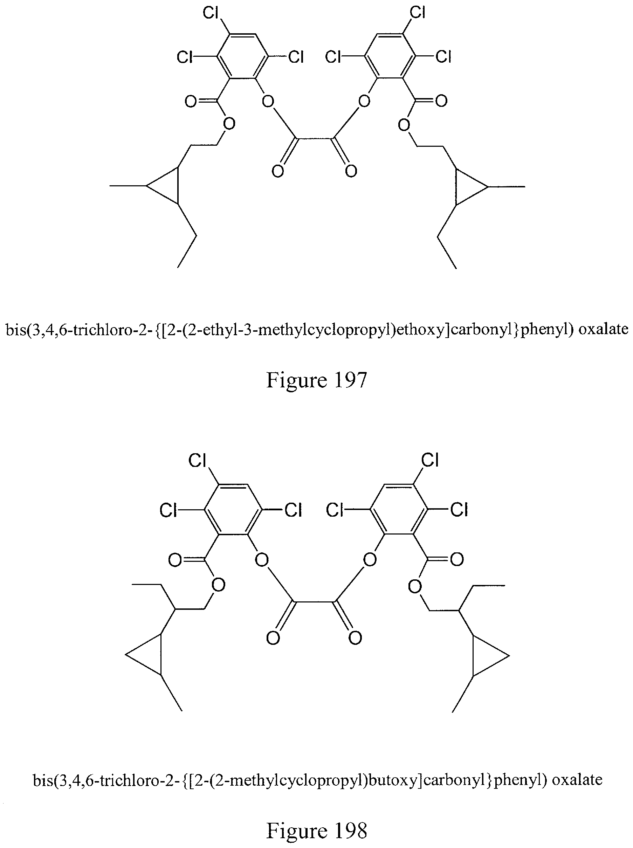

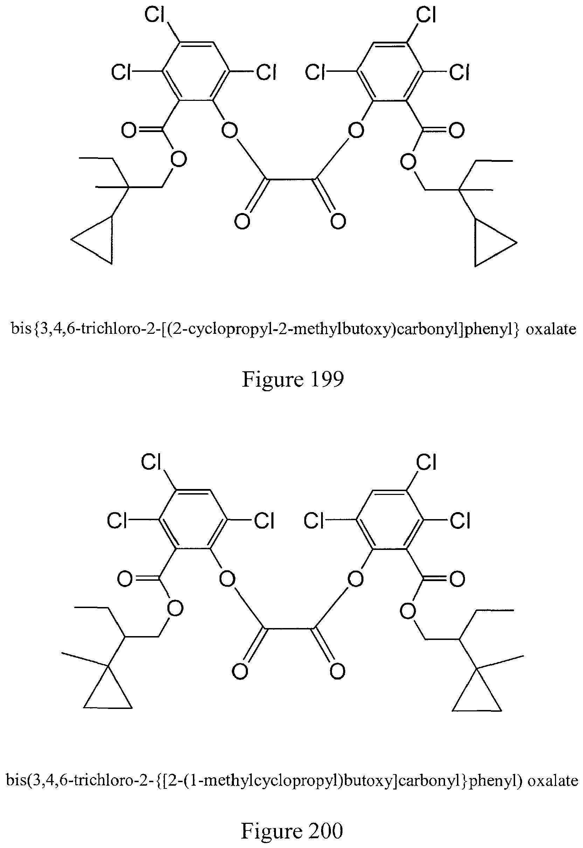

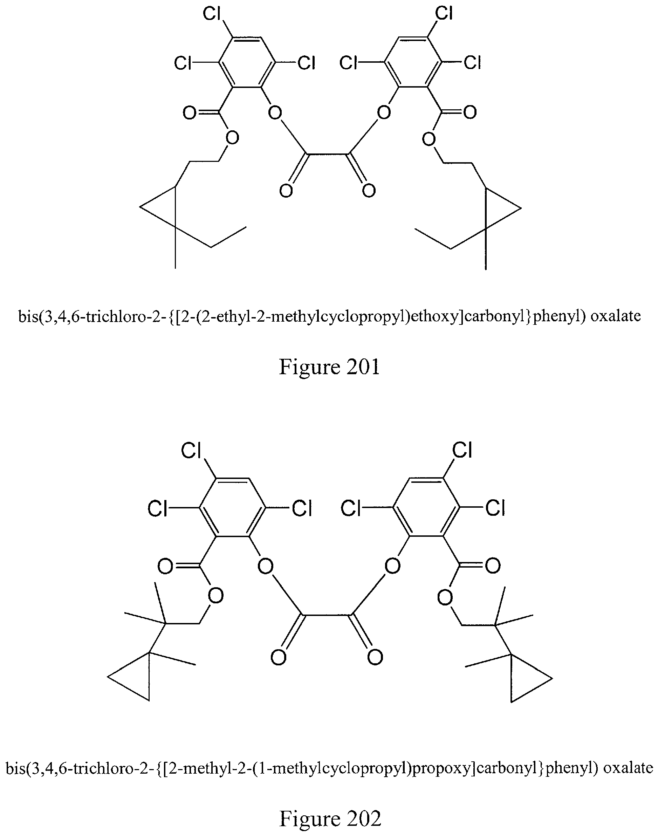

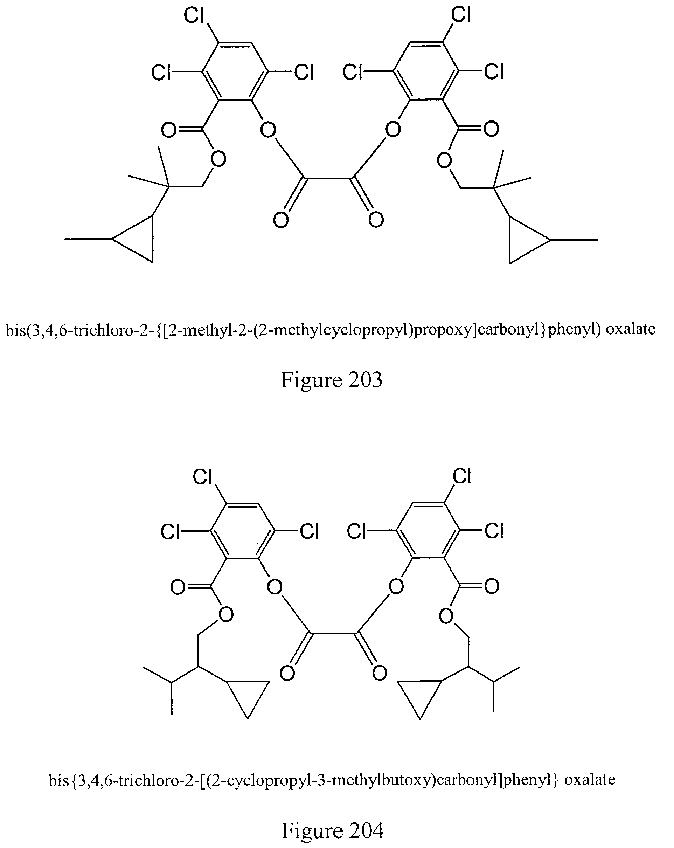

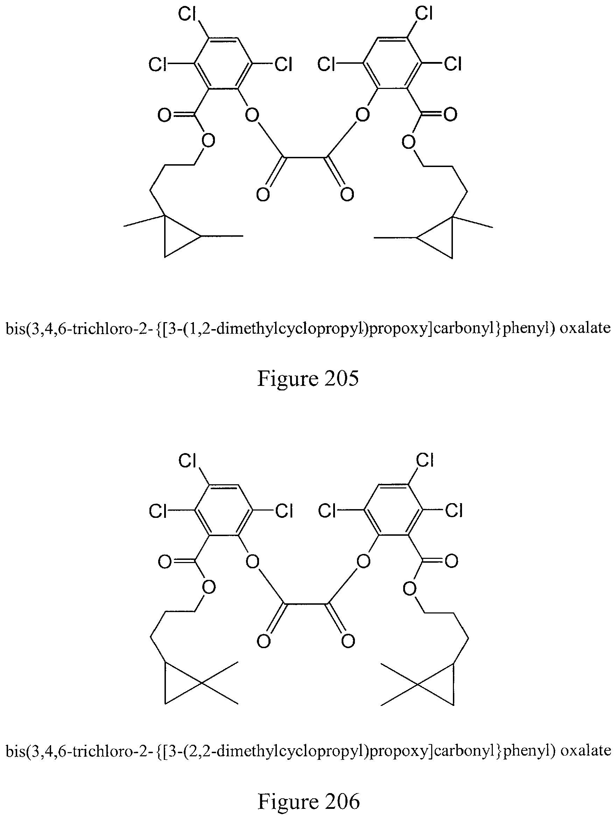

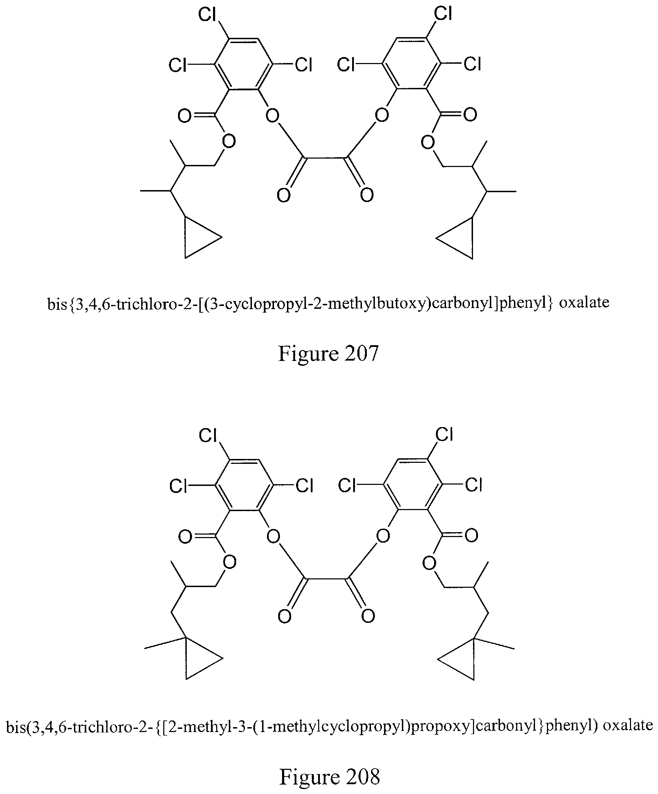

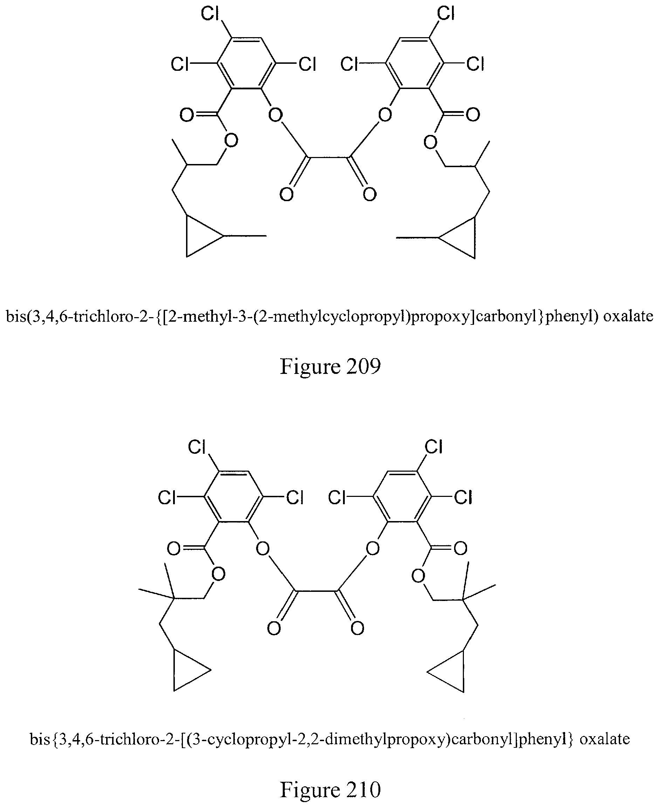

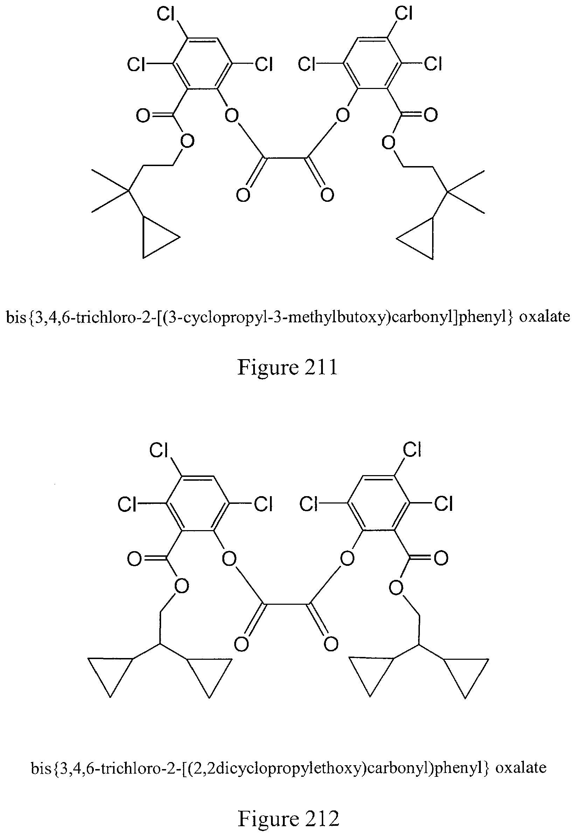

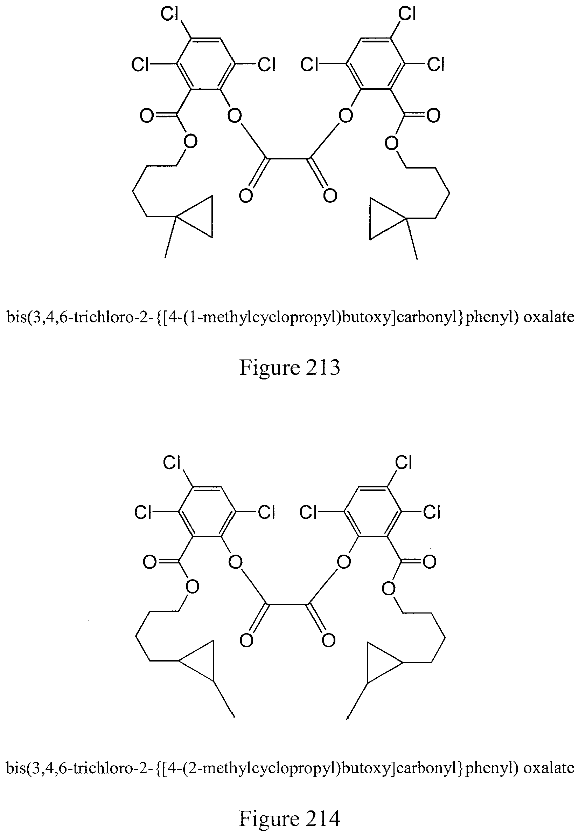

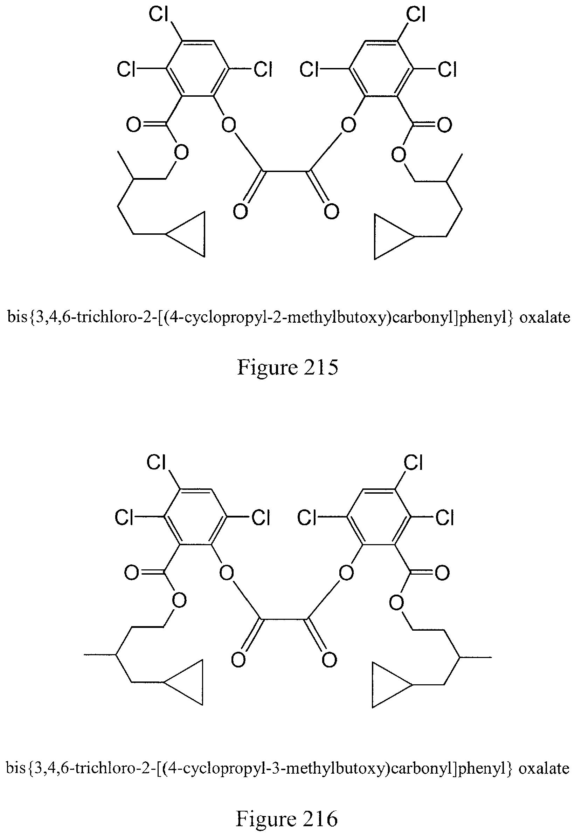

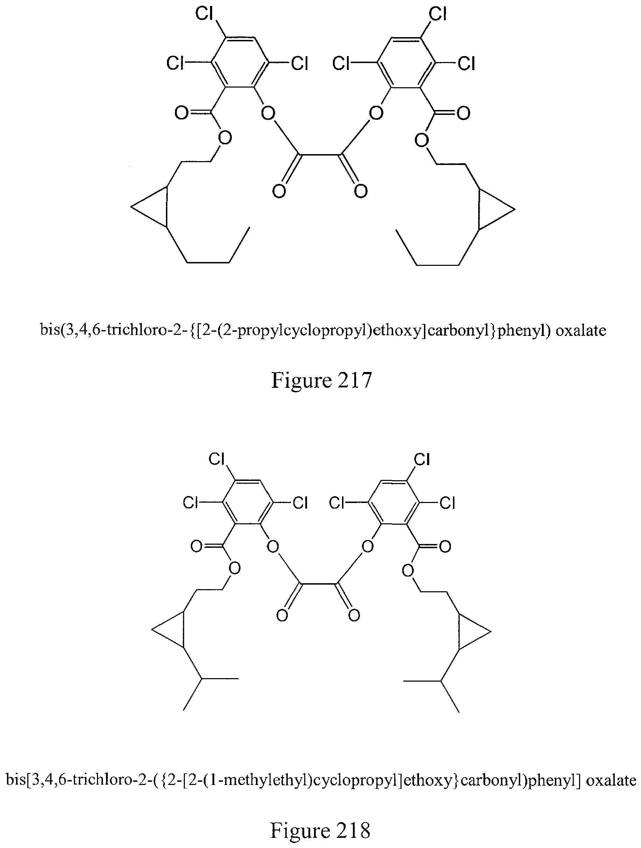

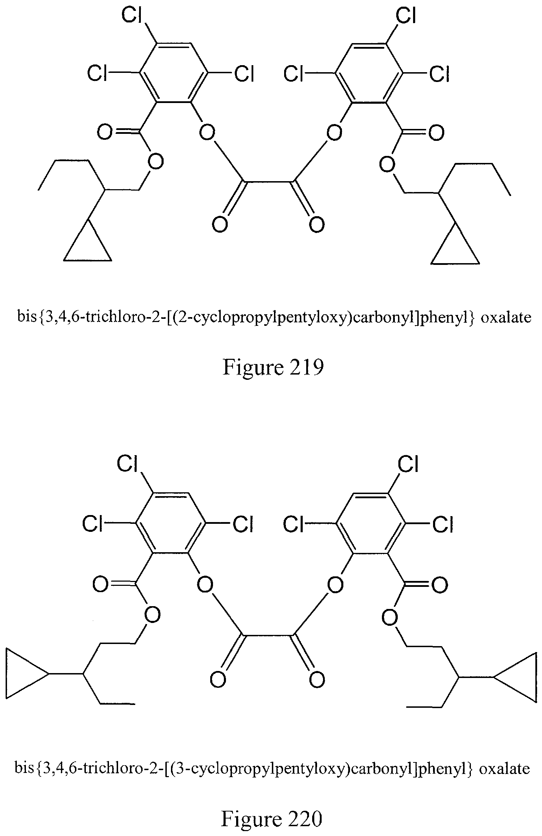

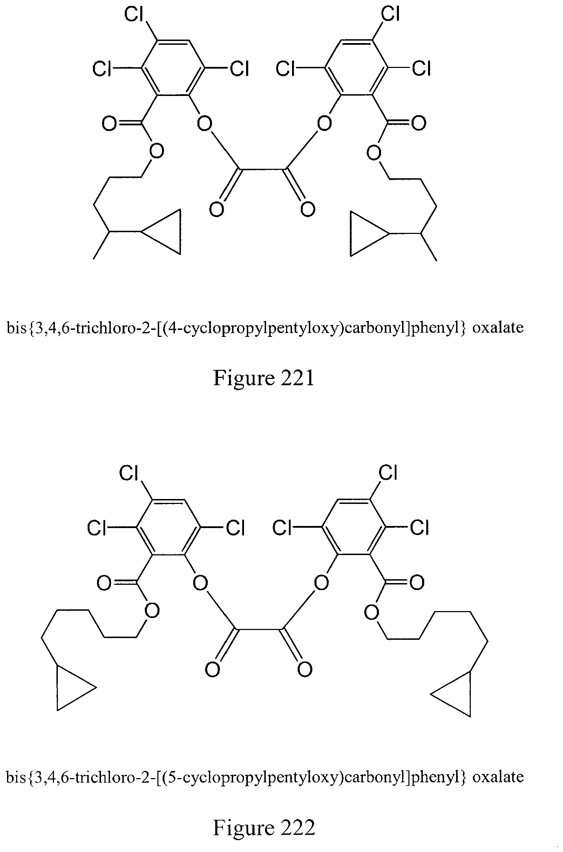

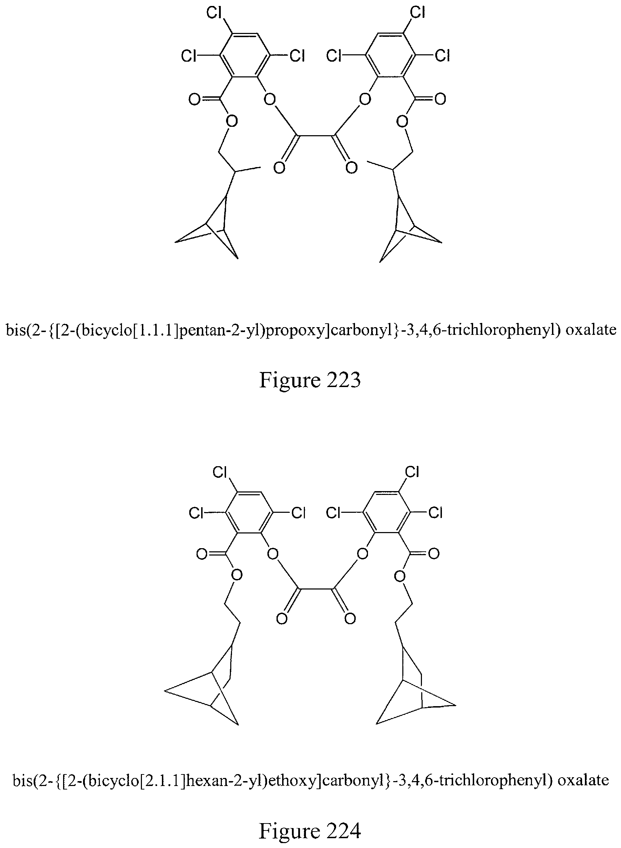

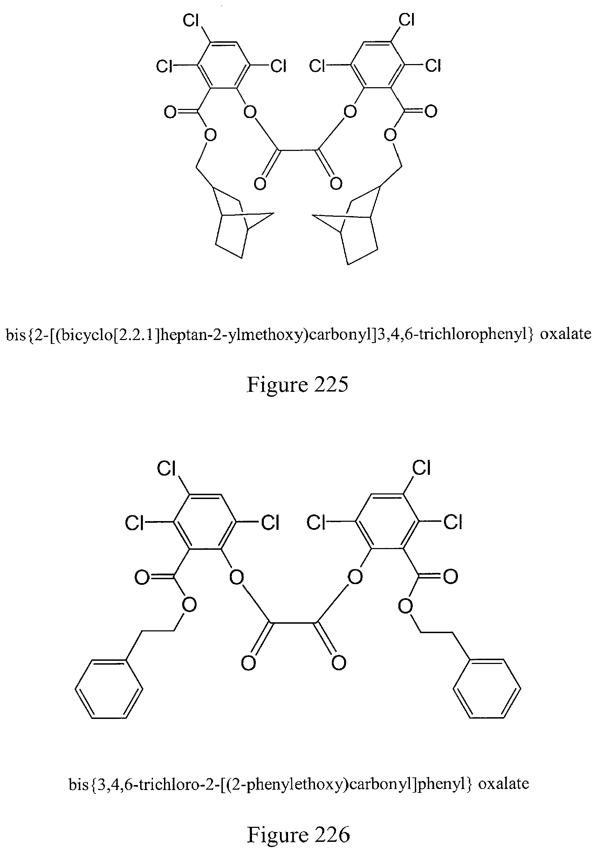

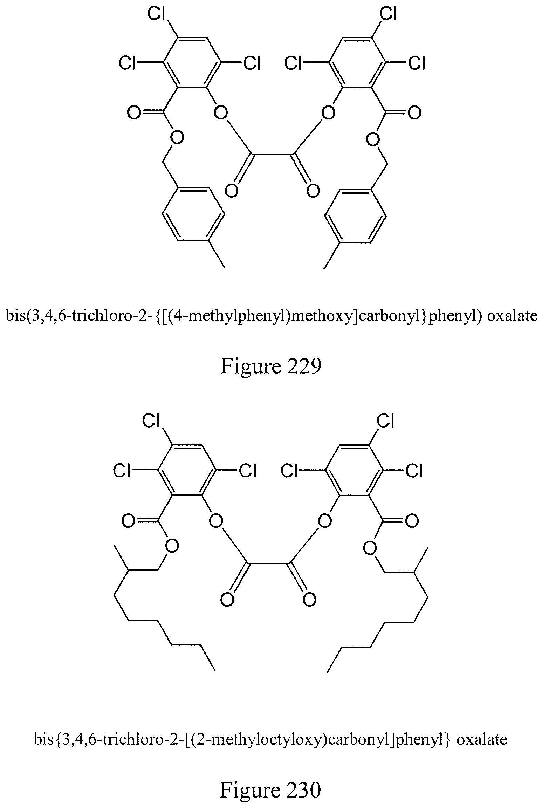

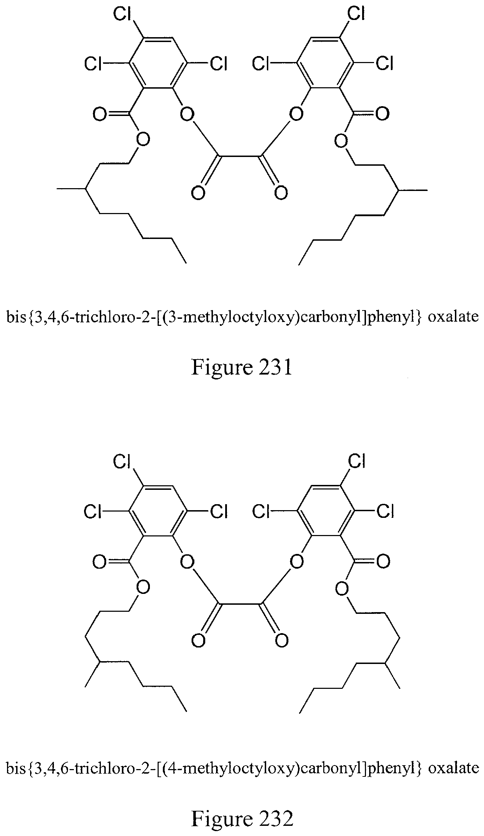

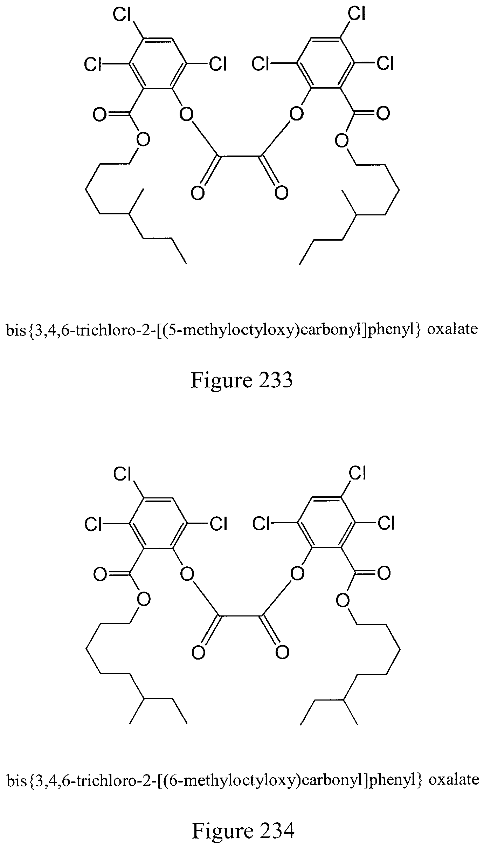

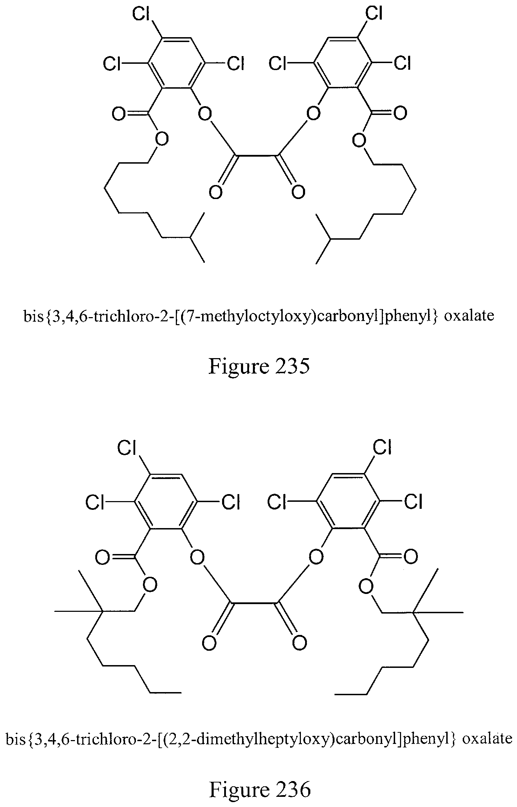

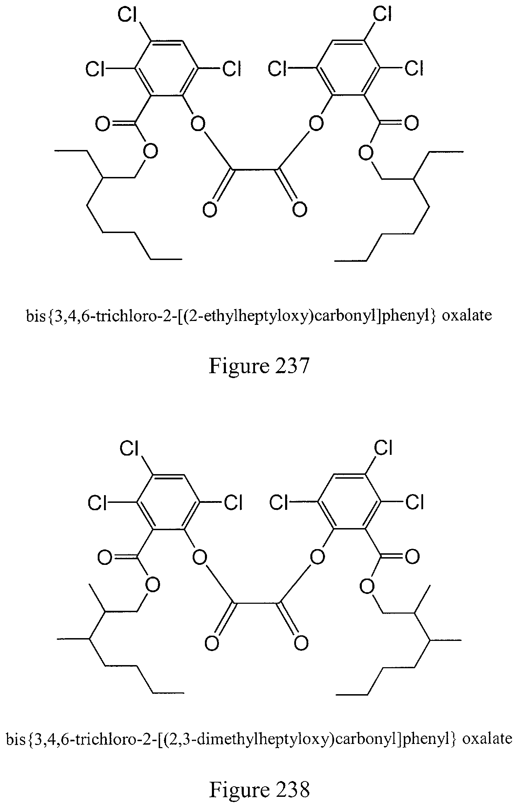

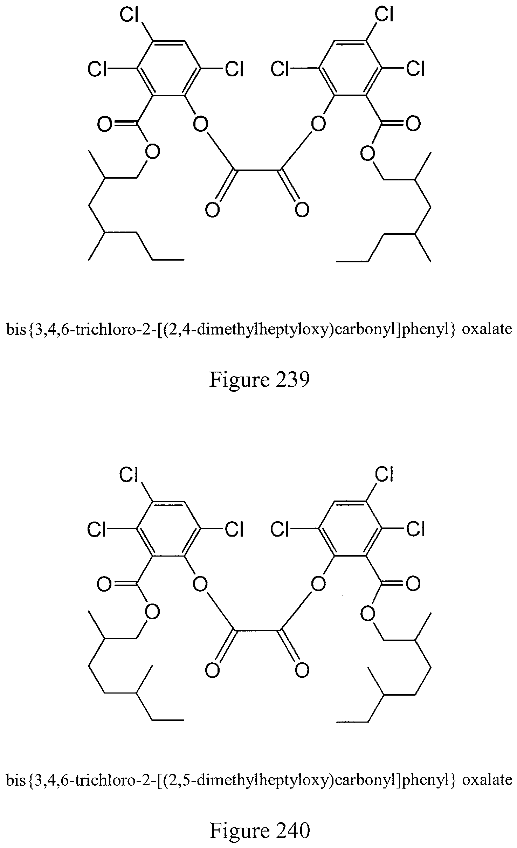

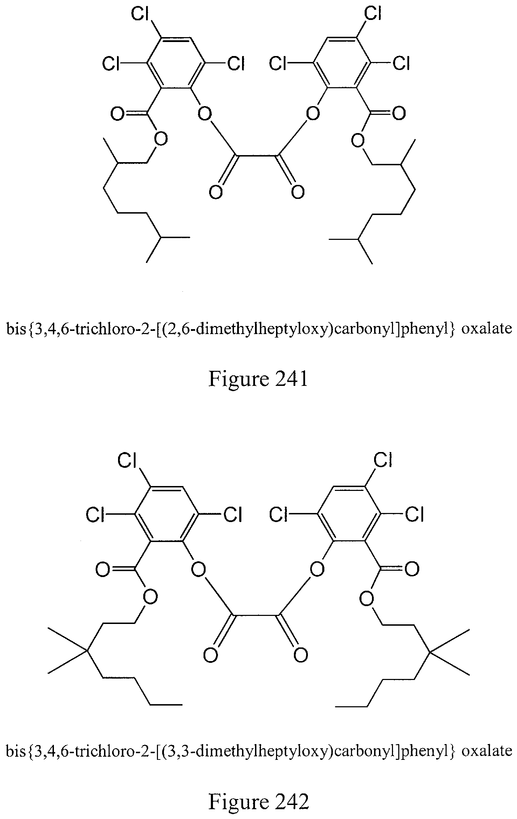

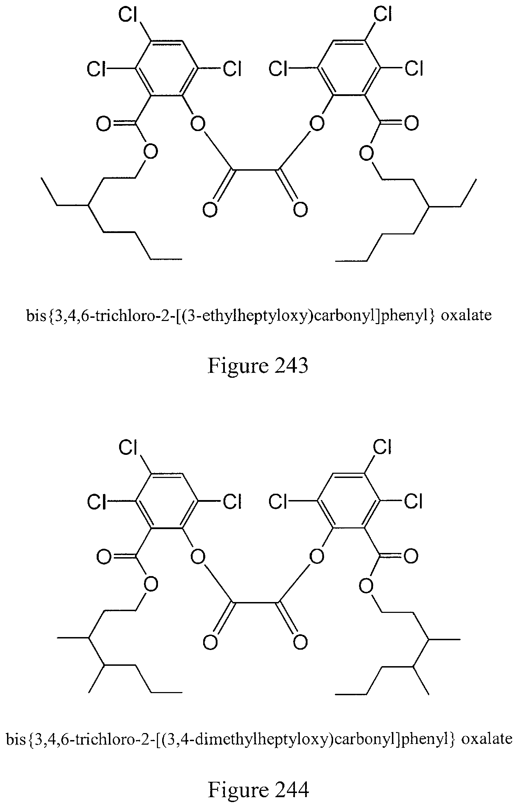

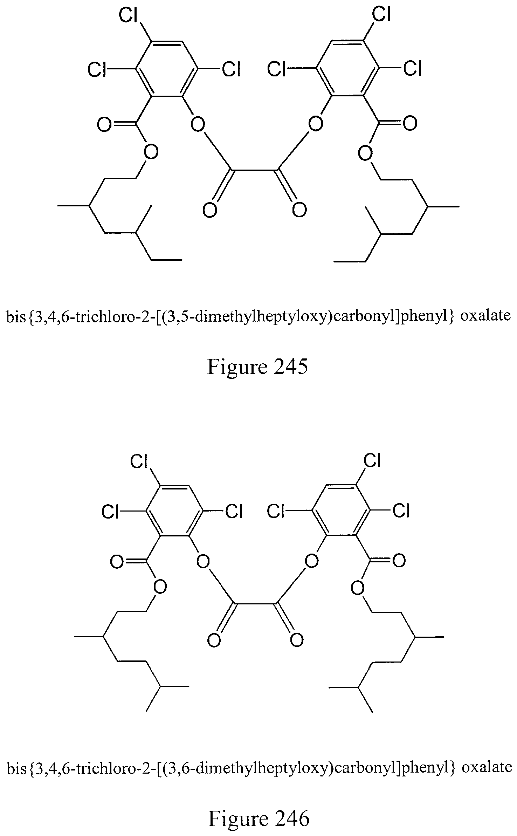

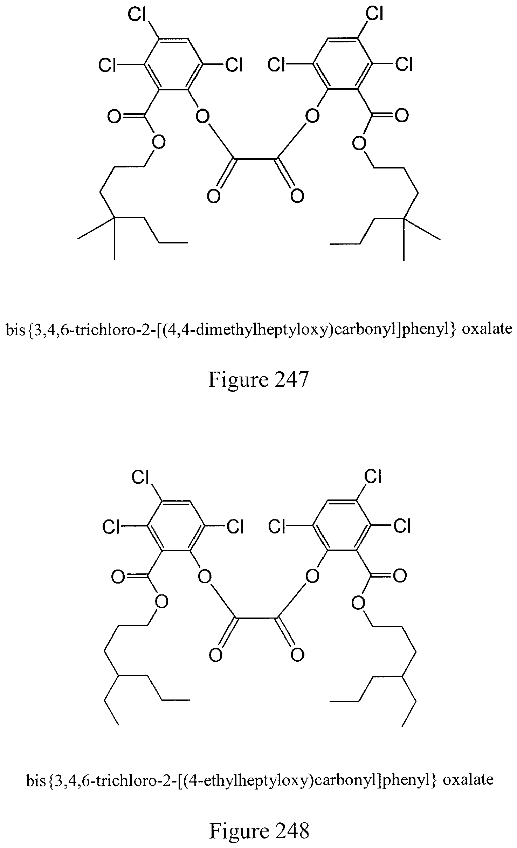

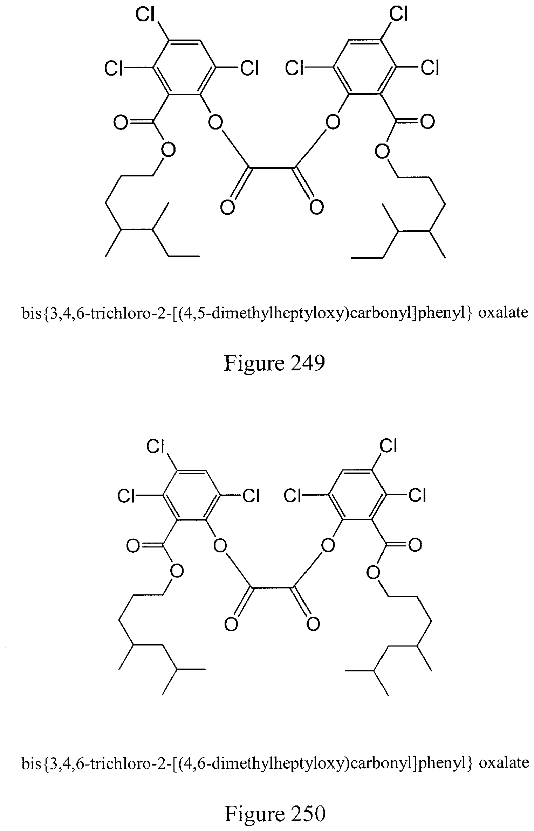

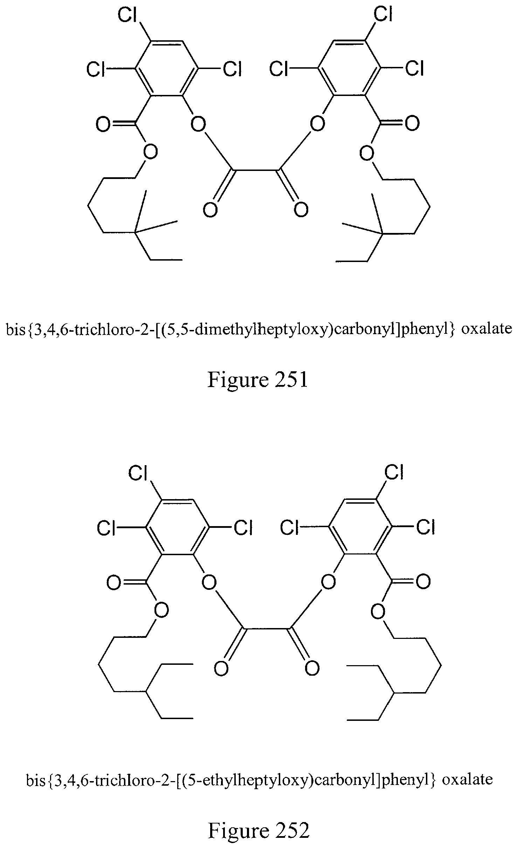

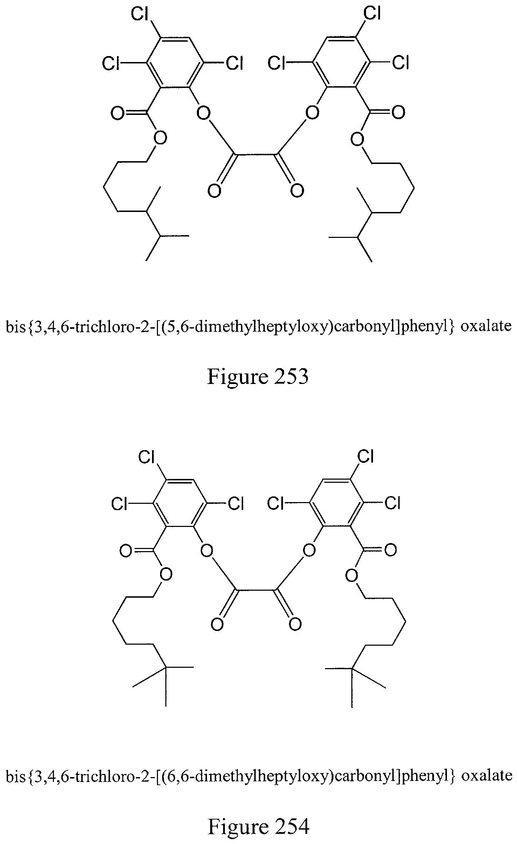

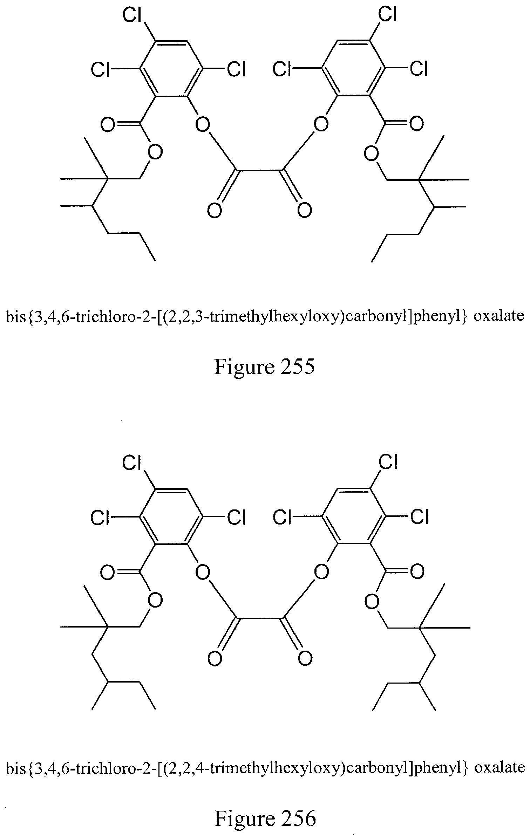

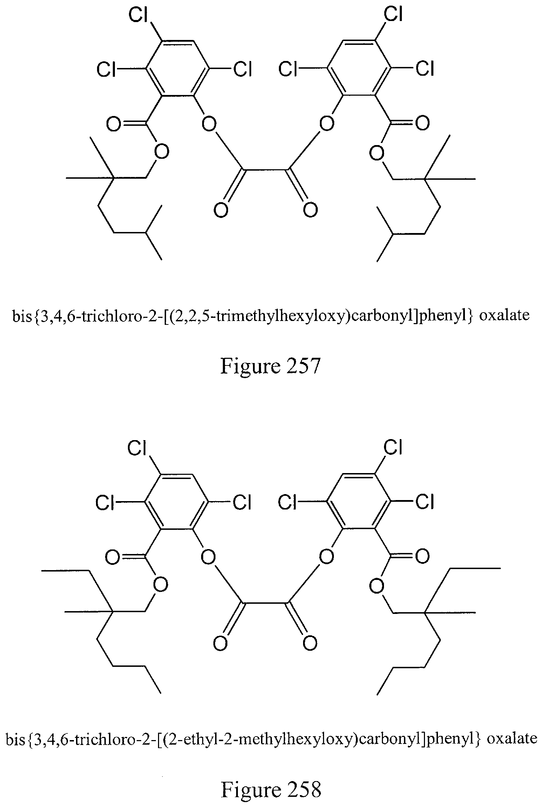

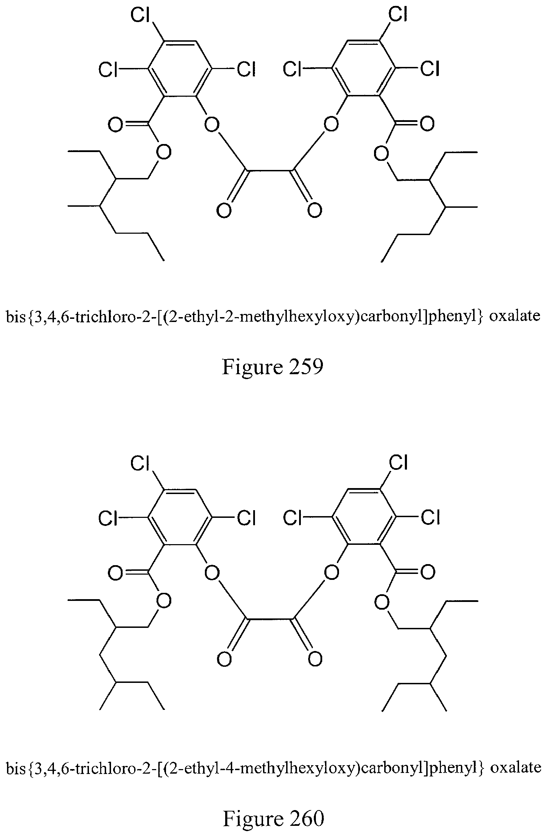

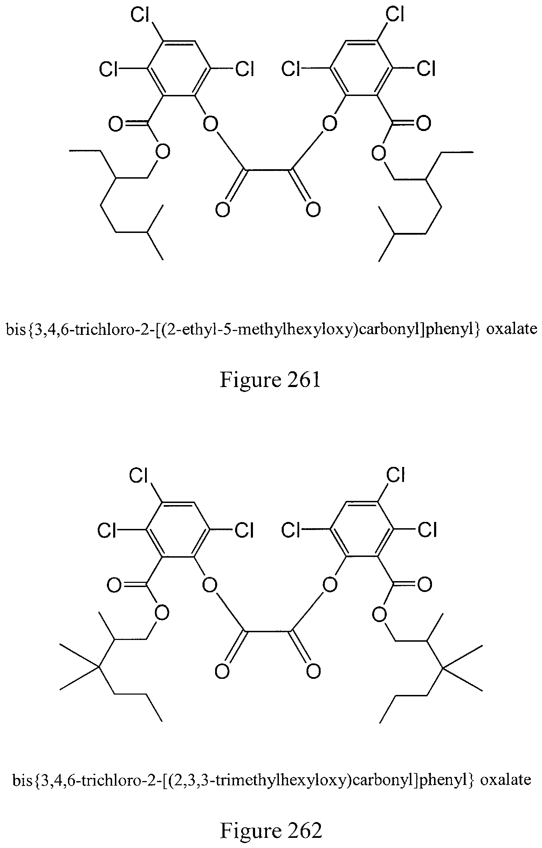

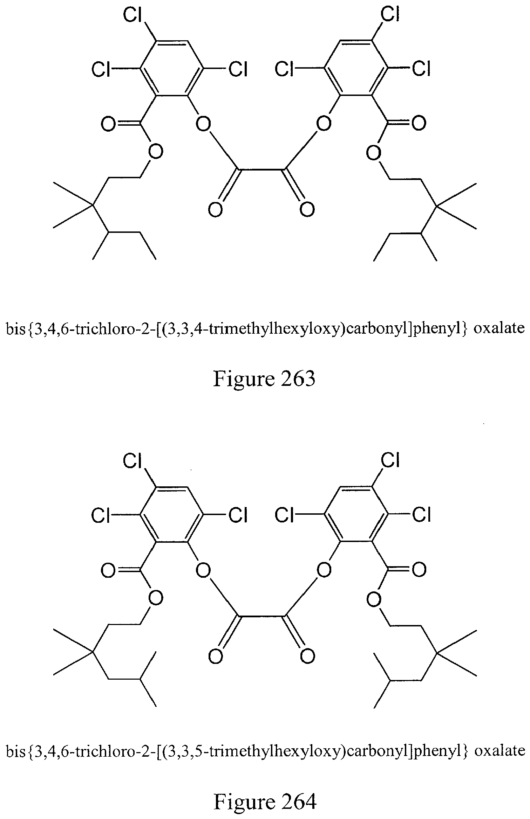

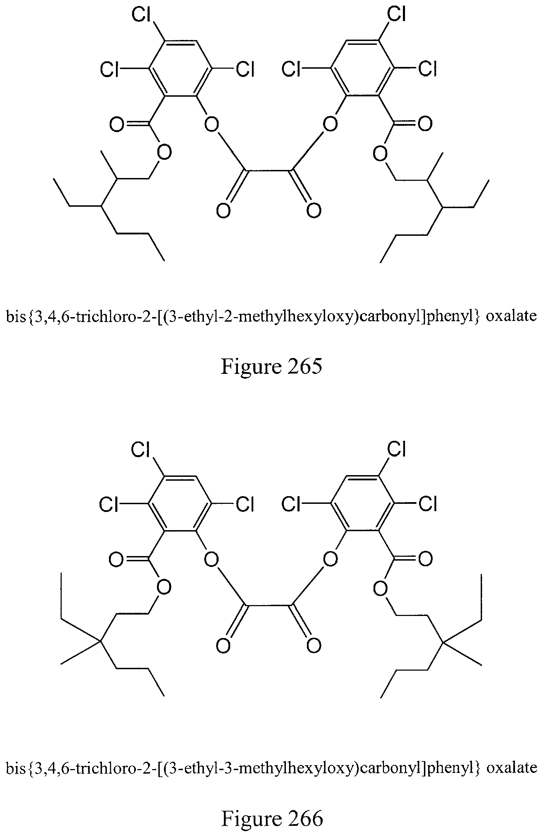

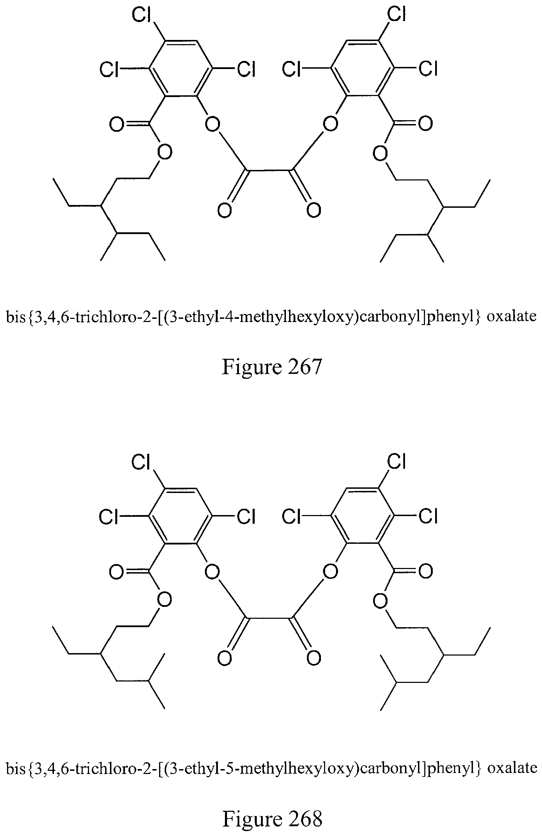

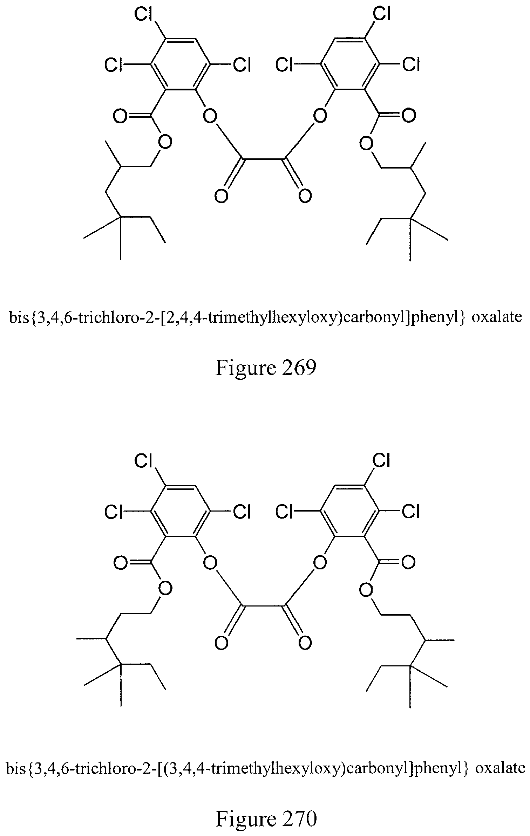

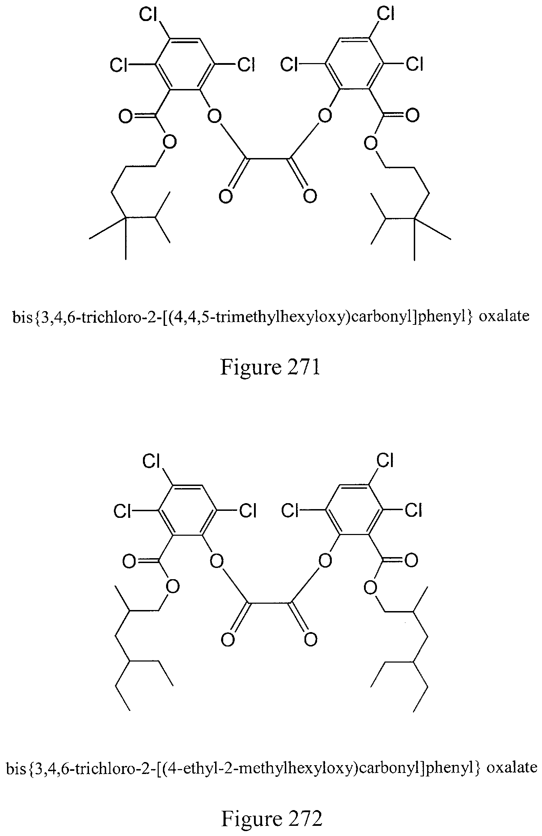

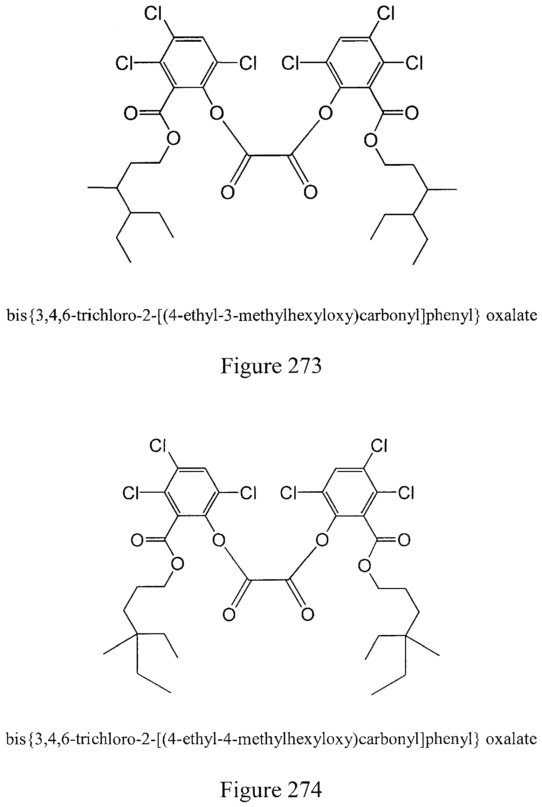

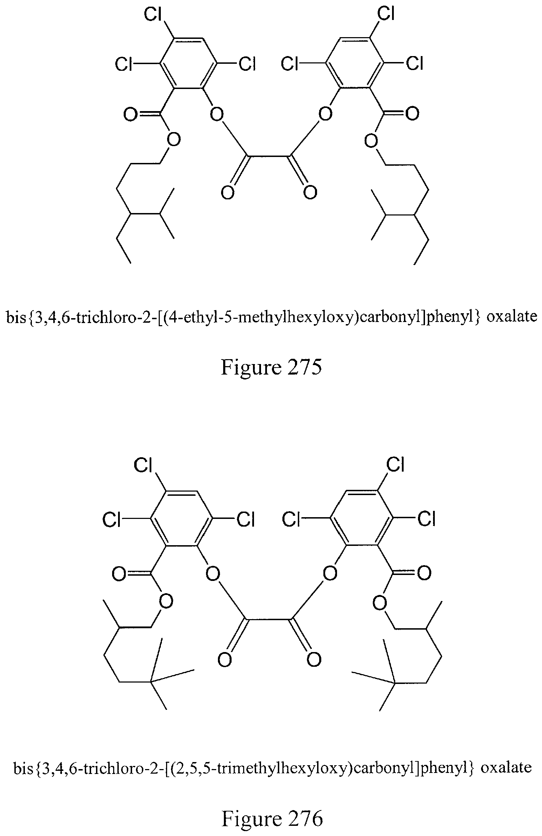

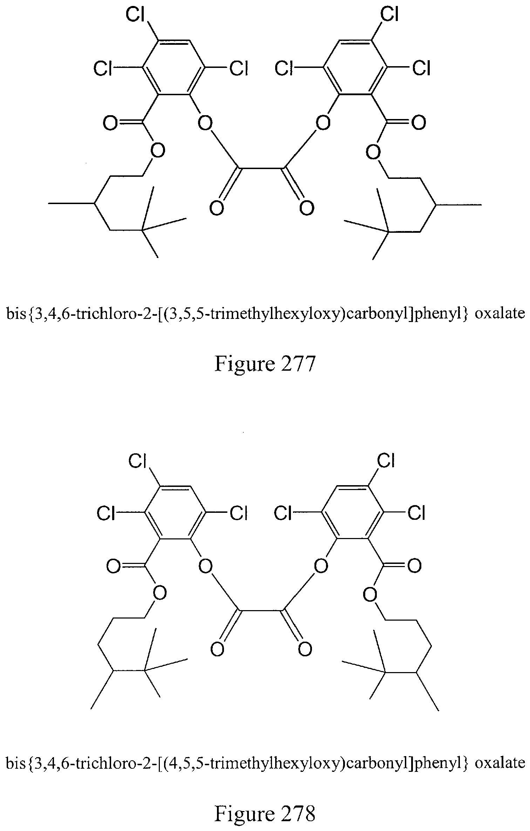

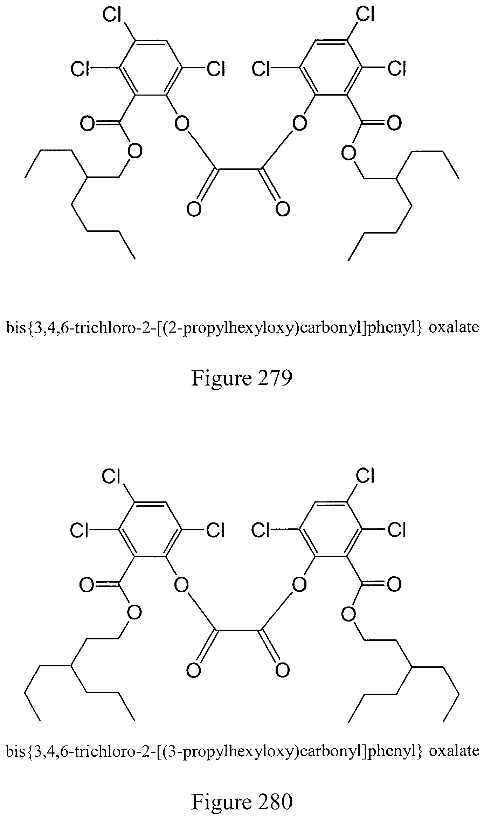

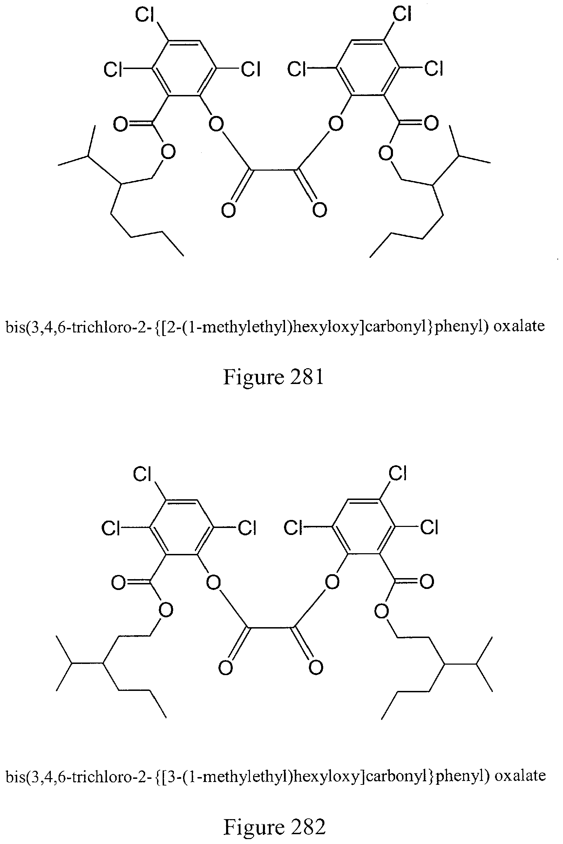

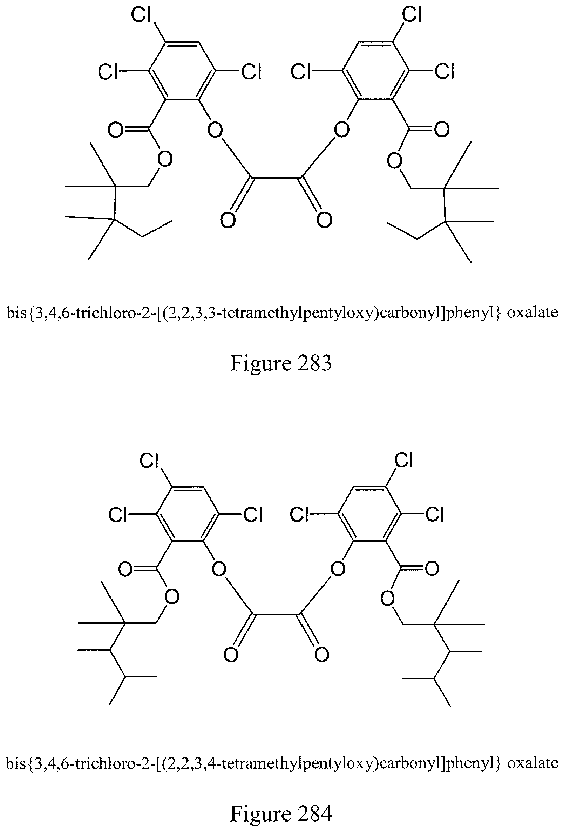

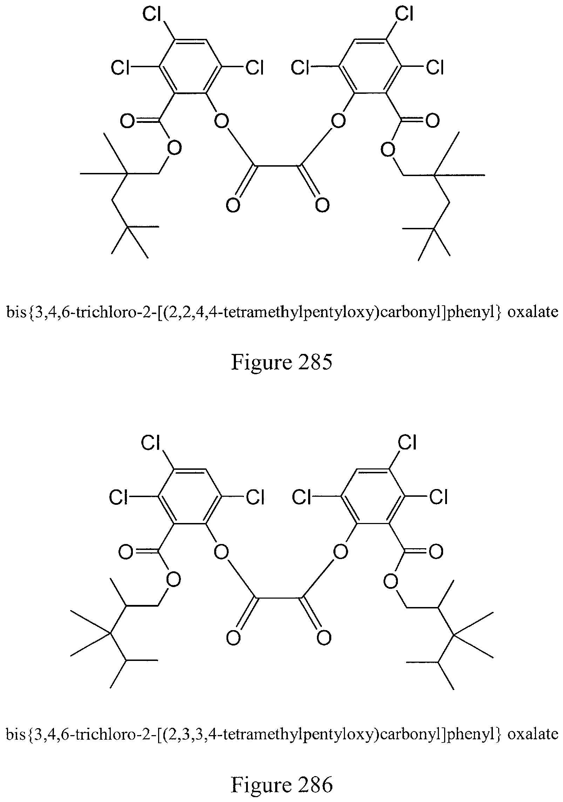

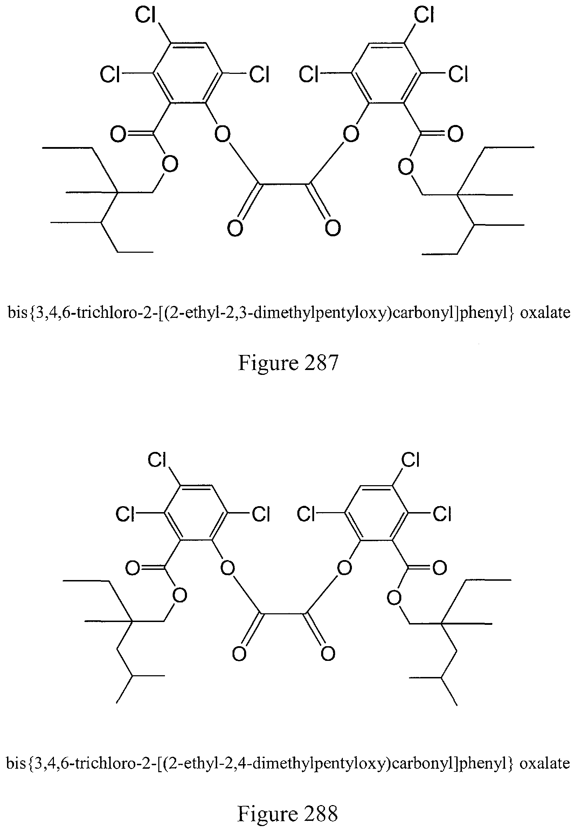

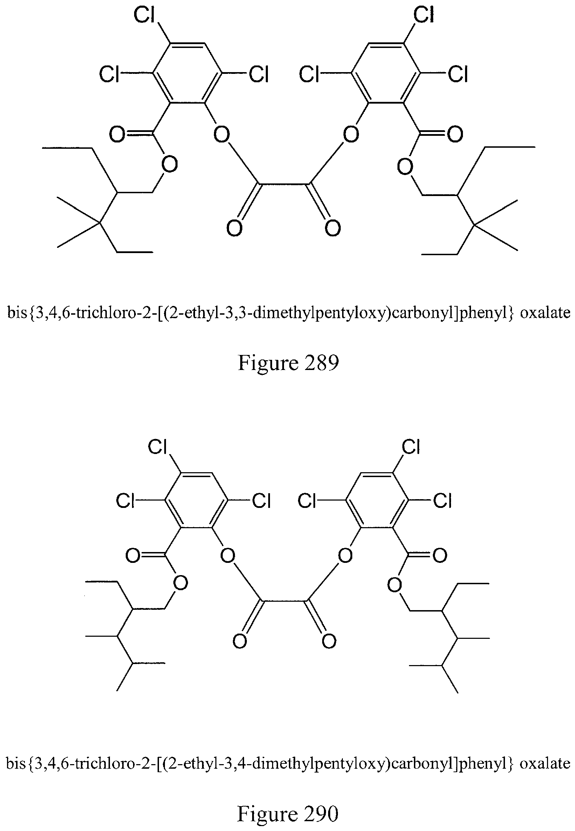

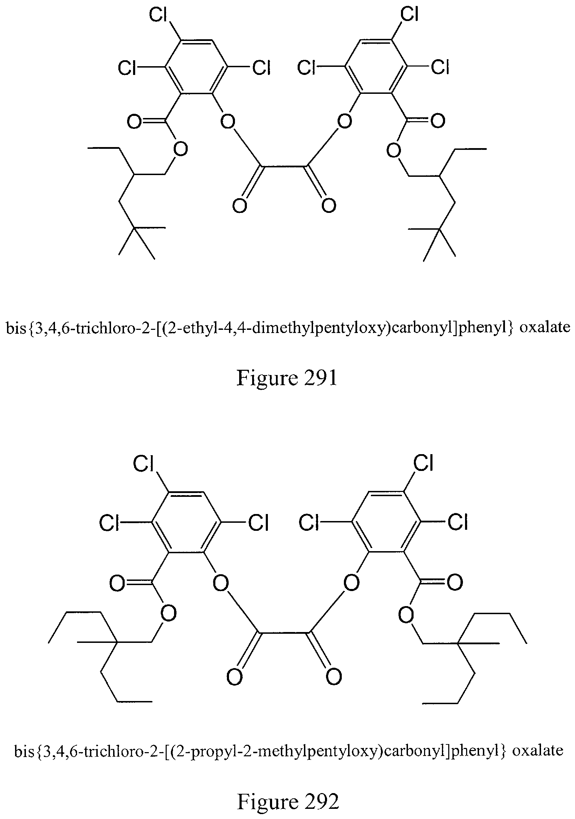

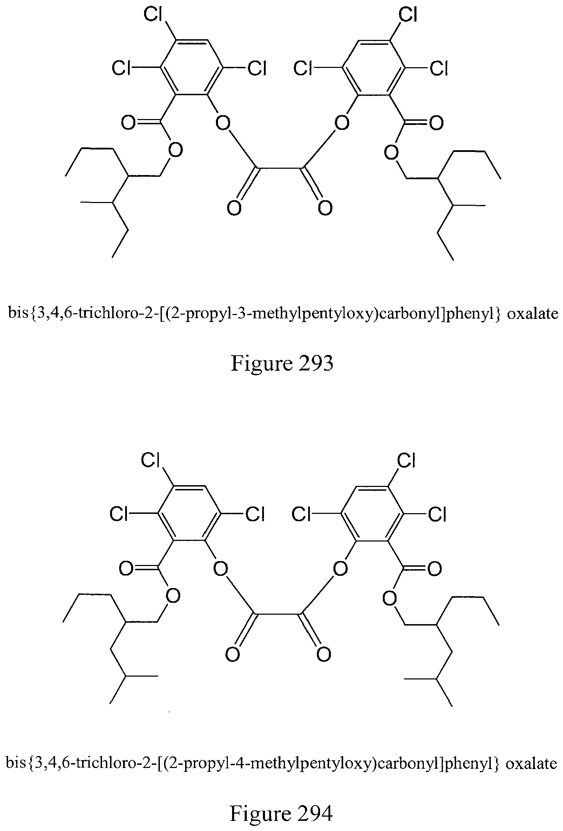

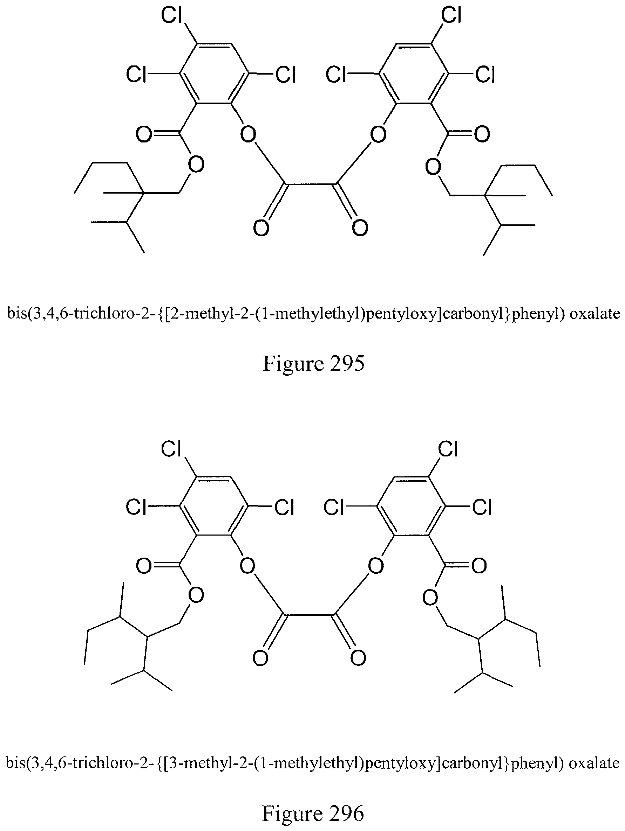

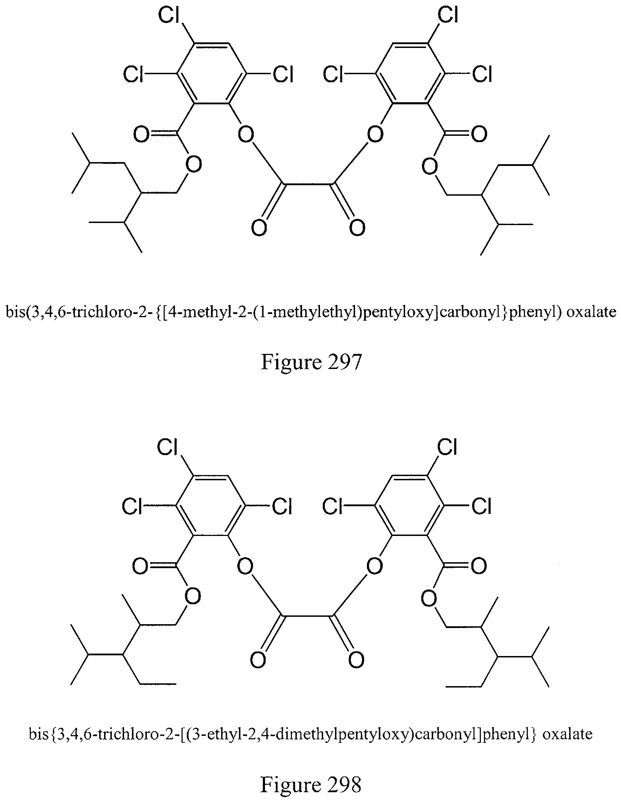

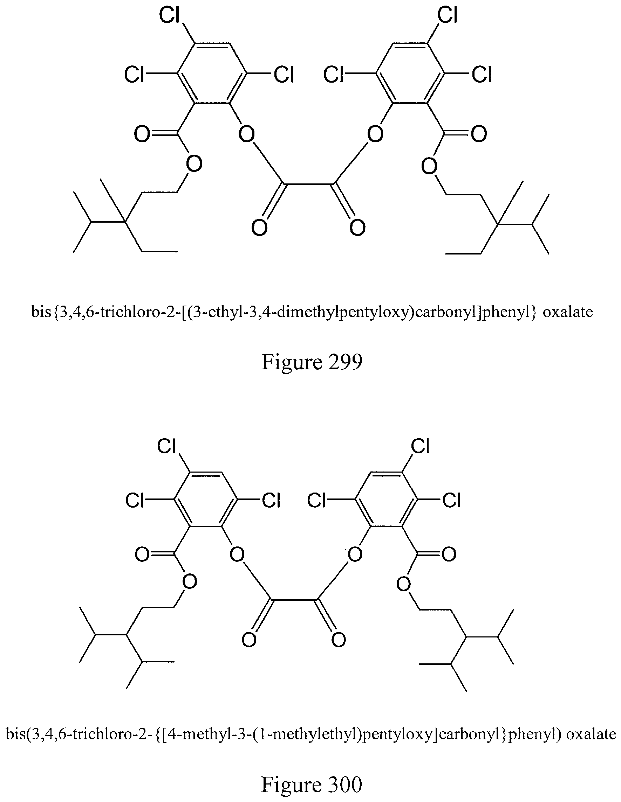

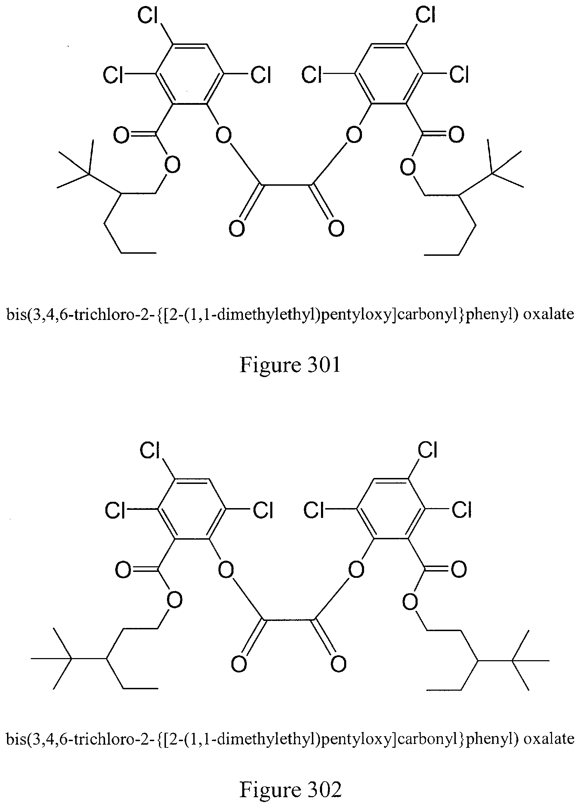

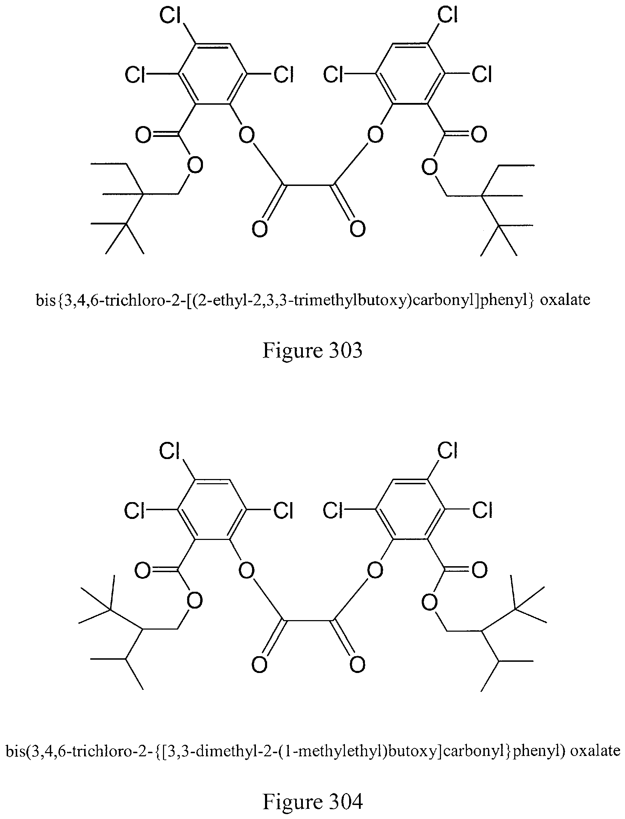

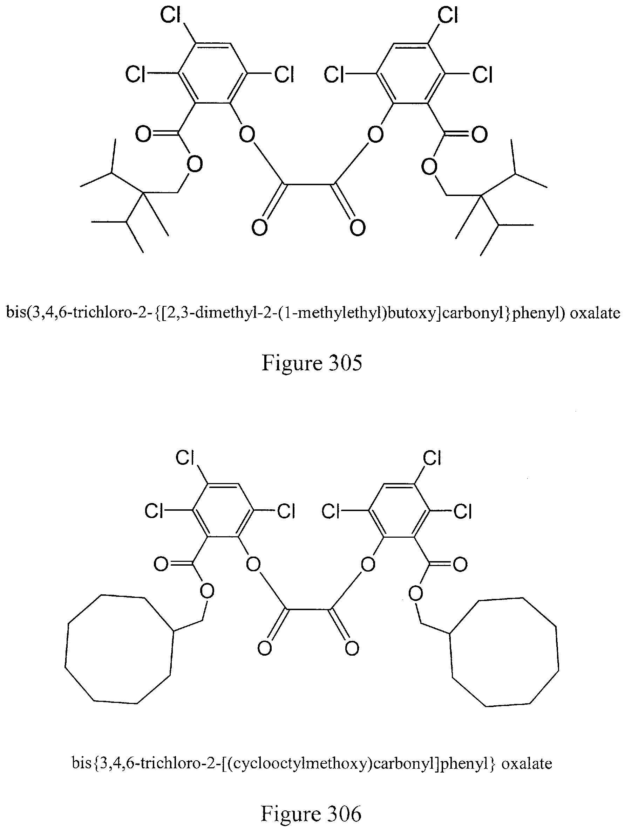

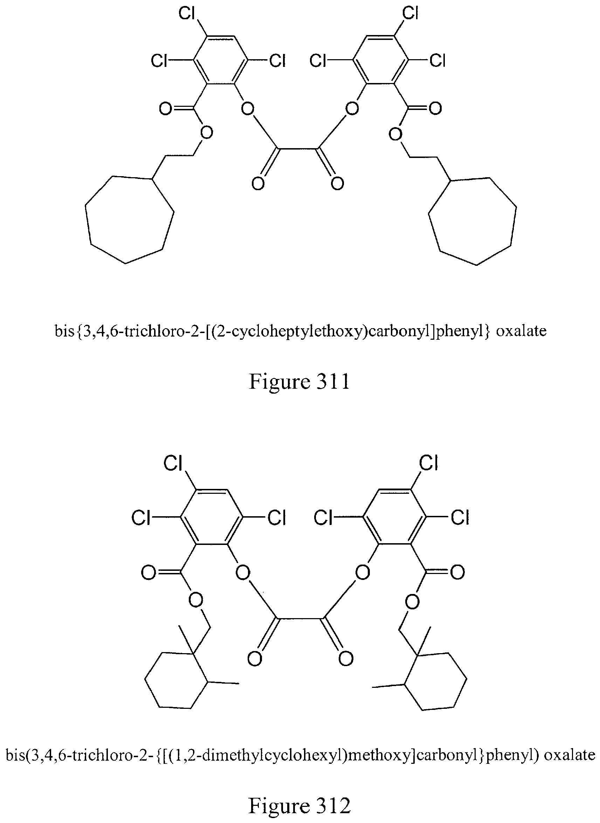

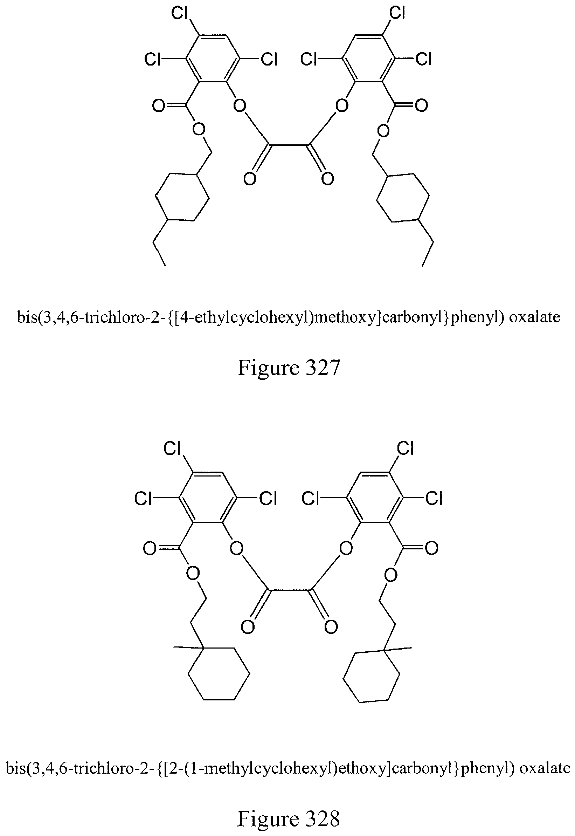

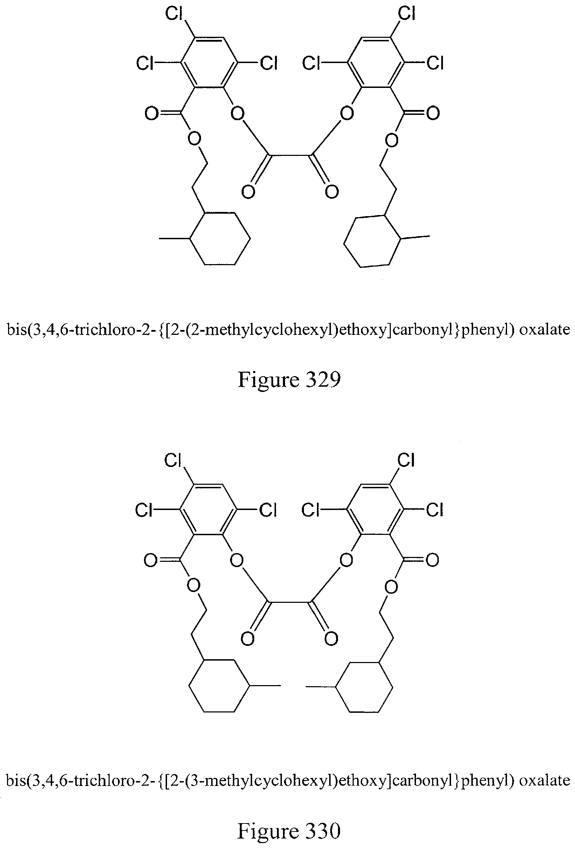

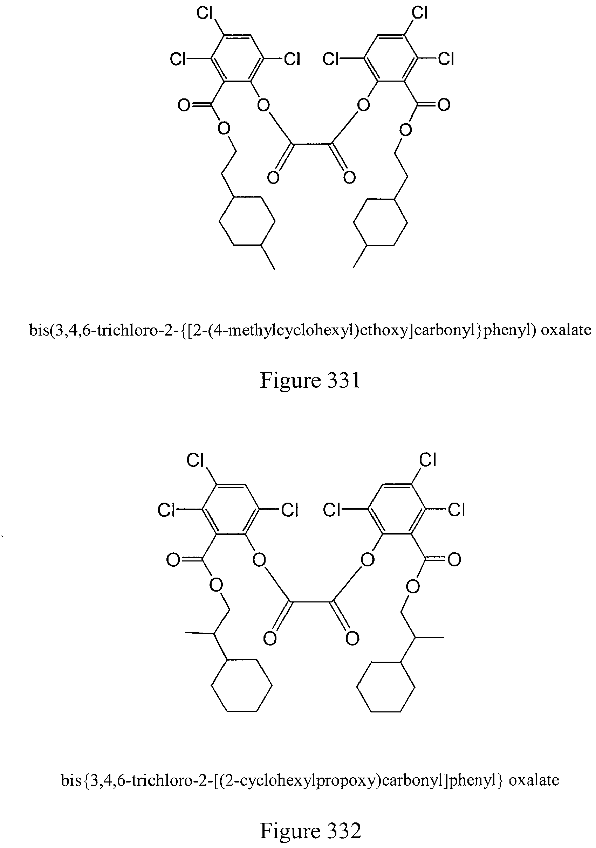

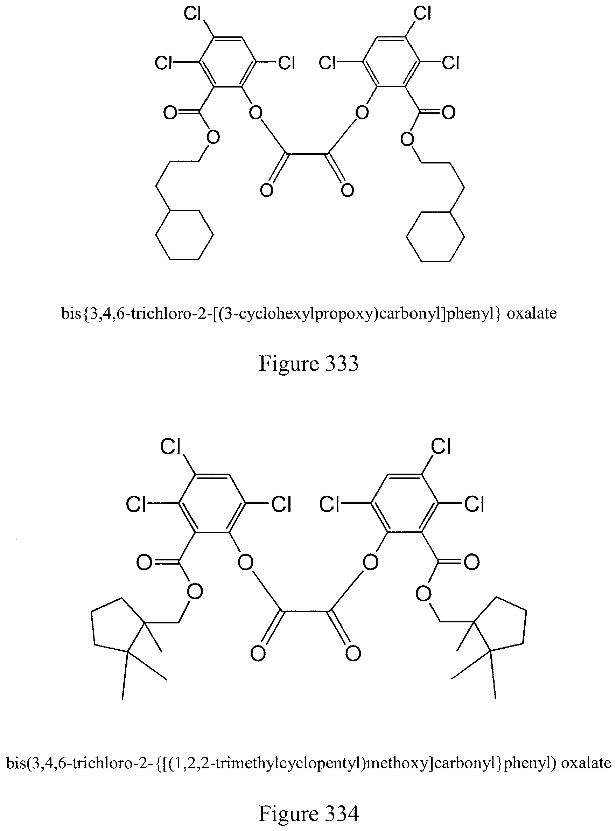

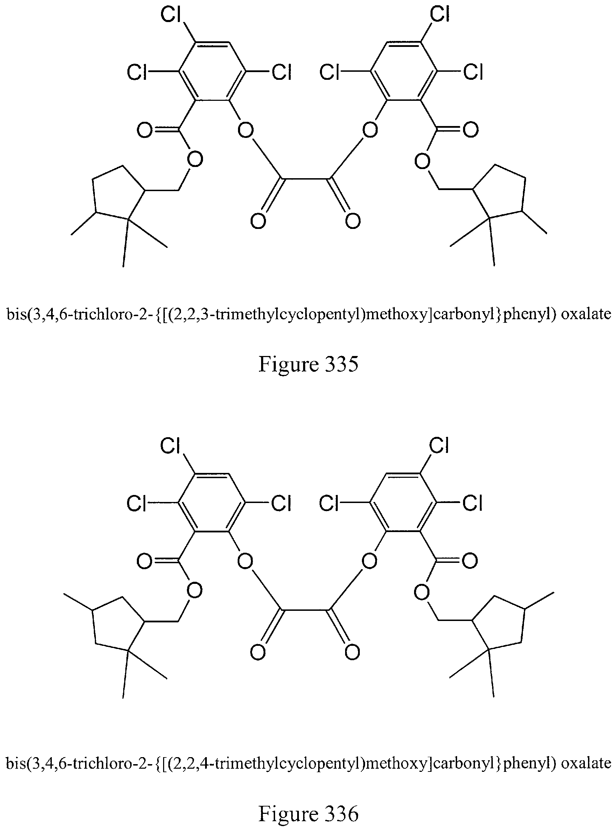

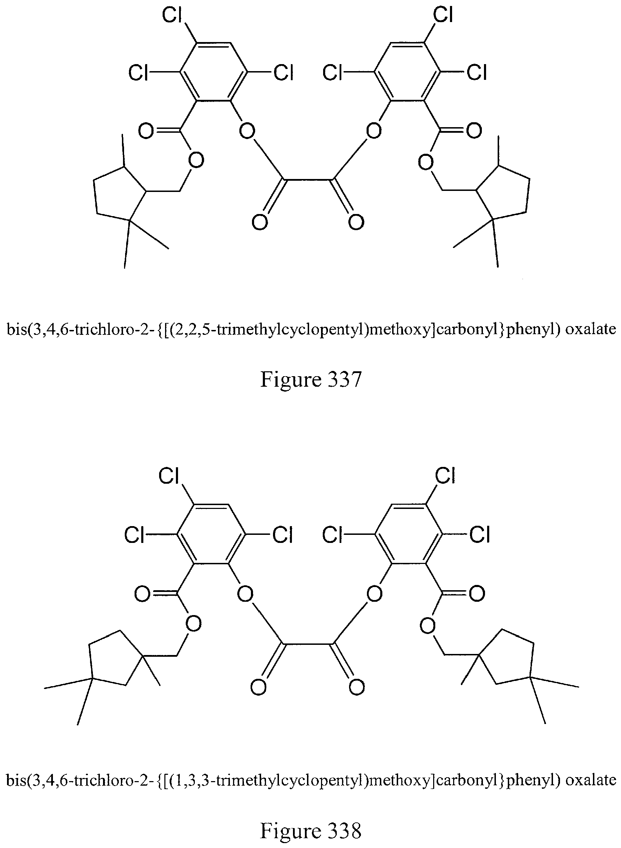

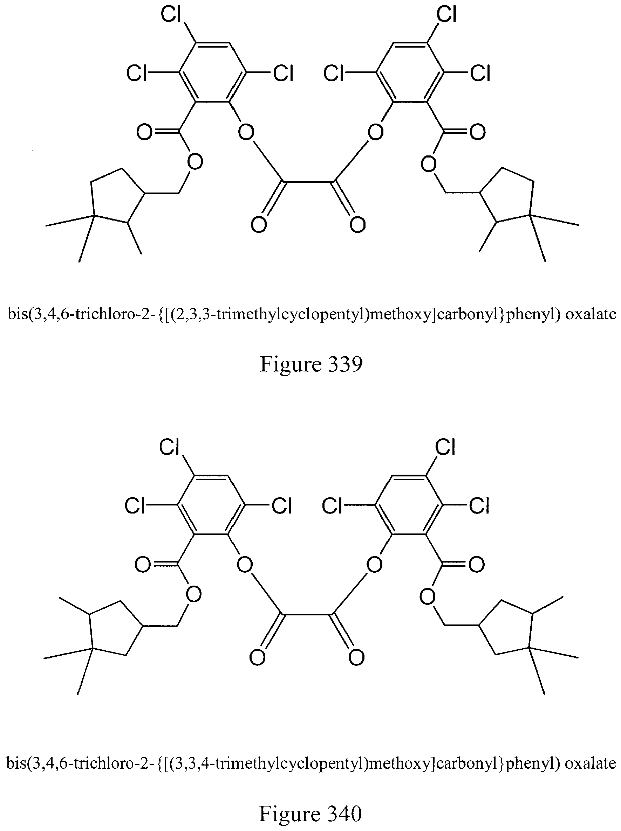

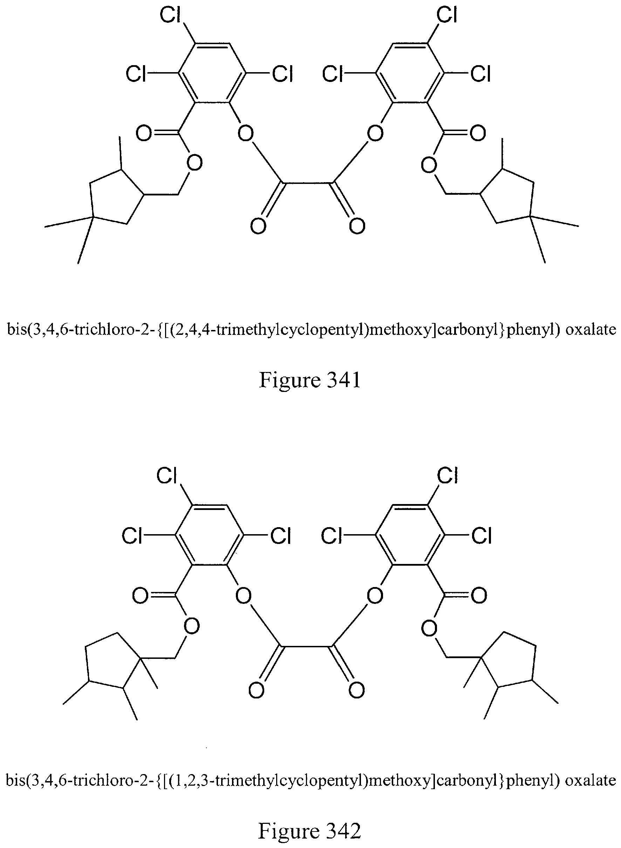

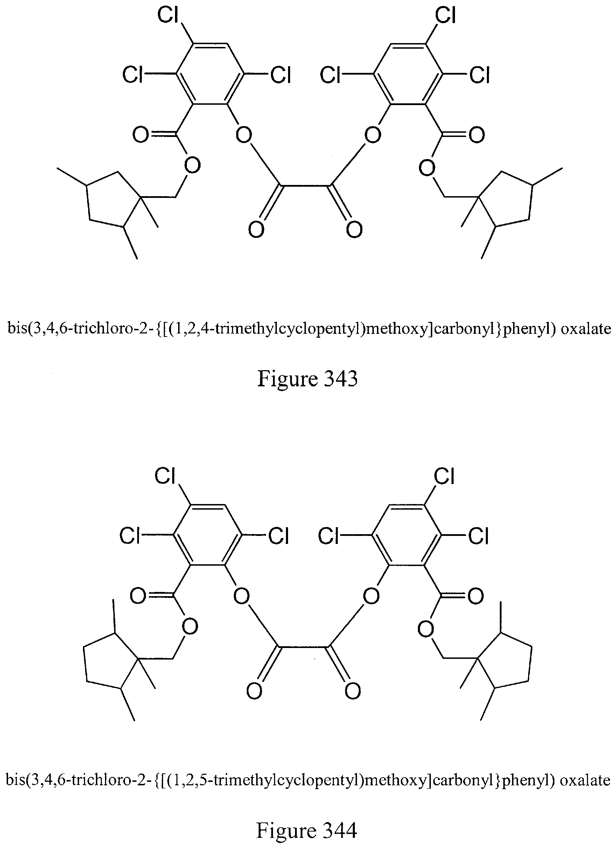

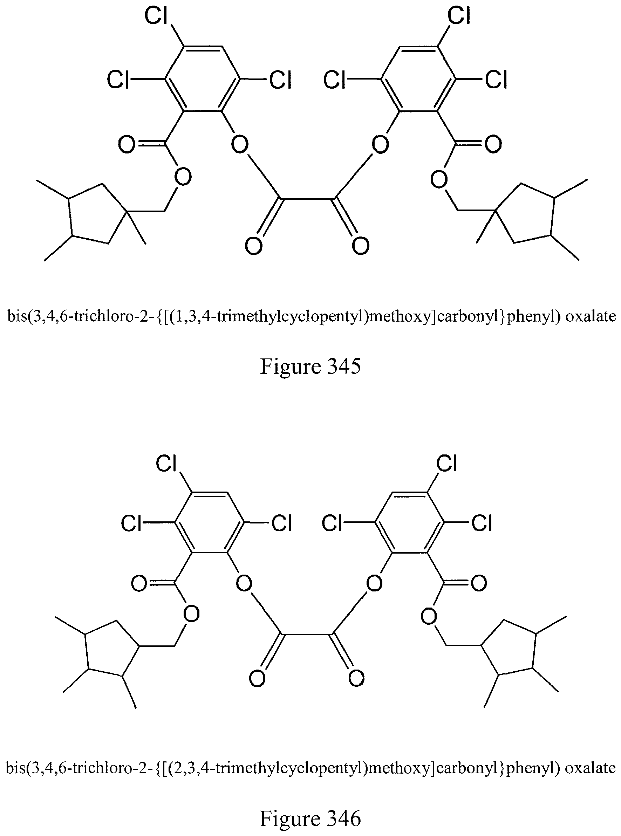

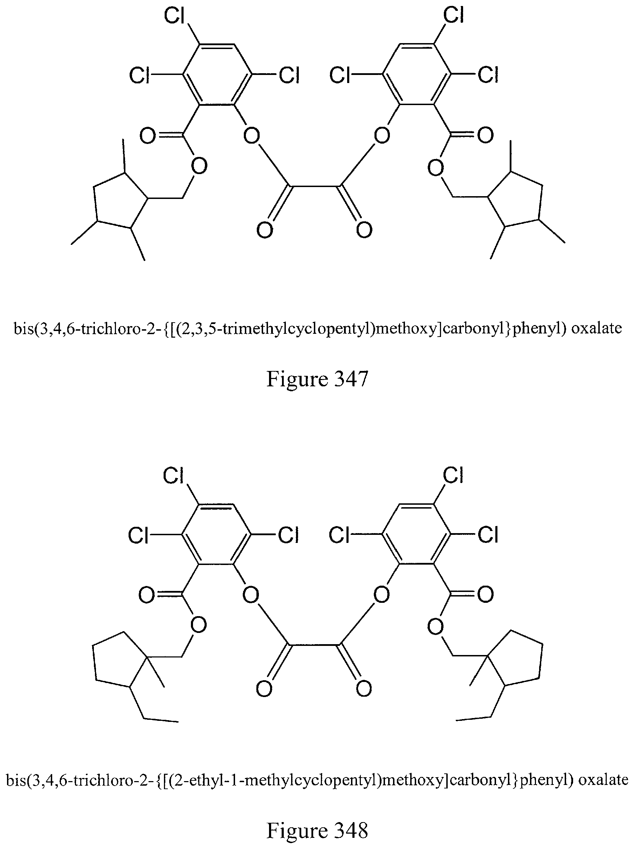

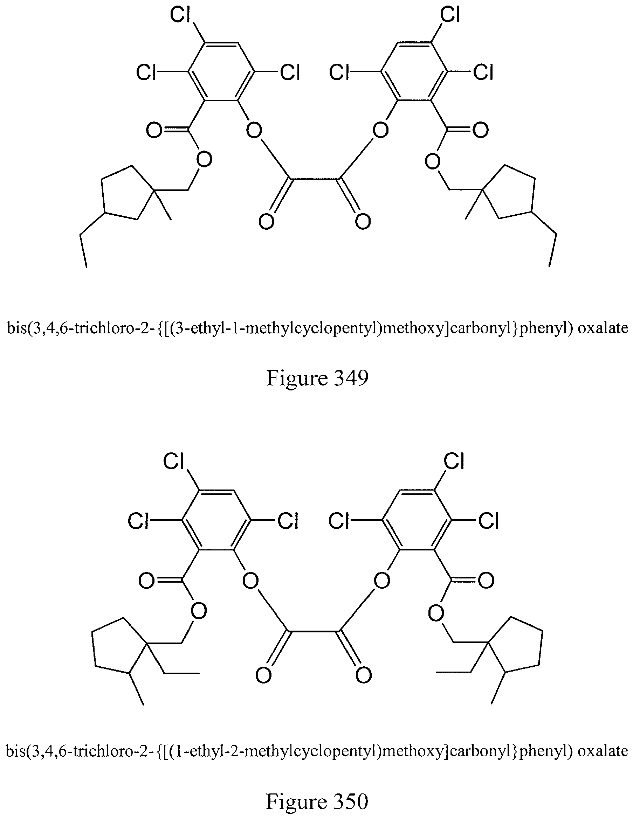

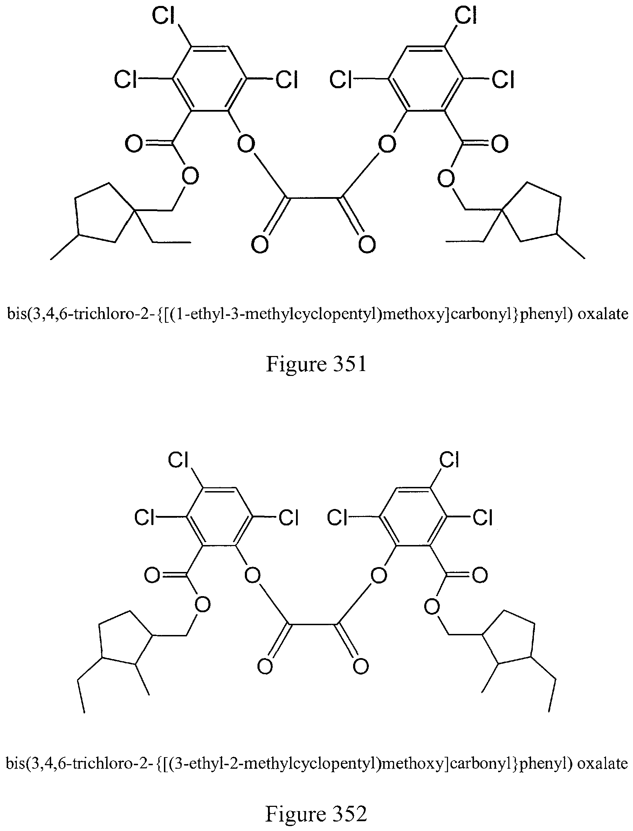

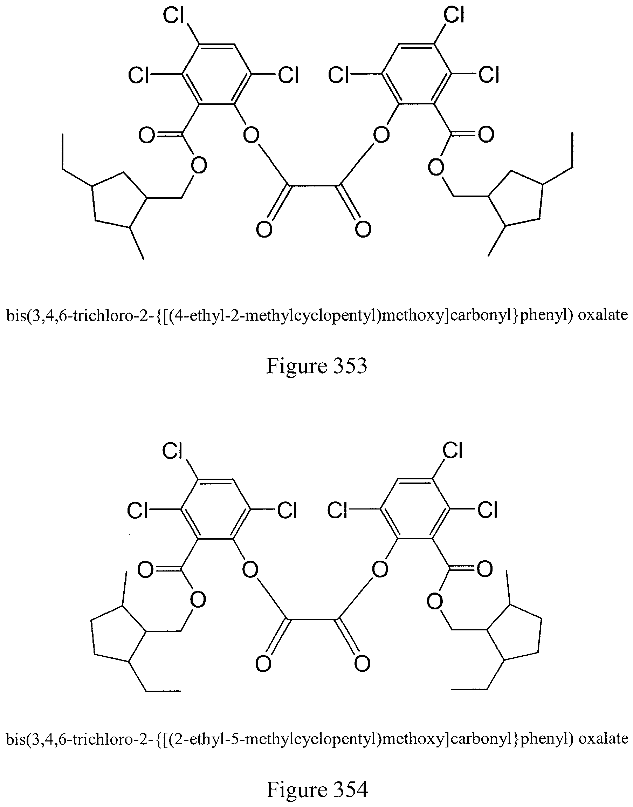

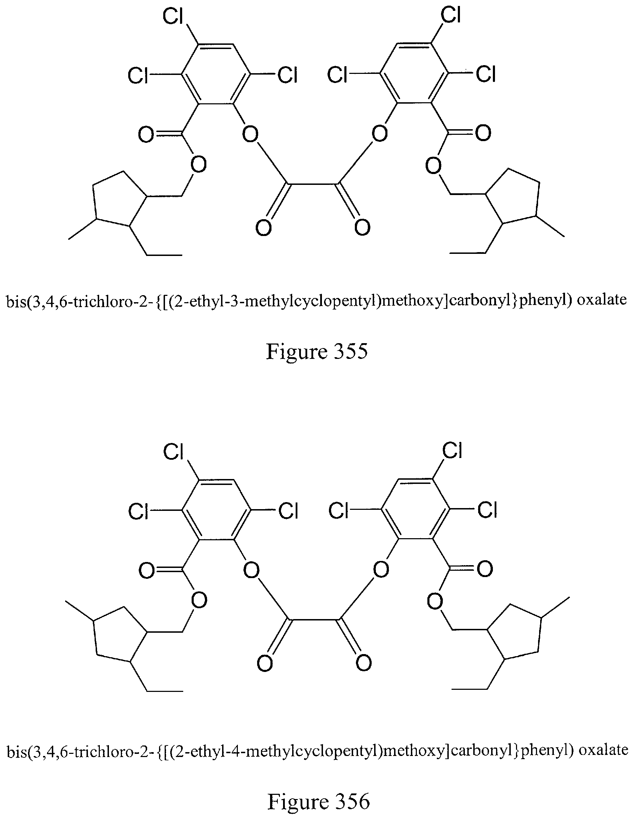

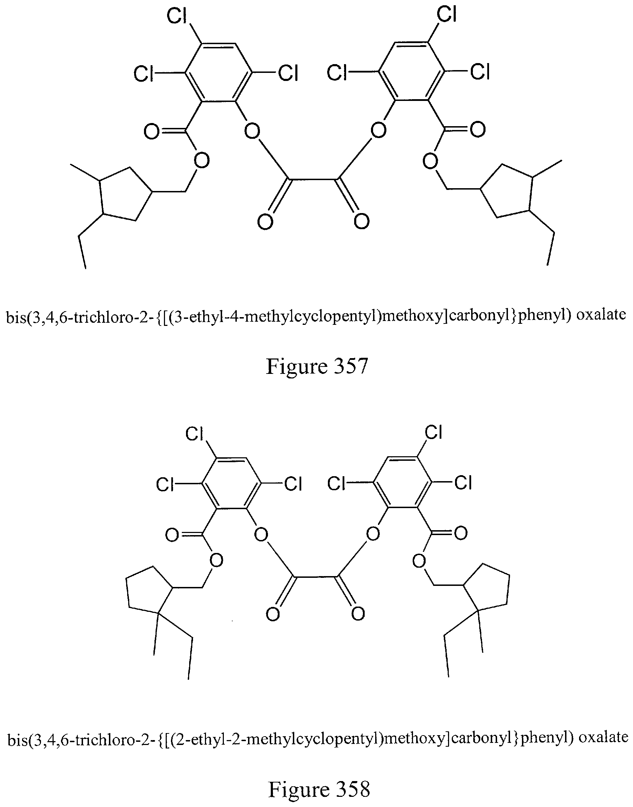

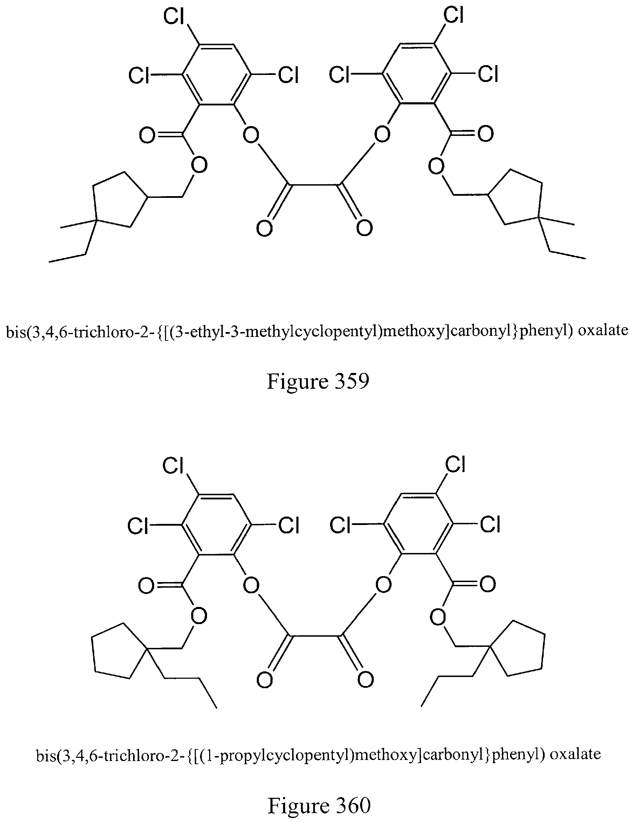

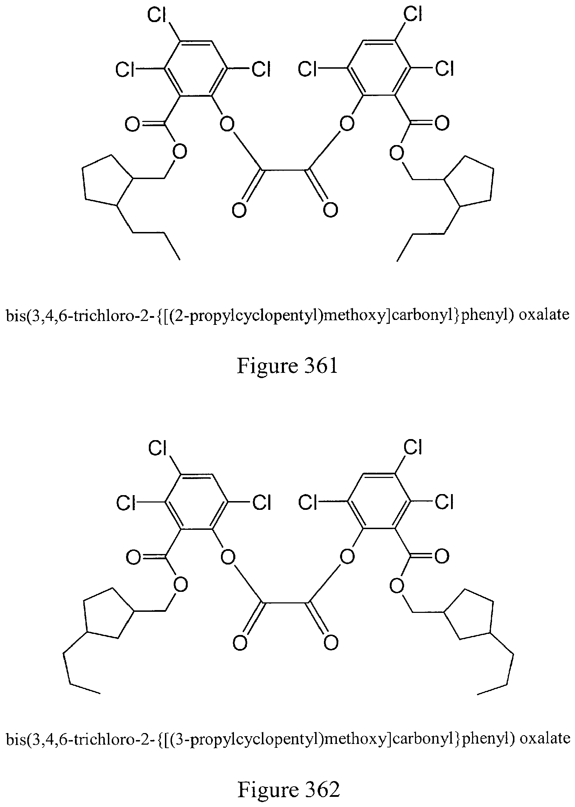

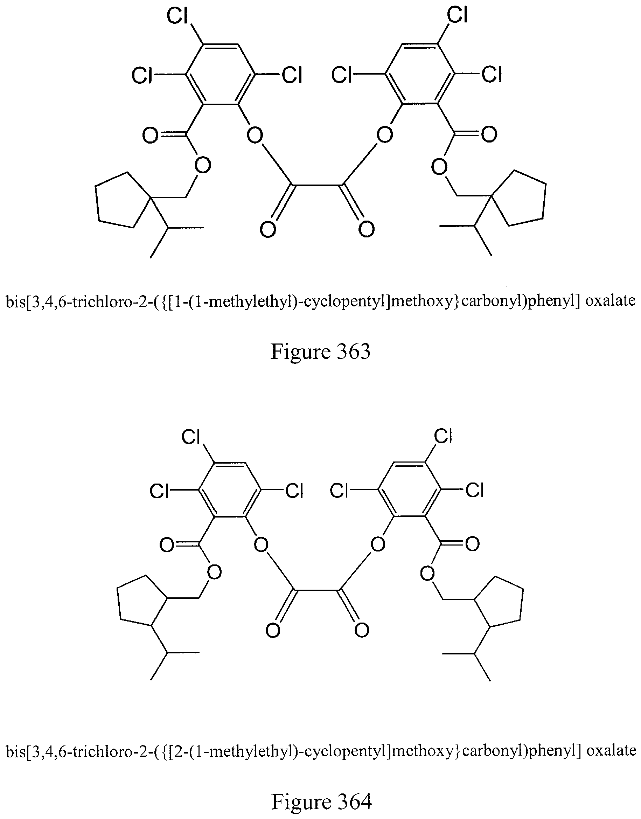

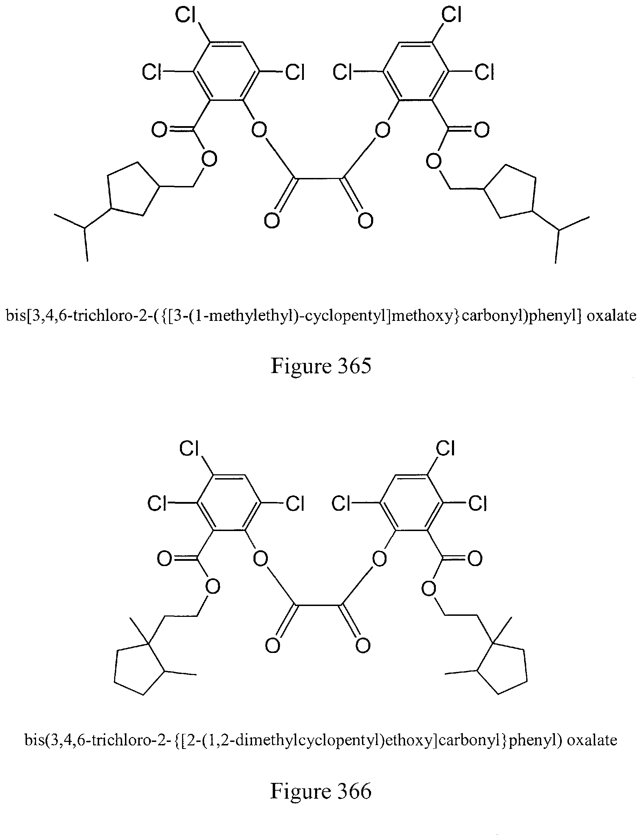

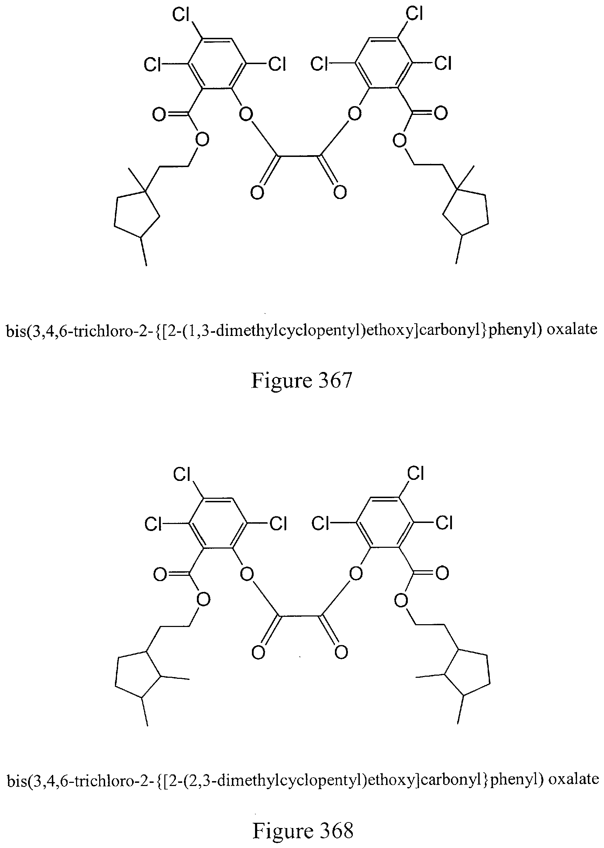

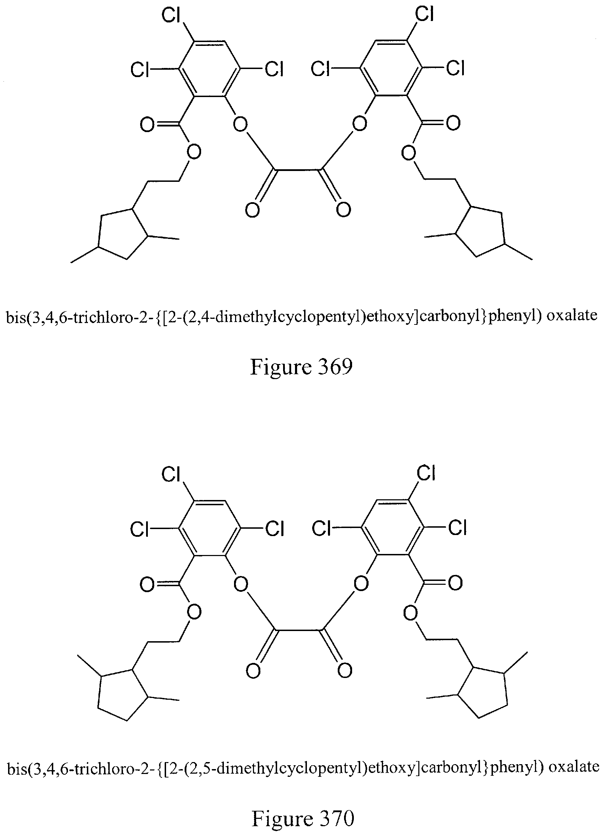

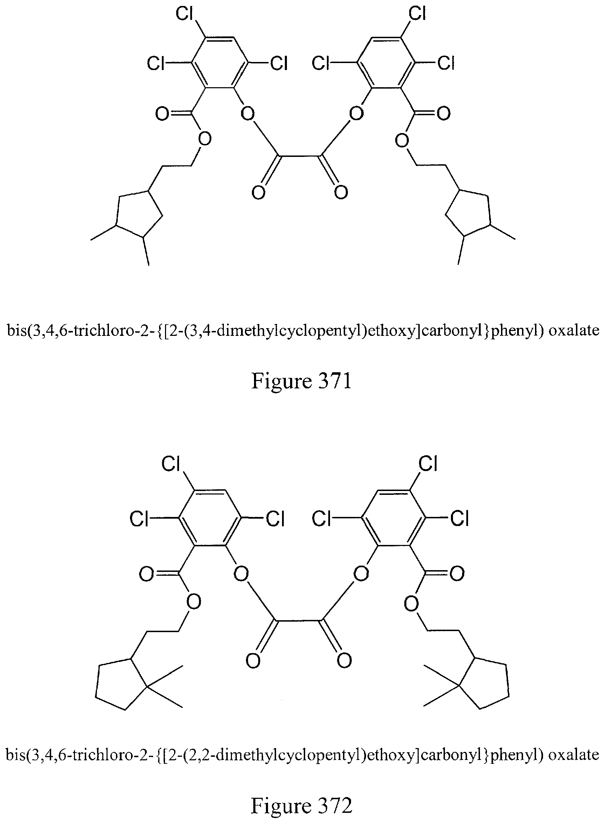

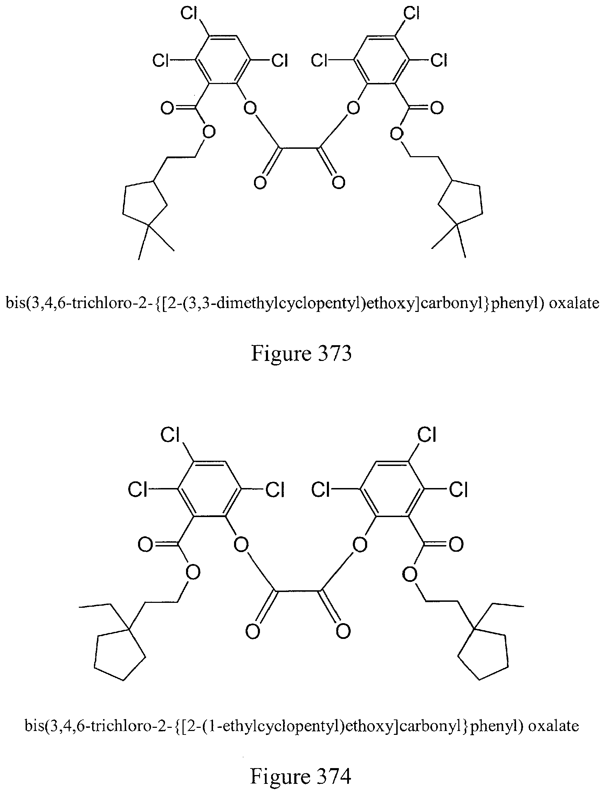

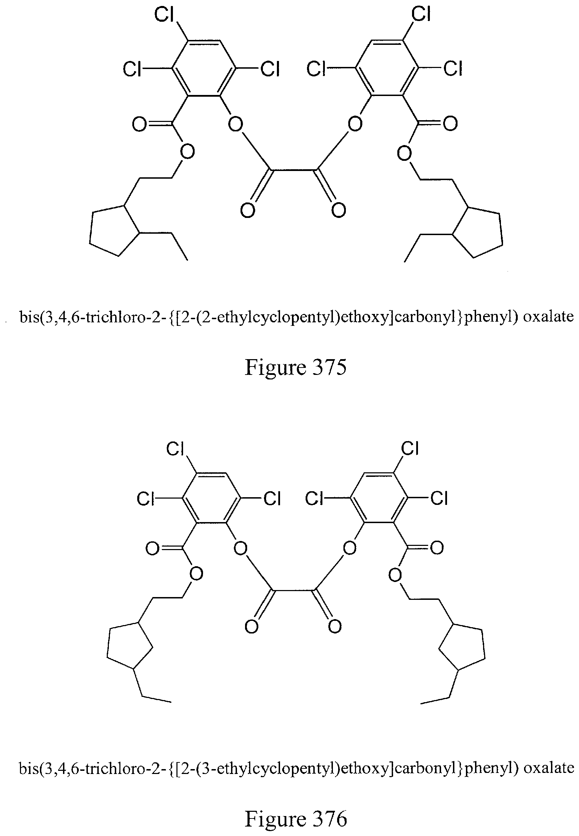

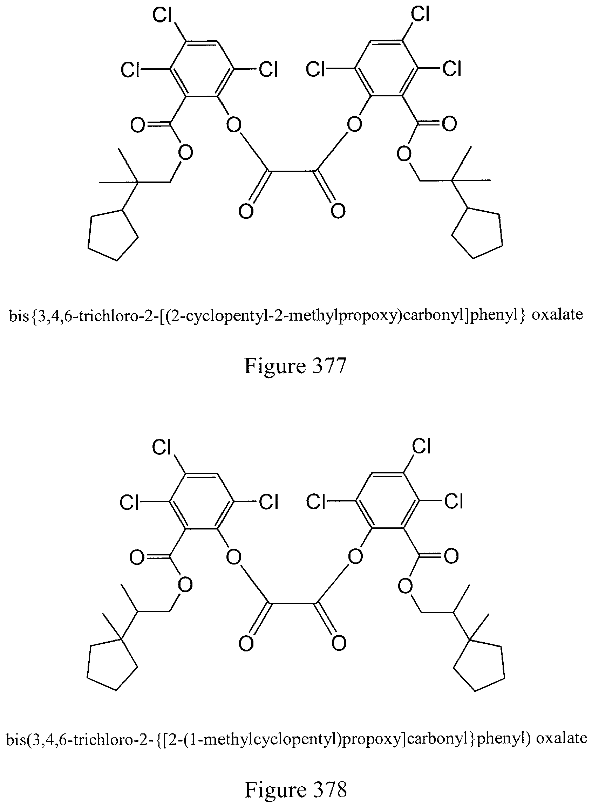

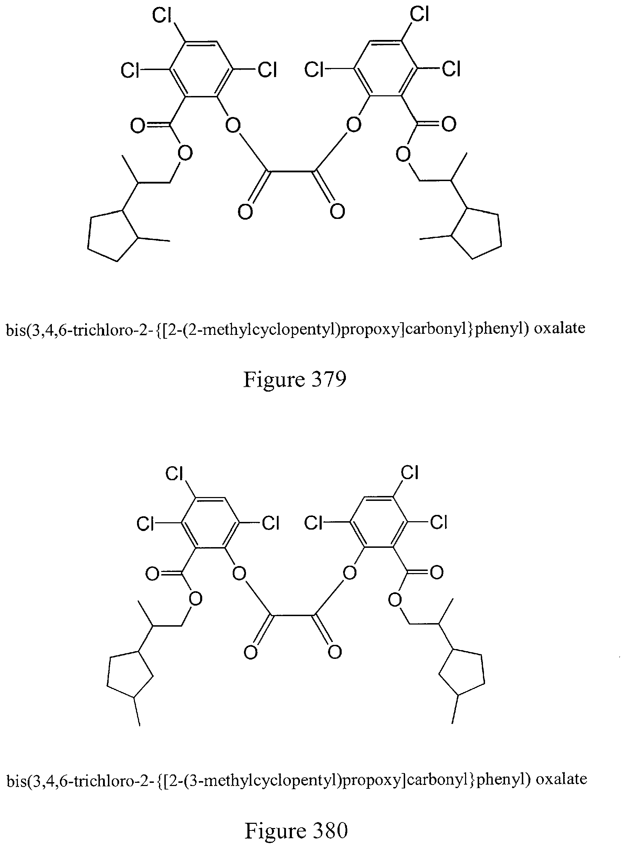

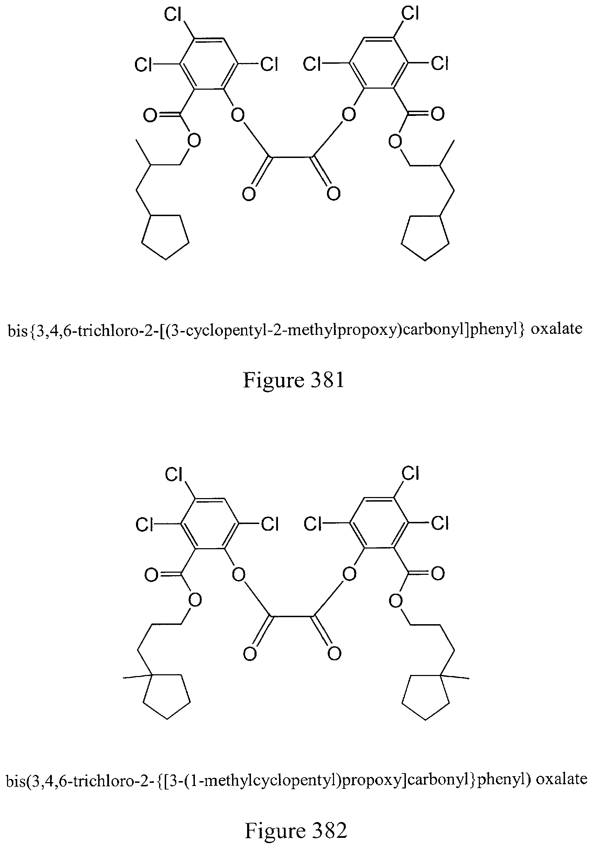

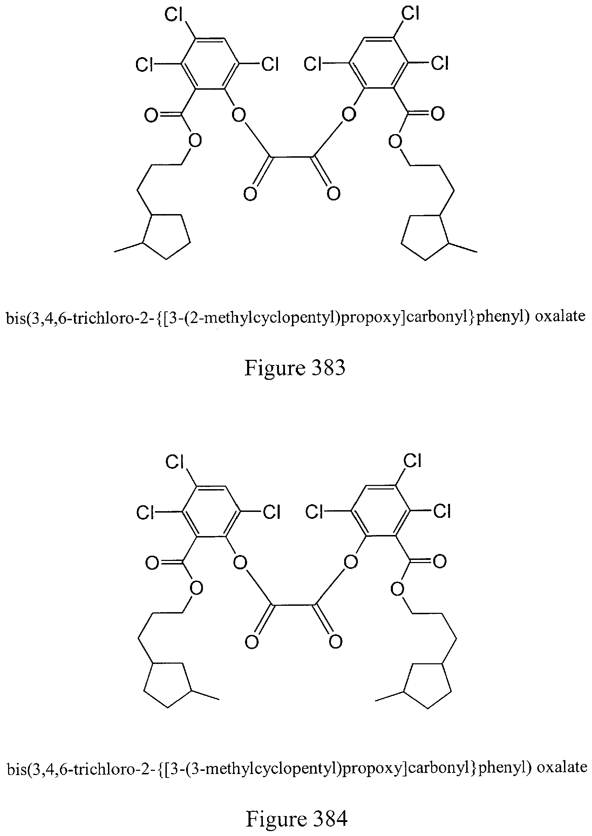

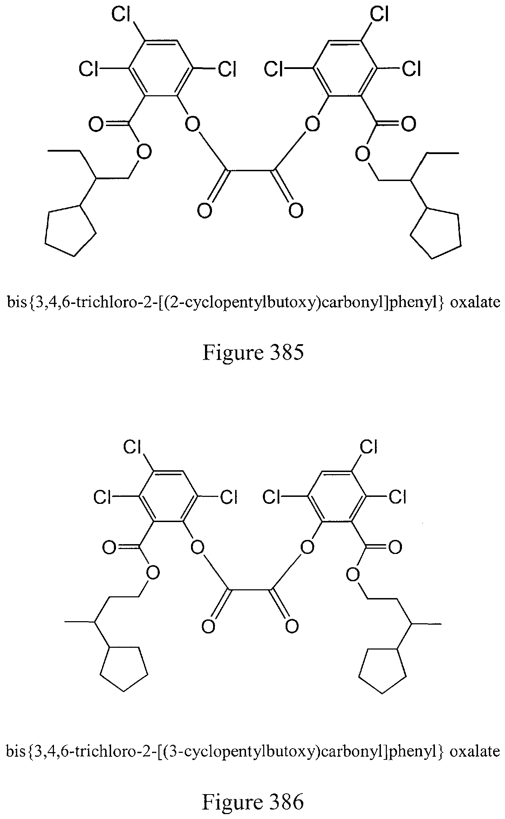

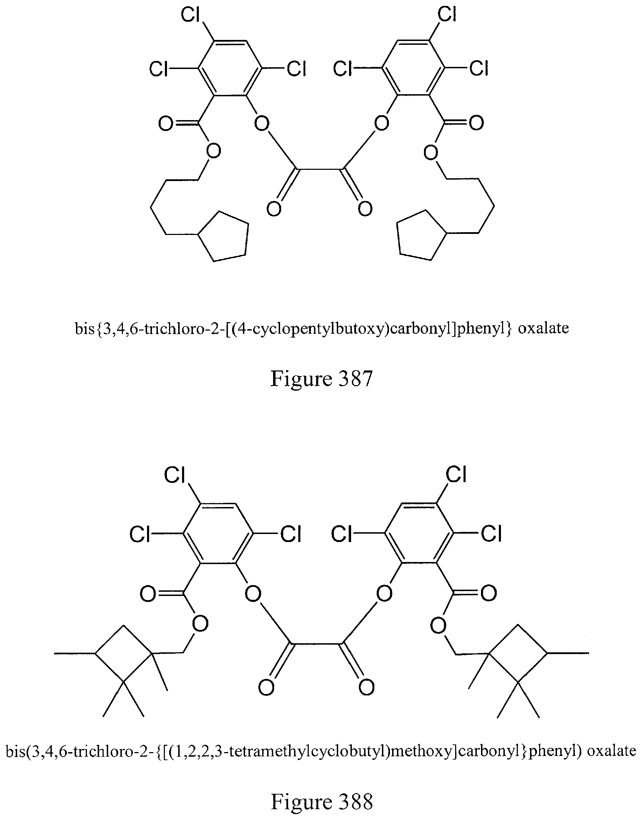

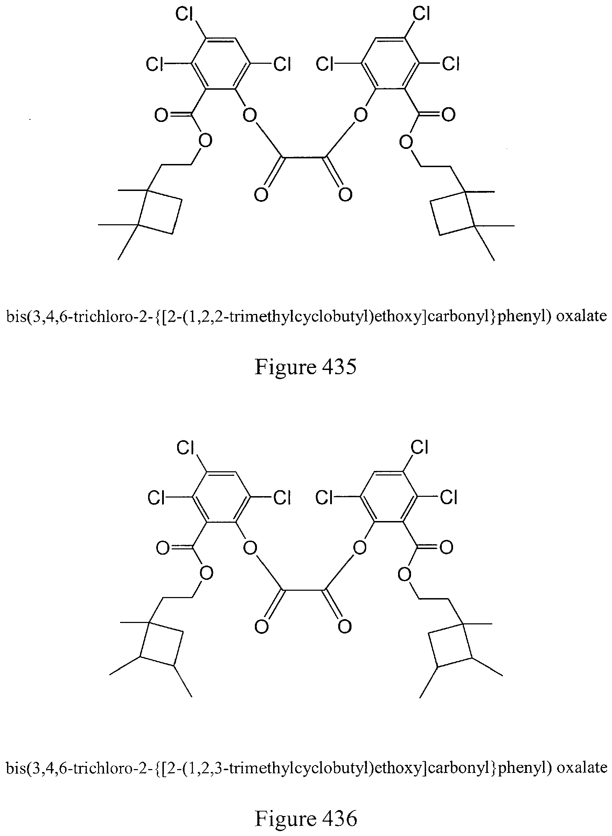

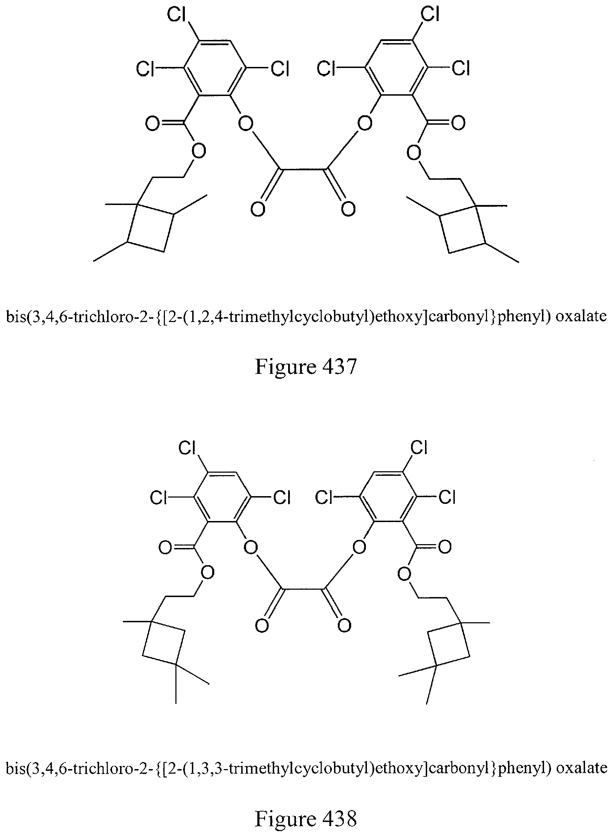

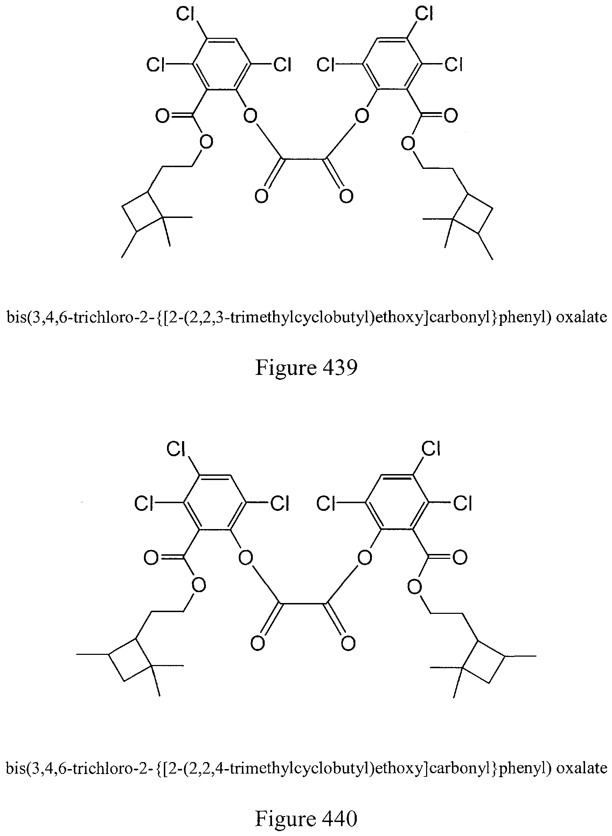

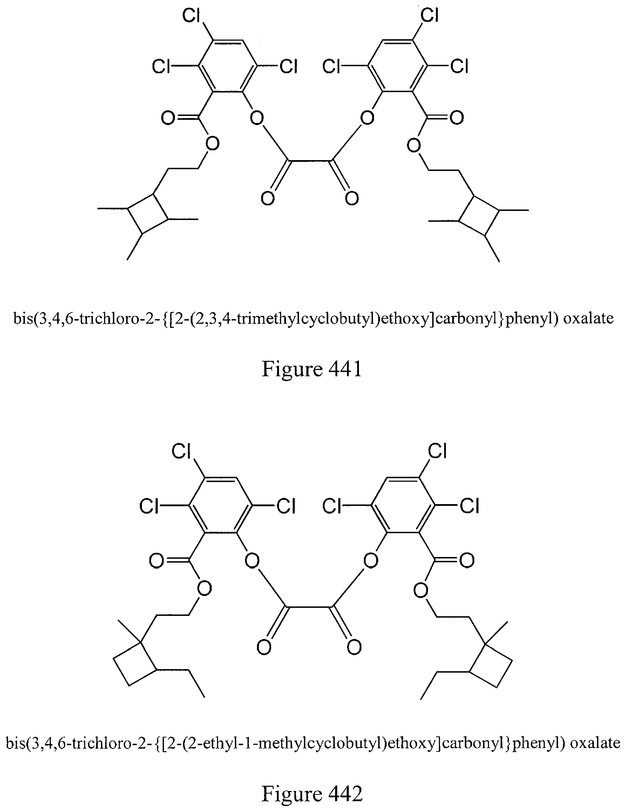

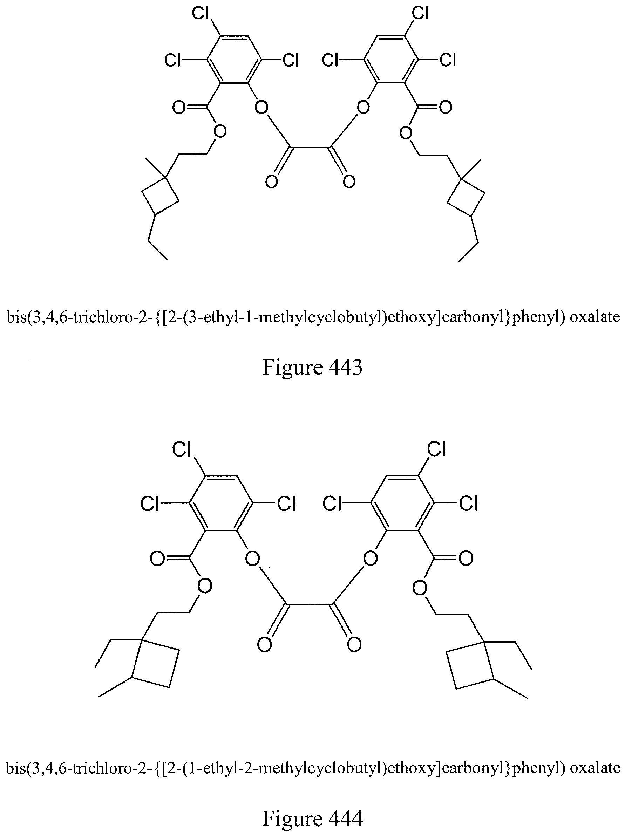

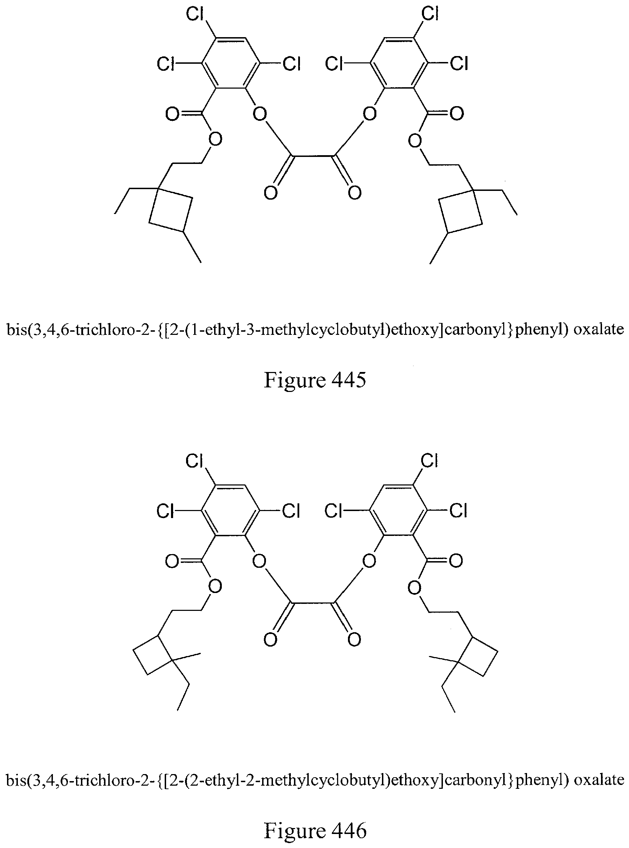

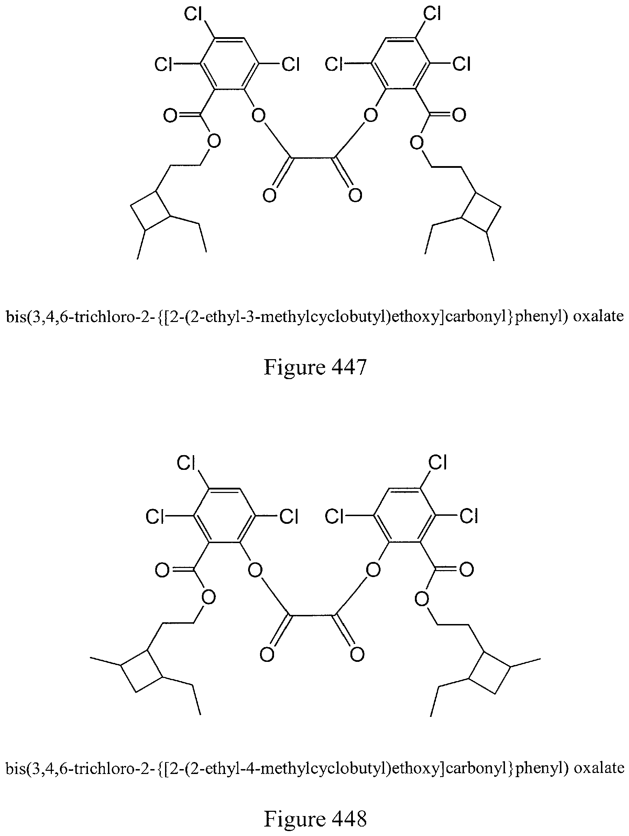

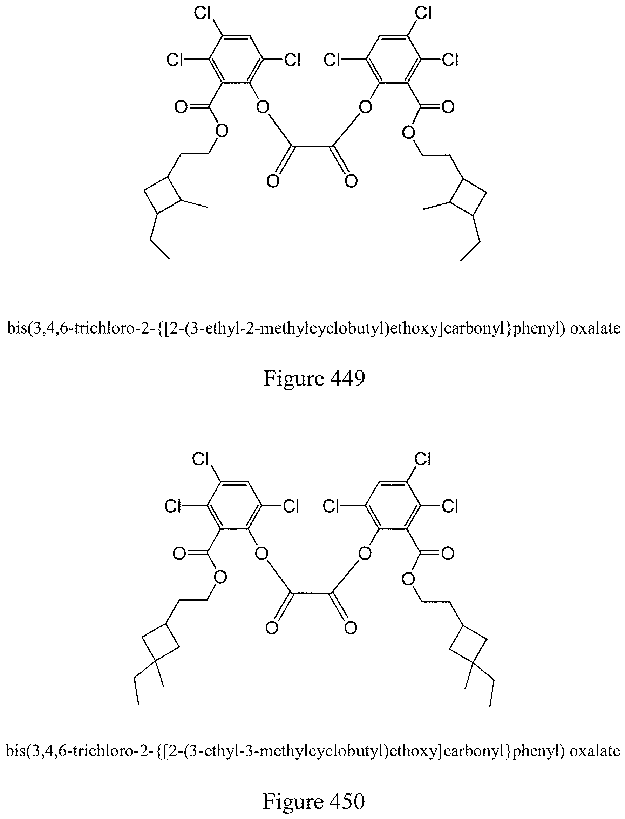

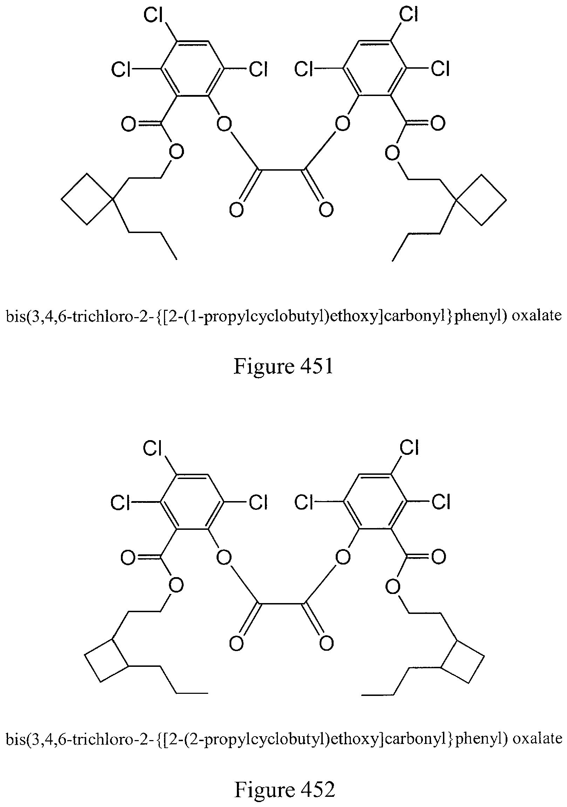

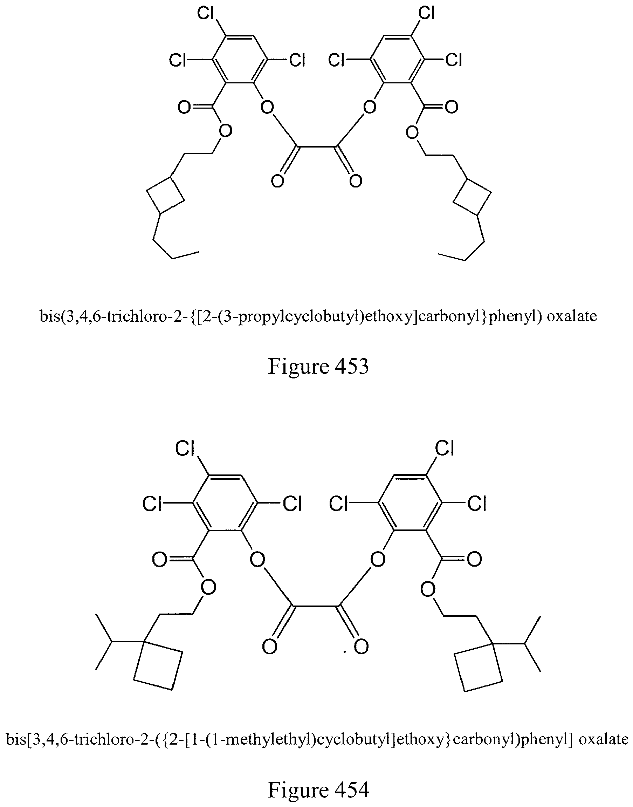

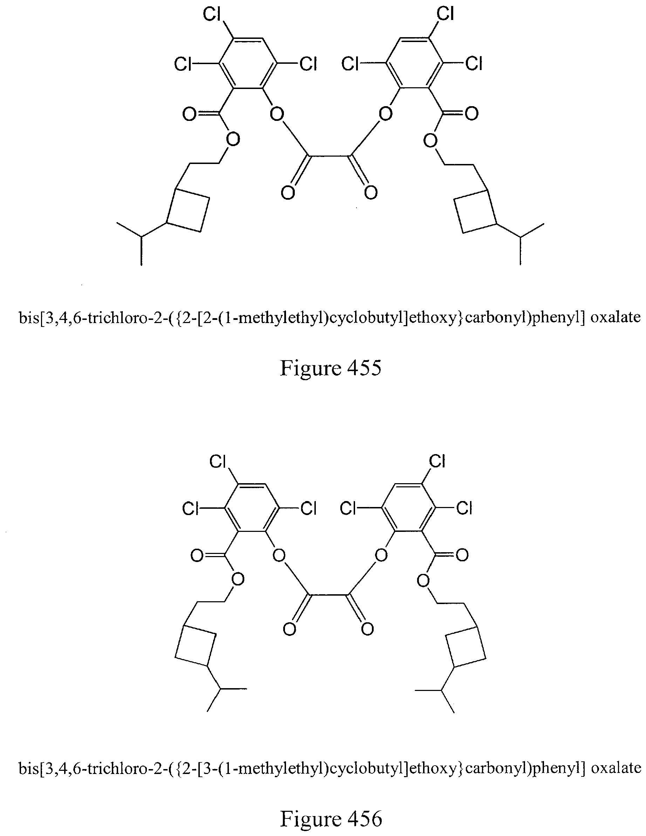

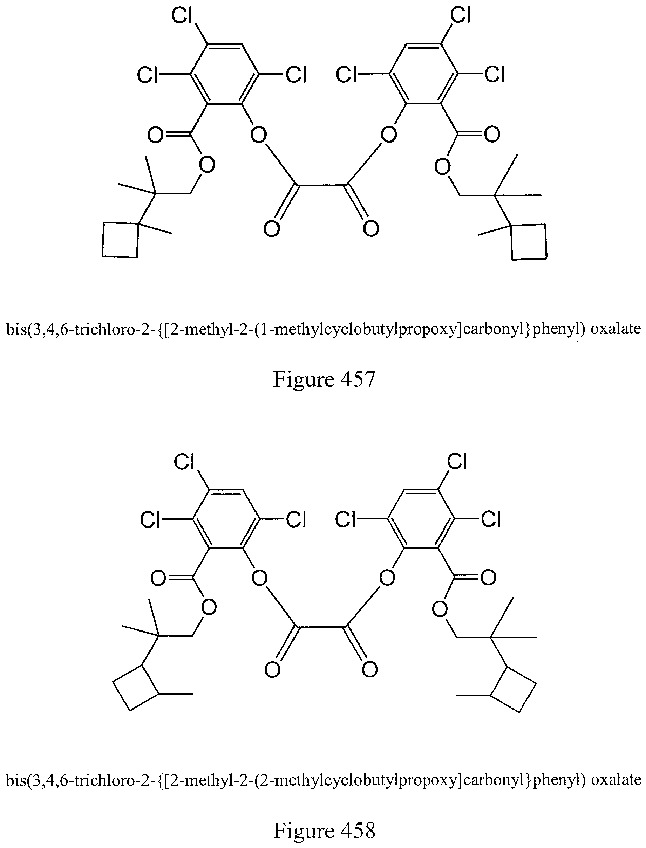

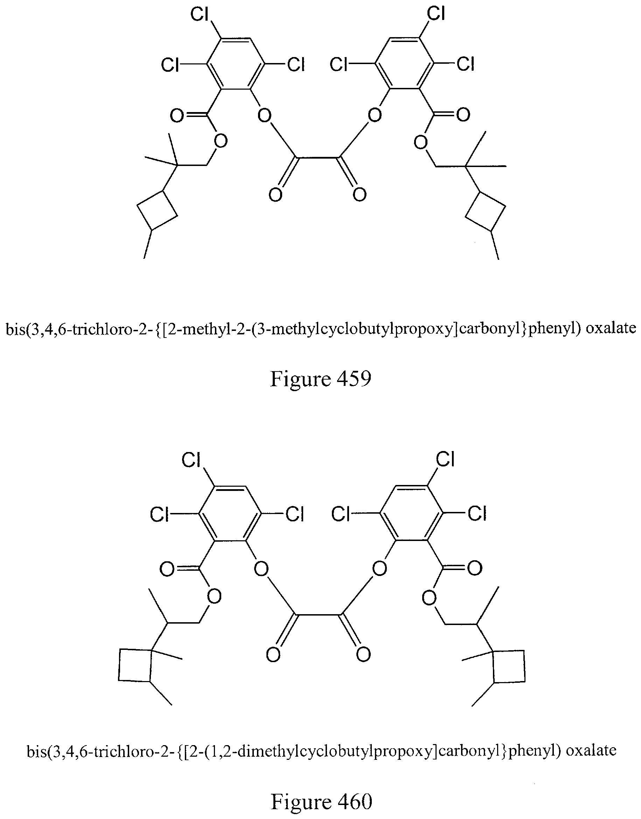

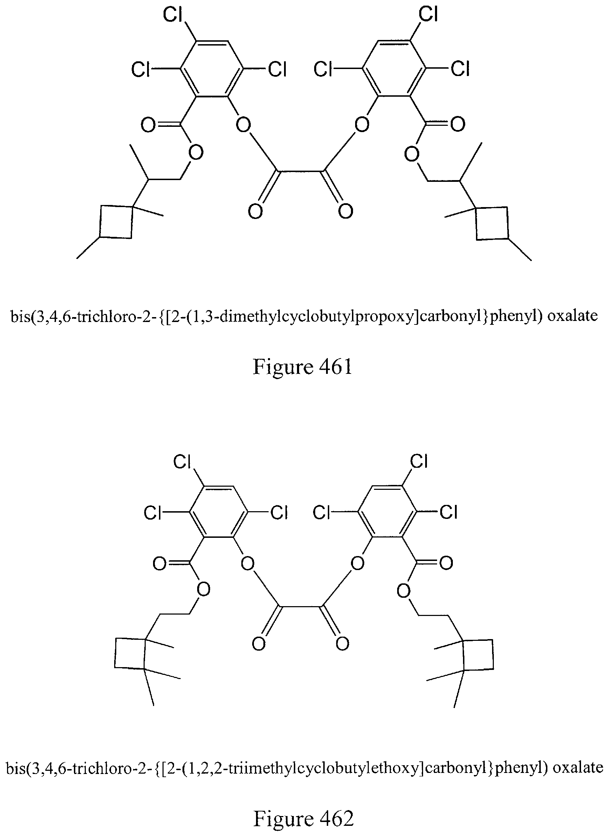

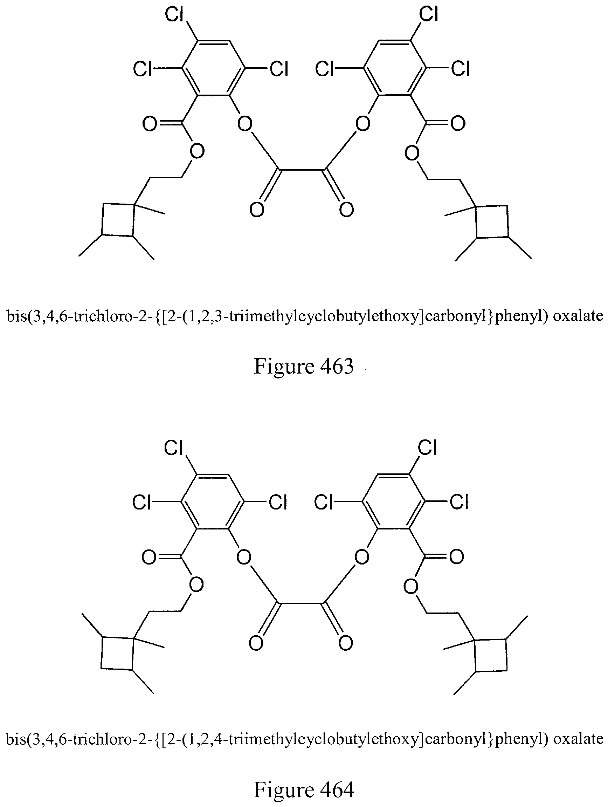

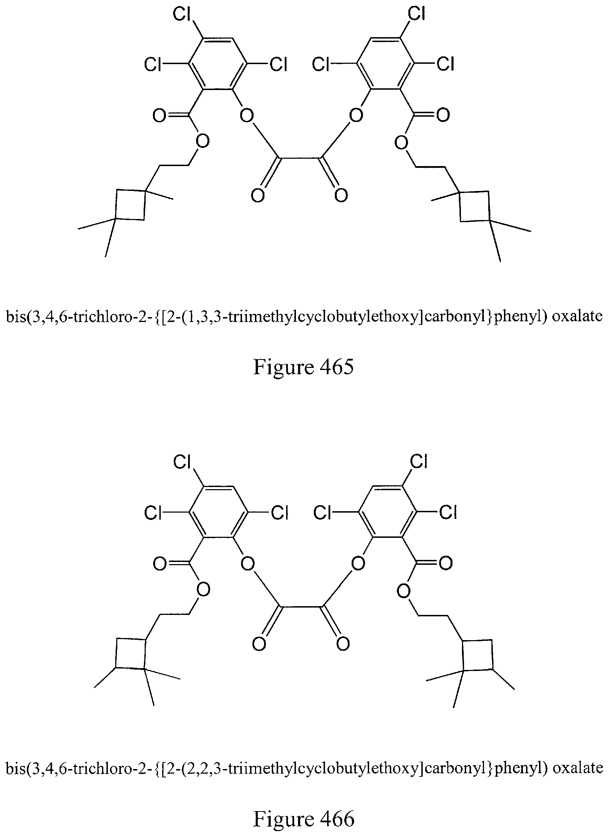

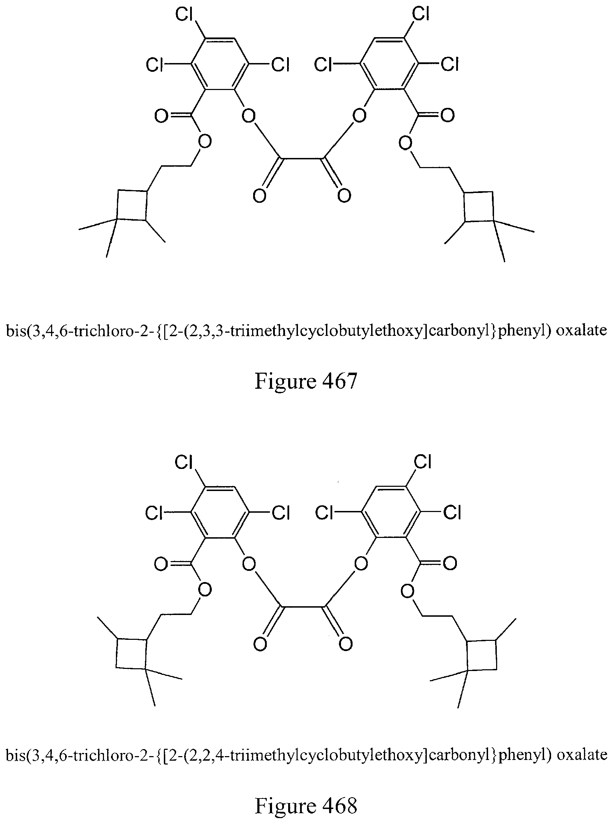

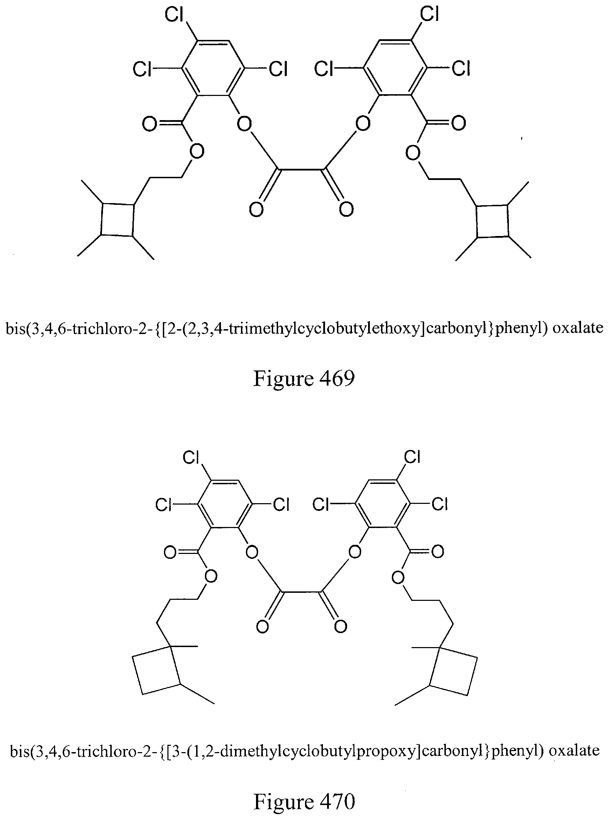

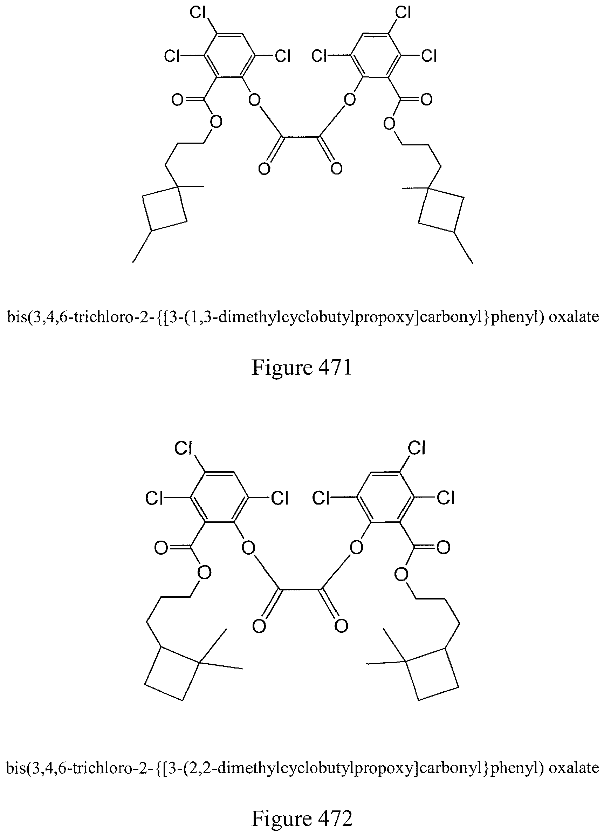

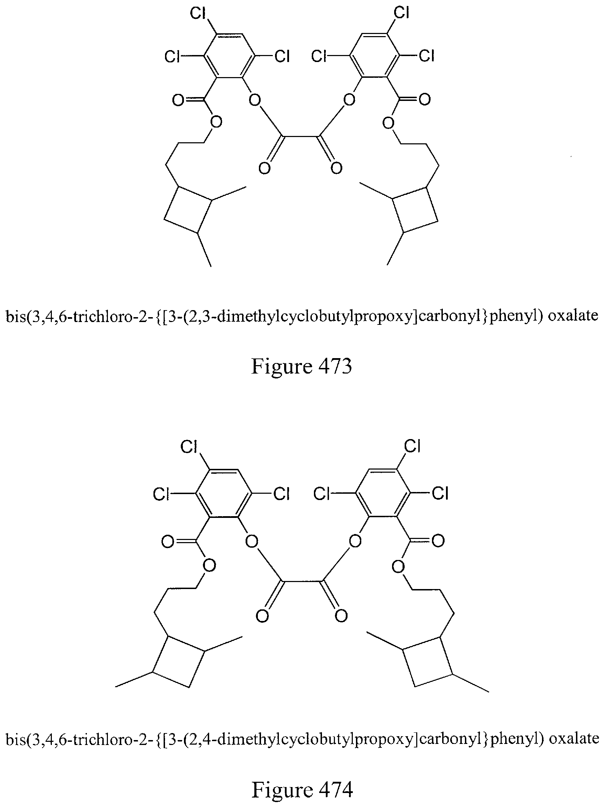

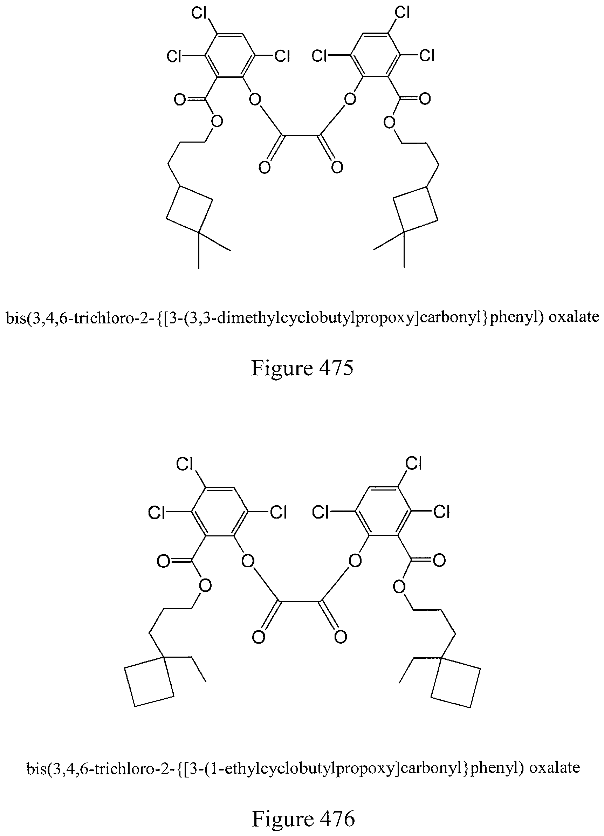

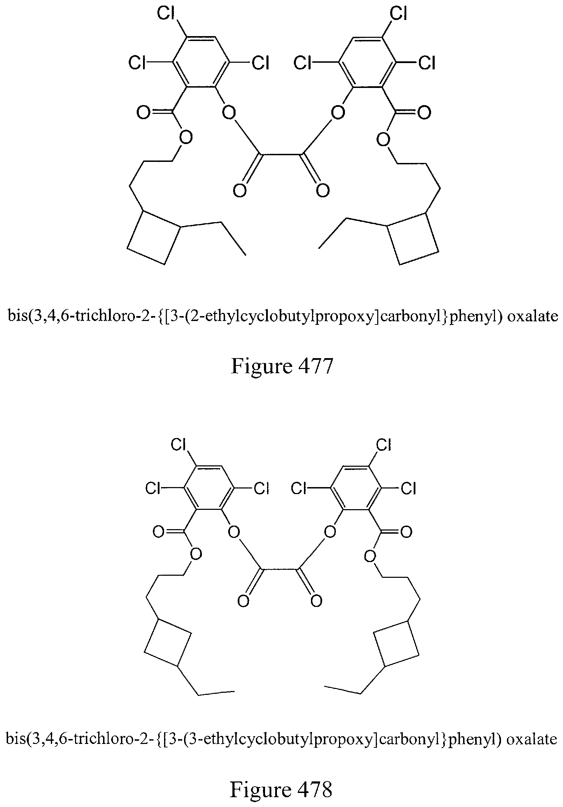

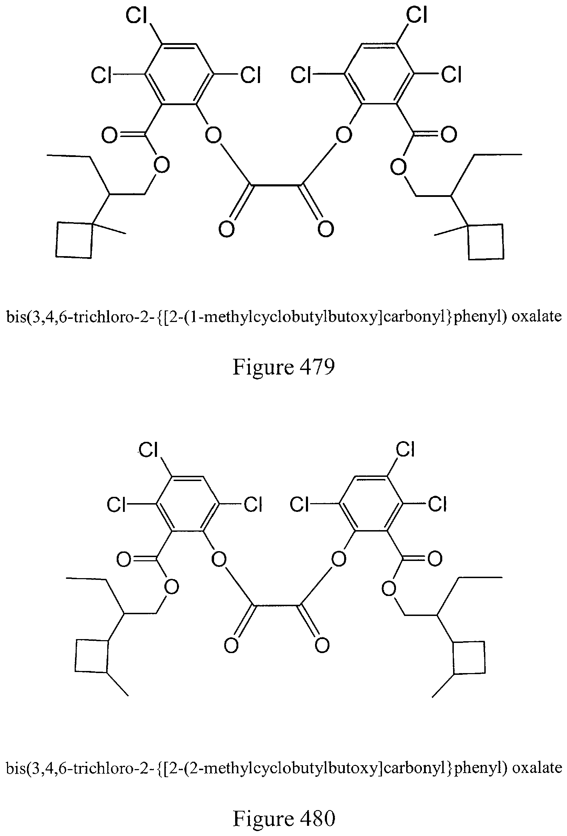

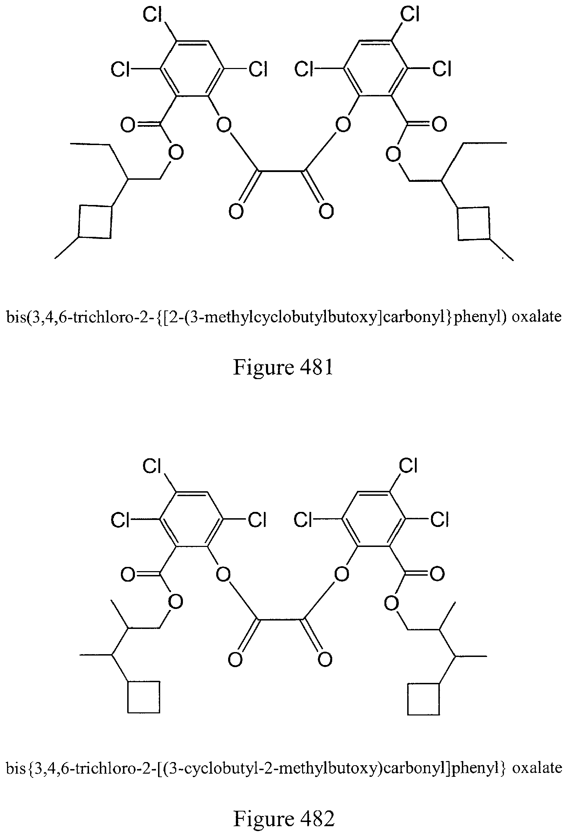

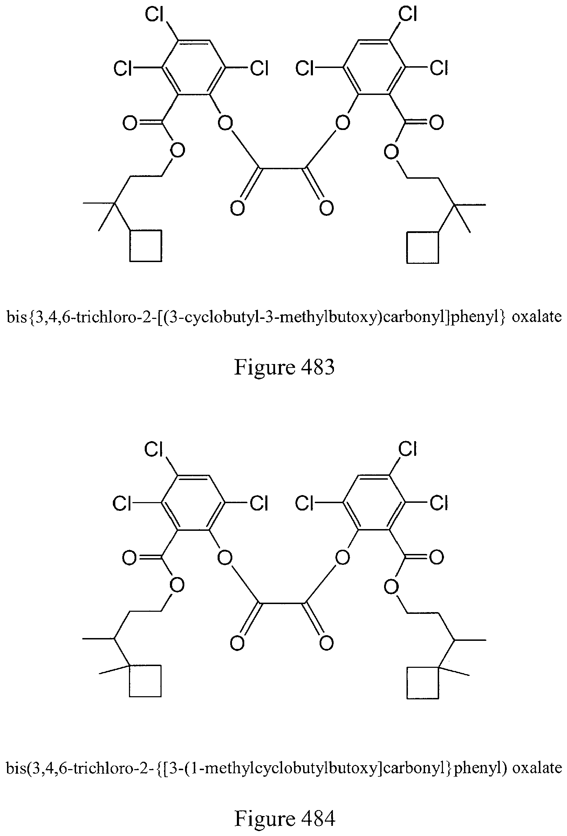

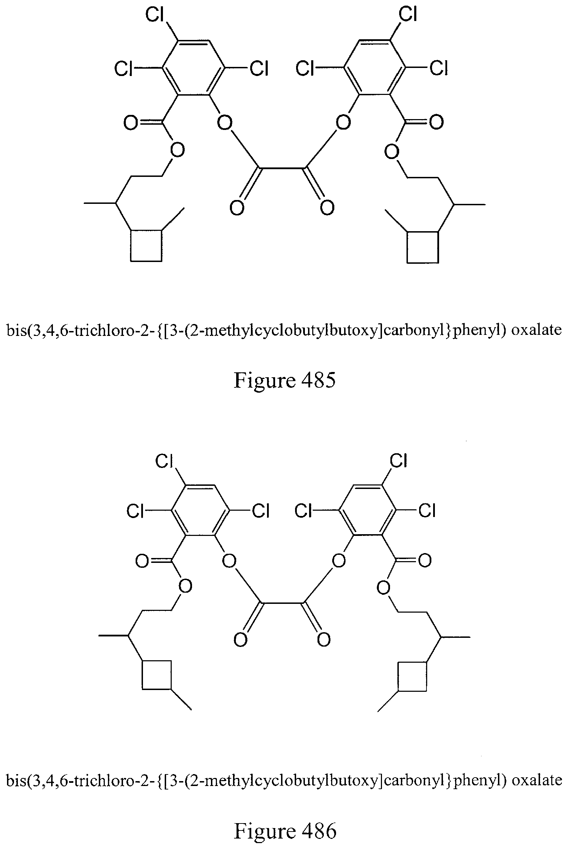

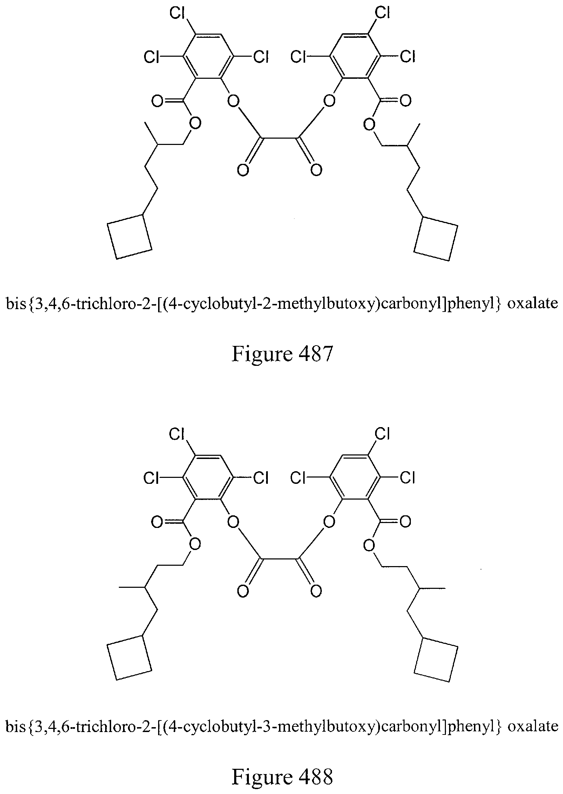

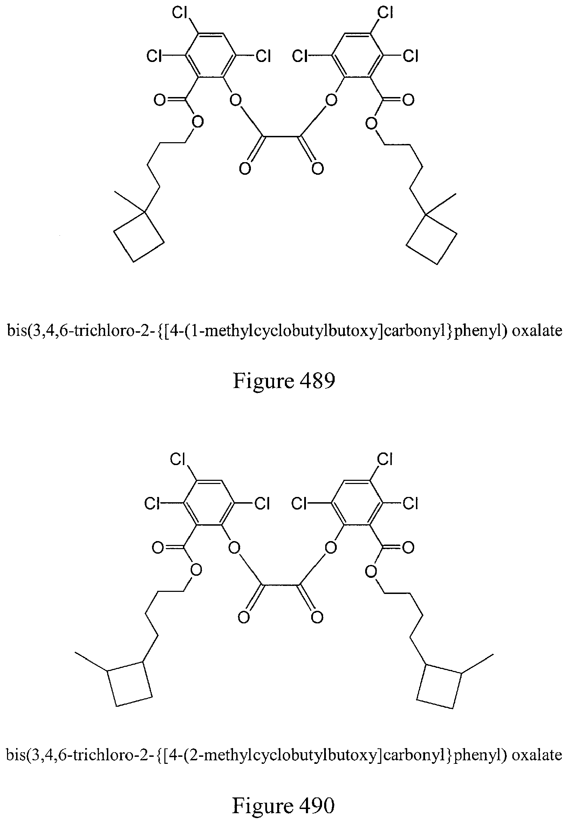

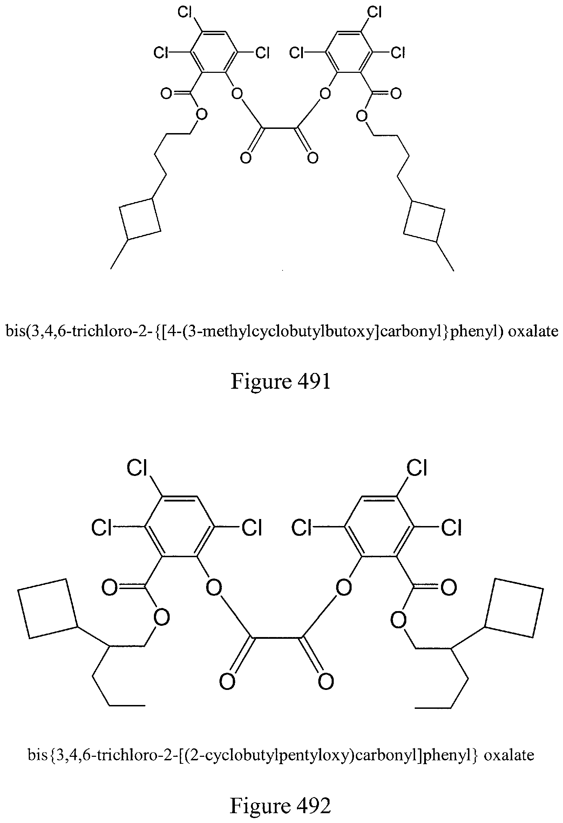

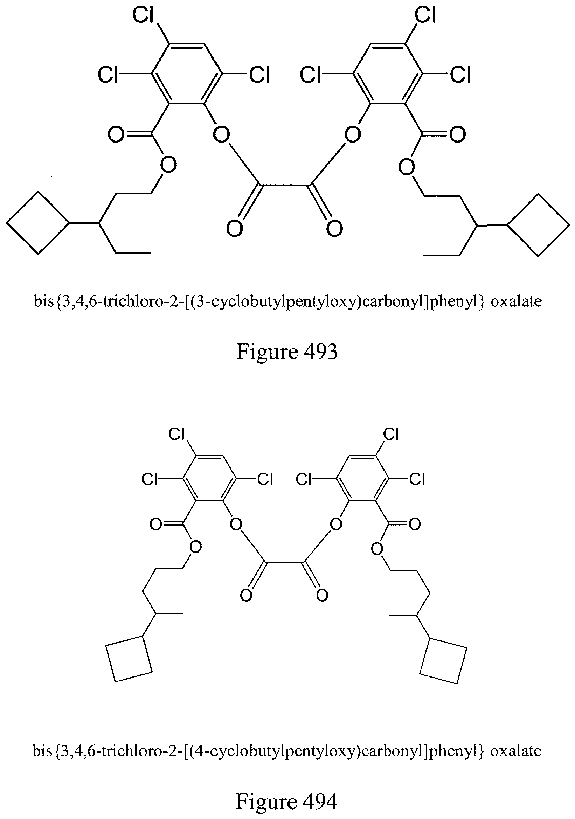

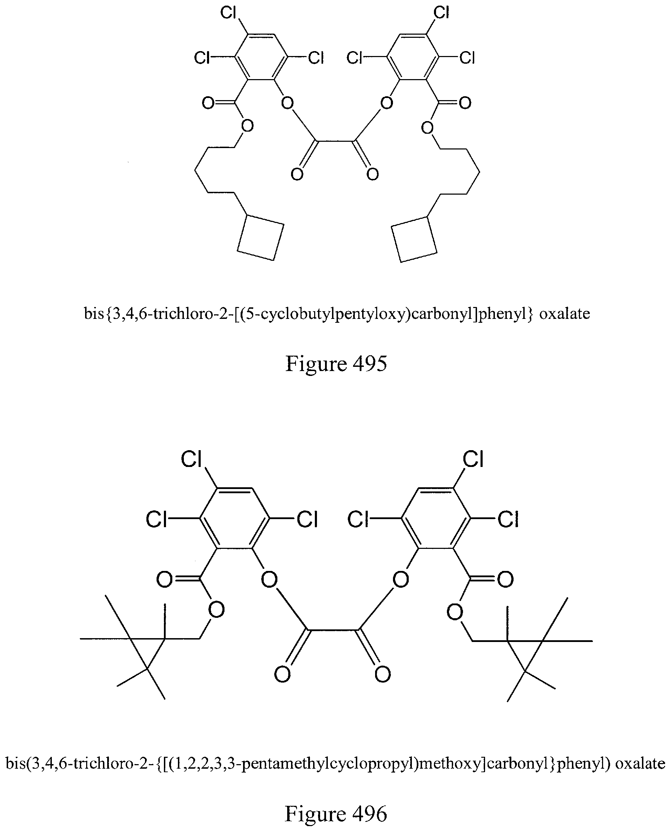

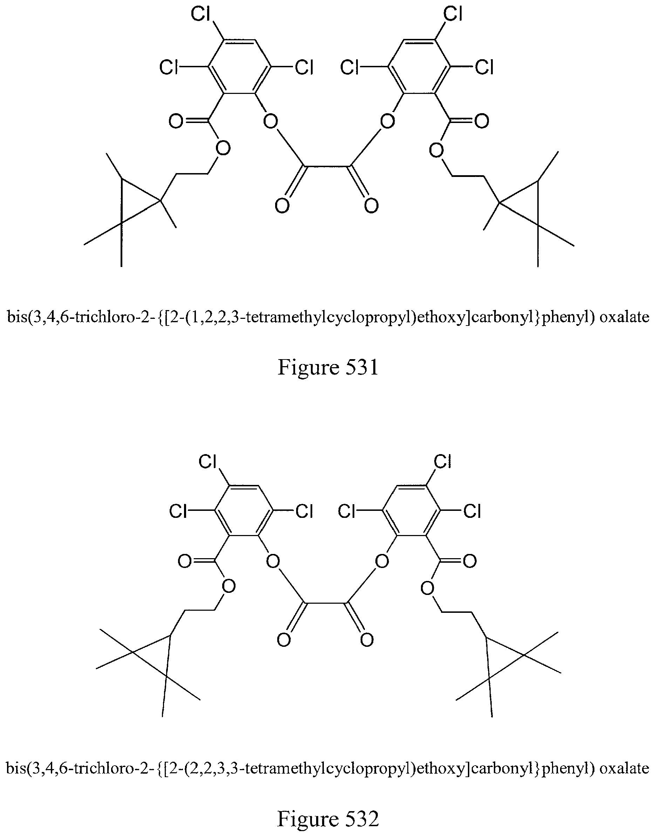

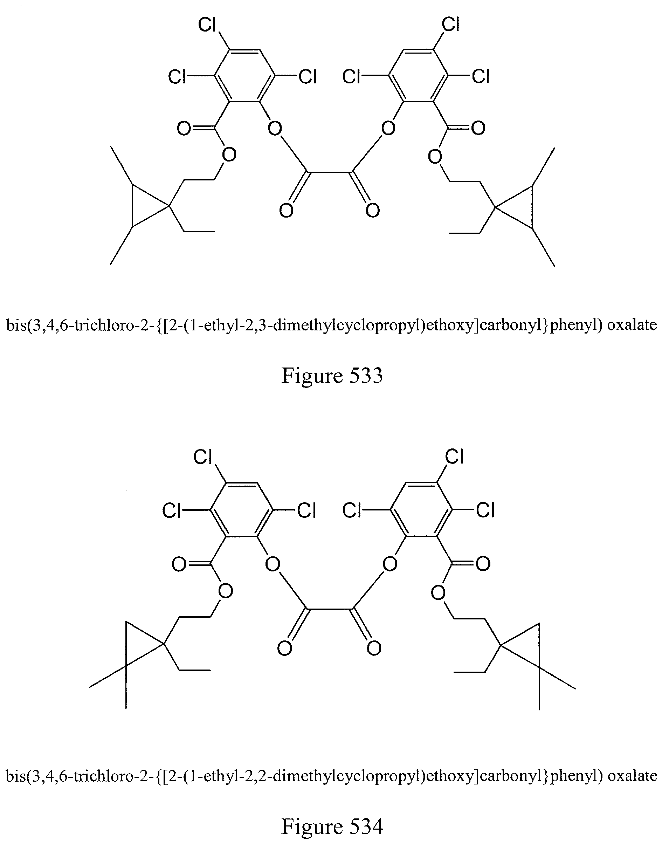

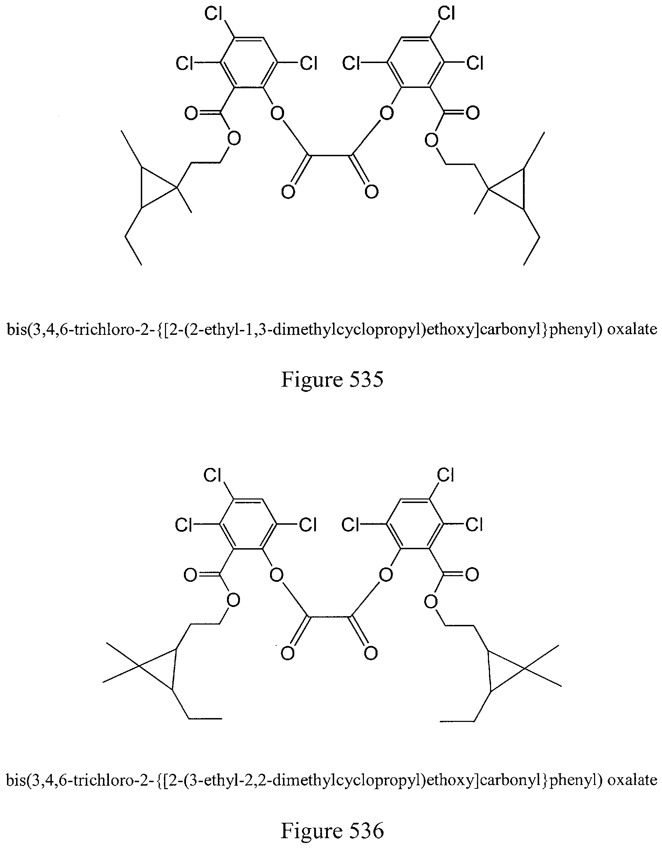

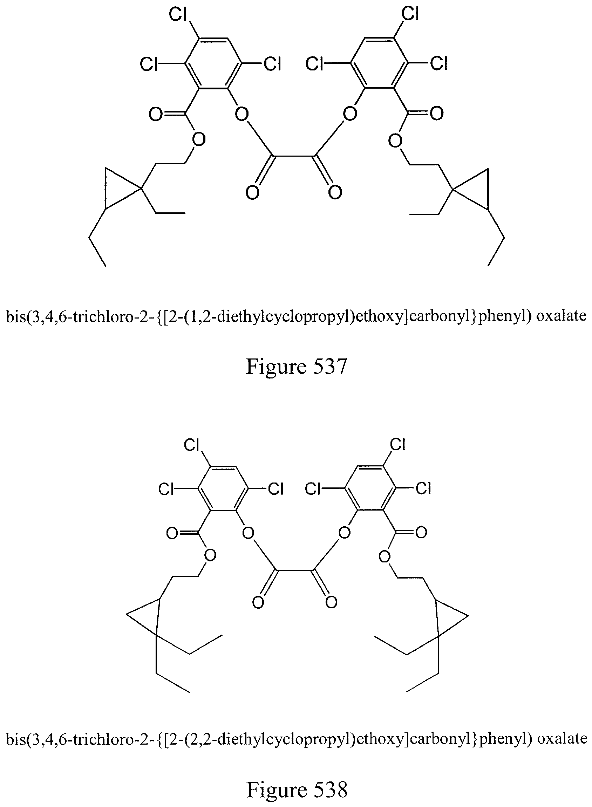

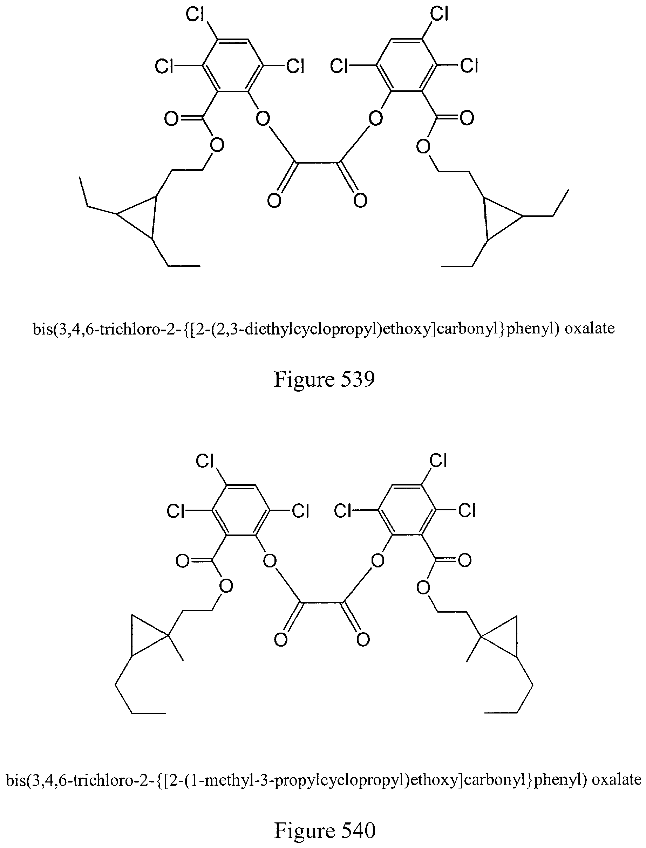

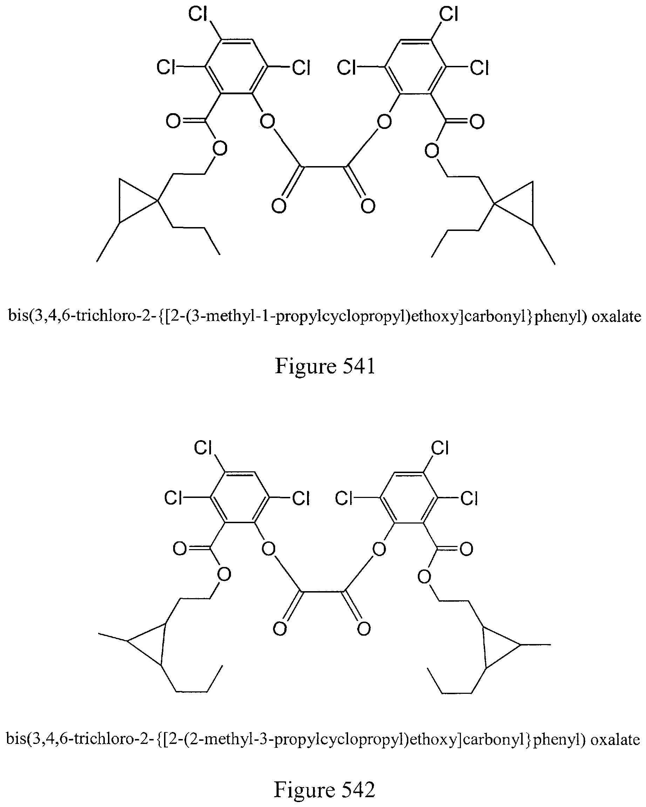

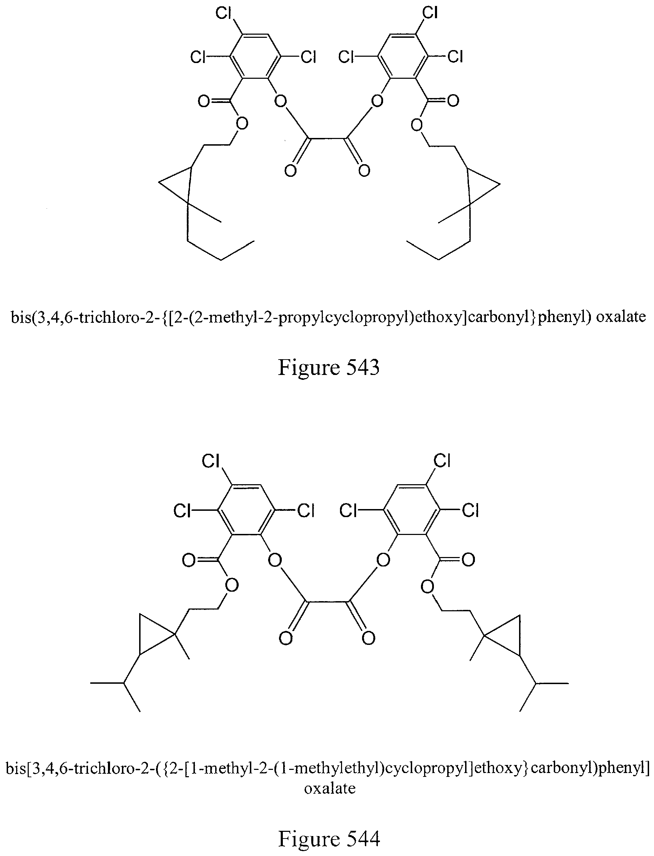

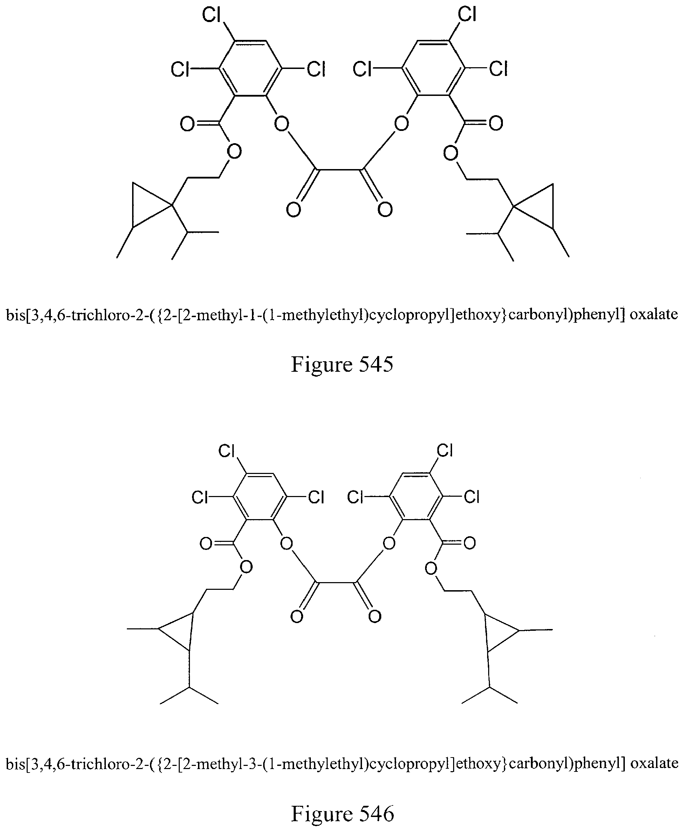

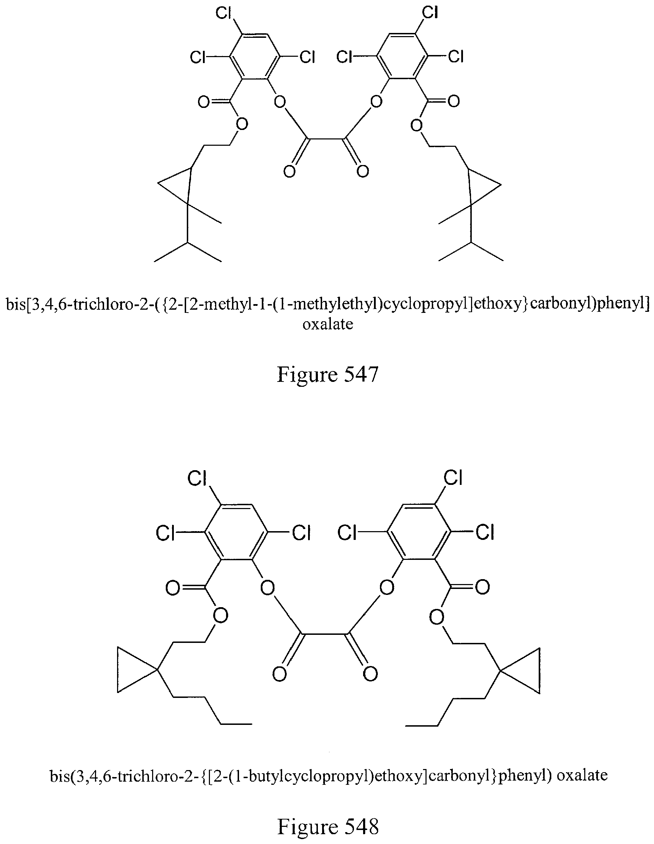

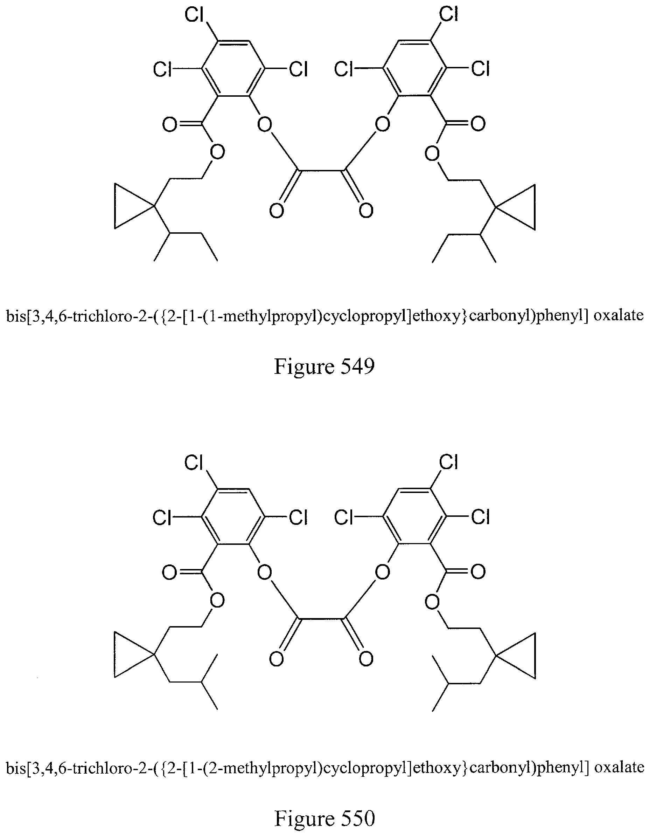

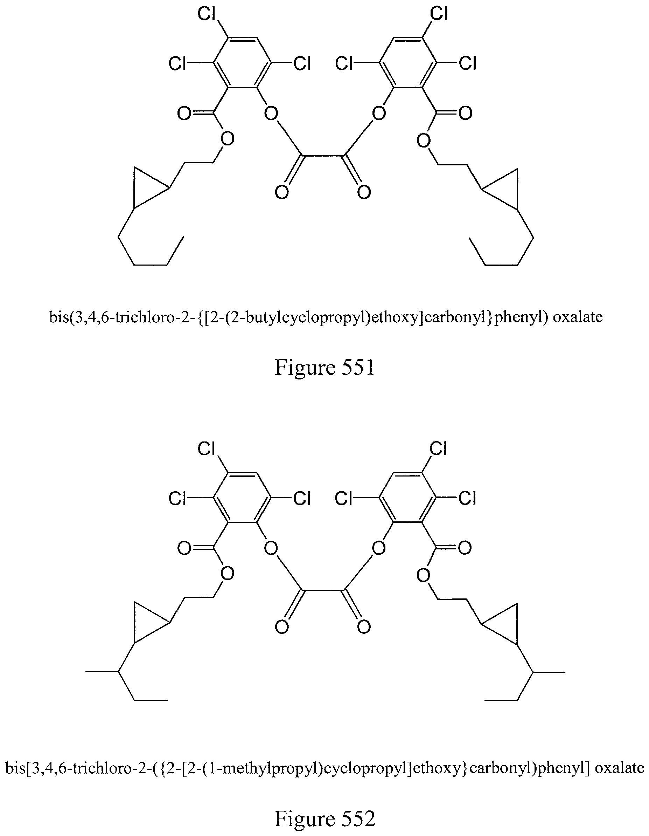

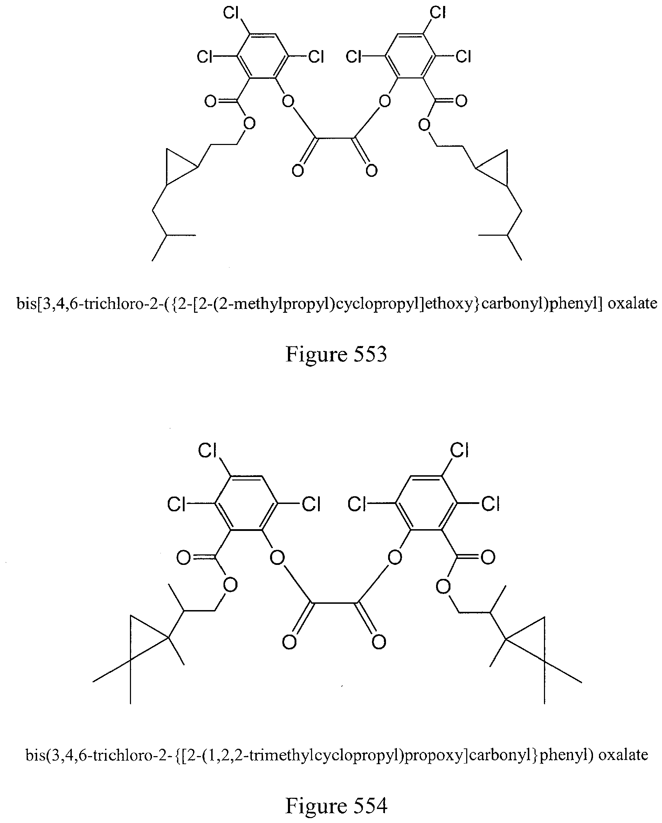

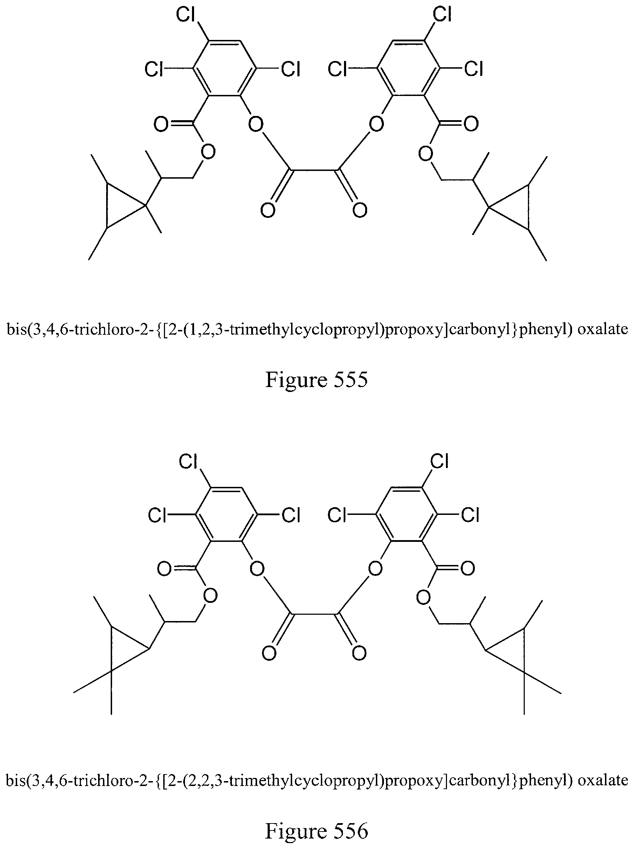

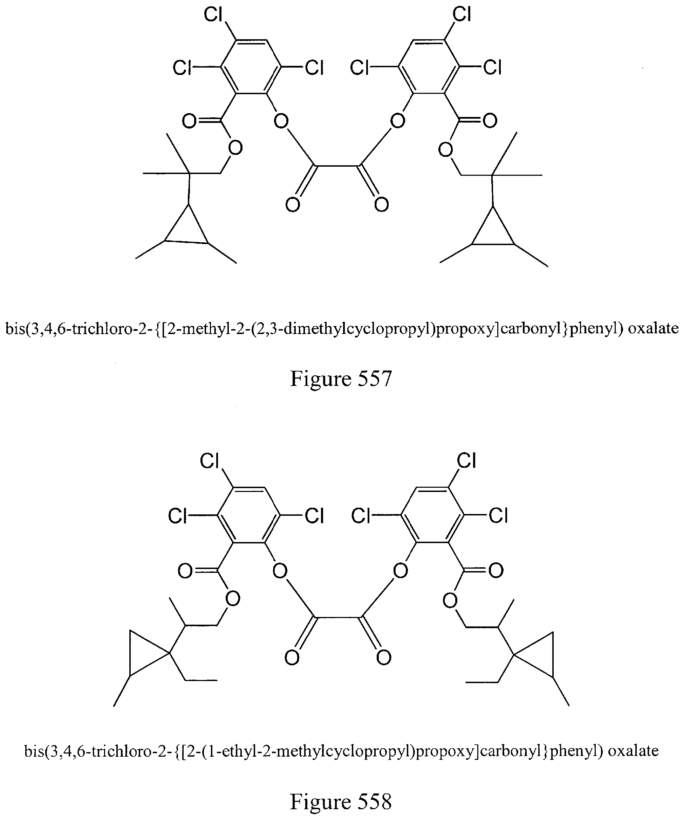

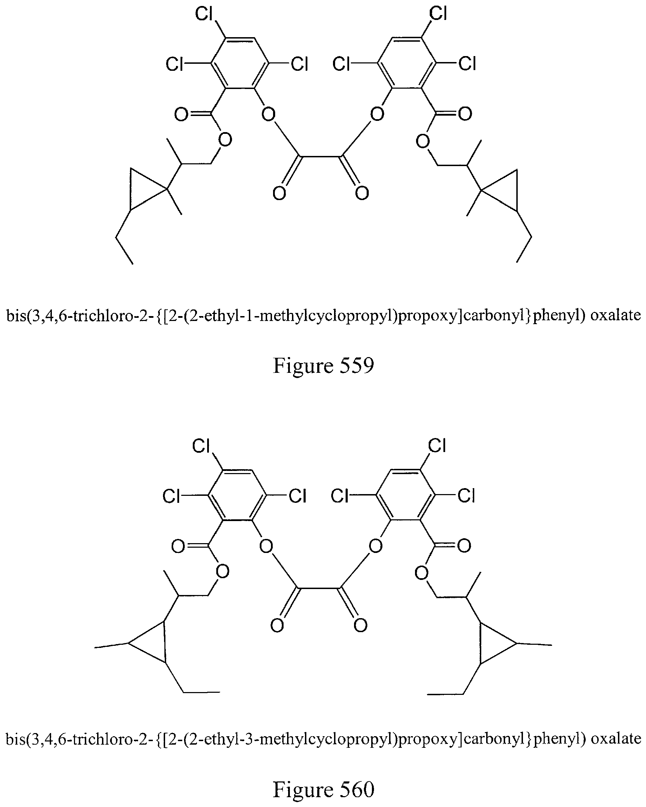

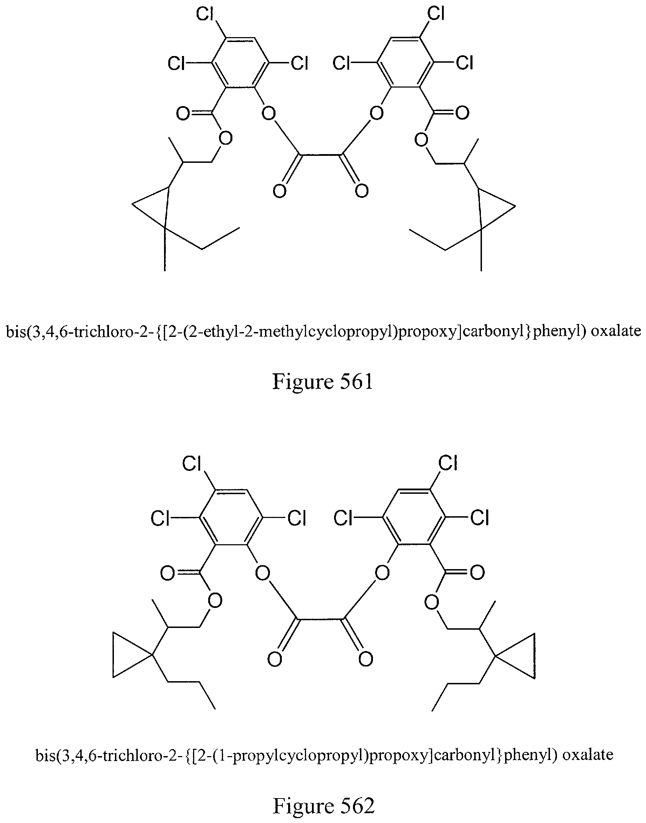

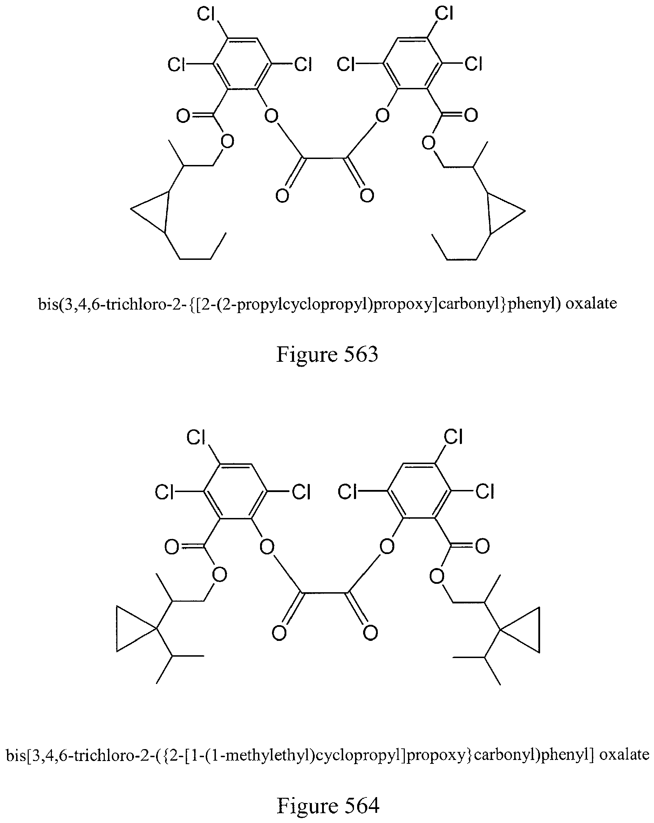

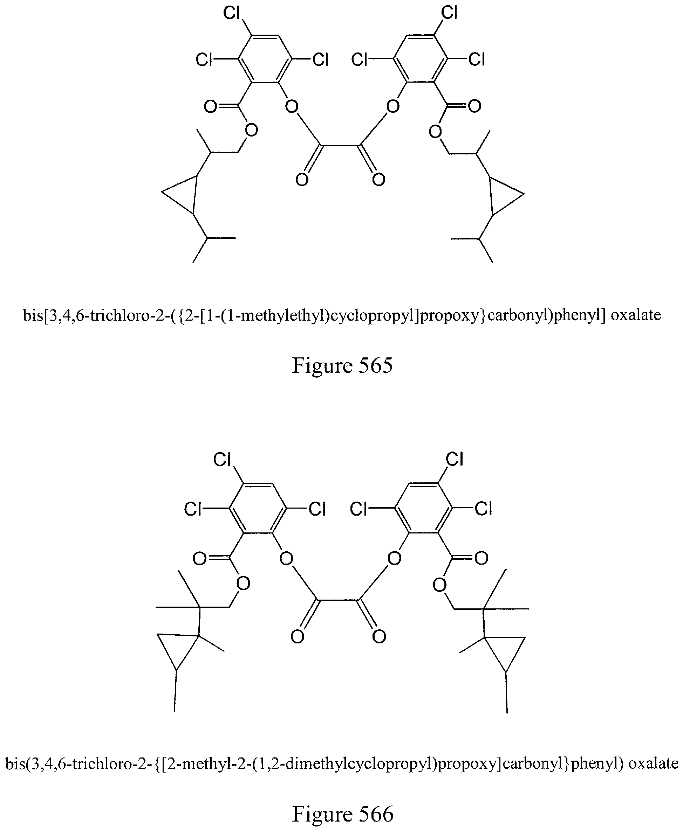

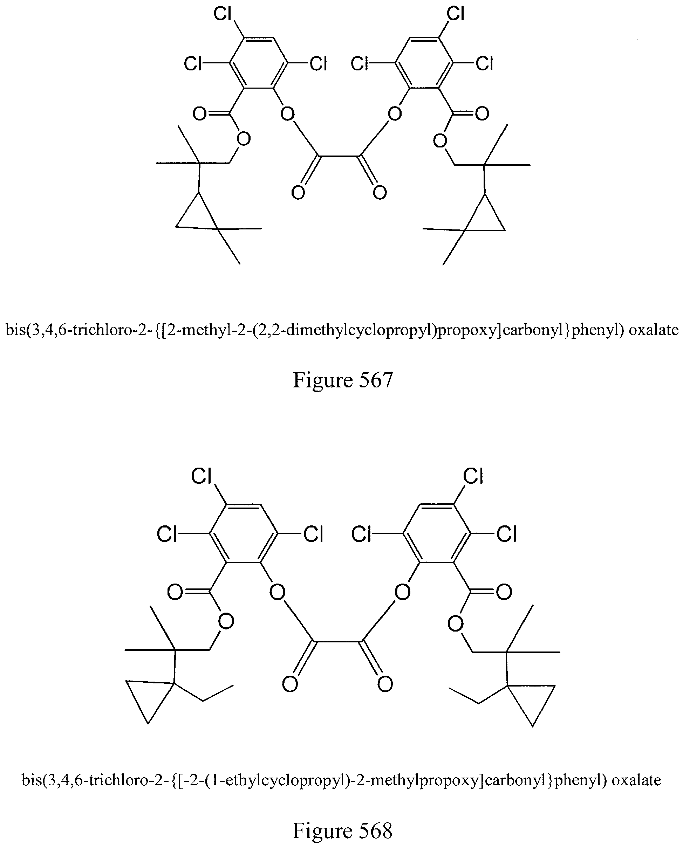

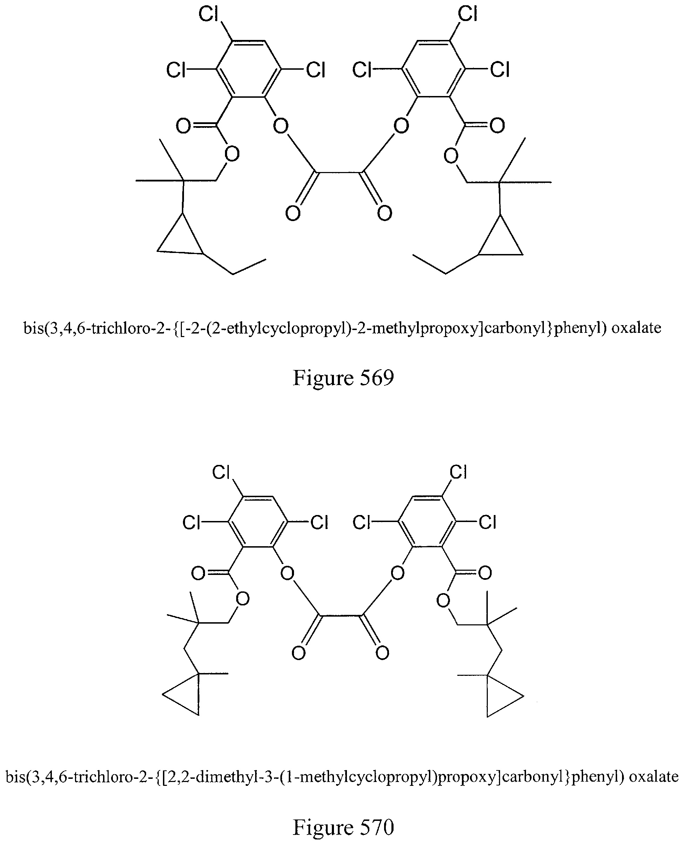

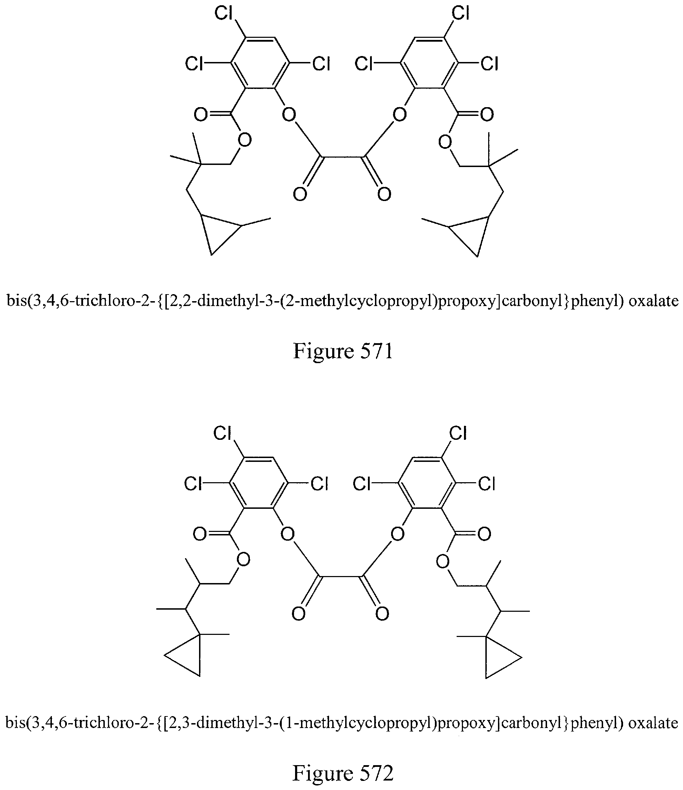

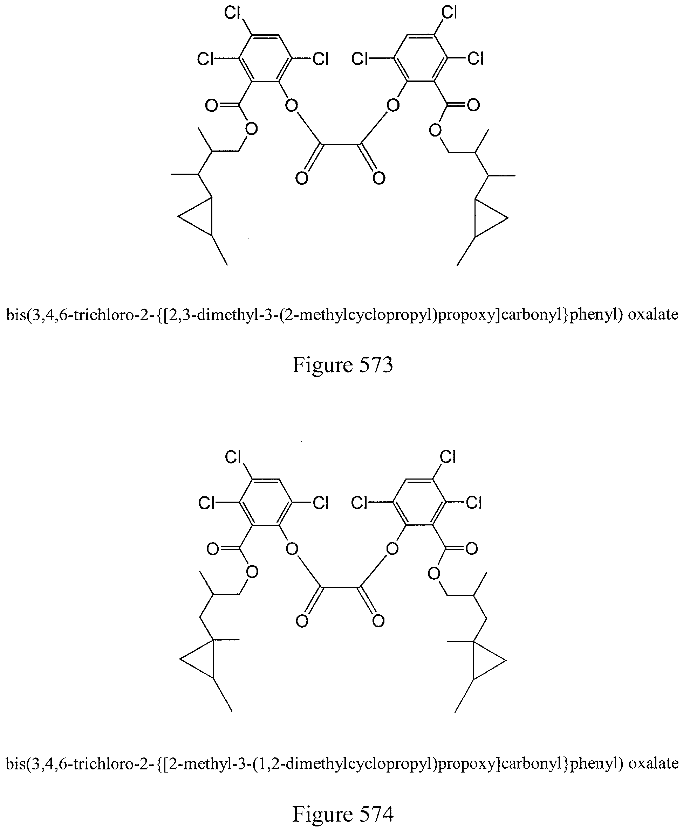

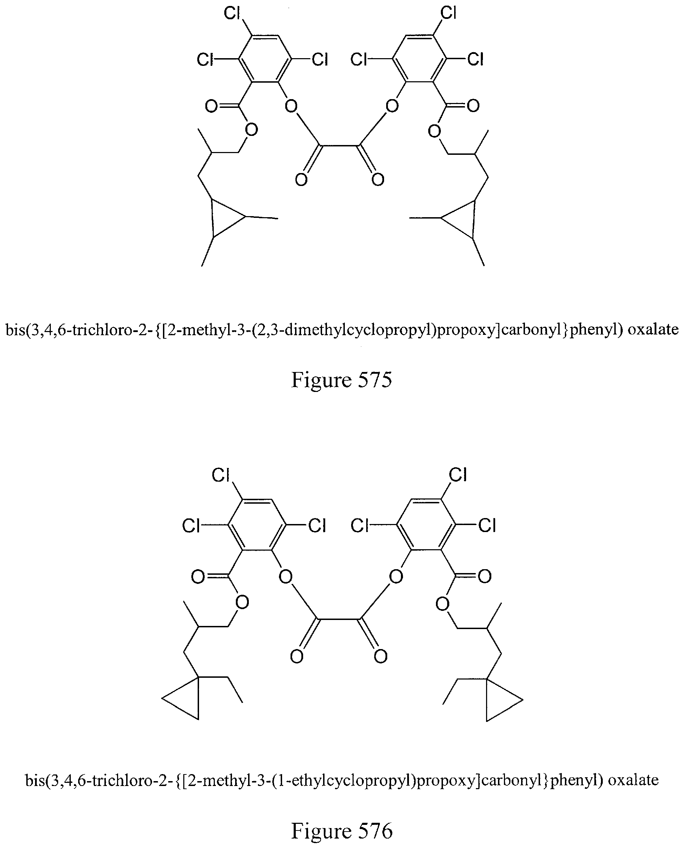

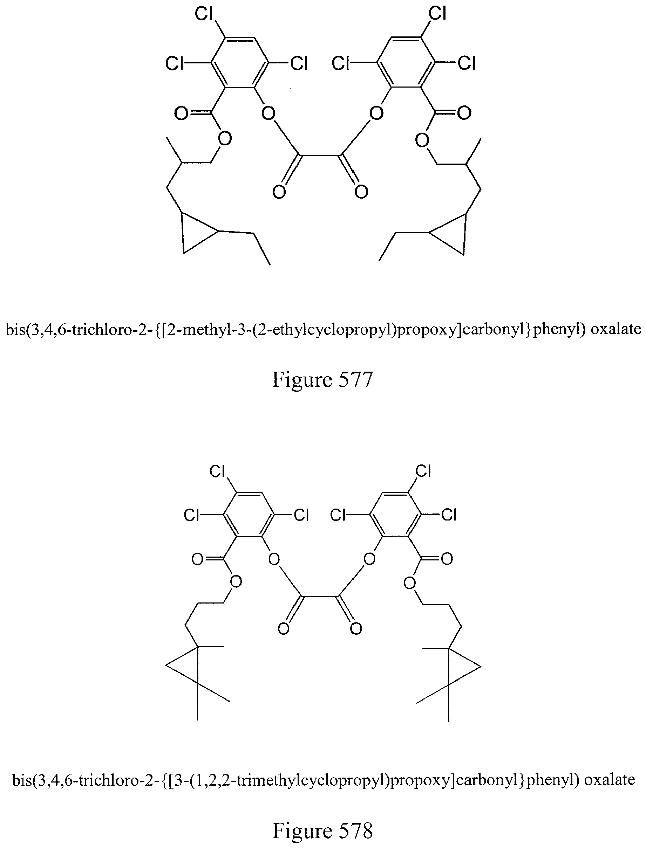

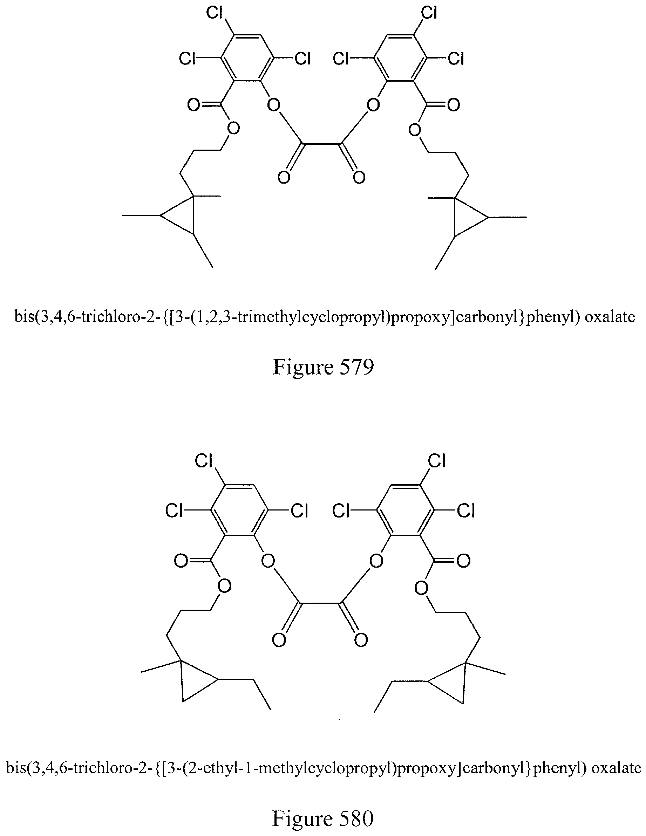

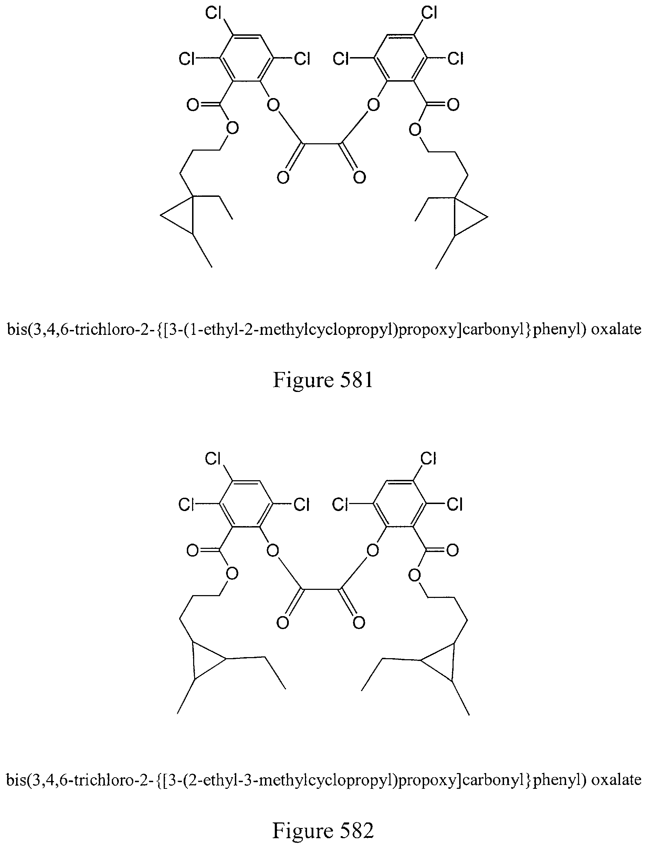

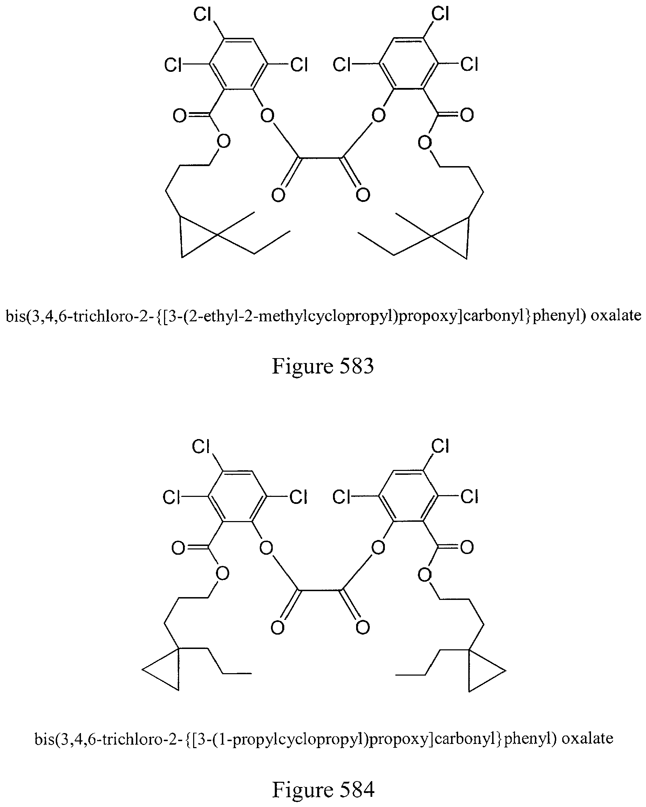

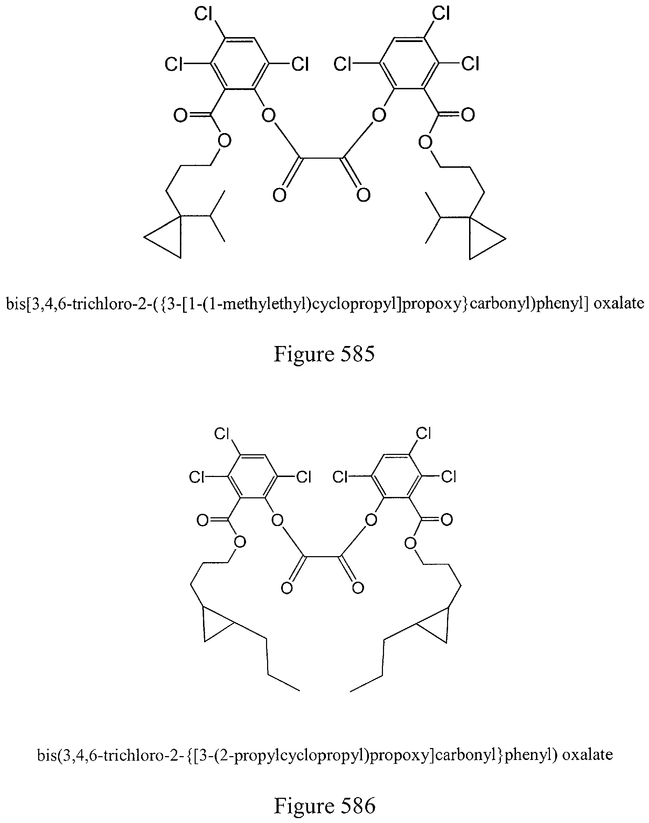

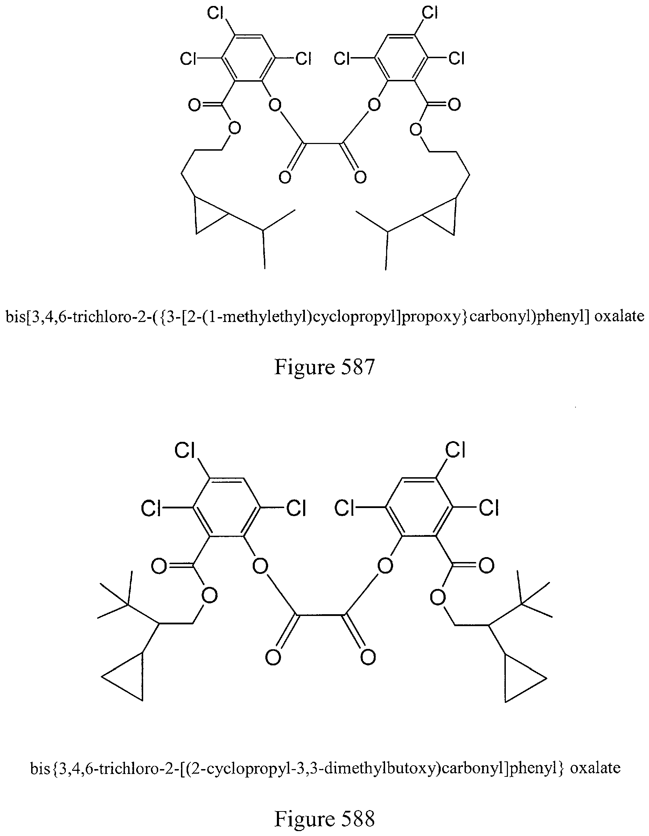

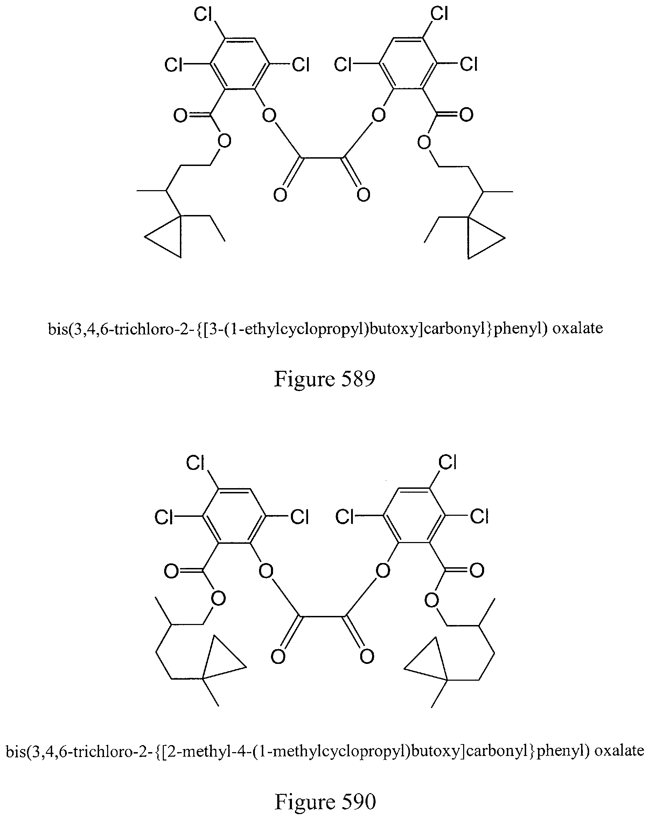

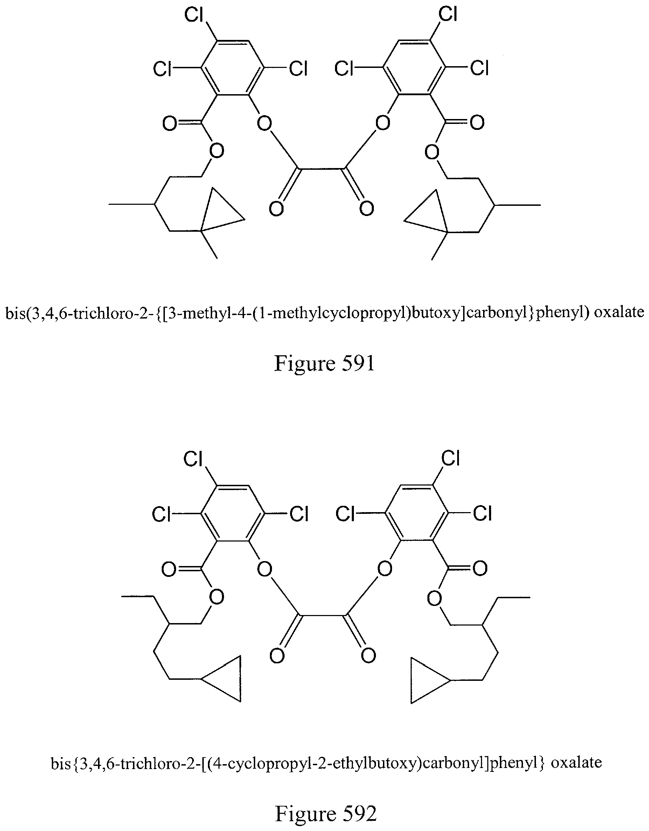

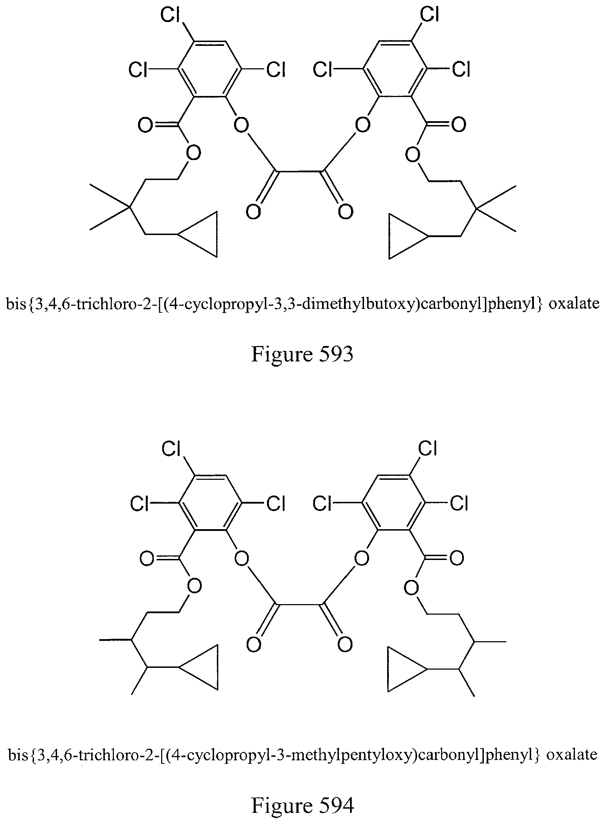

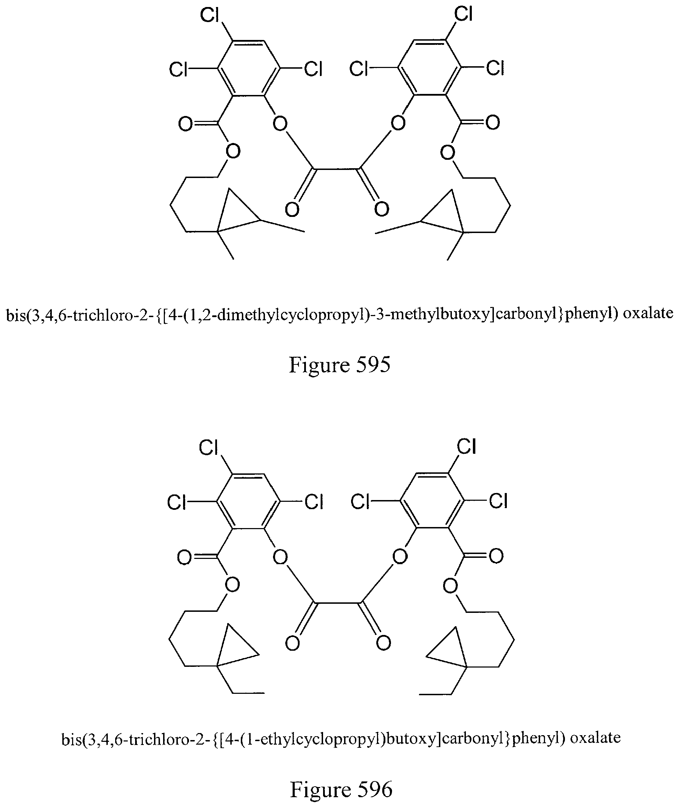

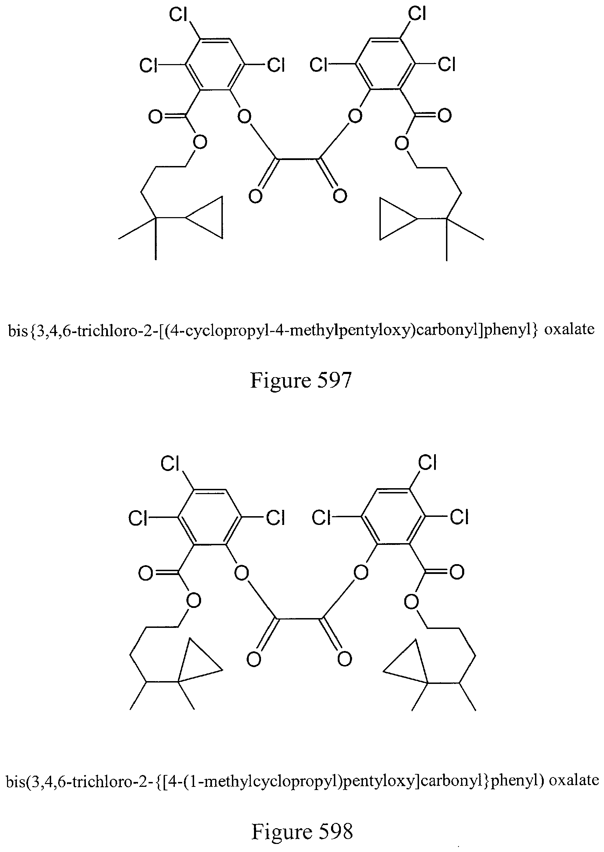

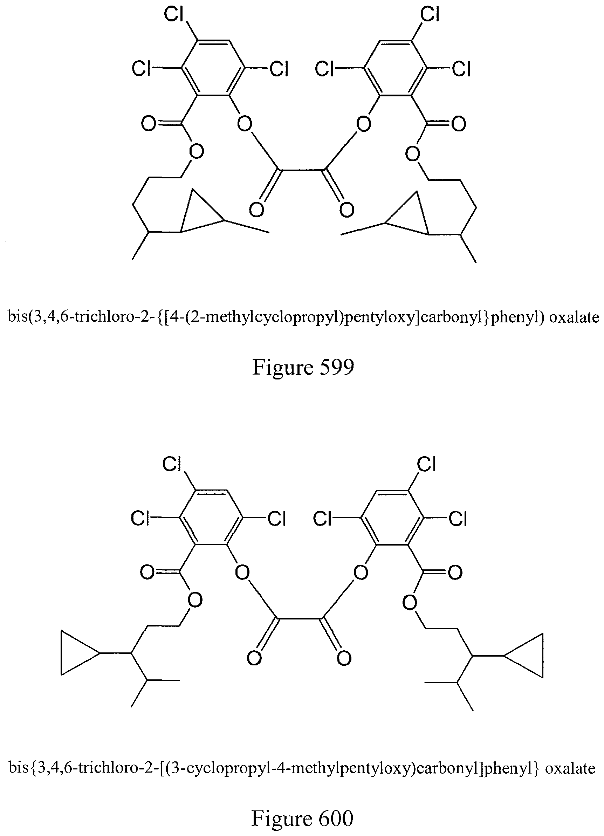

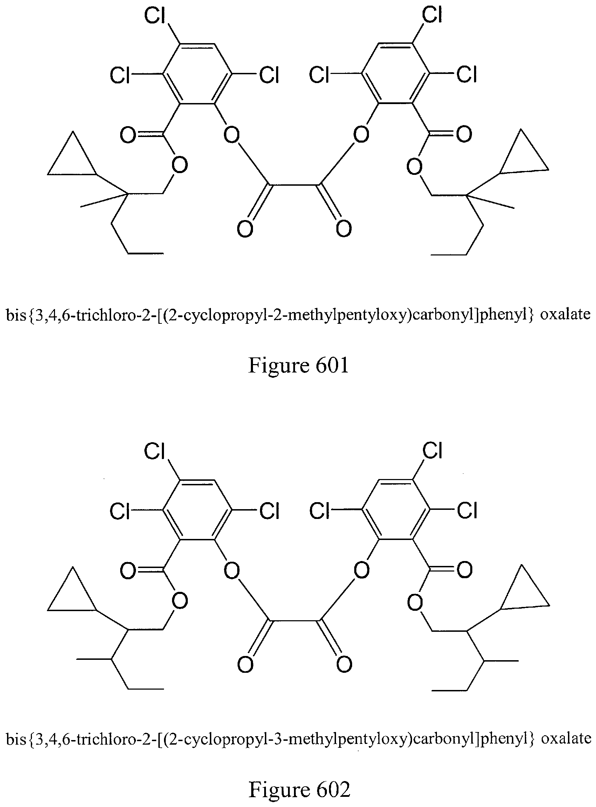

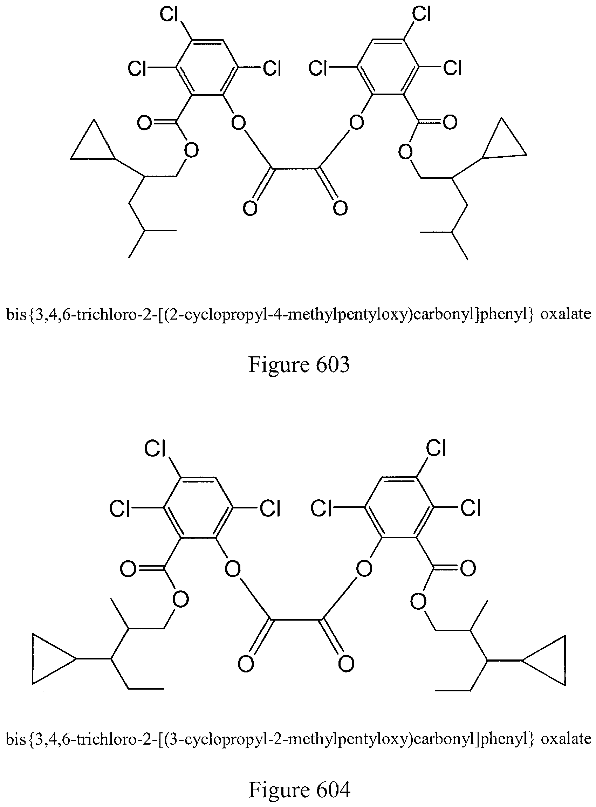

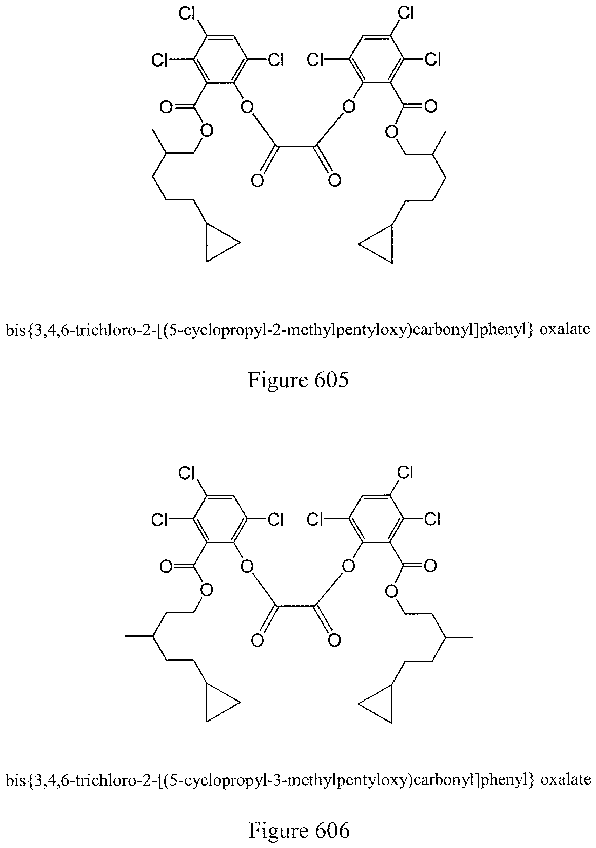

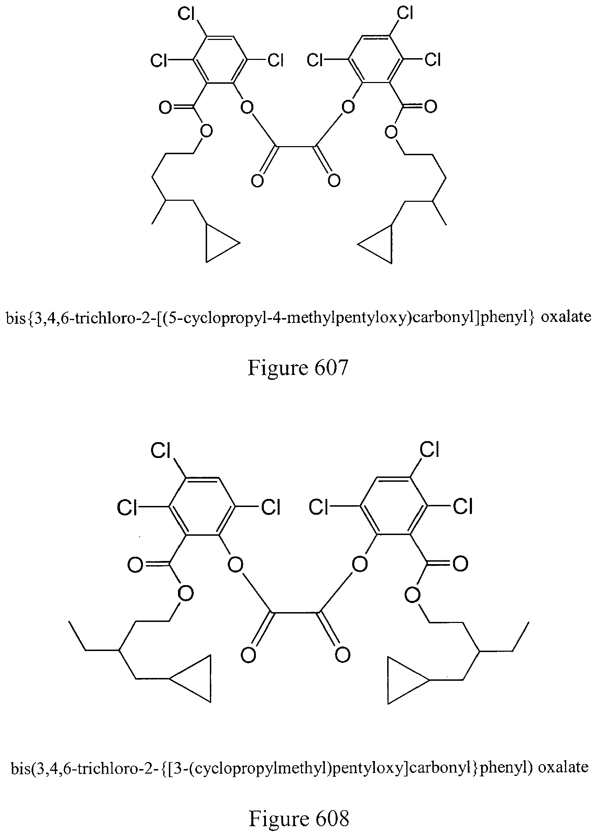

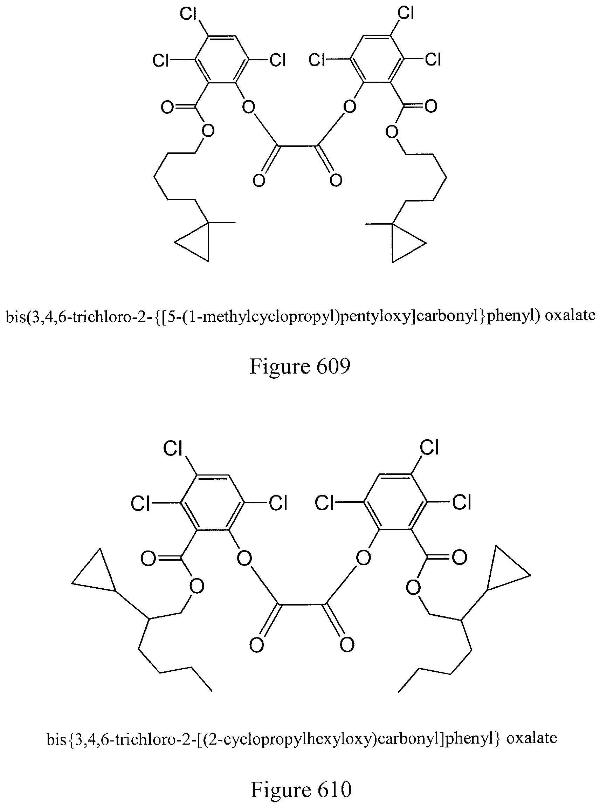

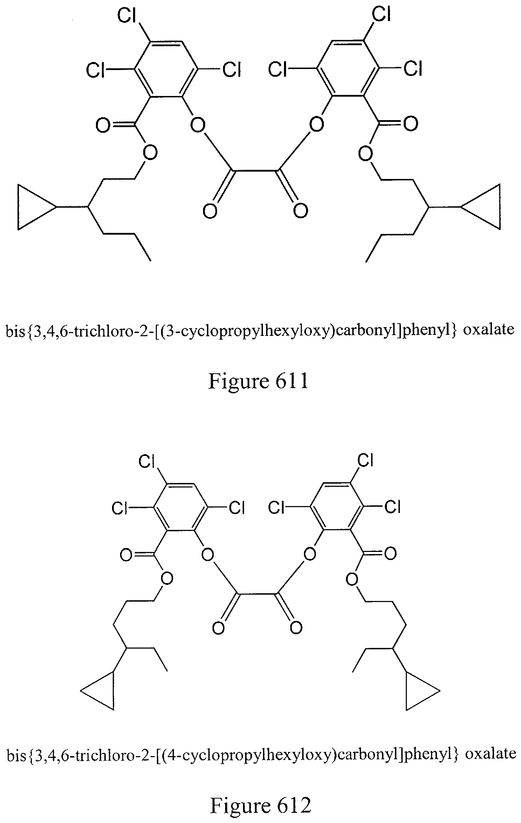

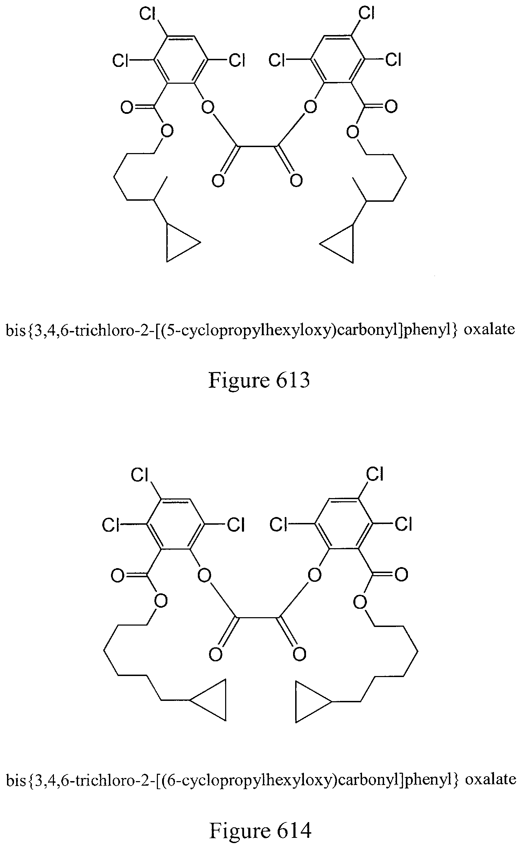

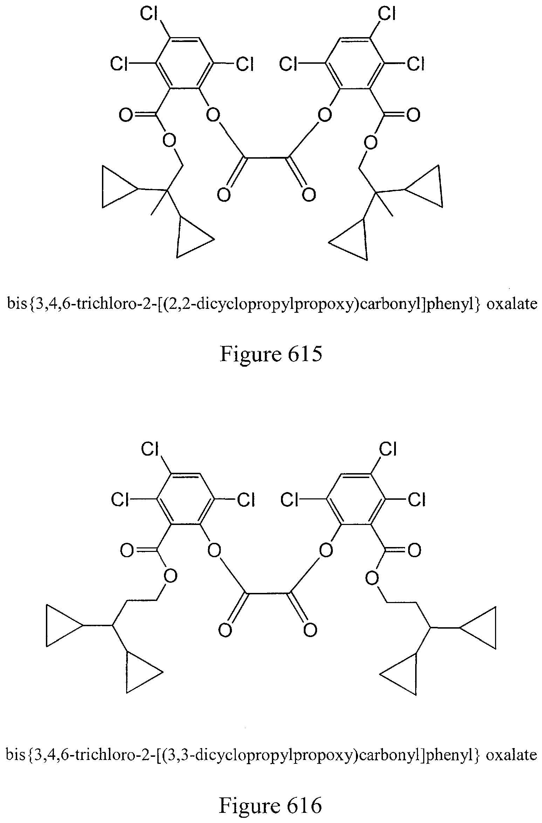

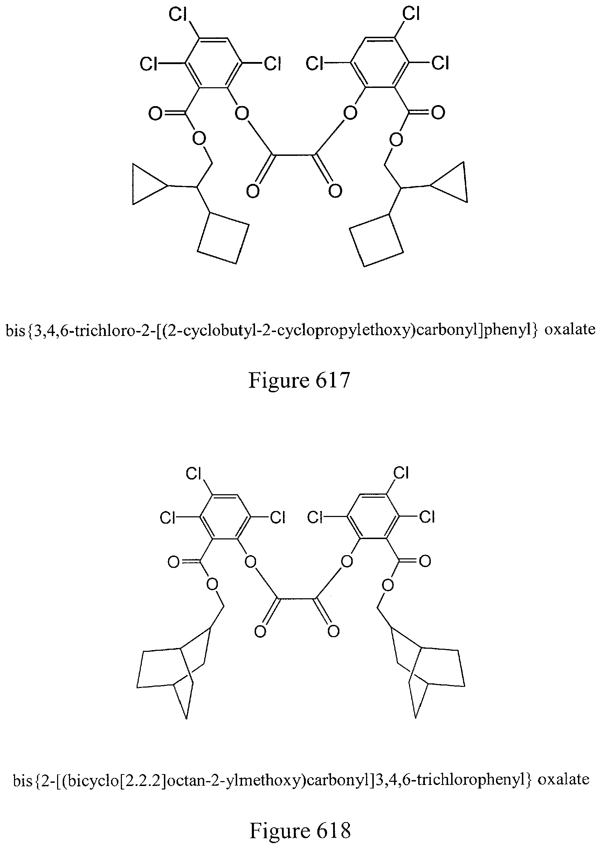

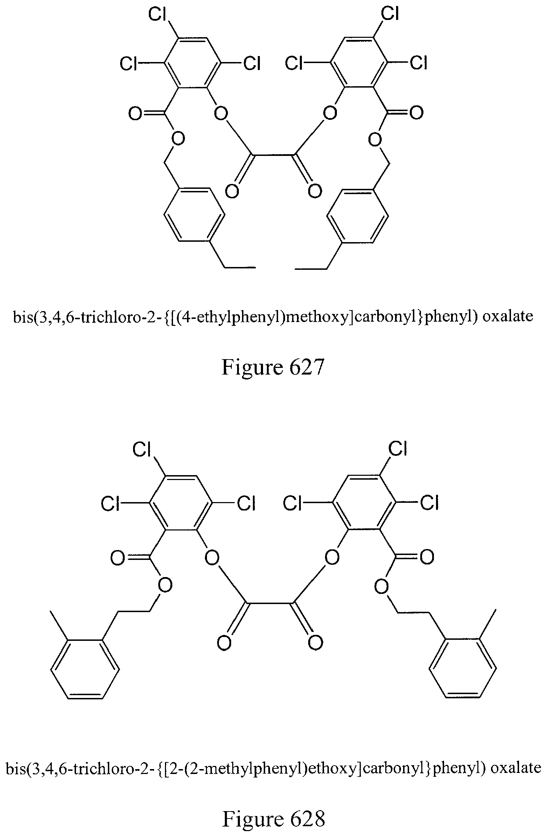

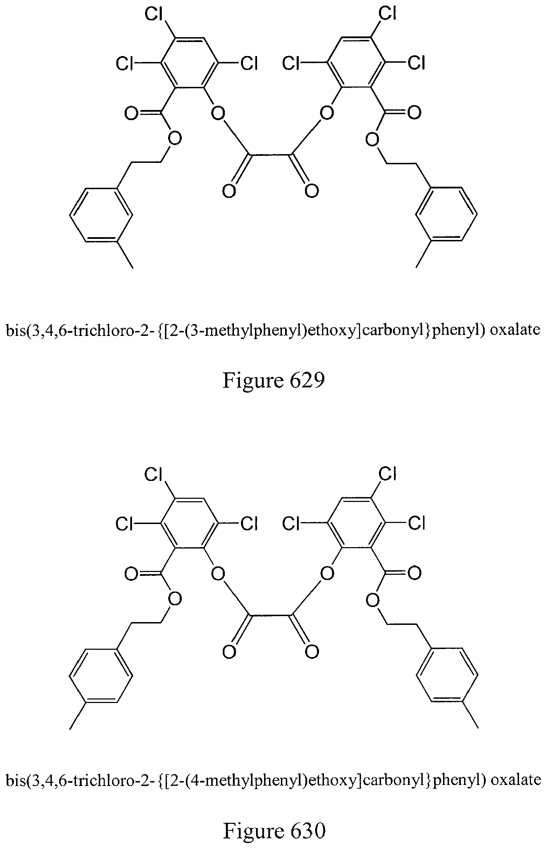

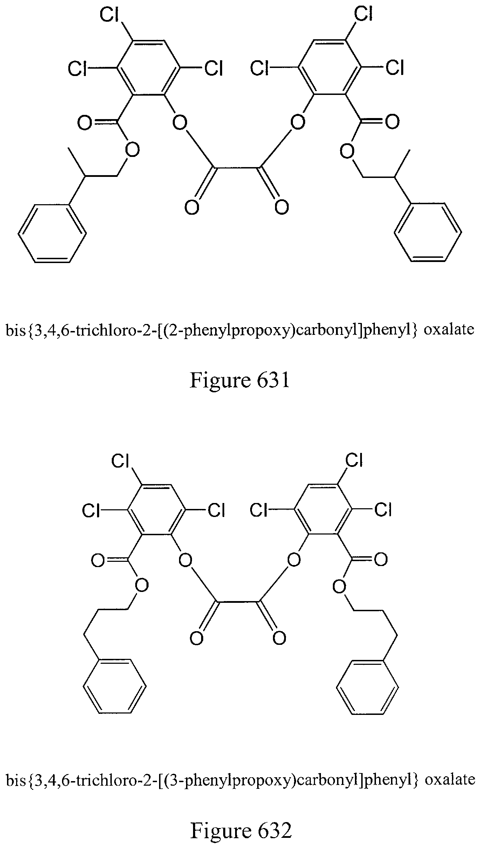

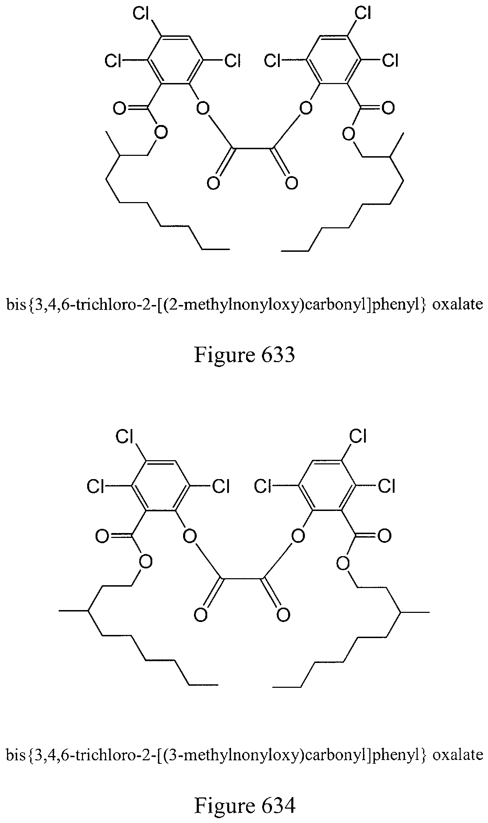

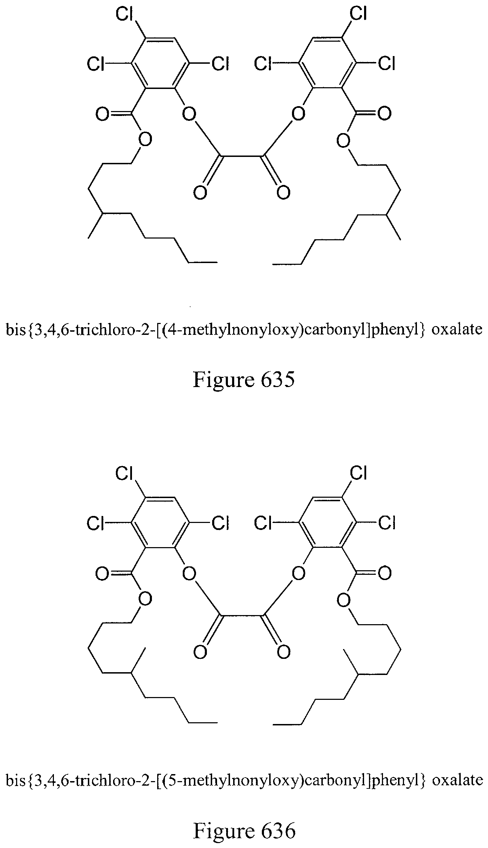

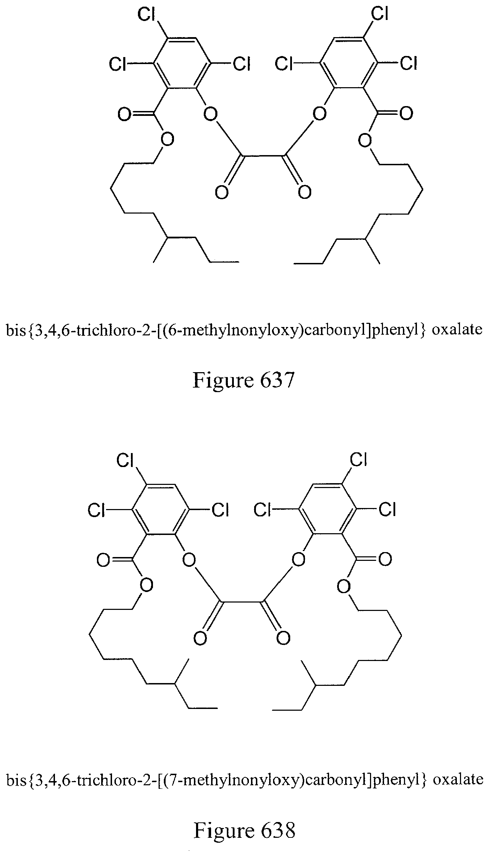

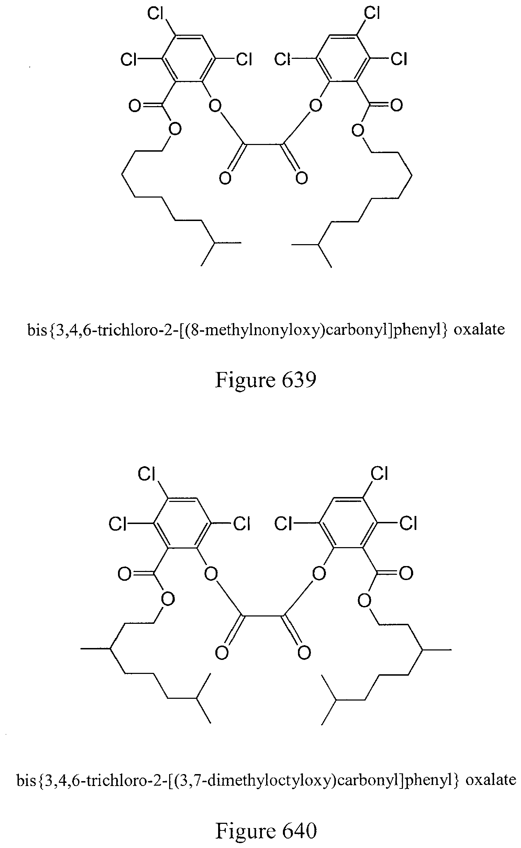

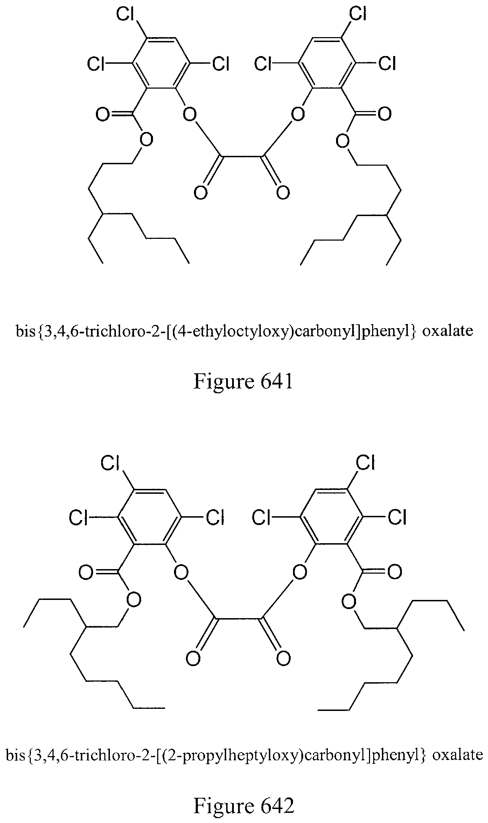

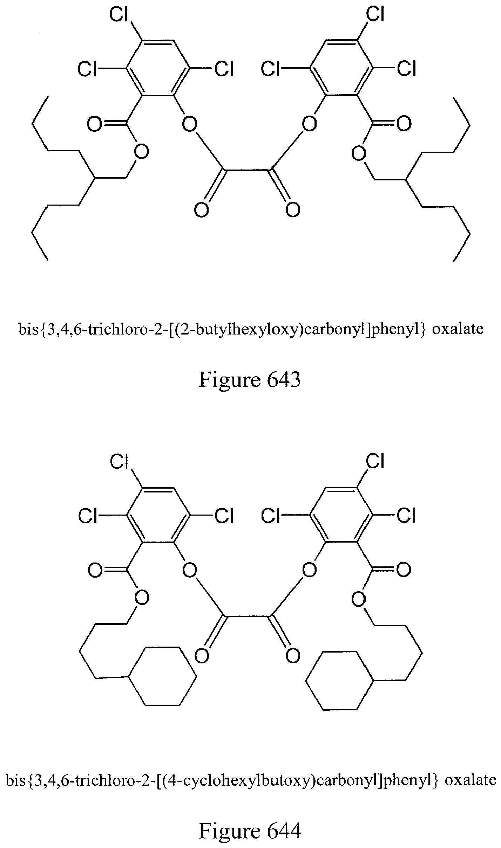

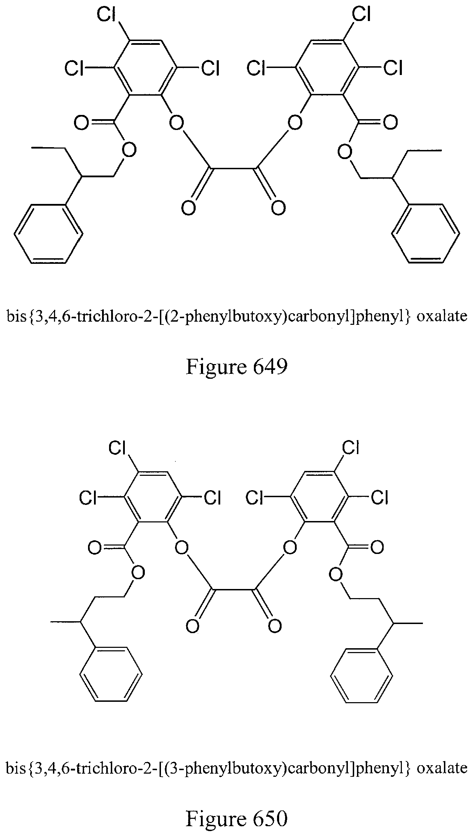

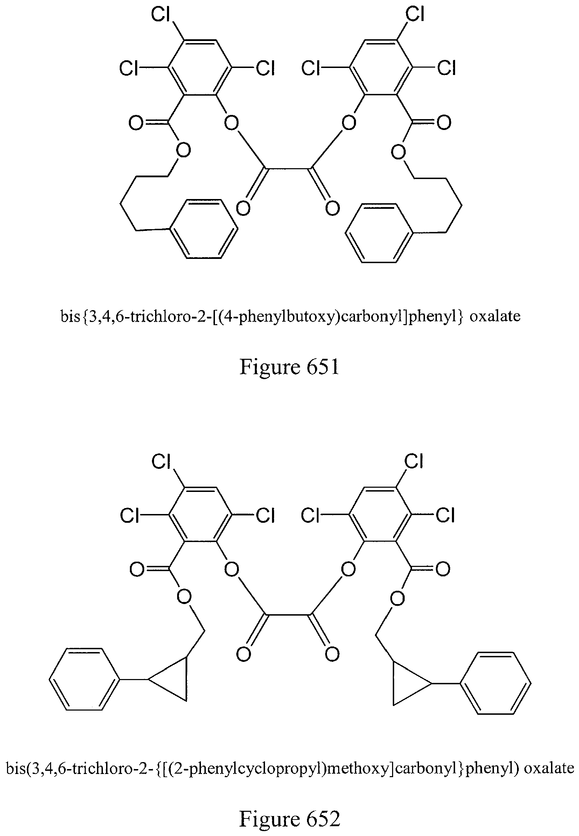

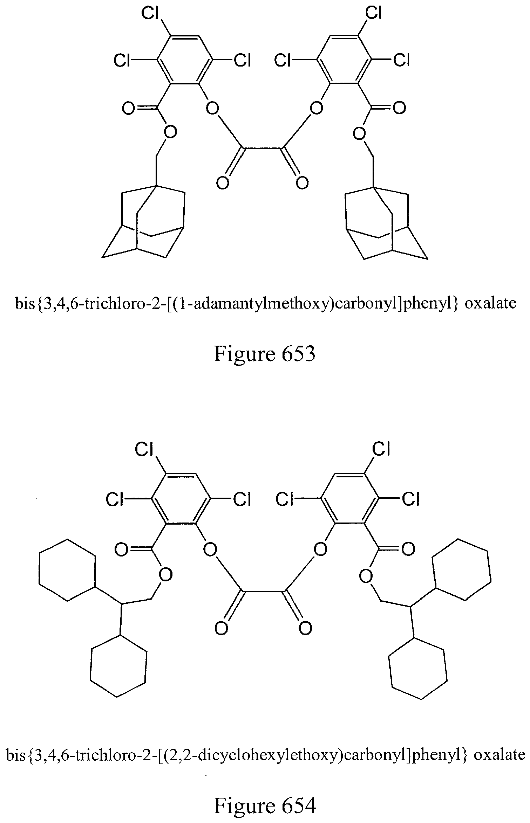

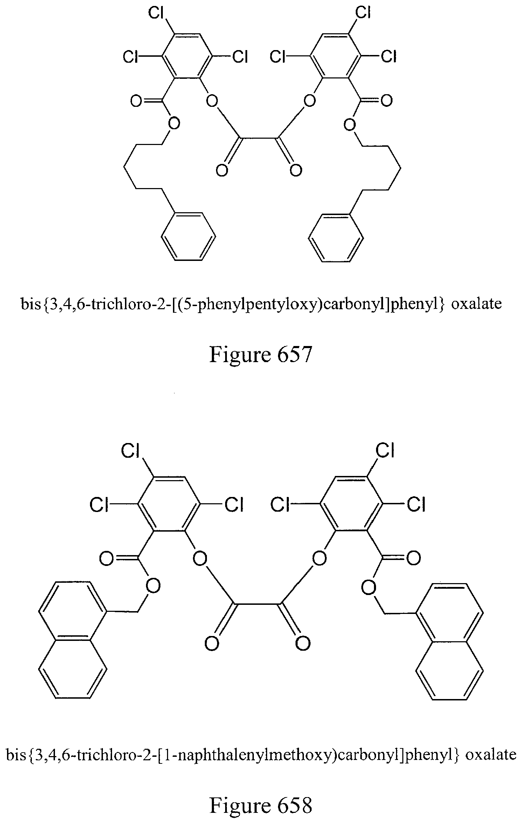

This method of synthesis was used for the compounds shown in Figures 1, 3-5, 9-16, 26-29, 31-46, 51, 60-64, 71-84, 87-120, 126, 138-142, 156-175, 192-224, 226, 230-305, 311, 328-333, 336-387, 435-496, 531-617, 628-644, 649-651, 654, and 657.

Additionally, xylene can be used for the first step and a tertiary amine other than triethylamine can be used for the oxalate, including tripropyl amine, tri-n-butyl amine, triisobutylamine, triisopropylamine, triisopentylamine, and N,N-diisopropylethyl amine.

### EXAMPLE 2

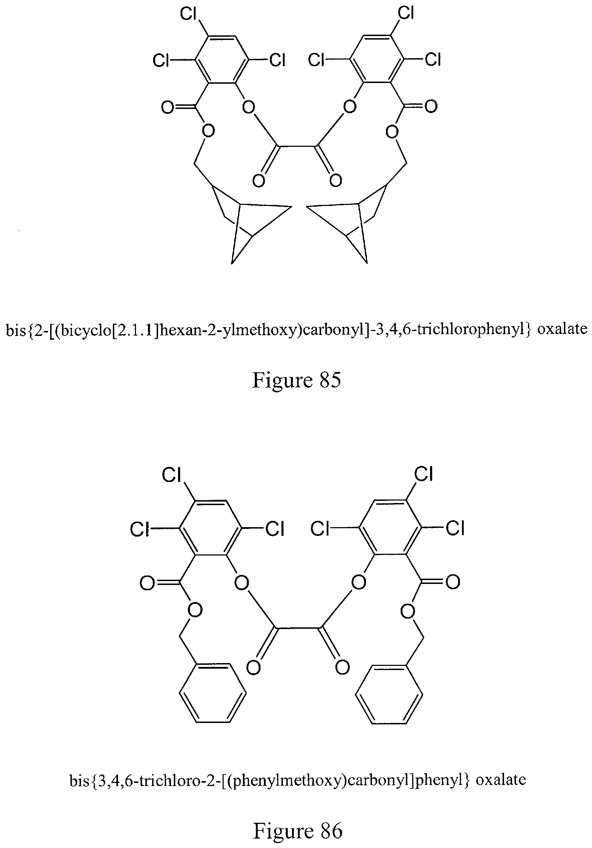

### Procedure For Producing Bis {3,4,6-Trichloro-2-[(Phenylmethoxy)Carbonyl]Phenyl} Oxalate (Figure 86)

A five liter 3-necked flask fitted with a mechanical stirrer and a Barrett or Dean-Stark trap was charged with 483 g (2.0 mol) of 3,5,6-trichlorosalicylic acid, 2000 mL of toluene, 228 mL (2.2 mol) of benzyl alcohol, and 7.86 g (30 mmol) of titanium oxide bis(acetylacetonate). The mixture was heated at reflux overnight or until the theoretical amount of water had been captured in the trap. After cooling to room temperature, the reaction product was treated with 750 mL of saturated aqueous sodium hydrogen carbonate and stirred for 1-2 hours. If the ester precipitates out at this time, add sufficient ethyl acetate or other water immiscible solvent to dissolve the material. After adding 750 mL of water and stirring for an additional 10-15 minutes, the mixture was filtered through a pad of CELITE. The mixture was separated in a filter funnel and the organic layer washed with 750 mL of brine. The organic layer was dried, filtered, and concentrated under reduced pressure. Once all of the material had been concentrated, an aliquot of xylene was added and removed under reduced pressure to ensure complete removal of any excess alcohol and water. If the product precipitated once the alcohol was removed, the residue was triturated with hexane, filtered, and dried. Yield was 254 g (76.5%). The material was analyzed by HPLC and used without further purification in the next step.

The oxalate, bis{3,4,6-trichloro-2-[(phenylmethoxy)carbonyl]phenyl} oxalate (Figure 86), can be made using processes described in the following: US 3,749,679. The recrystallization solvent of choice is ethyl acetate and one of the following: hexane, heptane, triethyl citrate, or toluene. The yield on this scale was 192.7 g (70%).

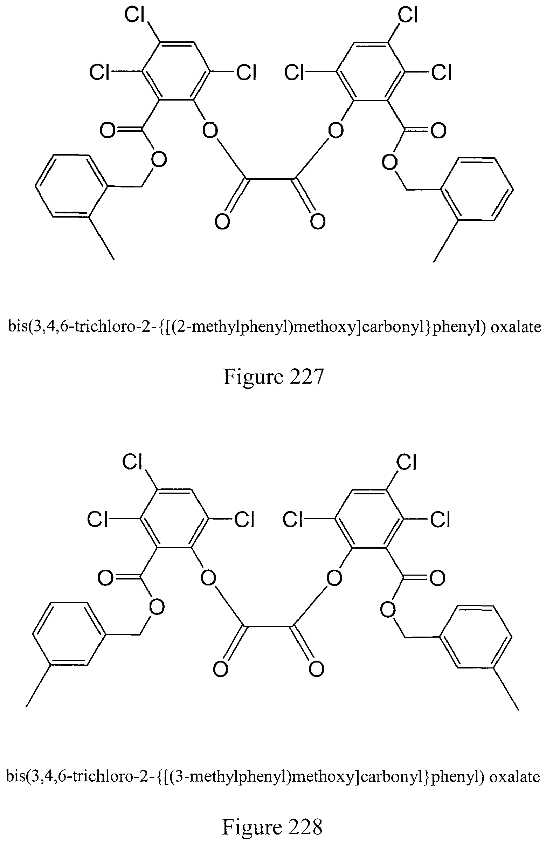

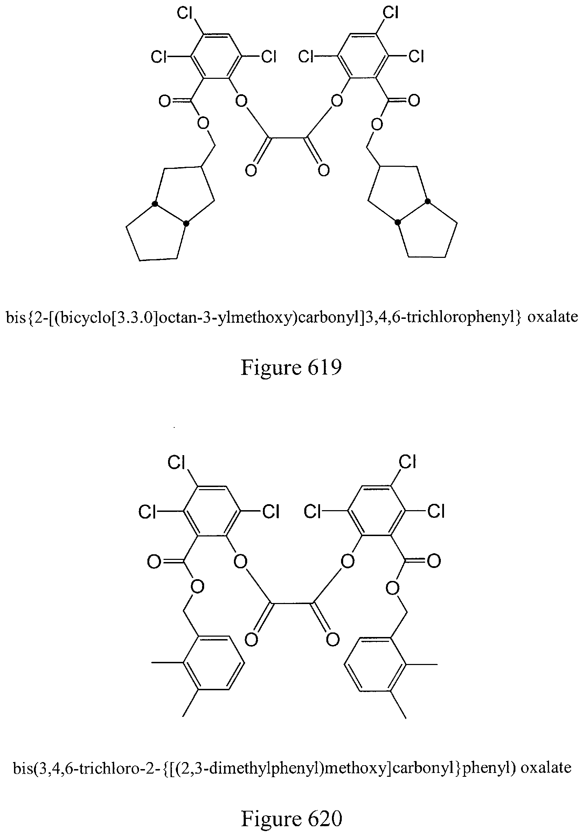

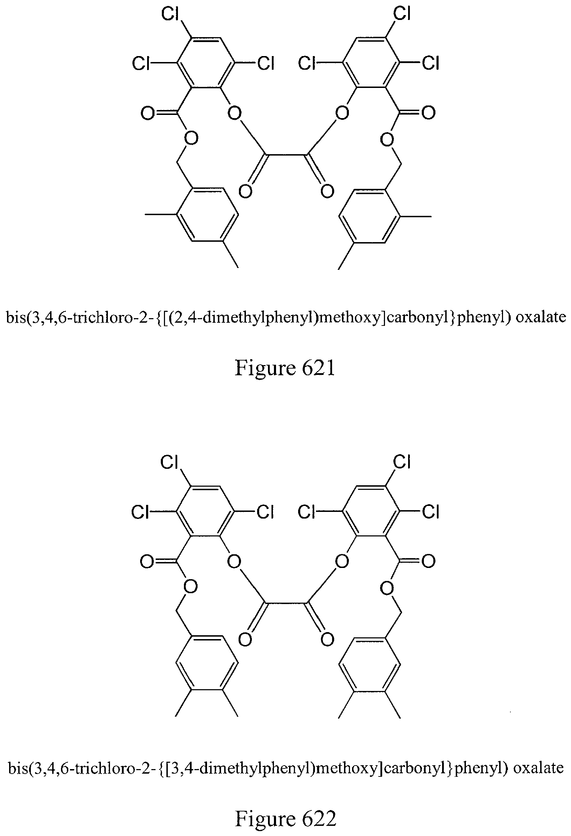

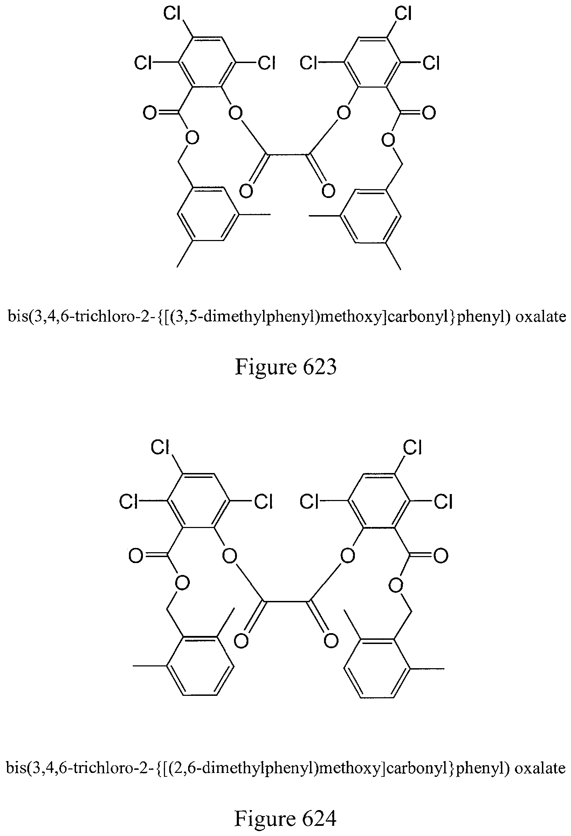

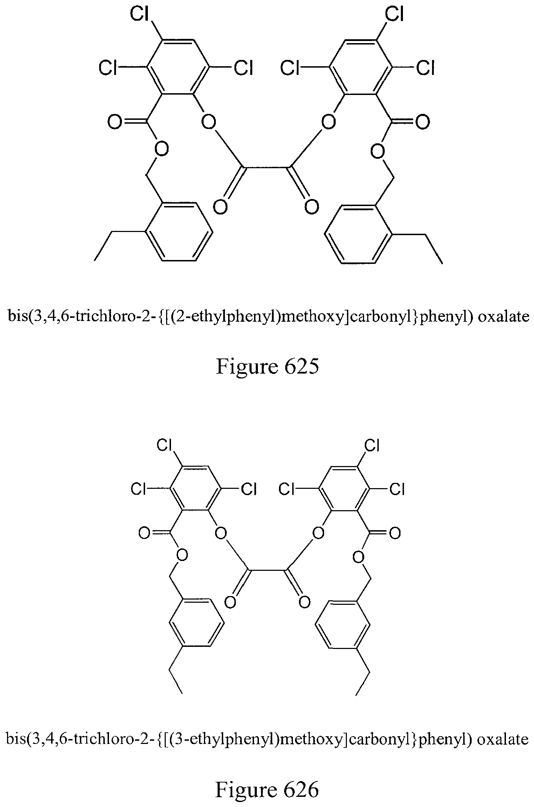

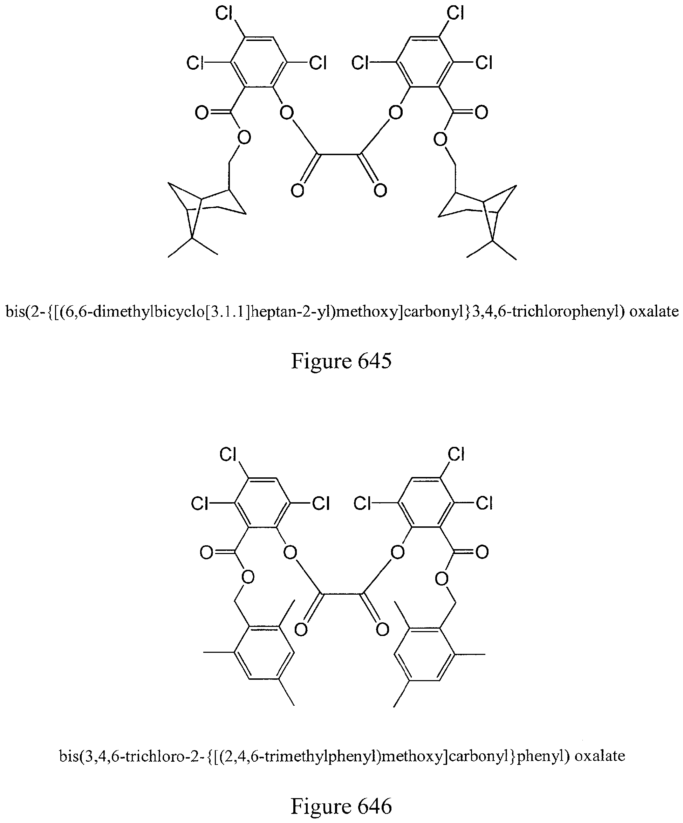

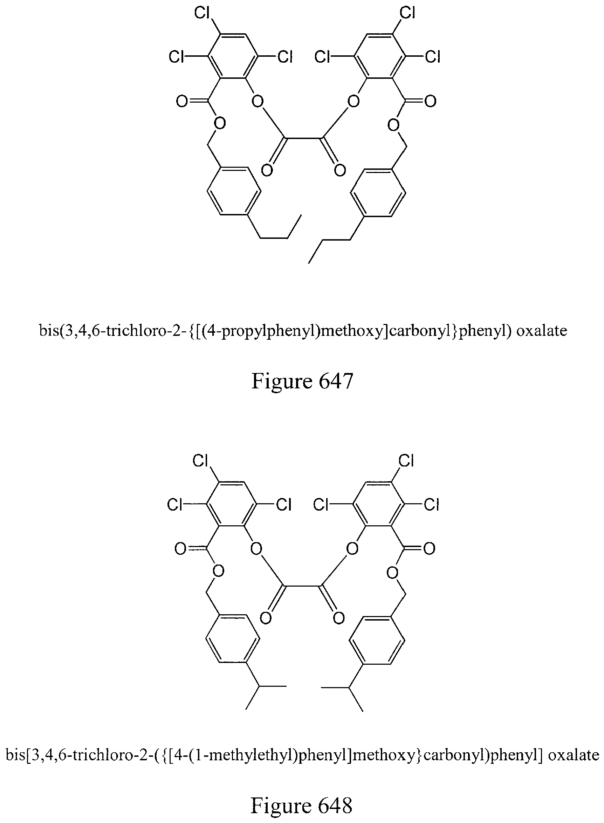

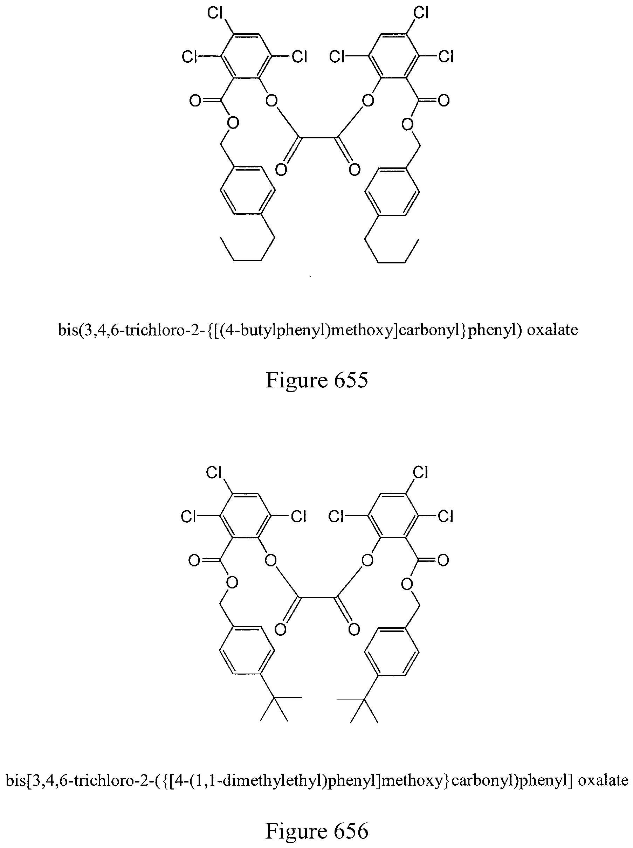

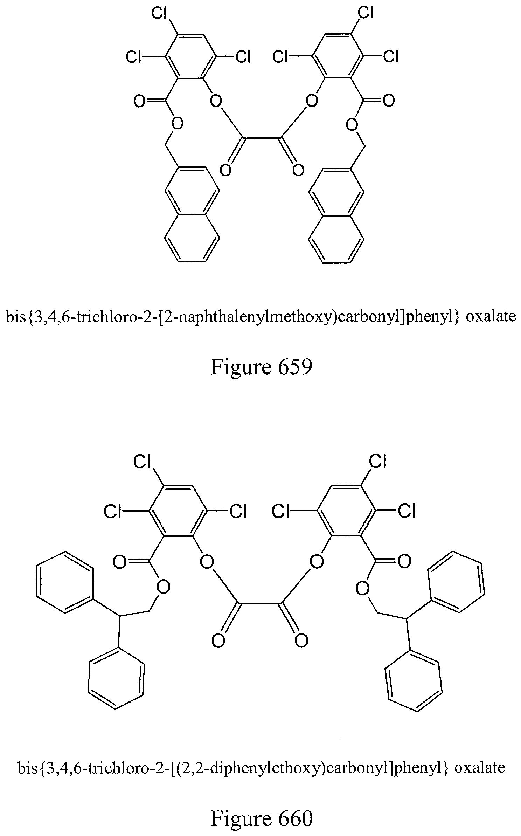

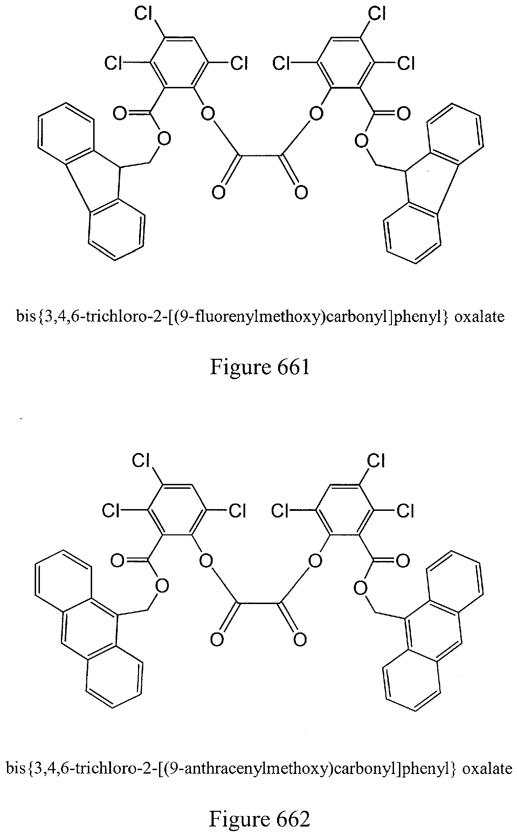

This method was used for the compounds shown in Figures 86, 227-229, 620-627, 646-648, 655-656, 658-659, and 661-662.

Additionally, xylene can be used for the first step and a tertiary amine other than triethylamine can be used for the oxalate, including tripropyl amine, tri-n-butyl amine, triisobutylamine, triisopropylamine, triisopentylamine, and N,N-diisopropylethyl amine.

### EXAMPLE 3 (REFERENCE EXAMPLE)

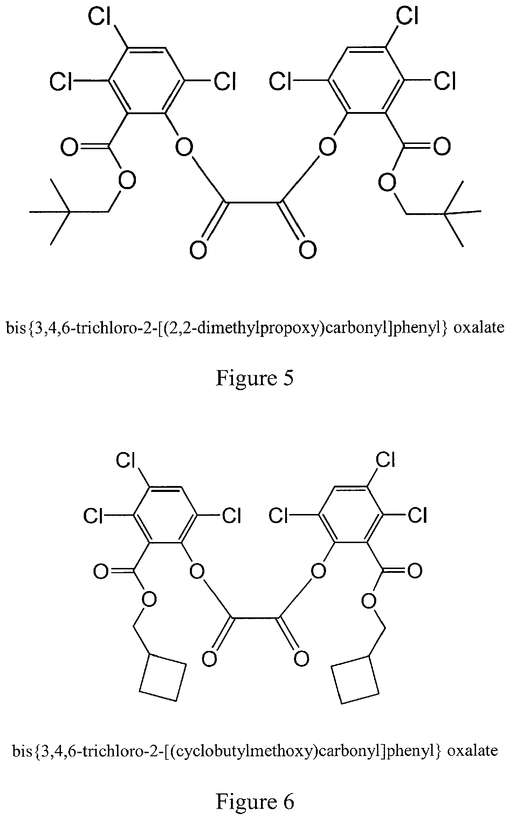

### Procedure For Producing Bis{3,4,6-Trichloro-2-[(2,2-Dimethylpropoxy)Carbonyl]Phenyl} Oxalate (Figure 5)

The first step for making 2,2-dimethylpropyl 3,4,6-trichlorosalicylate, was carried out in xylene using the procedure found in Example 2. The yield was 310 g (99%). The material was analyzed by HPLC and used without further purification in the next step.

Bis{3,4,6-trichloro-2-[(2,2-dimethylpropoxy)carbonyl]phenyl} oxalate can be made using processes described in Example 1. The yield was 180.1 g (53%).

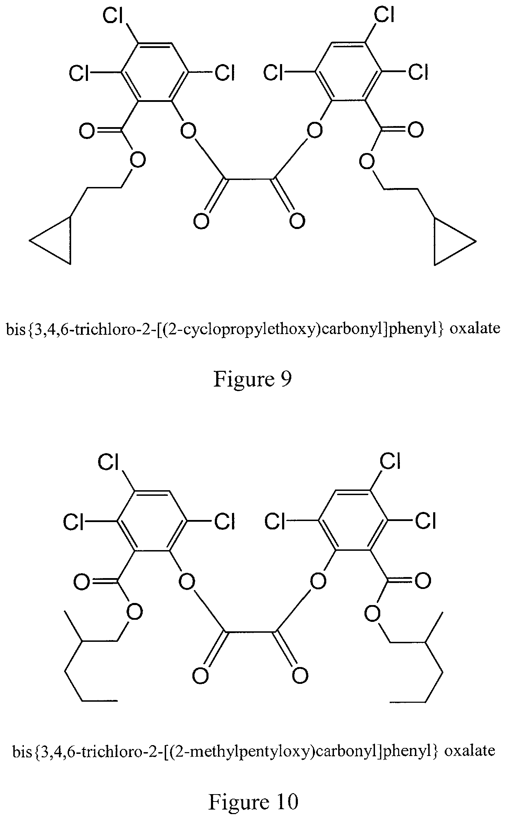

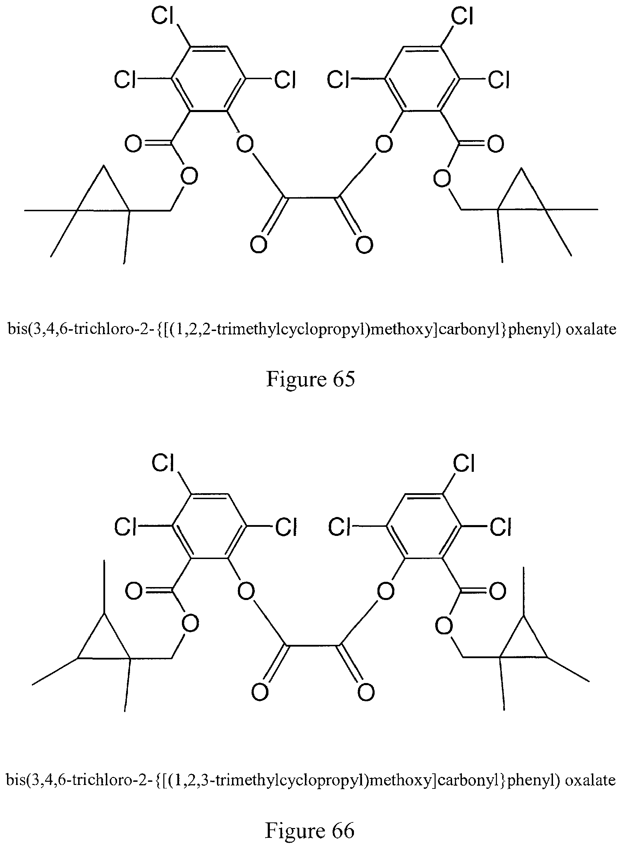

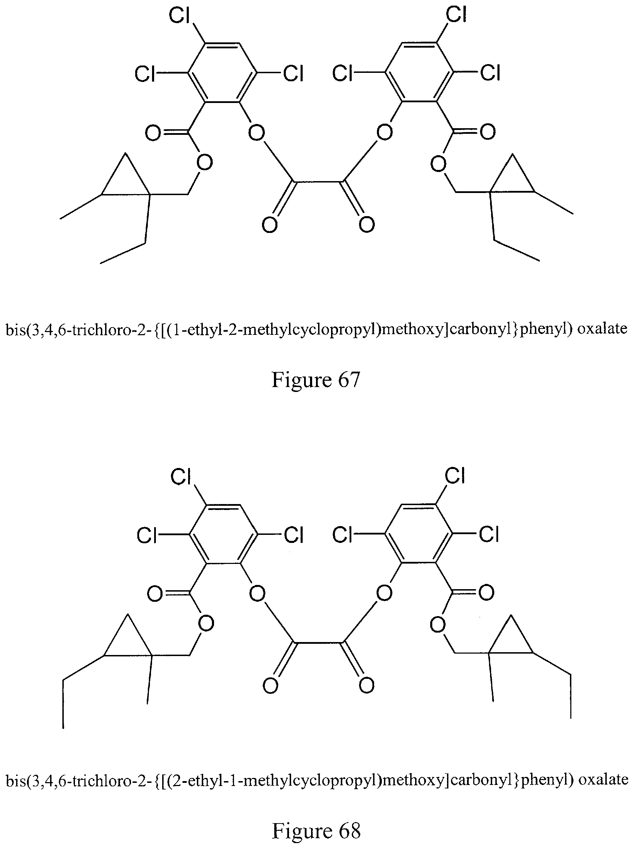

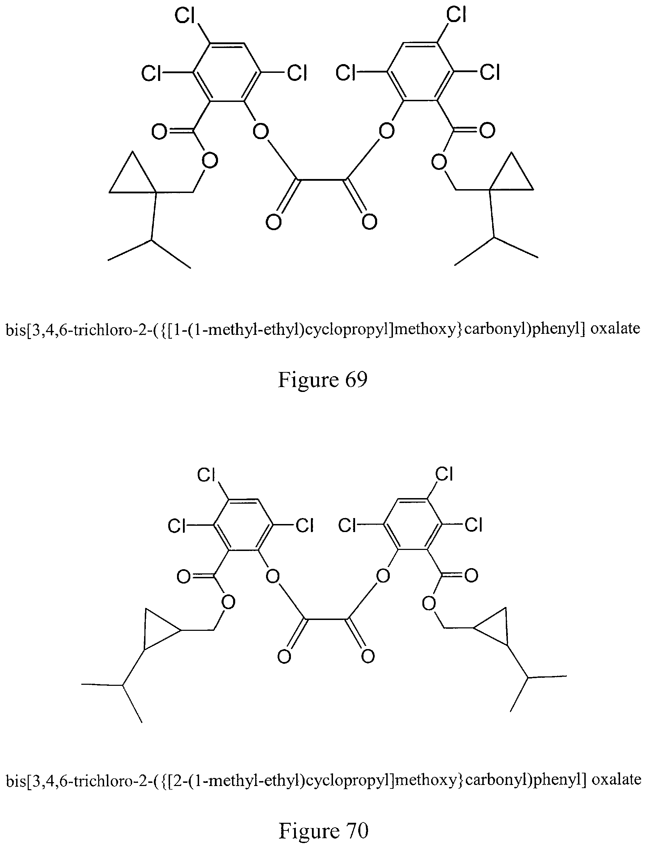

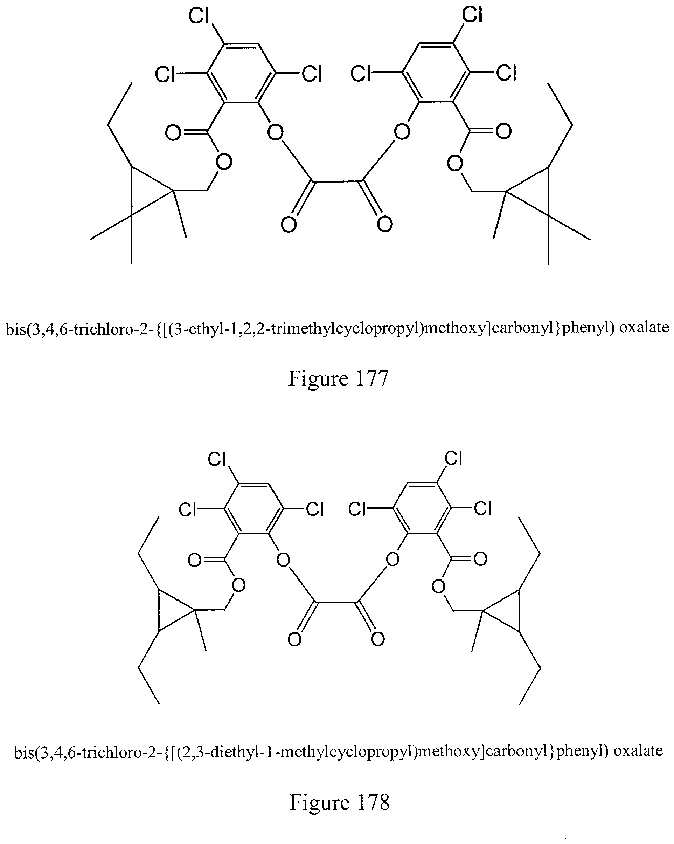

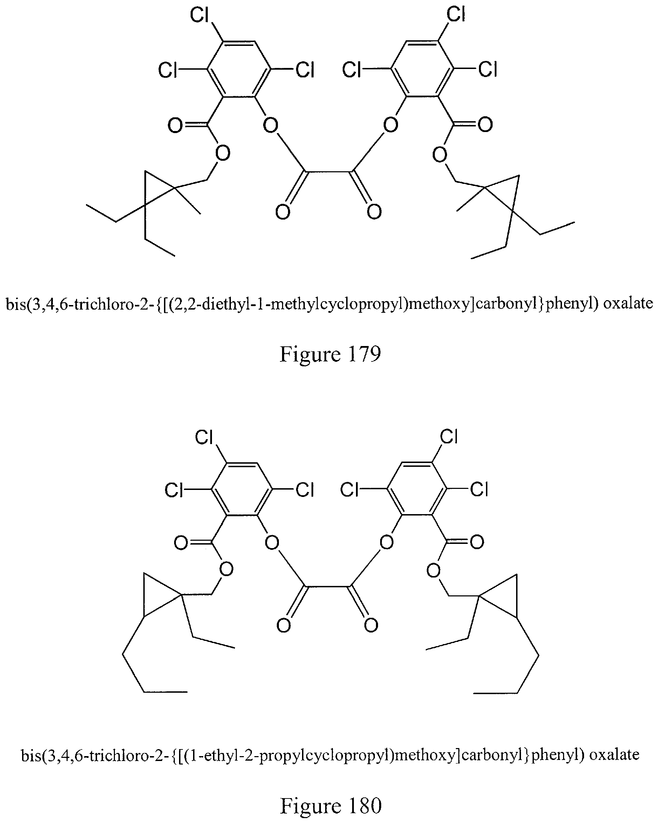

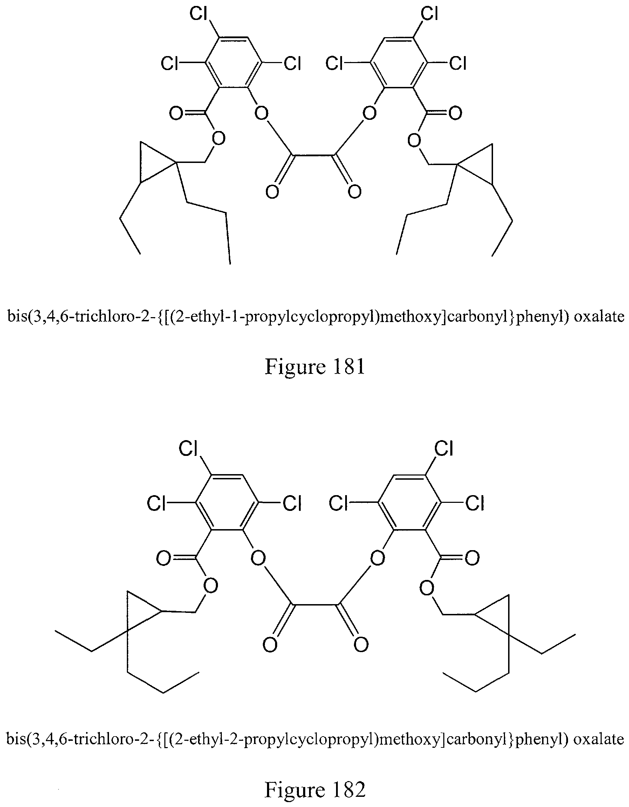

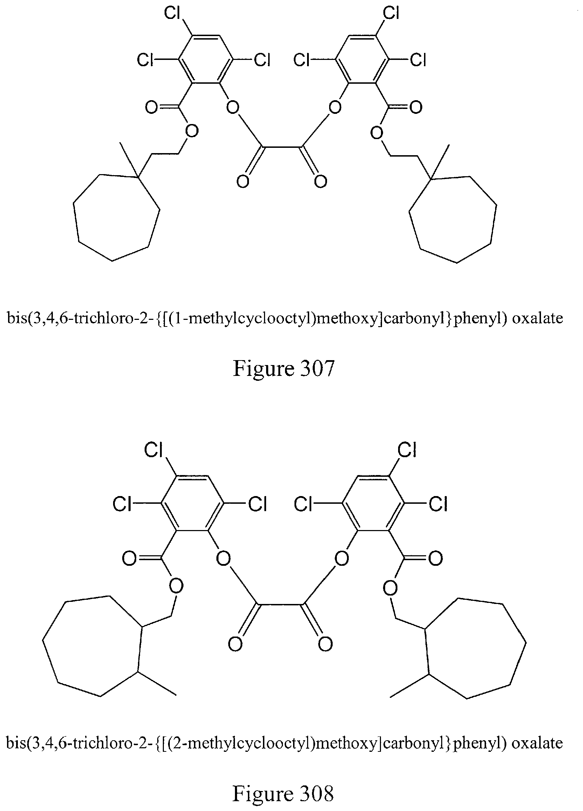

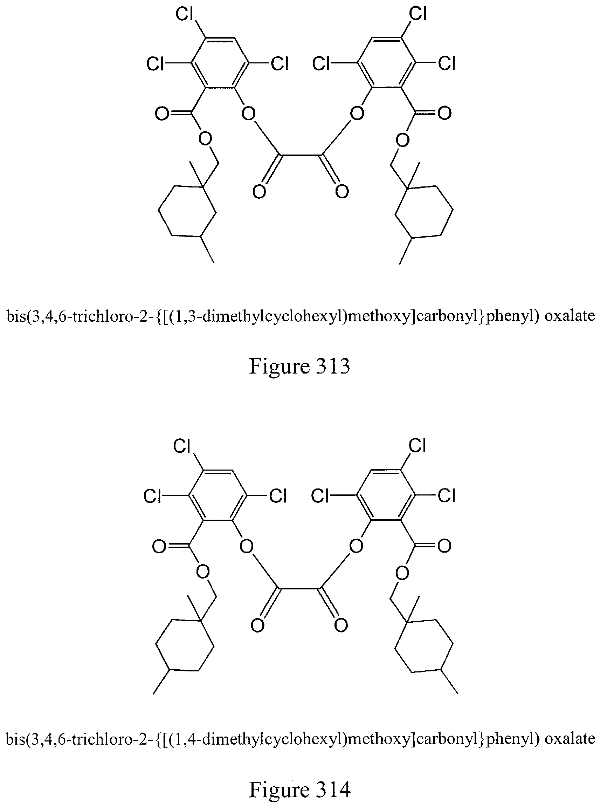

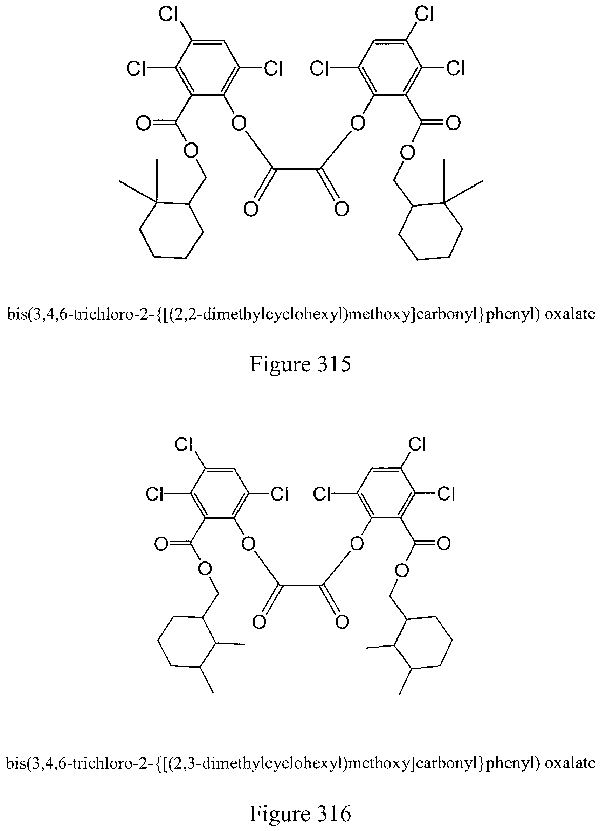

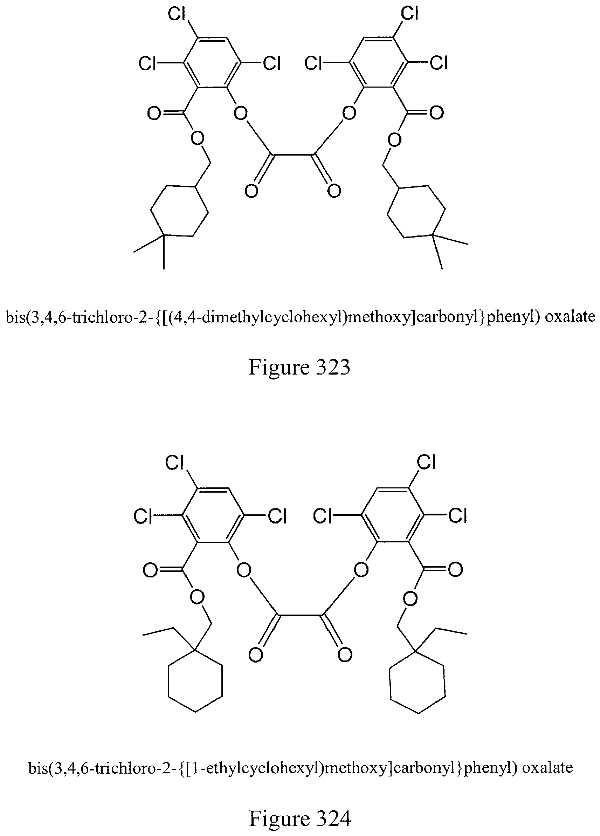

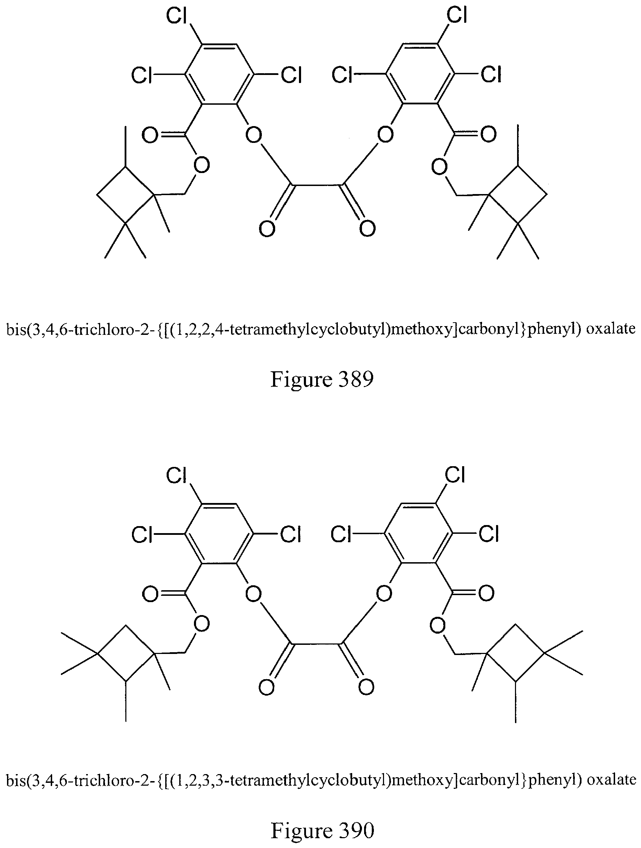

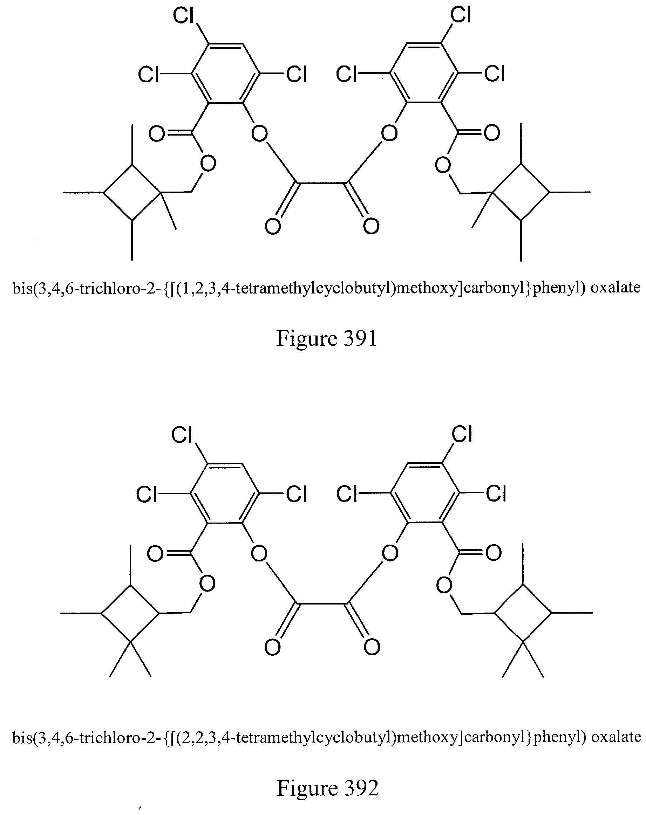

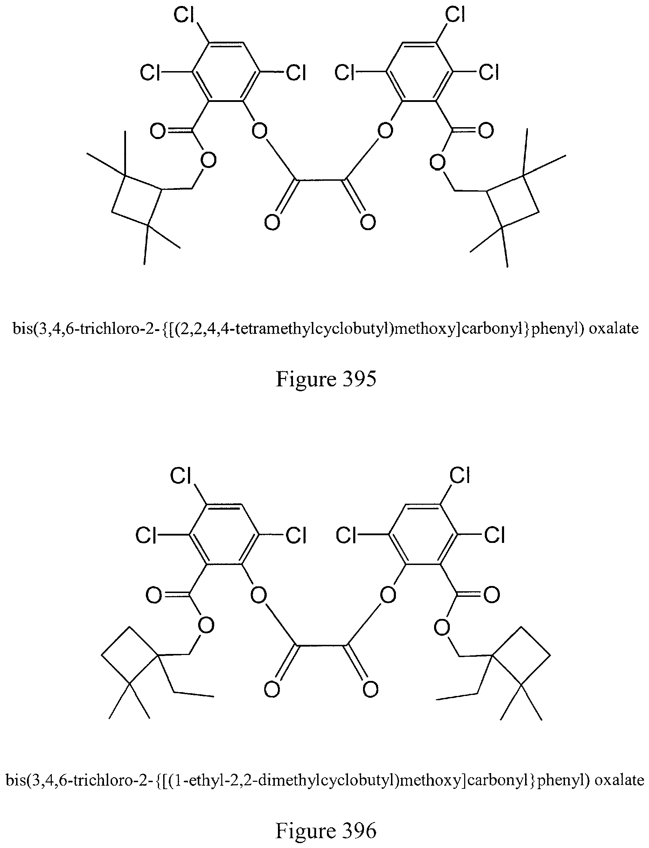

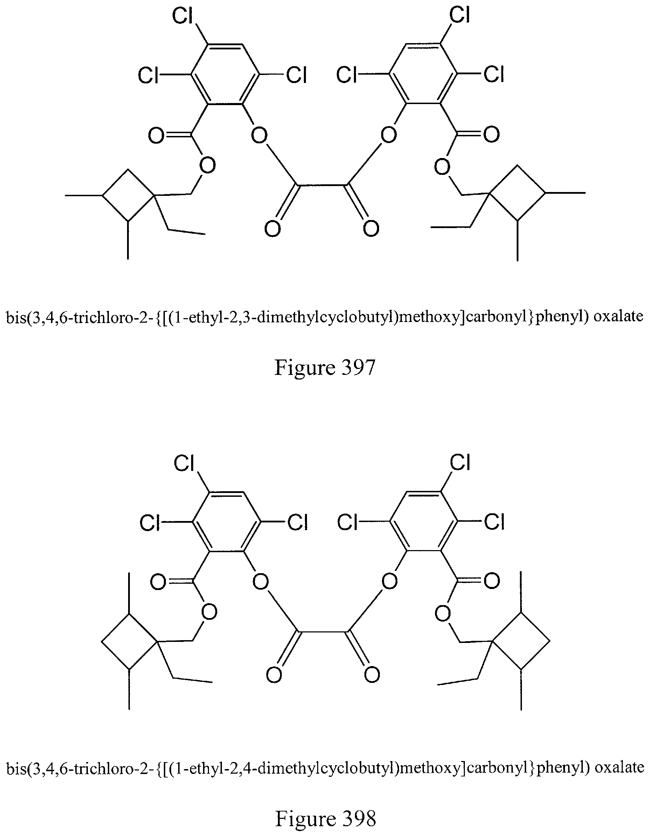

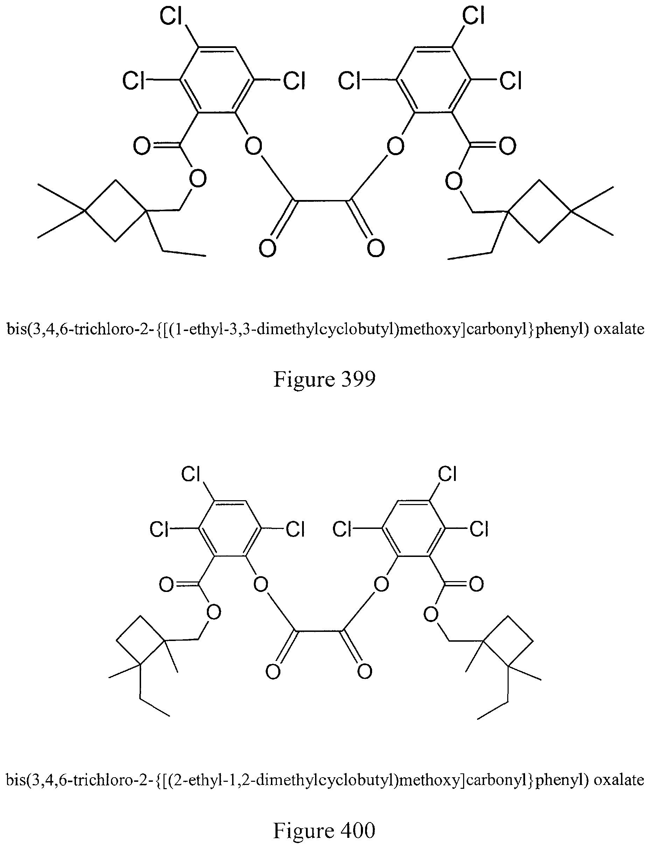

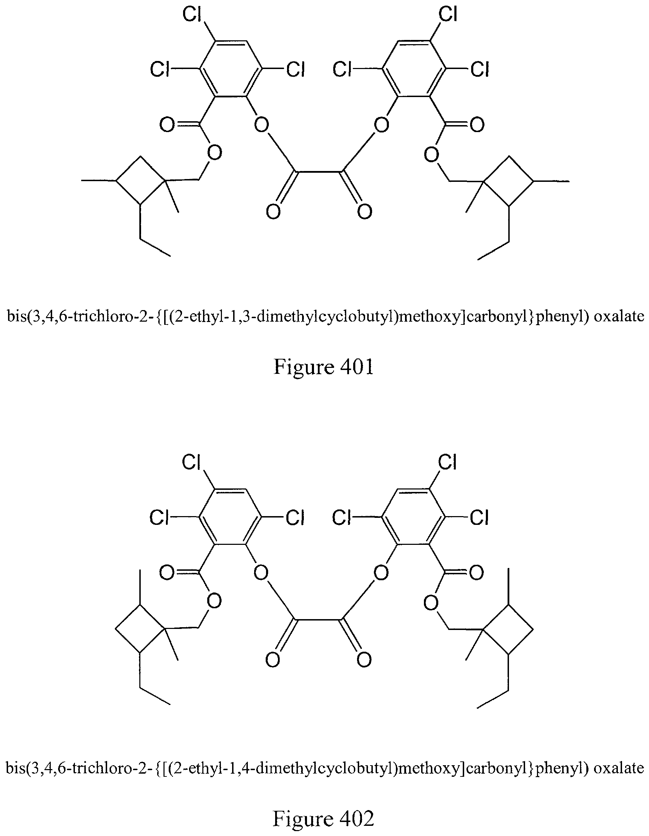

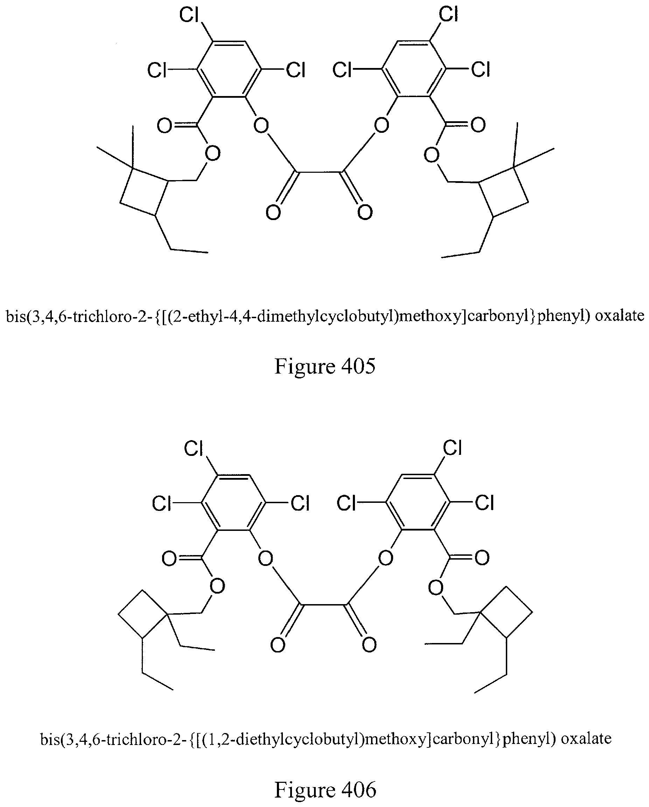

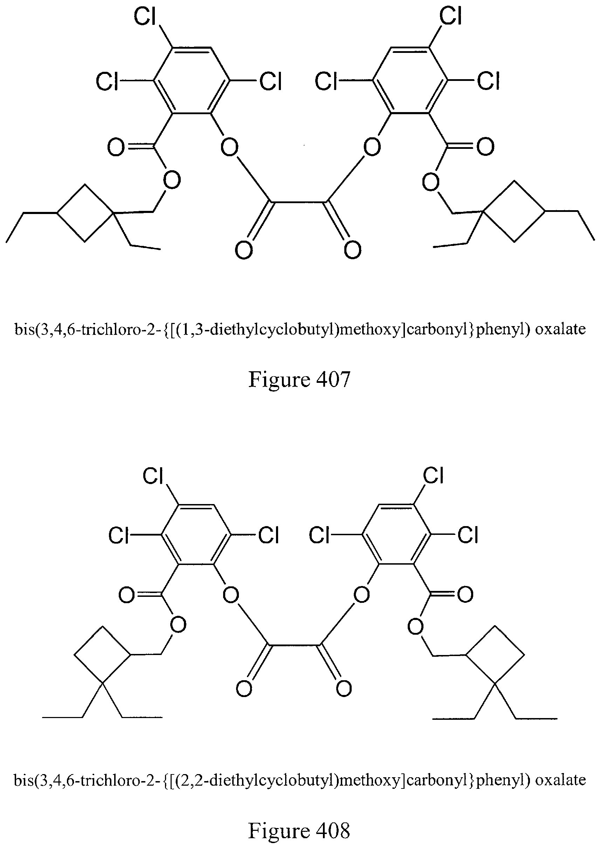

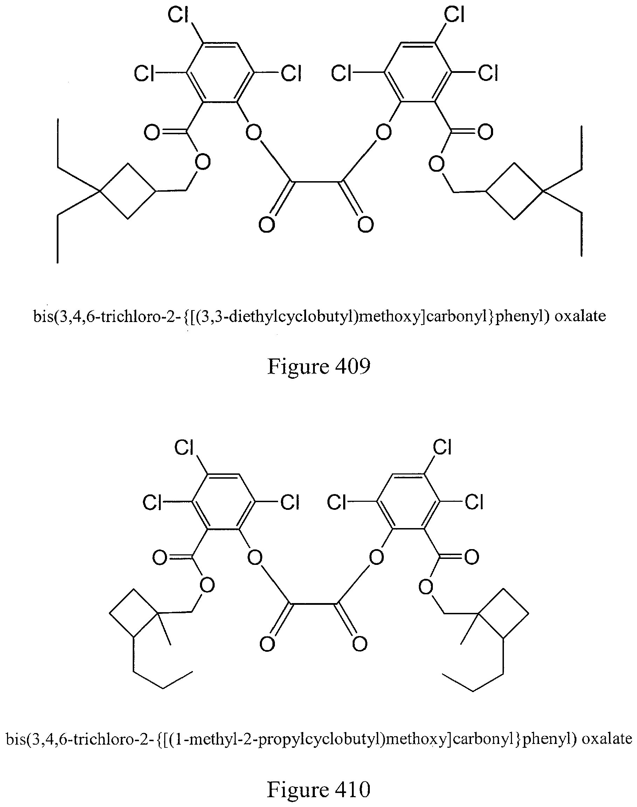

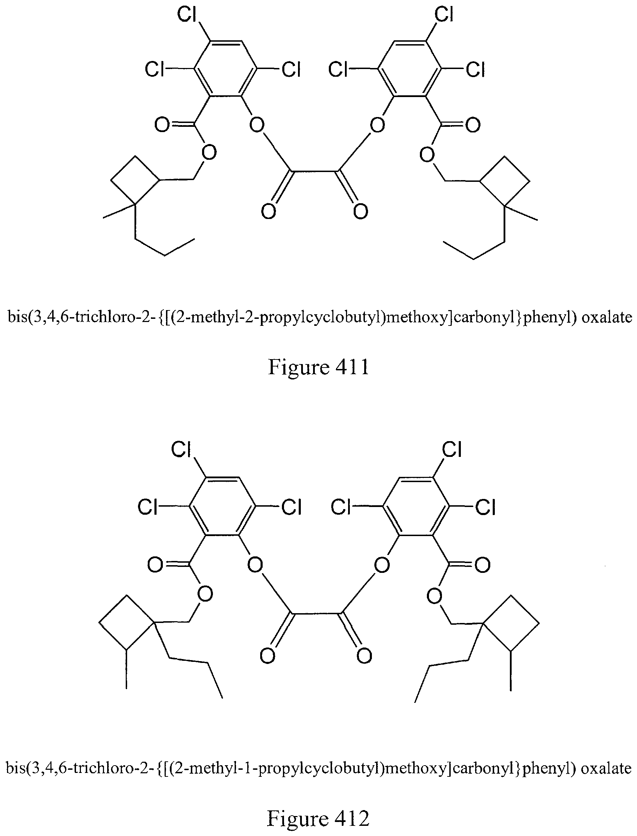

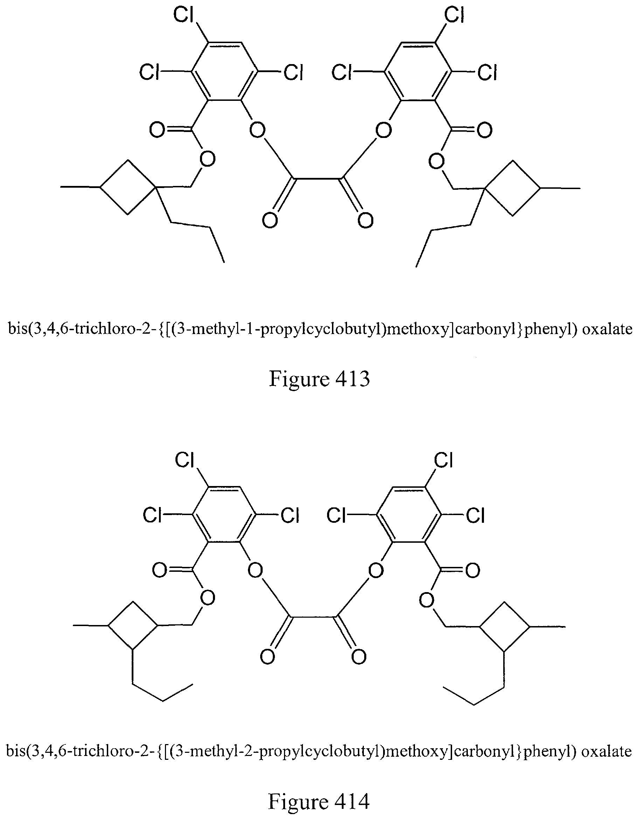

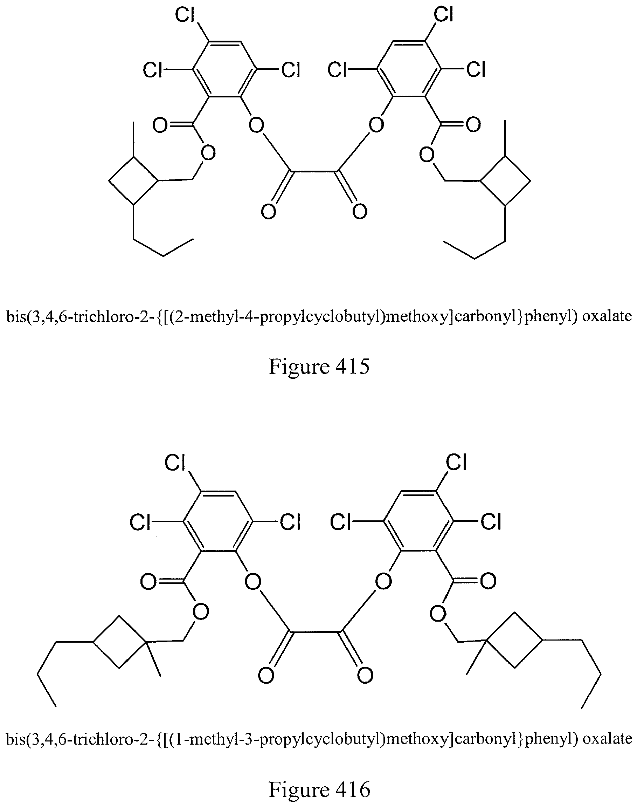

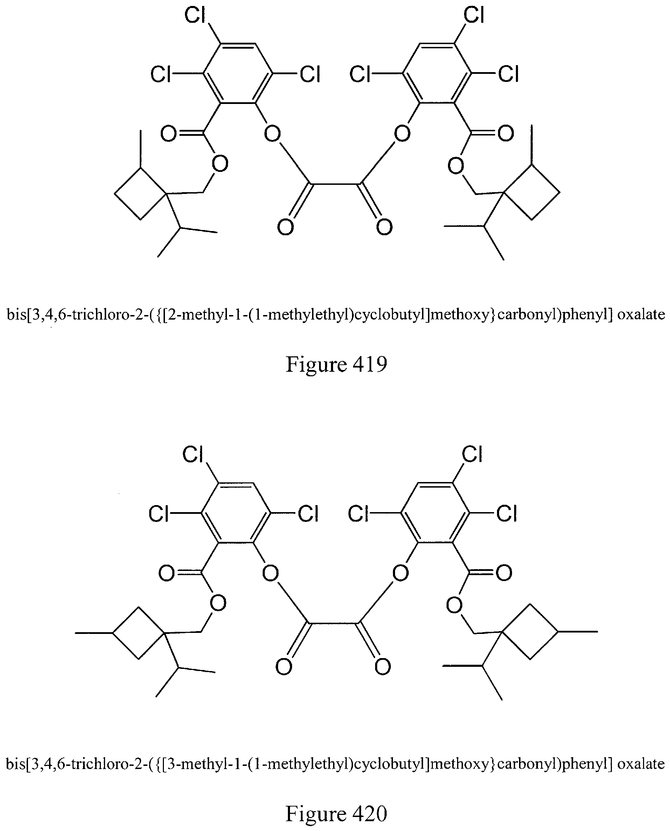

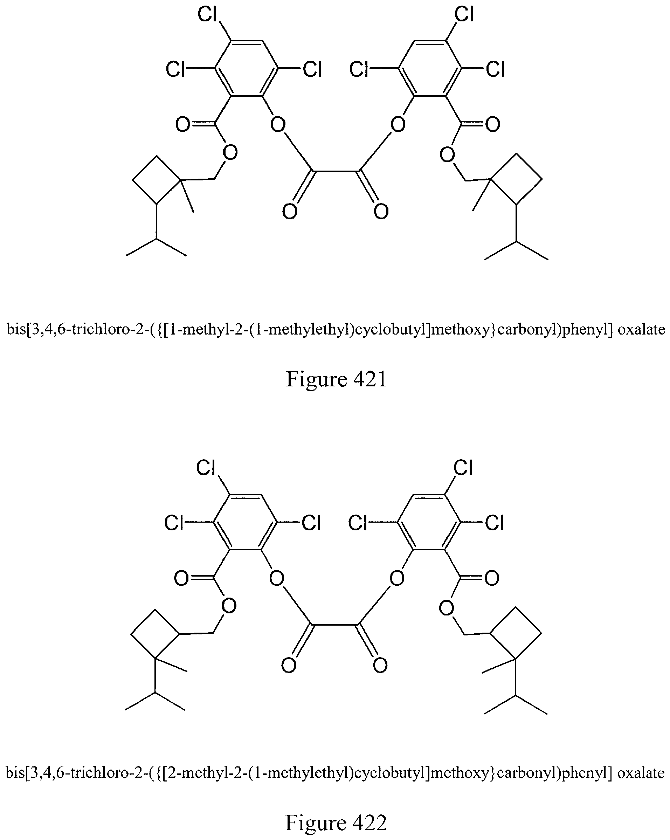

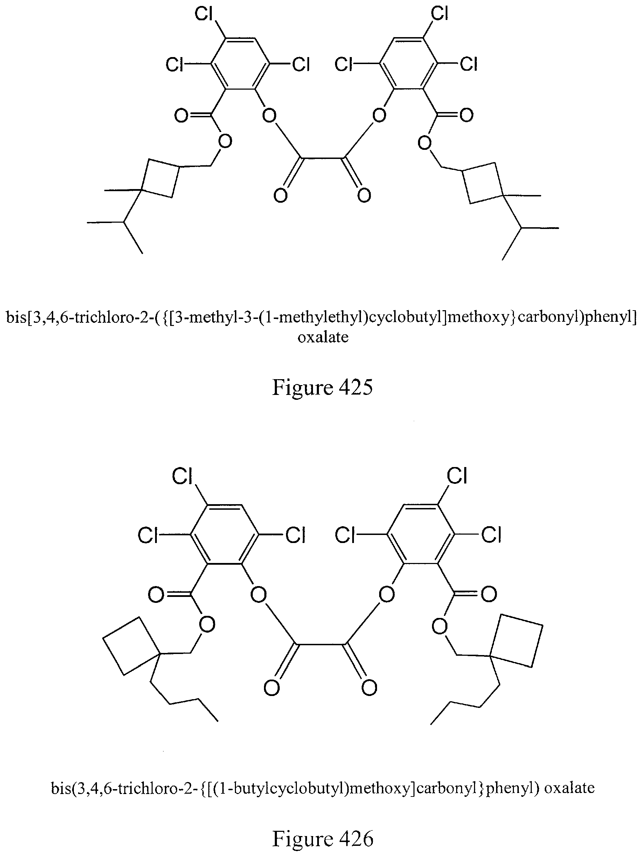

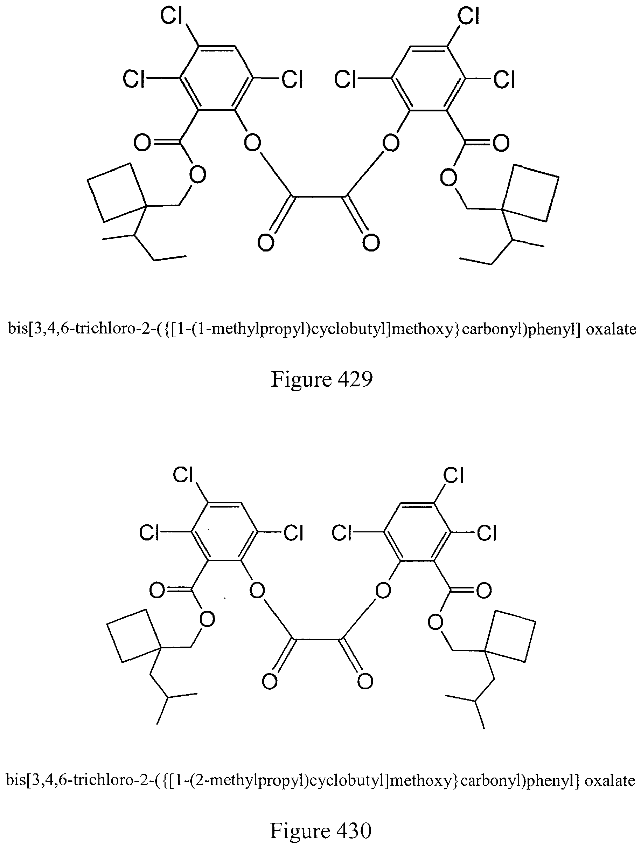

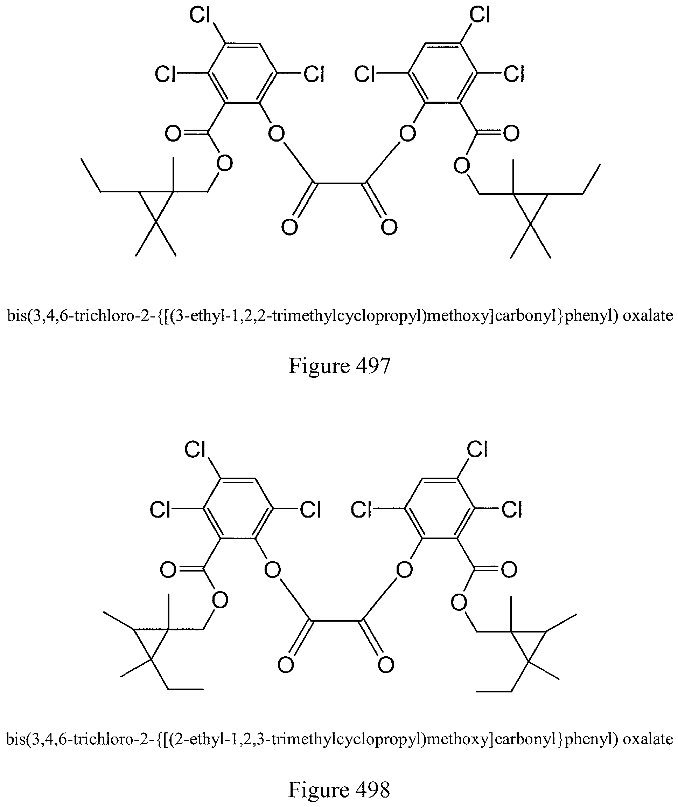

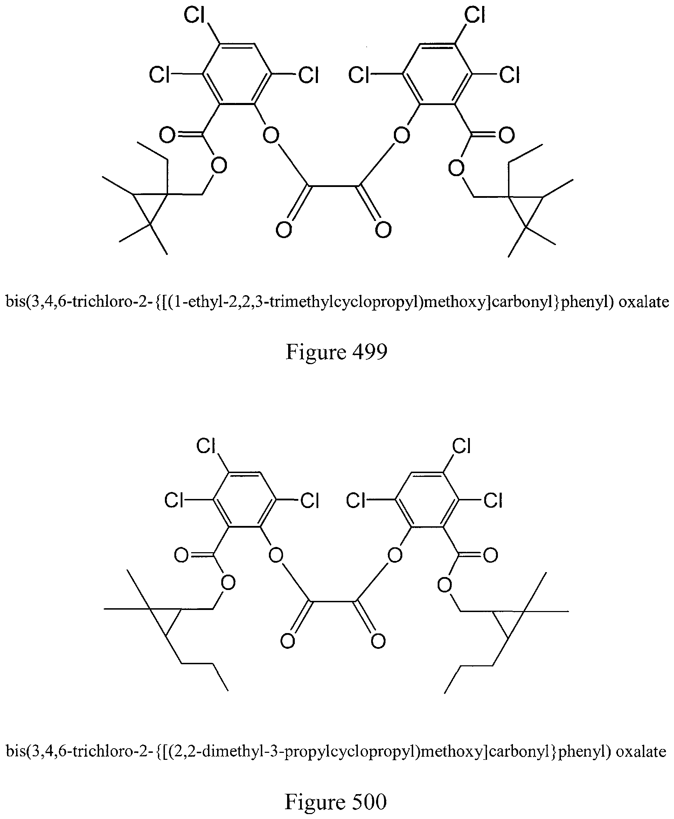

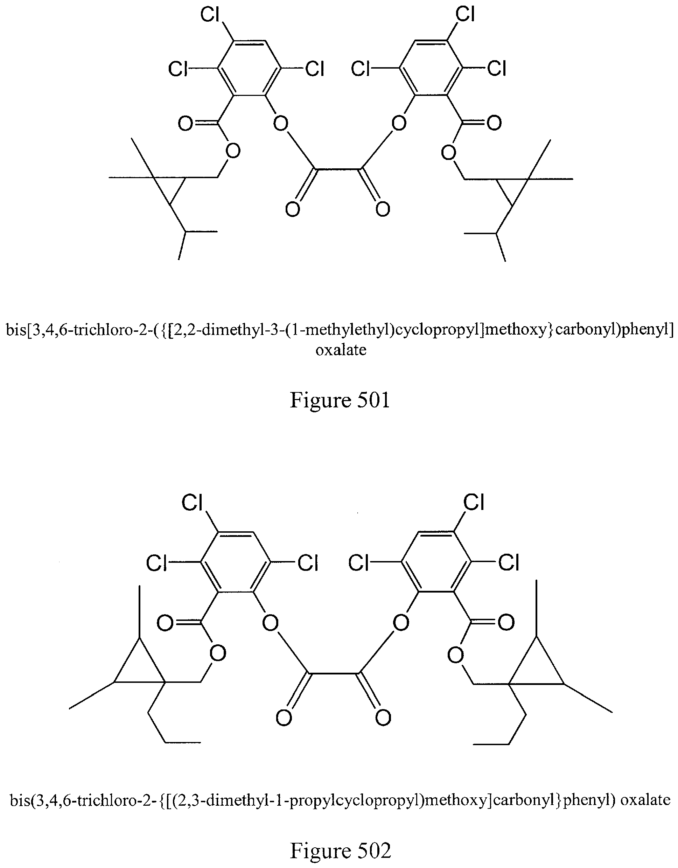

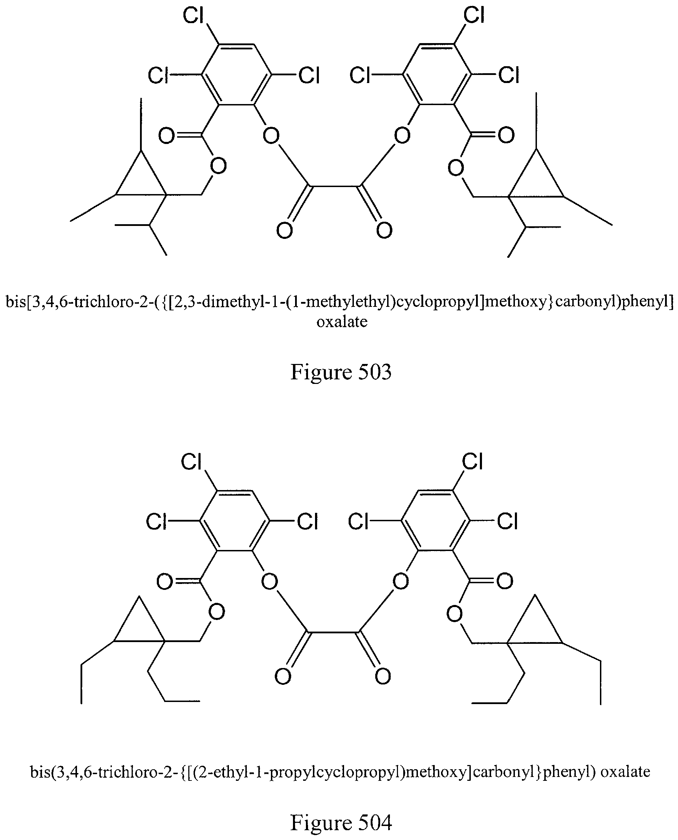

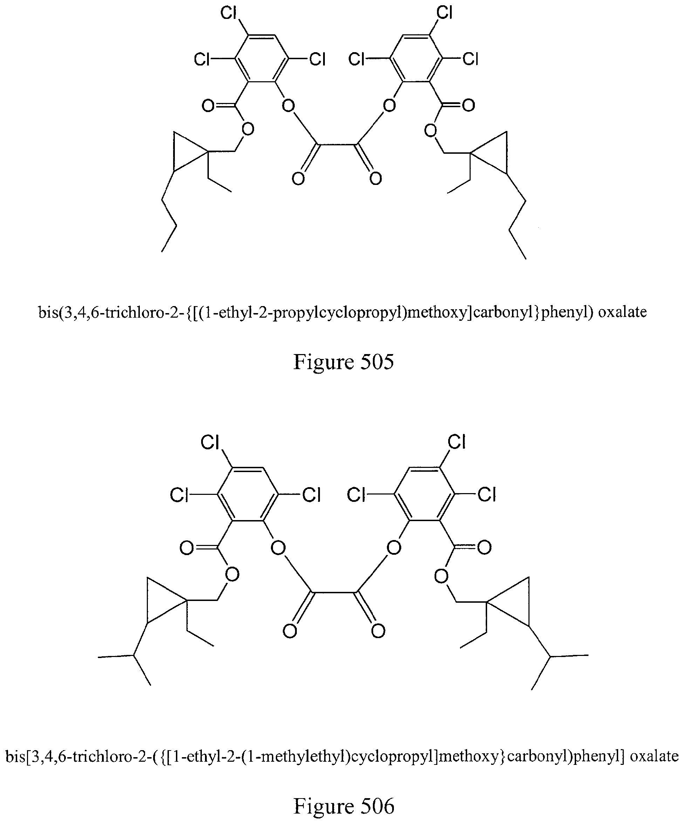

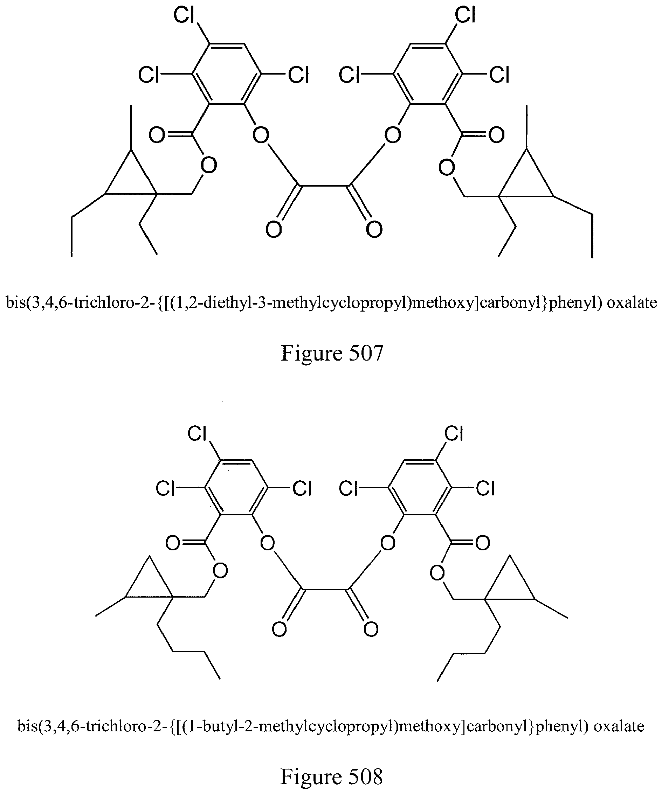

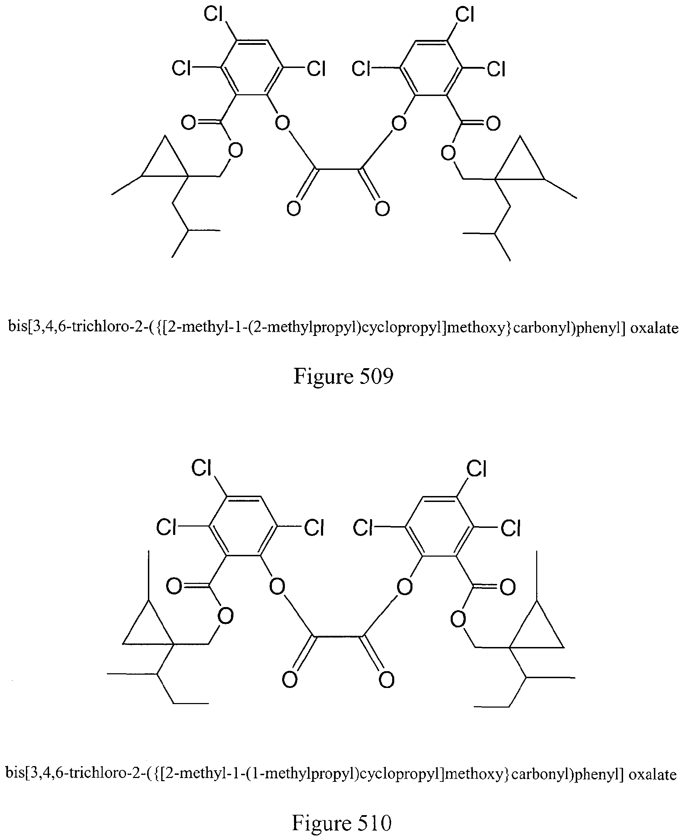

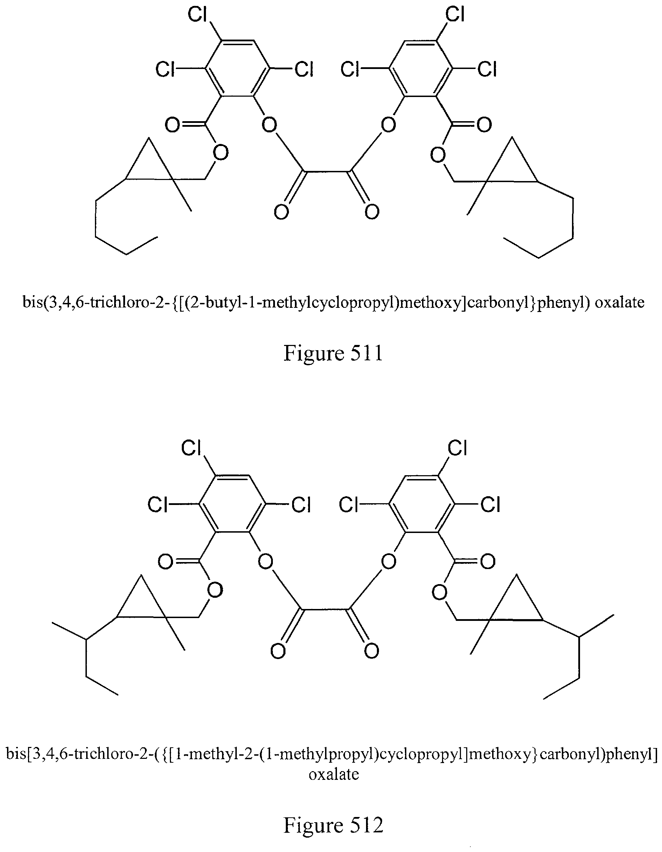

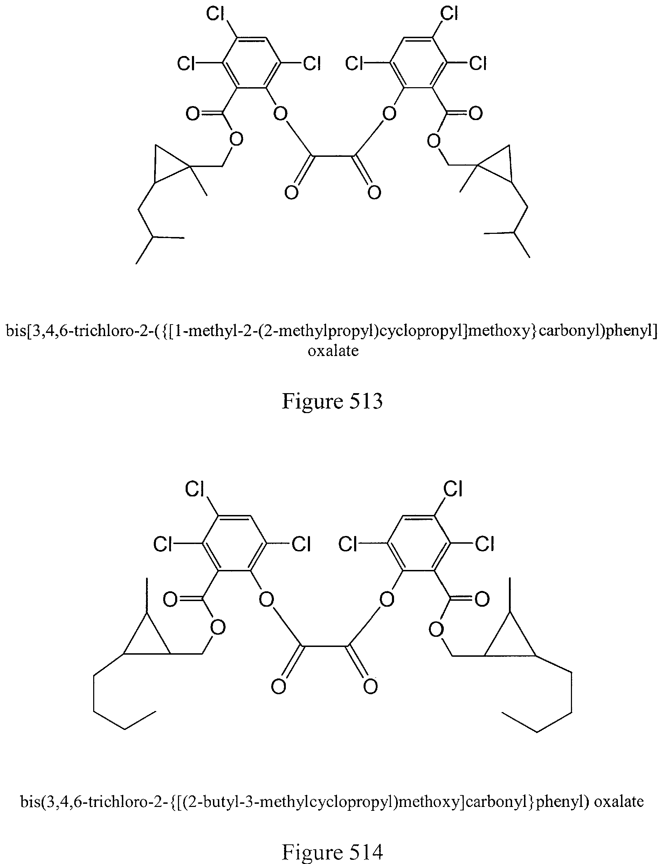

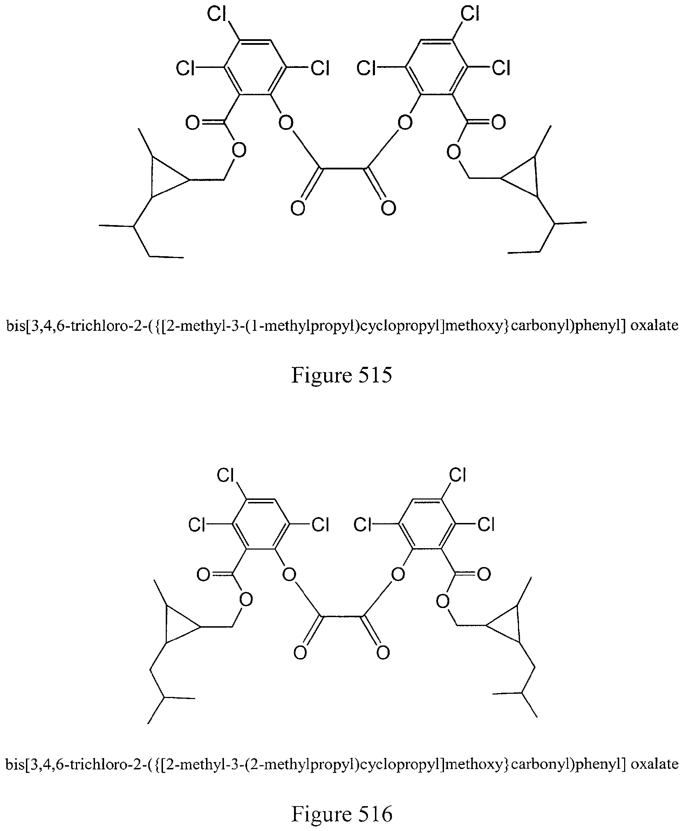

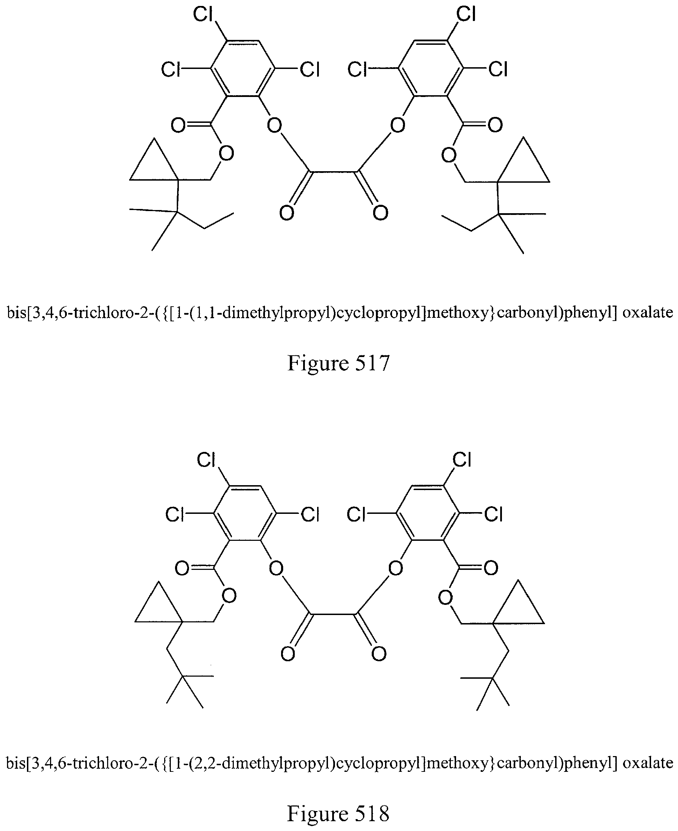

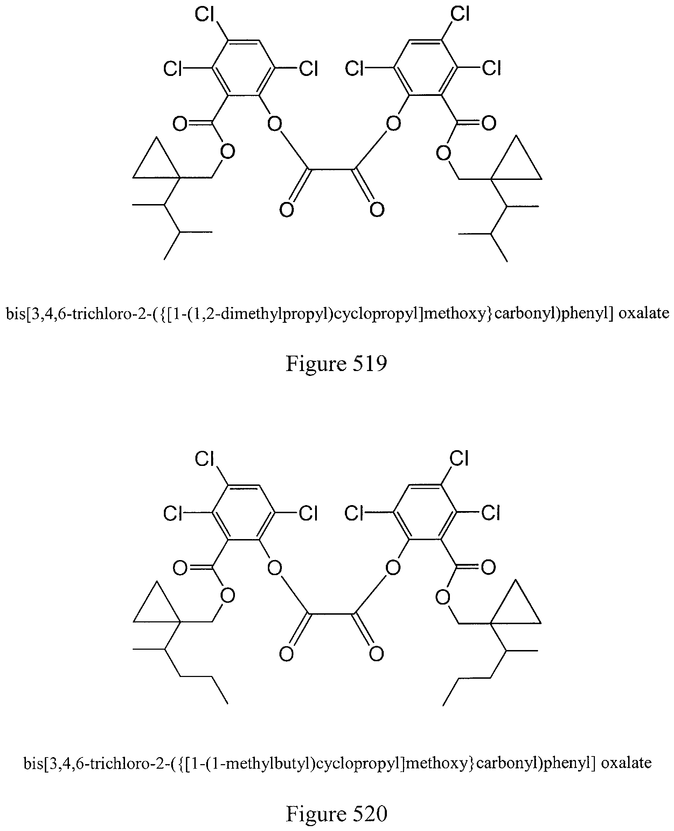

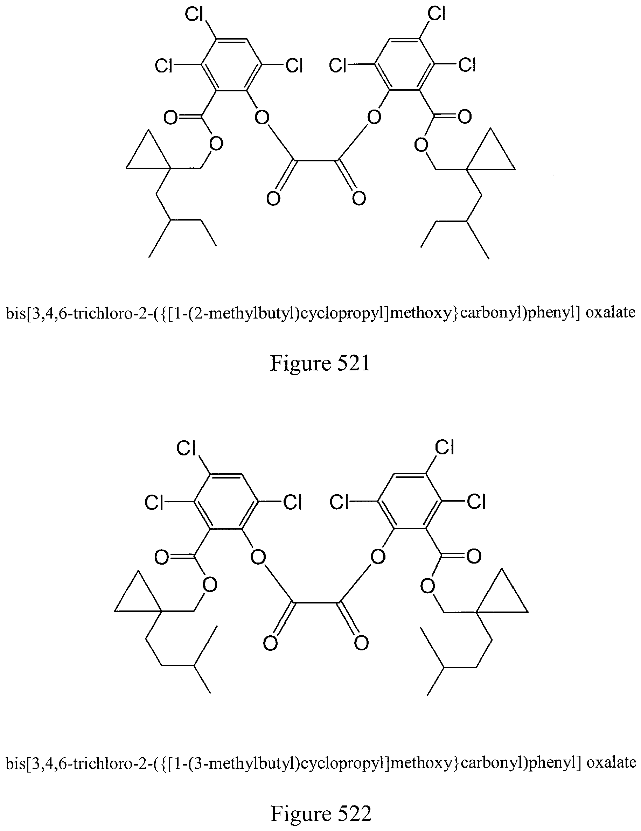

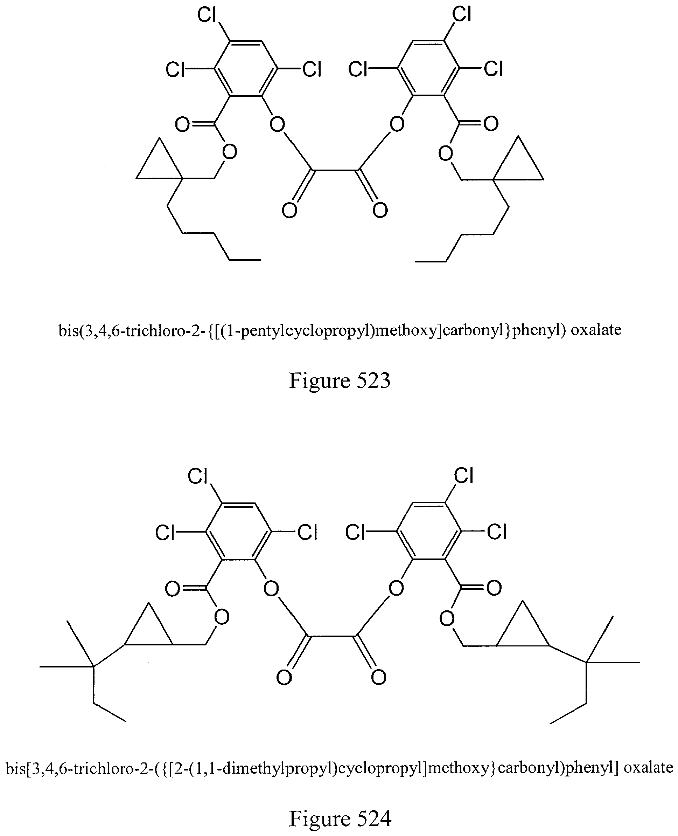

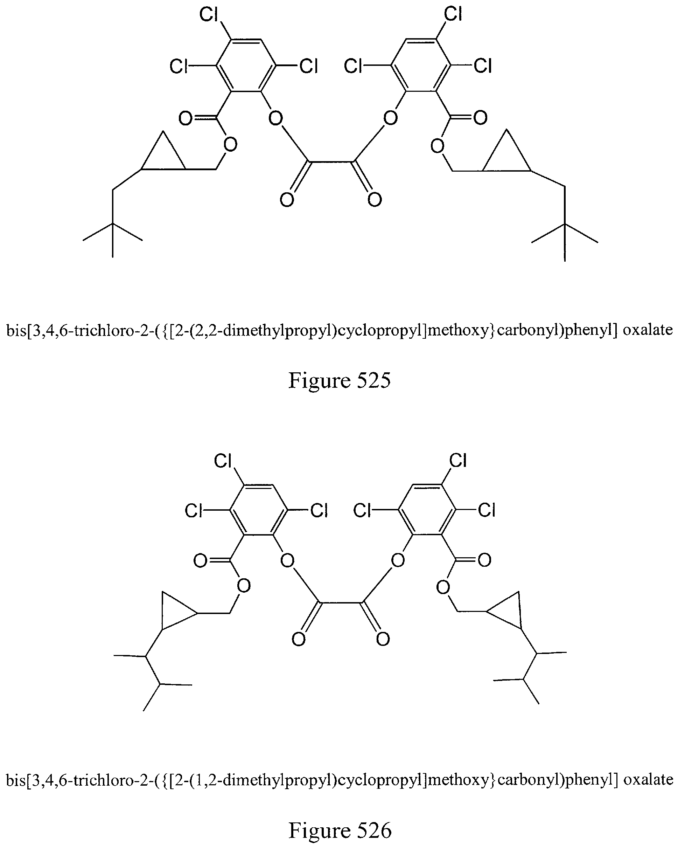

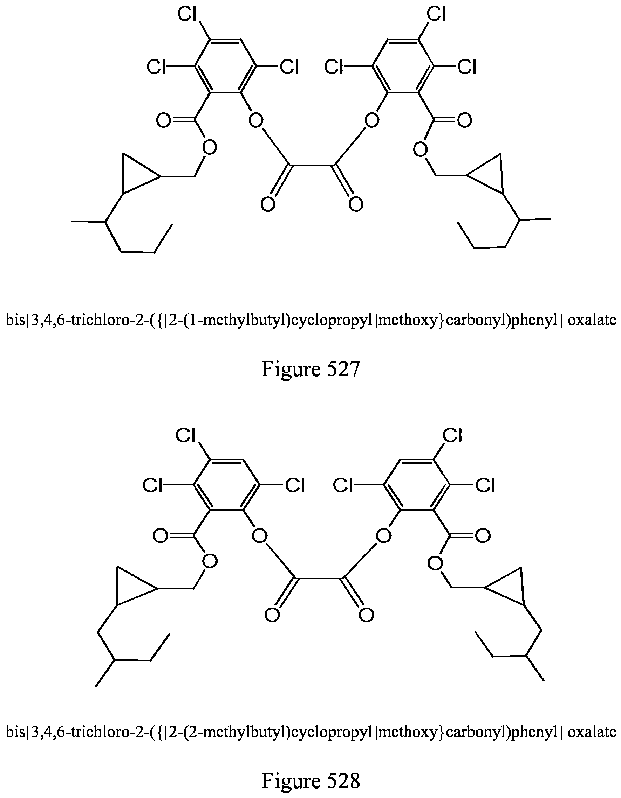

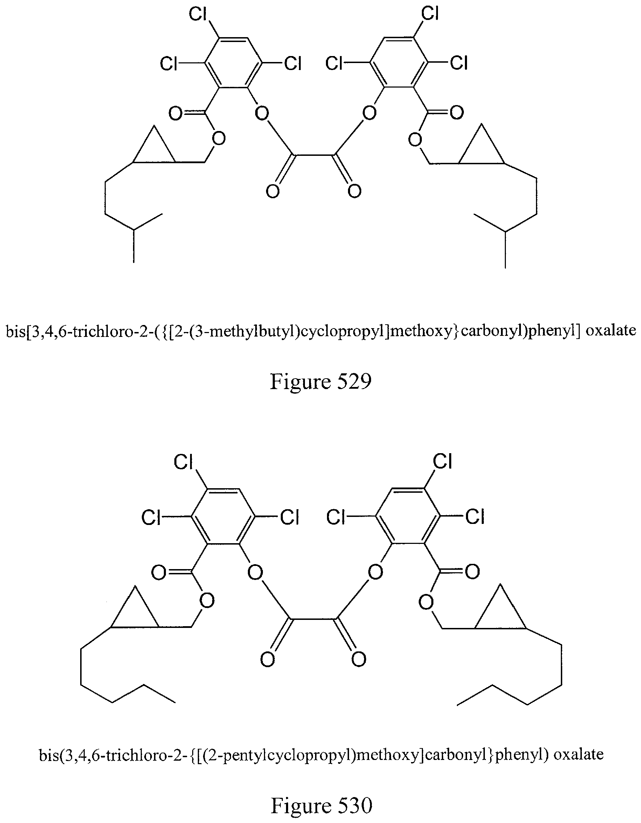

This method was used for the compounds shown in Figures 9, 13, 26-29, 35, 51, 60-69, 71-84, 92, 105, 107, 116, 118-119, 126, 138-142, 156-181, 192-224, 226, 236, 255-258, 283-285, 287-288, 295, 303, 305, 307, 311-315, 324, 328-334, 338, 342-345, 348-351, 358, 360, 363, 366-367, 371-391, 396-402, 406-407, 410, 412-413, 416, 419-422, 426, 429-430, 435-617, 628-632, 644, 649-652, 654, 657, and 660.

Additionally, a tertiary amine other than triethylamine can be used, including tripropyl amine, tri-n-butyl amine, triisobutylamine, triisopropylamine, triisopentylamine, and N,N-diisopropylethyl amine.

### EXAMPLE 4

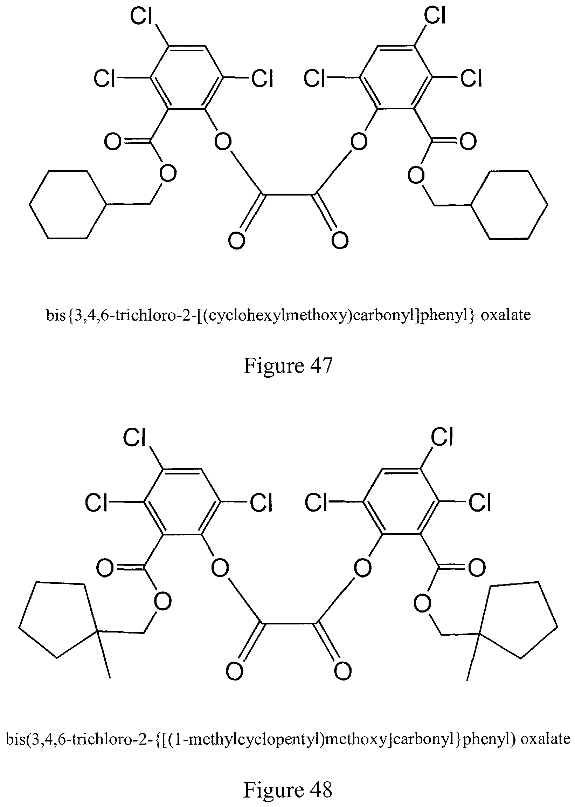

### Procedure For Producing Bis{3,4,6-Trichloro-2-[(Cyclohexylmethoxy)Carbonyl]Phenyl} Oxalate (Figure 47)

The 3,4,6-trichlorosalicylic acid (178.7 g, 0.74 mole) was placed in a 2 L 3-necked flask equipped with a mechanical stirrer and a Dean-Stark trap with an efficient reflux condenser. Xylene (490 mL), cyclohexylmethanol (100.2 mL, 0.81 mole), and titanium oxide bis(acetylacetonate) (2.9 g, 11 mmol) were added in the indicated order. The reaction mixture was heated at reflux overnight, and the water removed from the Dean-Stark trap. The reaction product was cooled to 35°C and 500 mL of saturated sodium bicarbonate solution was added. The mixture was stirred for 2 hours. Material started to come out of solution after 1/2 hour. Ethyl acetate (800 mL) was added, which dissolved the ester, and stirring was continued. The layers were separated, washed with brine, and dried with MgSO4. The organic layer was filtered and concentrated under reduced pressure. Once all of the material had been concentrated, an aliquot of xylene was added and removed under reduced pressure to ensure complete removal of any excess alcohol and water. The yield was 248 g (99%). The material was analyzed by HPLC and used without further purification in the next step.

Bis{3,4,6-trichloro-2-[(cyclohexylmethoxy)carbonyl]phenyl} oxalate can be made using processes described Example 1. The yield using the product from the step above was 153.8 g (57%).

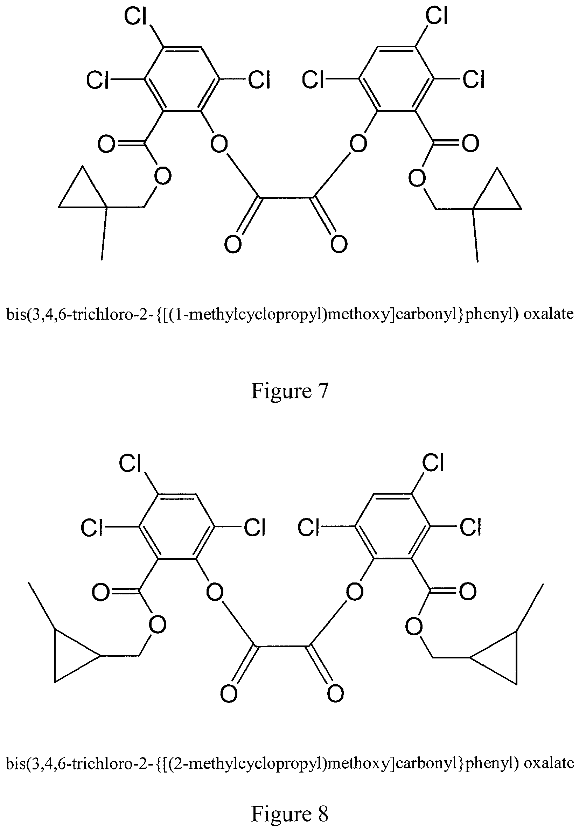

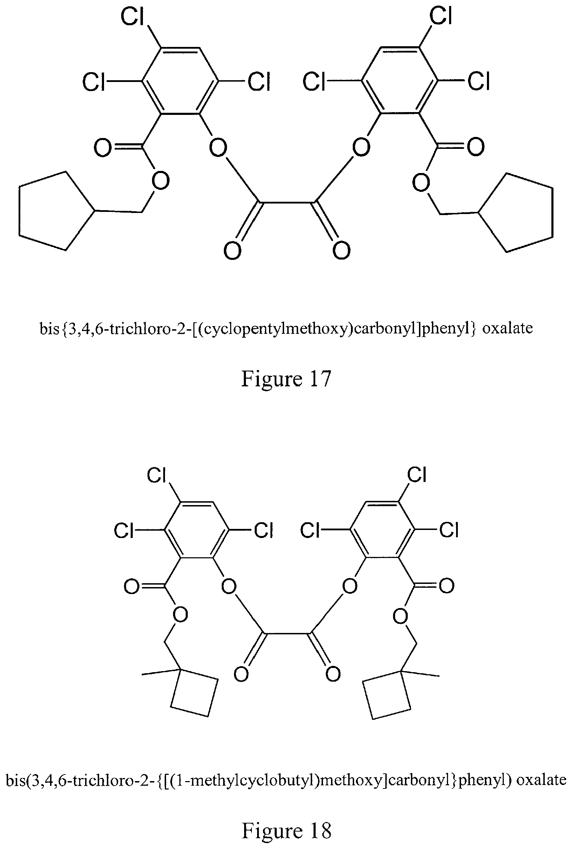

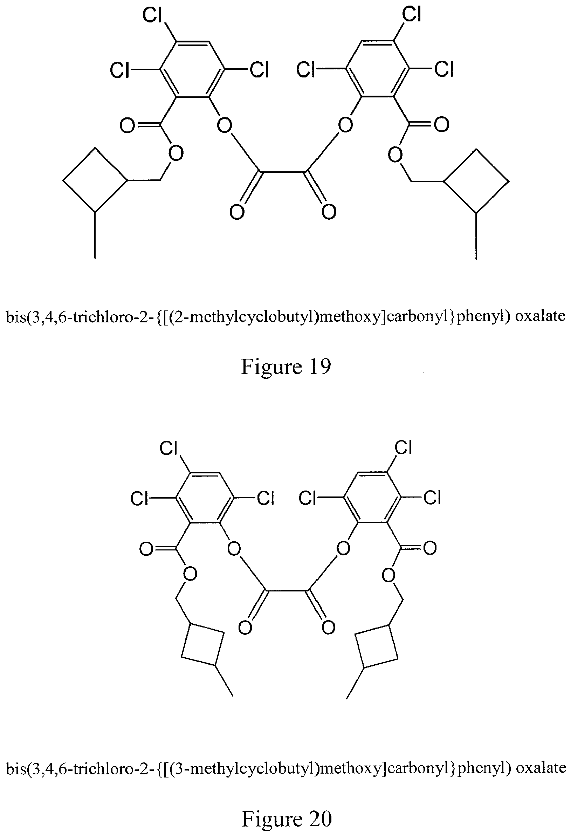

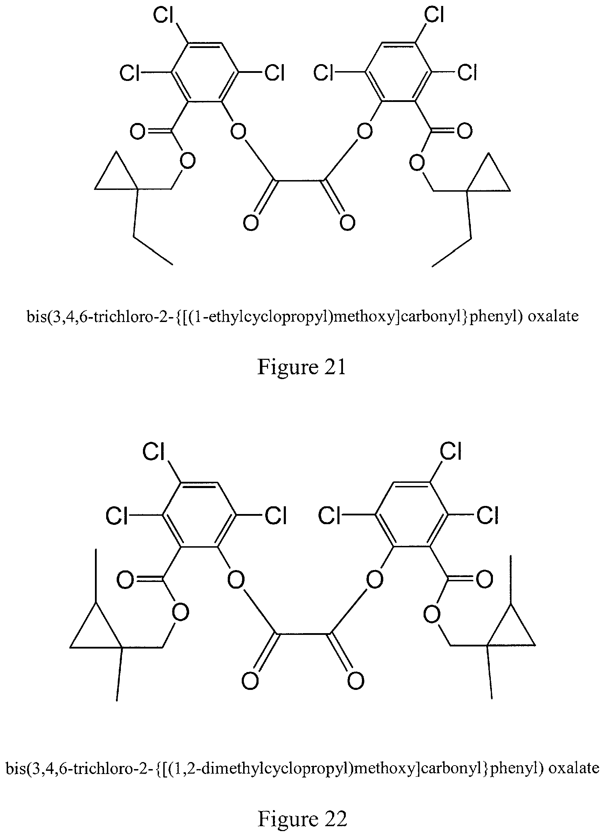

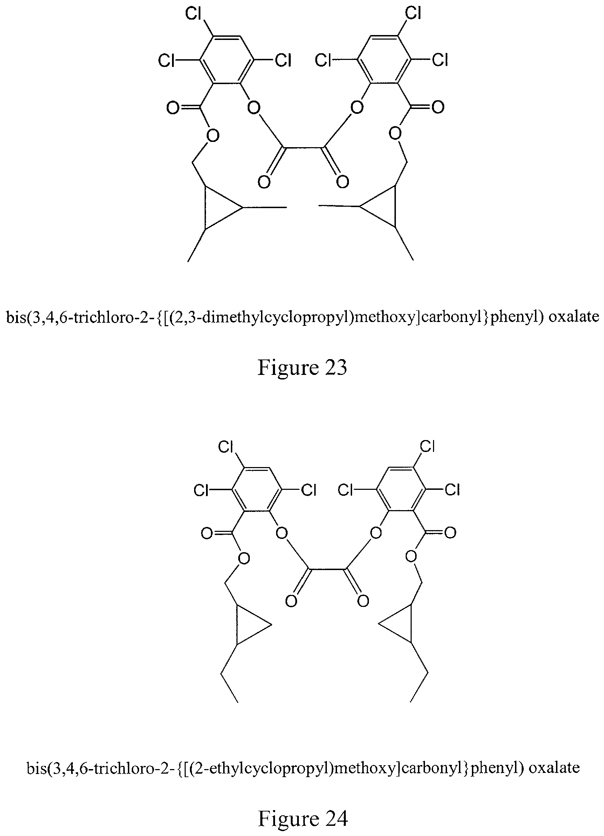

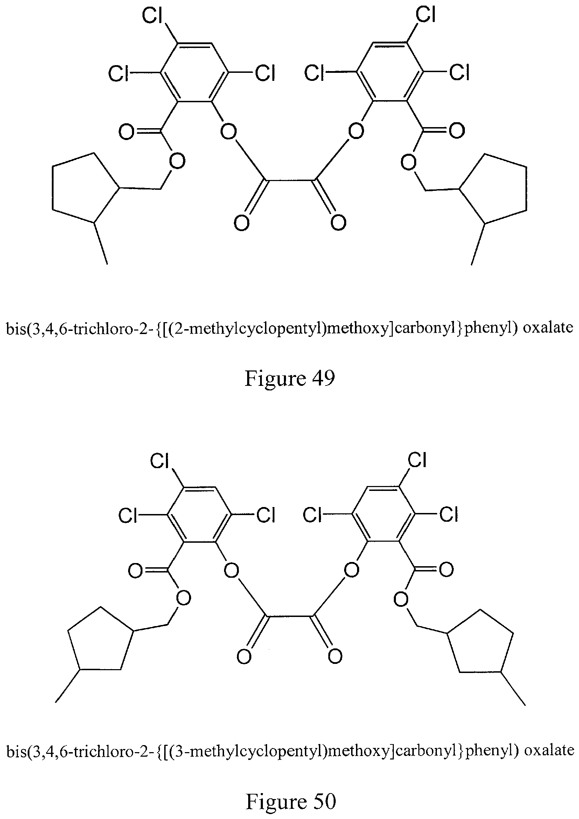

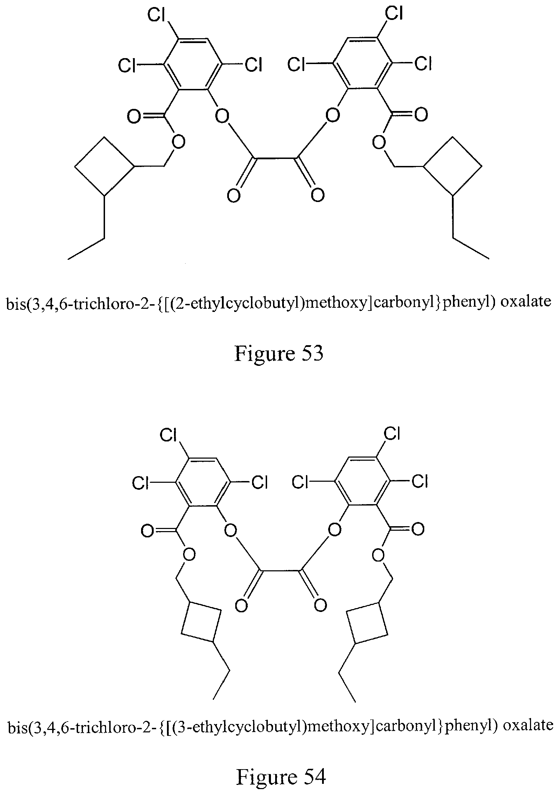

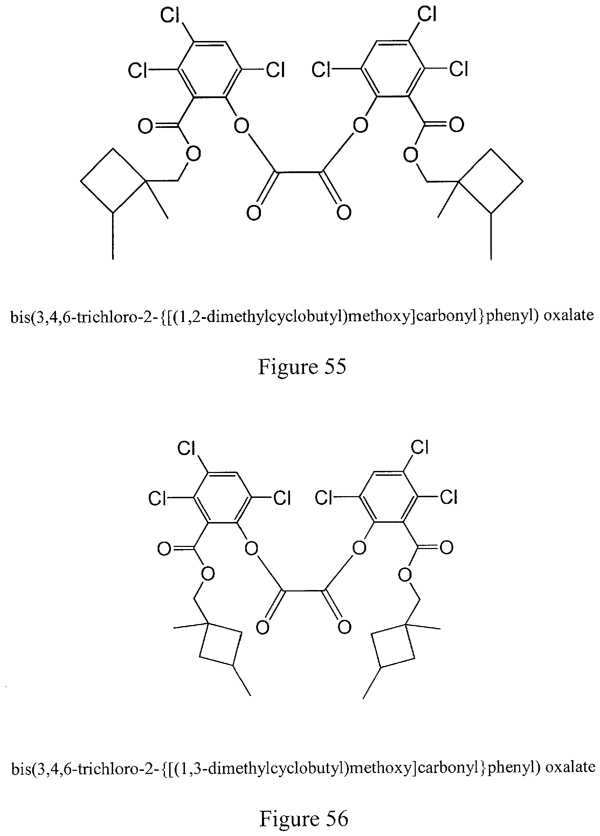

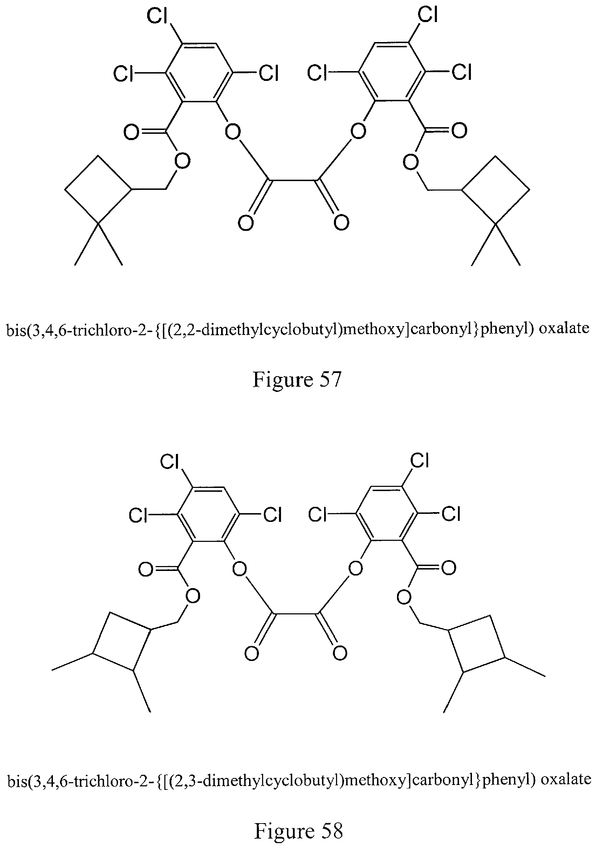

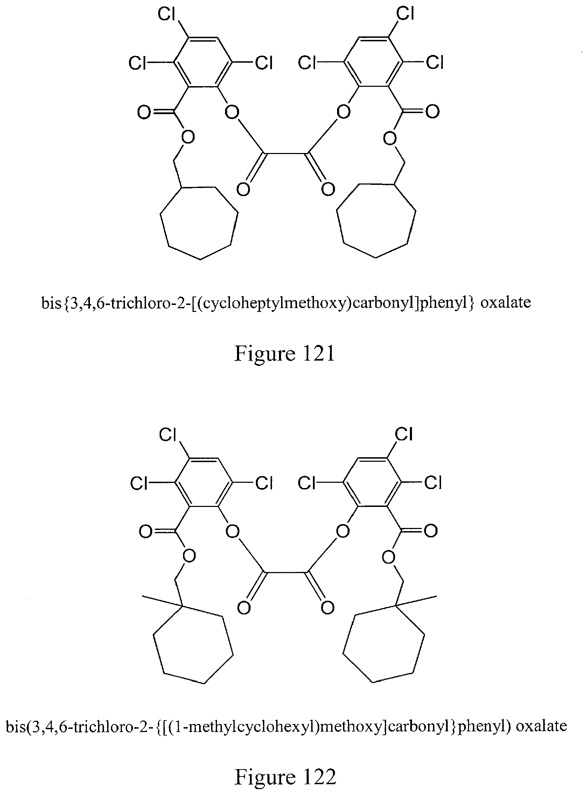

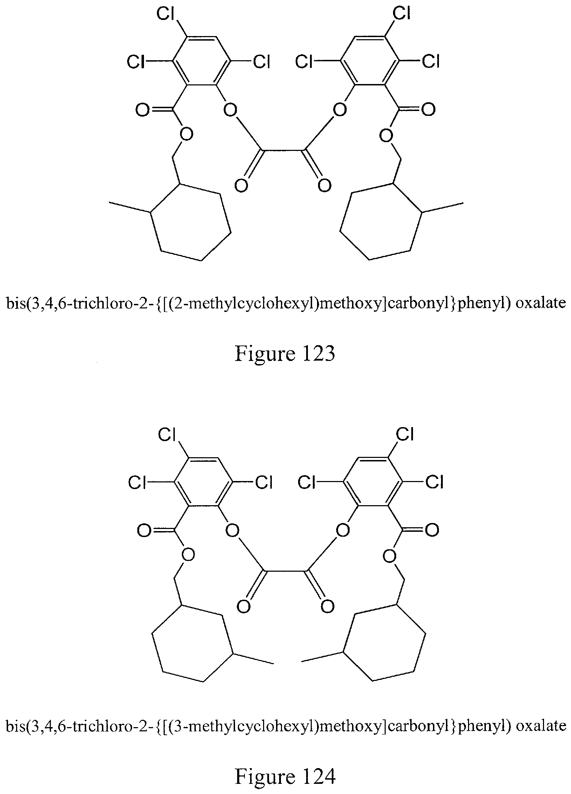

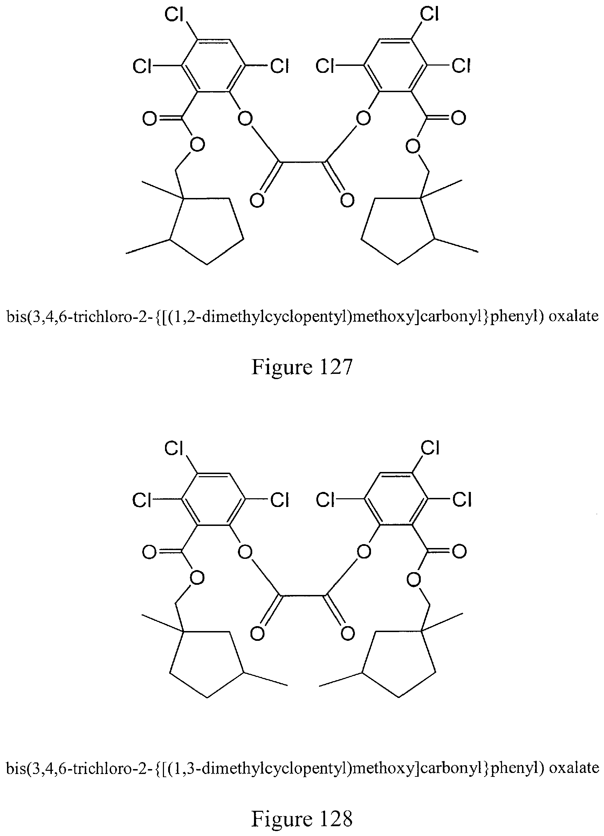

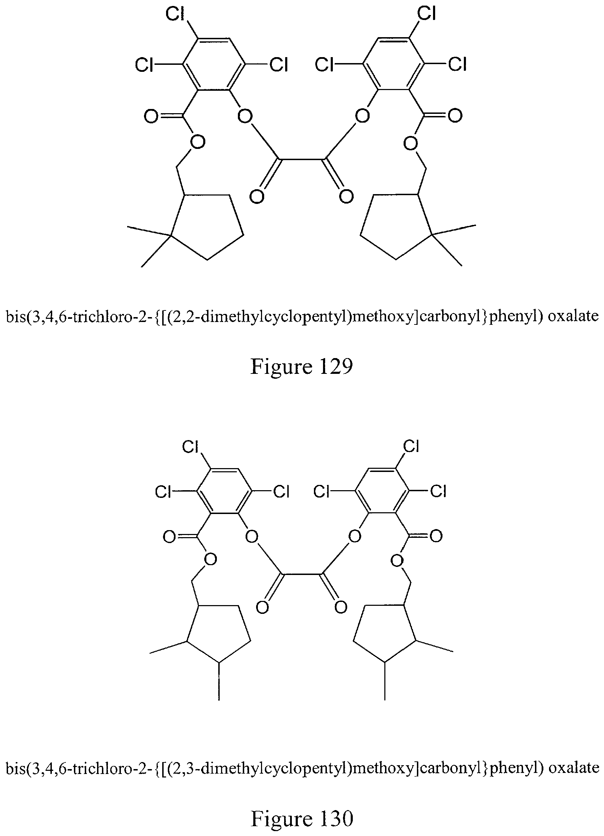

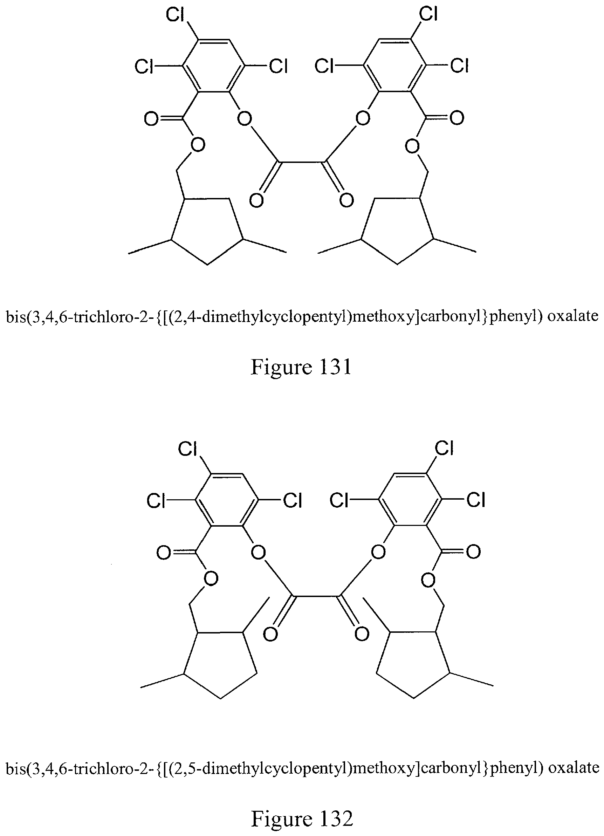

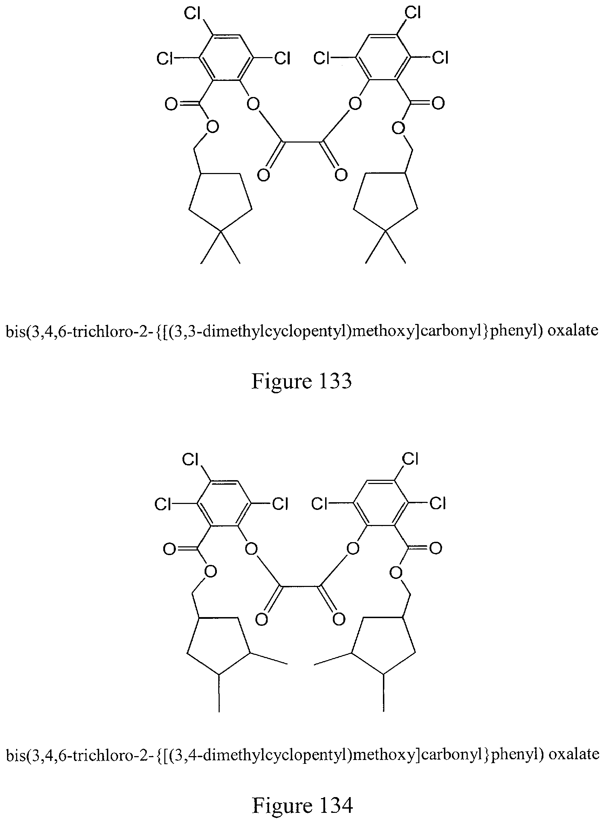

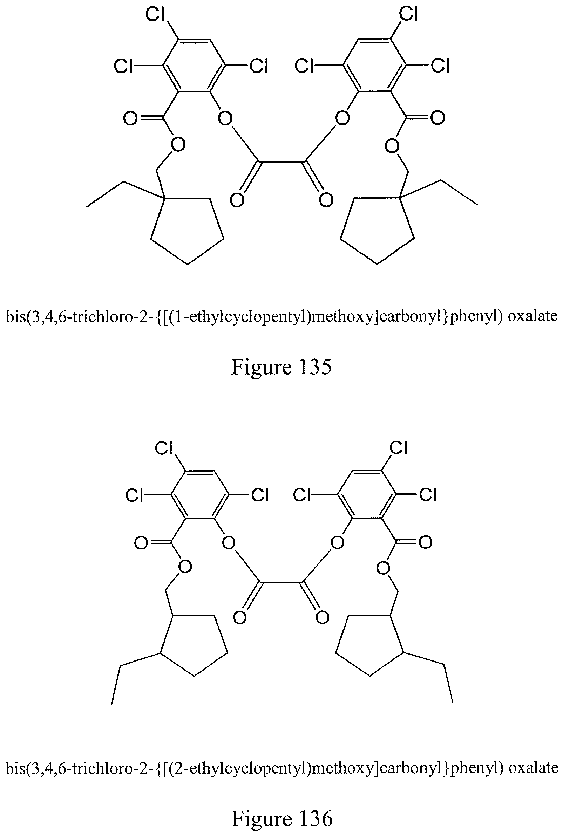

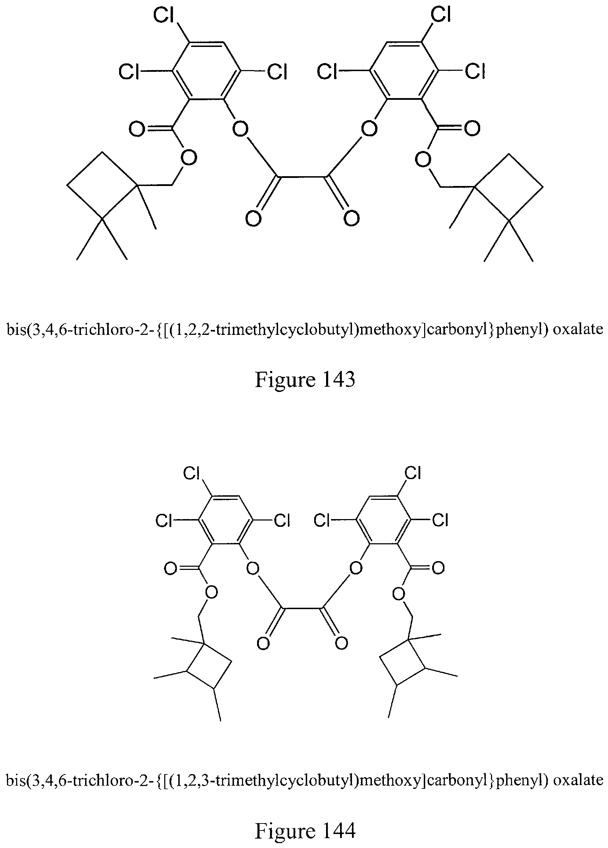

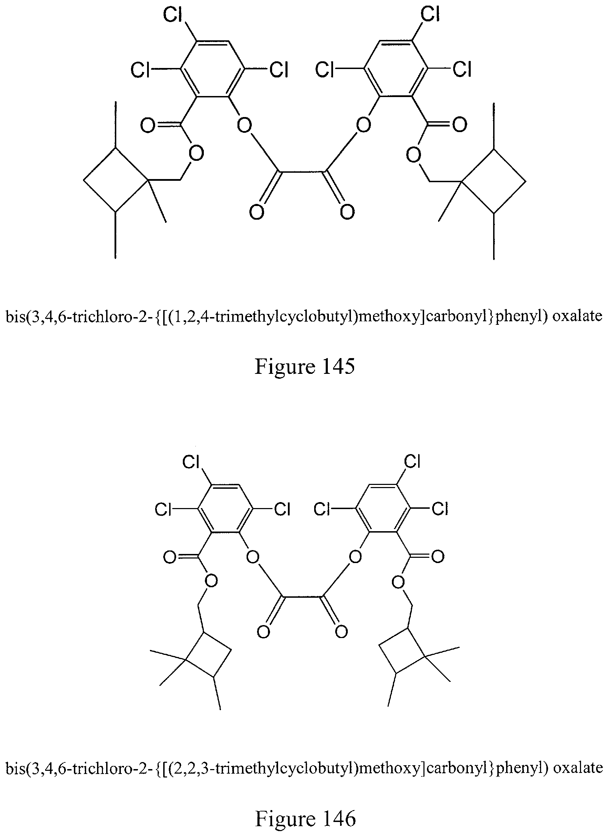

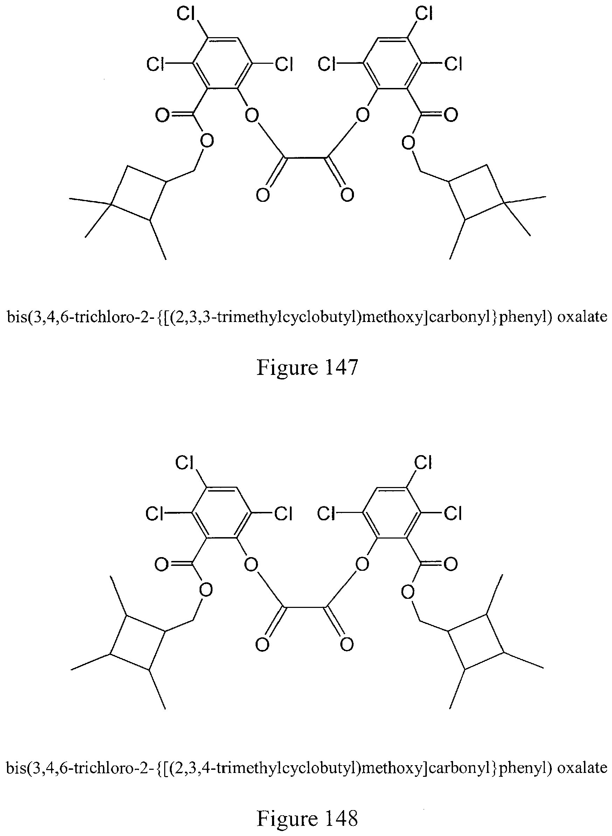

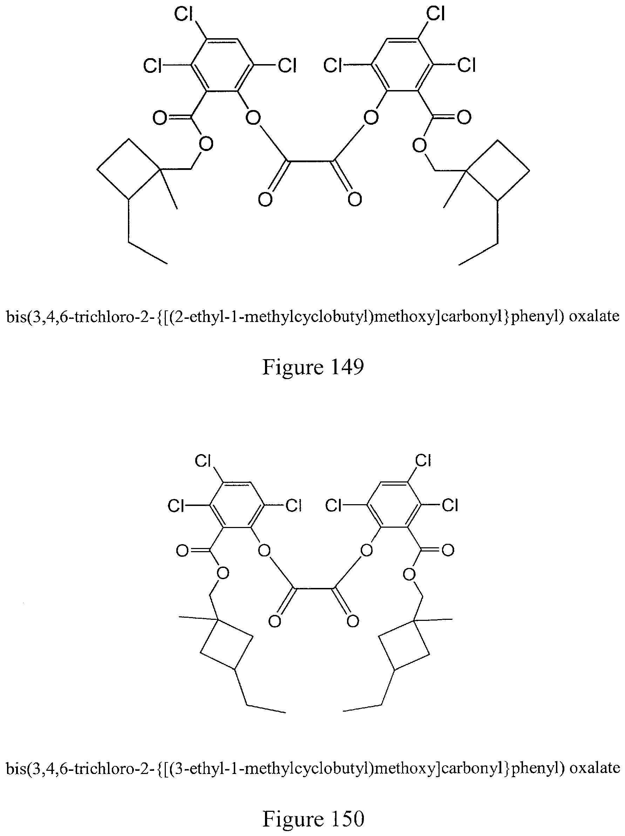

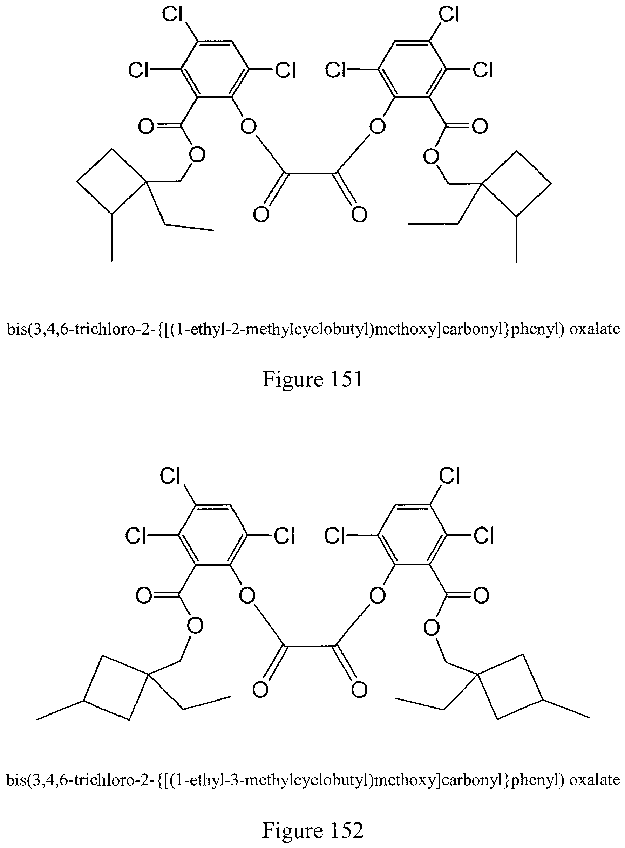

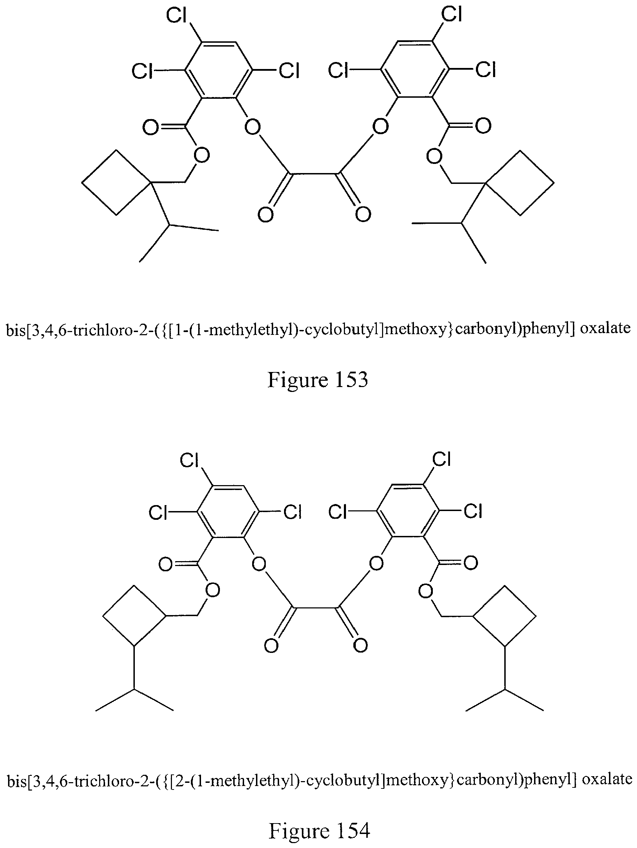

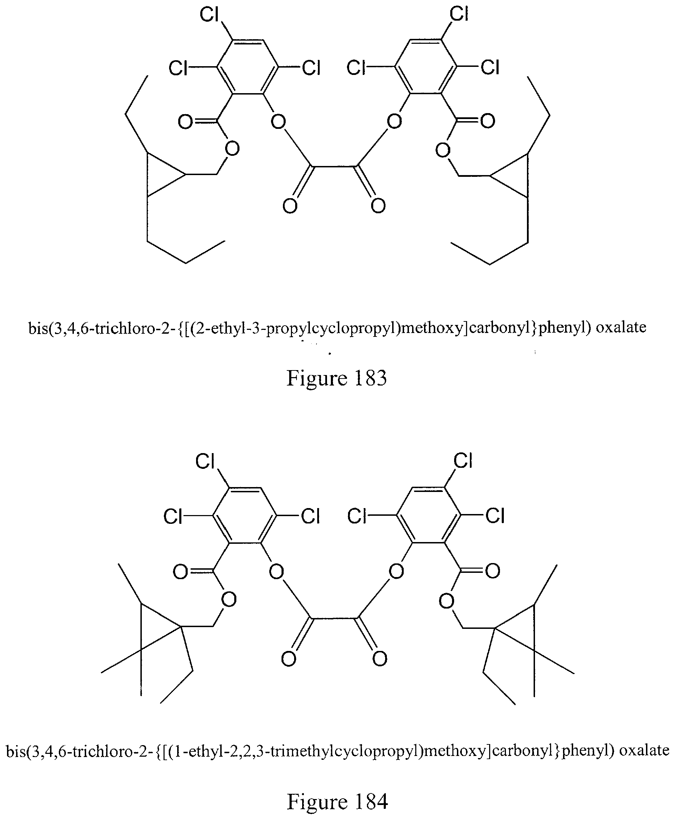

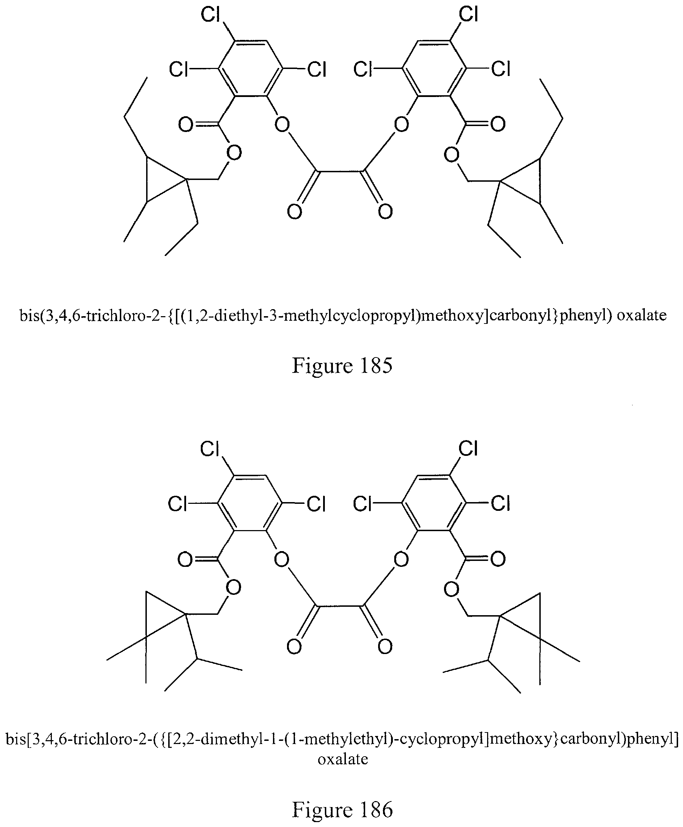

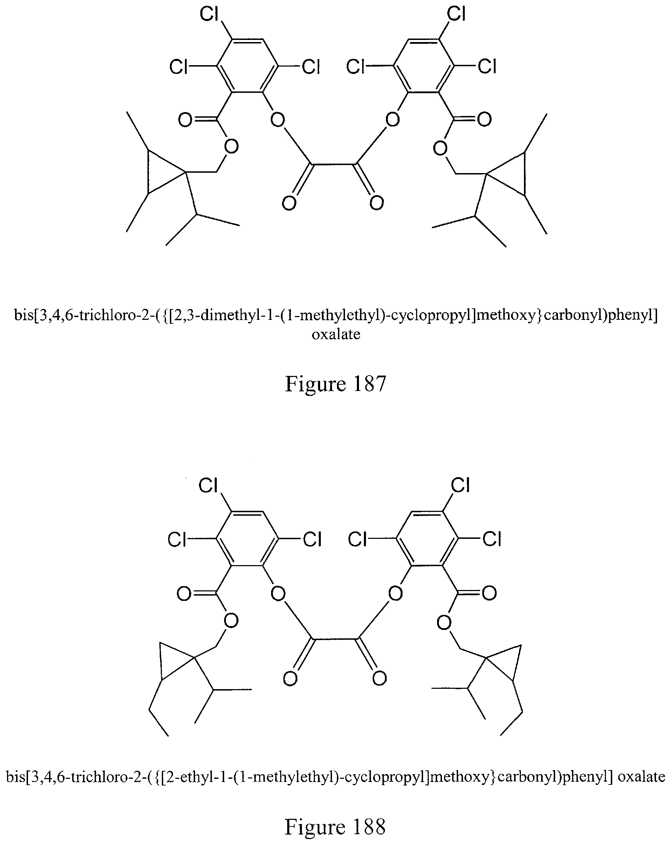

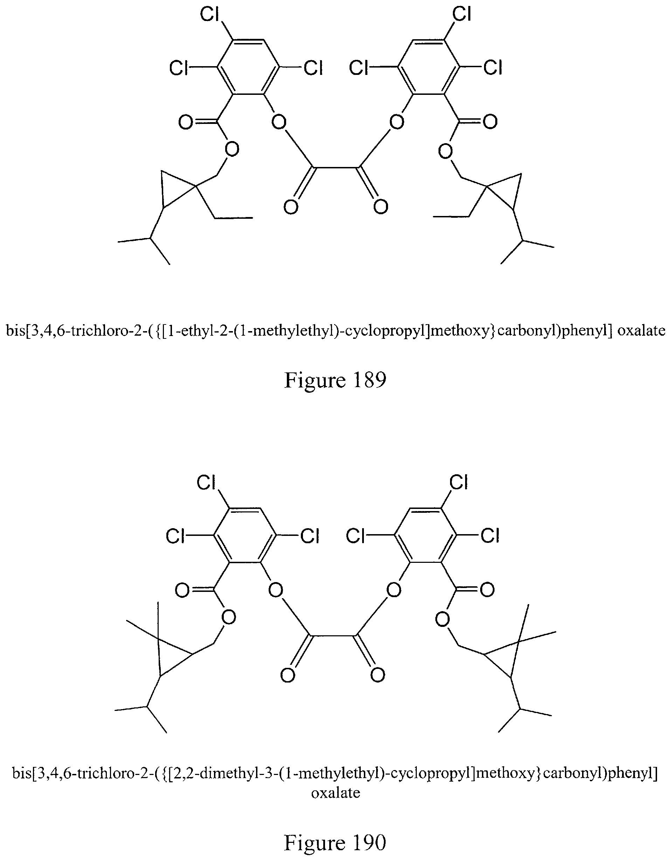

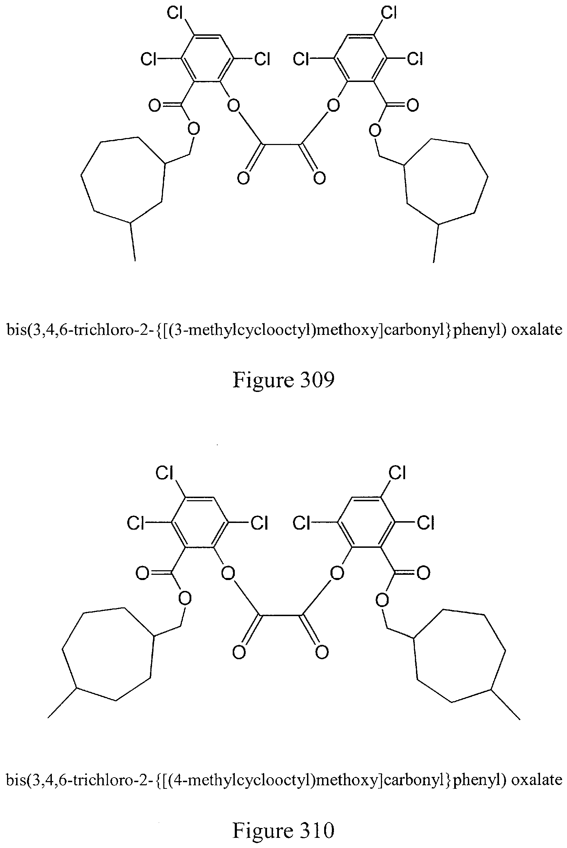

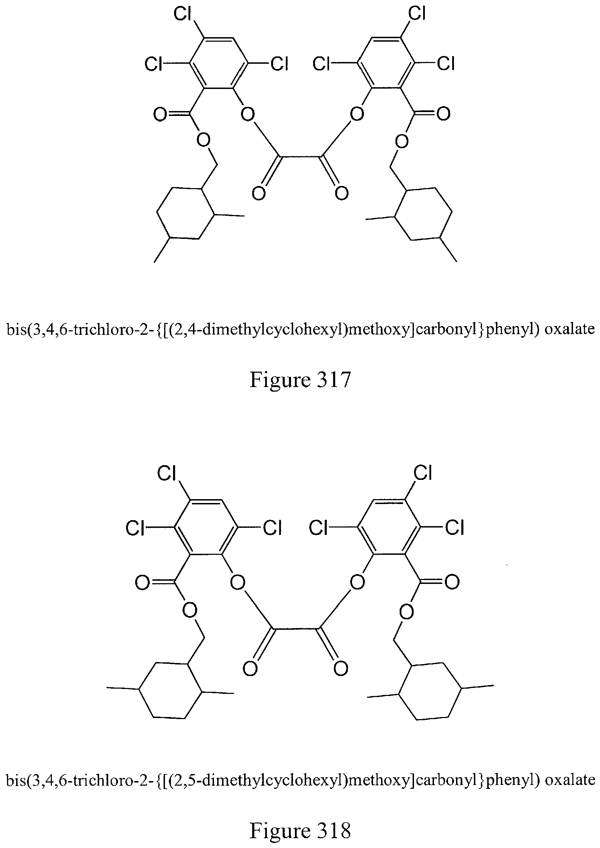

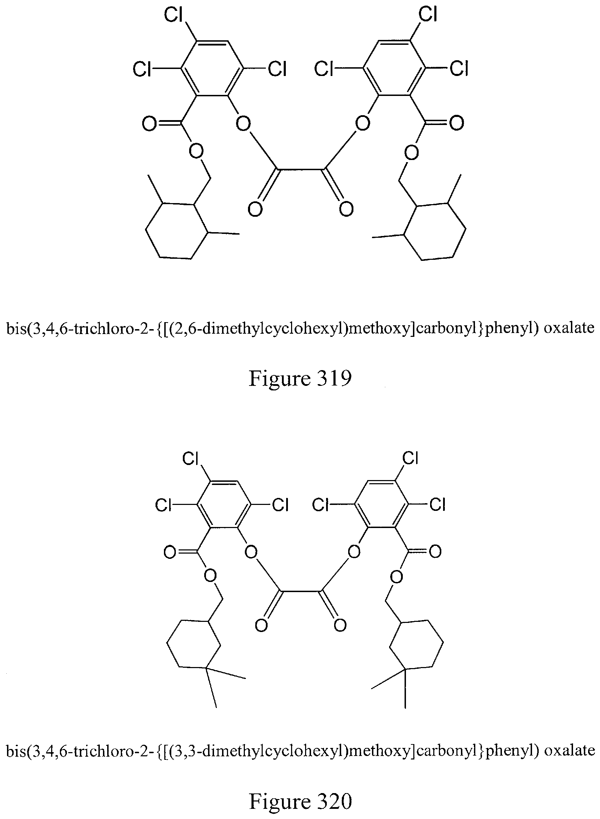

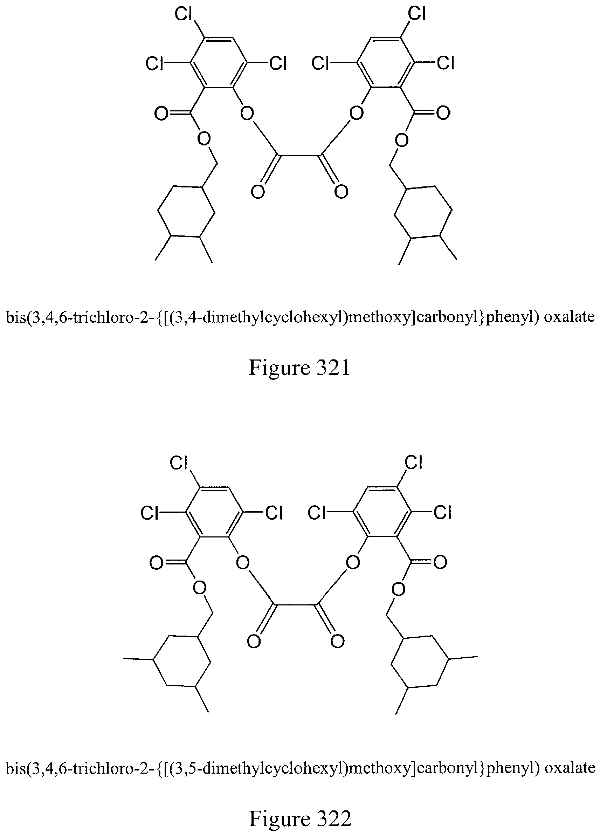

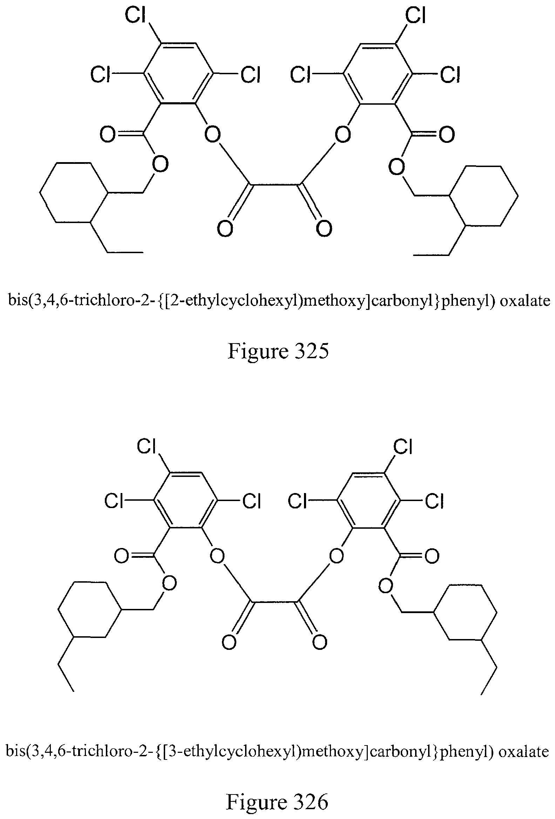

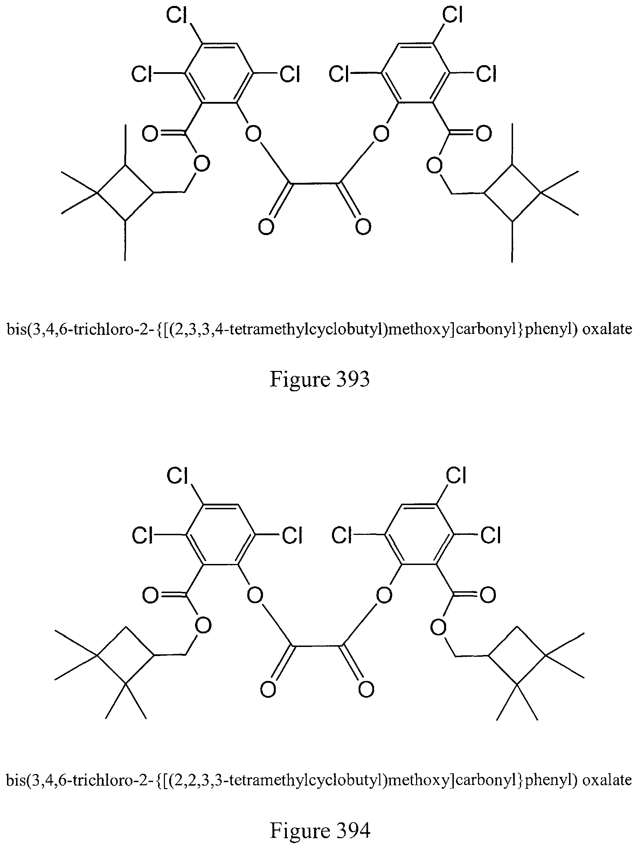

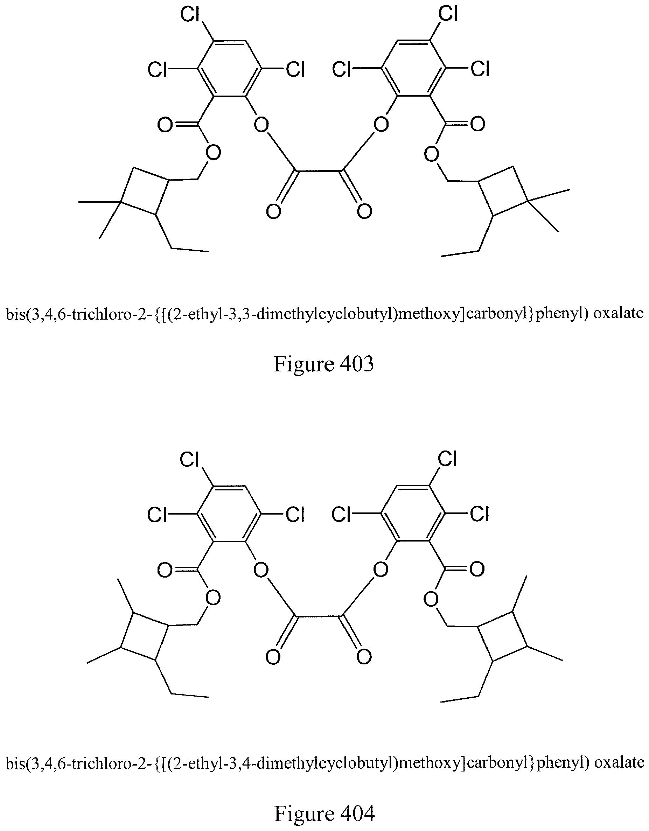

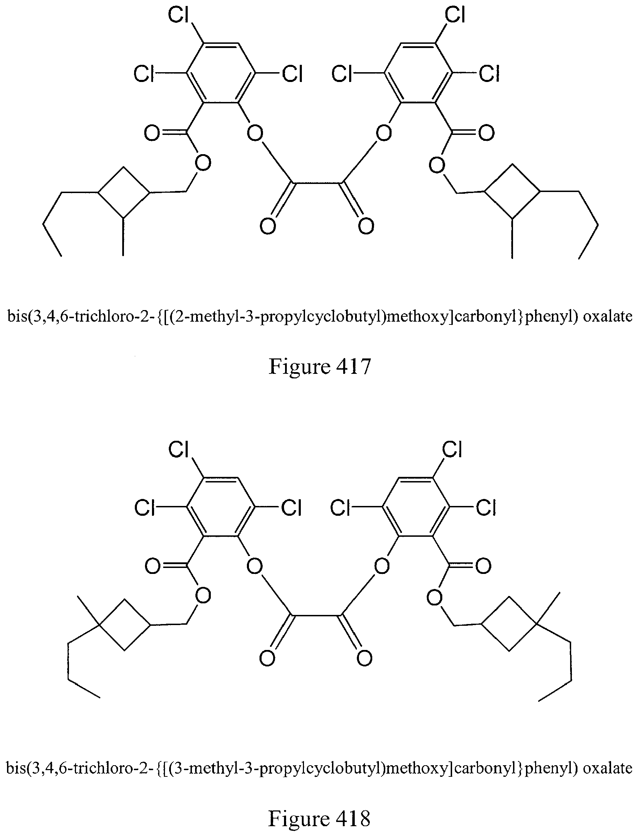

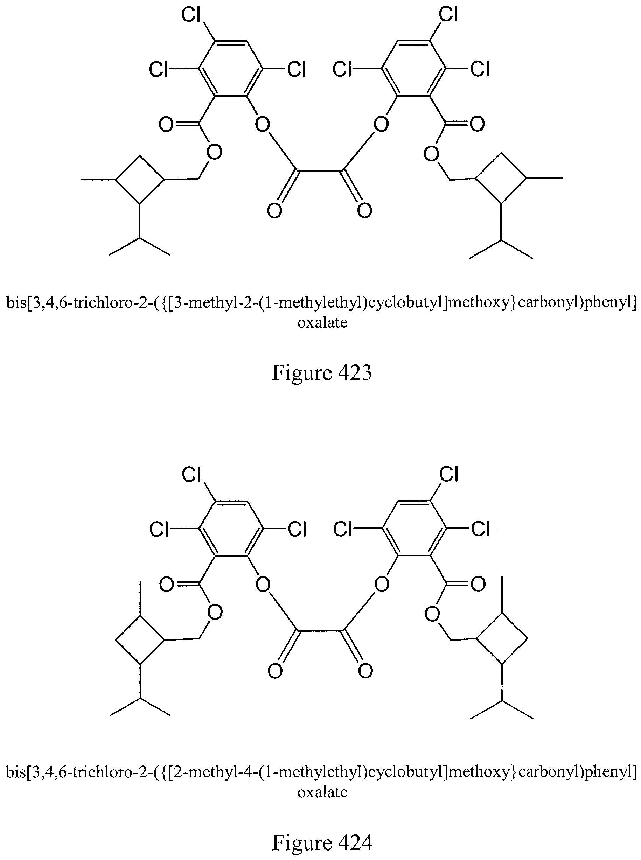

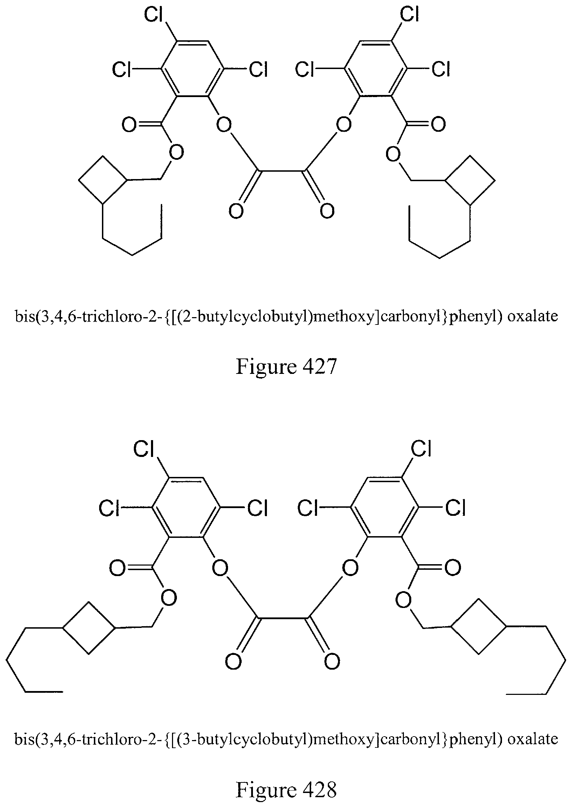

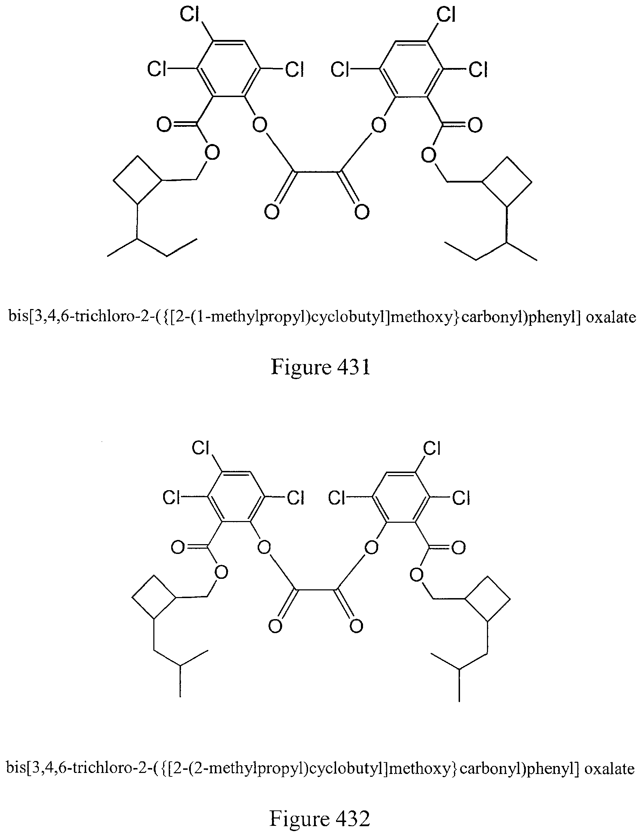

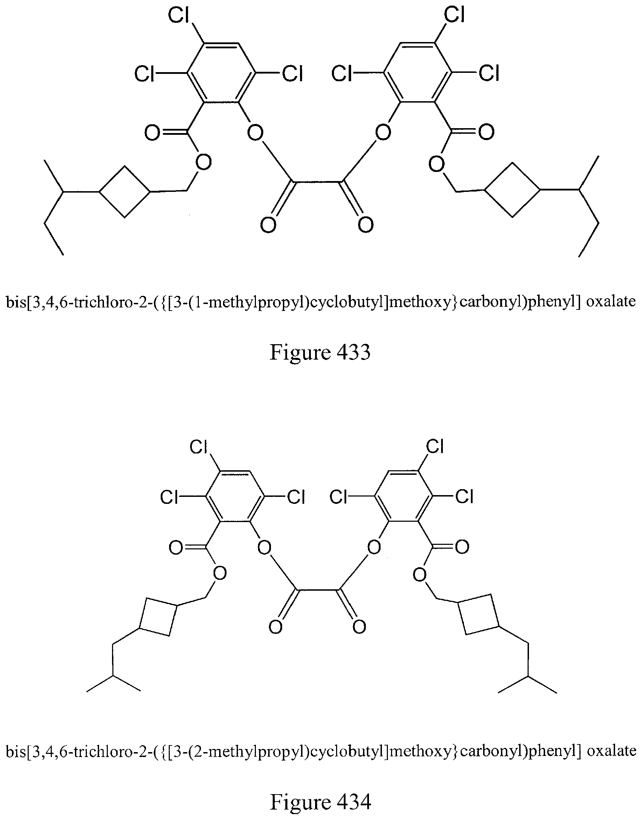

This method was used for the compounds shown in Figures 2, 6-8, 17-25, 30, 47-50, 52-59, 70, 85, 121-125, 127-137, 143-155, 182-191, 225, 306, 308-310, 316-323, 325-327, 335-337, 339-341, 346-347, 352-357, 359, 361-362, 364-365, 368-370, 392-395, 403-405, 408-409, 411, 414-415, 417-418, 423-425, 427-428, 431-434, 618-619, 645, and 653.

Additionally, a tertiary amine other than triethylamine can be used, including tripropyl amine, tri-n-butyl amine, triisobutylamine, triisopropylamine, triisopentylamine, and N,N-diisopropylethyl amine.

### EXAMPLE 5 (REFERENCE EXAMPLE)

Bis{3,4,6-trichloro-2-[(pentoxy)carbonyl] phenyl} oxalate, a straight chain oxalate ester, and bis{3,5,6-trichloro-2-[(3-methylbutoxy)carbonyl] phenyl} oxalate made via the route described in Example 1, were examined. Both materials were placed within open glass bottles and stored in a cabinet in a room where the humidity was maintained at 50% +/- 5% Relative Humidity. Within two weeks the bis{3,4,6-trichloro-2-[(pentoxy)carbonyl] phenyl} oxalate had changed significantly in physical appearance. HPLC analysis revealed that this material had reverted to n-pentyl trichlorosalicylic acid. Compounding this material into an oxalate solution and admixing with an activator solution resulted in no light. The bis{3,5,6-trichloro-2-[(3-methylbutoxy)carbonyl] phenyl} oxalate was also compounded into an oxalate solution and admixed with an activator solution resulting in the generation of light.

### EXAMPLE 6

Bis{3,4,6-trichloro-2-[(pentoxy)carbonyl] phenyl} oxalate, a straight chain oxalate ester was made via the route described in Example 1. Bis{3,5,6-trichloro-2[(benzylmethoxy) carbonyl] phenyl} oxalate was made via the route described in Example 2. Both materials were placed within open glass bottles and stored in a cabinet in a room where the humidity was maintained at 50% +/- 5% Relative Humidity. Within two weeks the bis{3,4,6-trichloro-2-[(pentoxy)carbonyl] phenyl} oxalate had changed significantly in physical appearance. HPLC analysis revealed that this material had reverted to n-pentyl trichlorosalicylic acid. Compounding this material into an oxalate solution and admixing with an activator solution resulted in no light. The bis{3,5,6-trichloro-2[(benzylmethoxy) carbonyl] phenyl} oxalate was also compounded into an oxalate solution and admixed with an activator solution resulting in the generation of light.

### EXAMPLE 7 (REFERENCE EXAMPLE)

Bis{3,4,6-trichloro-2-[(pentoxy)carbonyl] phenyl} oxalate, a straight chain oxalte ester, and bis{3,5,6-trichloro-2-[(2-methylbutoxy)carbonyl] phenyl} oxalate were made via the route described in Example 1. Both materials were placed within open glass bottles and stored in a cabinet in a room where the humidity was maintained at 50% +/- 5% Relative Humidity Within two weeks the bis{3,4,6-trichloro-2-[(pentoxy)carbonyl] phenyl} oxalate had changed significantly in physical appearance. HPLC analysis revealed that this material had reverted to n-pentyl trichlorosalicylic acid. Compounding this material into an oxalate solution and admixing with an activator solution resulted in no light. Bis {3,5,6-trichloro-2-[(2-methylbutoxy)carbonyl] phenyl} oxalate was also compounded into an oxalate solution and admixed with an activator solution resulting in the generation of light.

### EXAMPLE 8

Bis{3,4,6-trichloro-2-[(pentoxy)carbonyl] phenyl} oxalate was made via the route described in Example 1. Bis{3,4,6-trichloro-2-[(cyclohexylmethoxy)carbonyl]phenyl} oxalate was made via the route described in Example 4. Both materials were placed within open glass bottles and stored in a cabinet in a room where the humidity was maintained at 50% +/- 5% Relative Humidity. Within two weeks the bis{3,4,6-trichloro-2-[(pentoxy)carbonyl] phenyl} oxalate had changed significantly in physical appearance. HPLC analysis revealed that this material had reverted to n-pentyl trichlorosalicylic acid. Compounding this material into an oxalate solution and admixing with an activator solution resulted in no light. The bis{3,4,6-trichloro-2-[(cyclohexylmethoxy)carbonyl]phenyl} oxalate was also compounded into an oxalate solution and admixed with an activator solution resulting in the generation of light.

Chemical light systems, also known as chemiluminescent systems, for production of a chemiluminescent light, generally utilize a two-component system to chemically generate light. Chemiluminescent light is produced by combining the two components, which are usually in the form of chemical solutions referred to as the "oxalate" component and the "activator" component. All suitable activator compositions, inclusive of the various additional fluorescers, catalysts and the like, known to be useful in the prior art, are contemplated for use with the branched chain oxalate species of the present invention.

All patents and publications mentioned in this specification are indicative of the levels of those skilled in the art to which the invention pertains.

## Claims

1. A branched chain oxalic acid ester selected from the group consisting of bis{3,4,6-trichloro-2-[(cyclopentylmethoxy)carbonyl]phenyl} oxalate, bis{3,4,6-trichloro-2-[(cyclohexylmethoxy)carbonyl]phenyl} oxalate, bis{3,4,6-trichloro-2-[(phenylmethoxy)carbonyl]phenyl} oxalate, bis{3,4,6-trichloro-2-[(2-phenylethoxy)carbonyl]phenyl} oxalate, bis(3,4,6-trichloro-2-{[(2-methylphenyl)methoxy]carbonyl}phenyl) oxalate, bis(3,4,6-trichloro-2-{[(3-methylphenyl)methoxy]carbonyl}phenyl) oxalate, bis(3,4,6-trichloro-2-{[(4-methylphenyl)methoxy]carbonyl}phenyl) oxalate, bis(3,4,6-trichloro-2-{[(2,3-dimethylphenyl)methoxy]carbonyl}phenyl) oxalate, bis(3,4,6-trichloro-2-{[(2,4-dimethylphenyl)methoxy]carbonyl}phenyl) oxalate, bis(3,4,6-trichloro-2-{[3,4-dimethylphenyl)methoxy]carbonyl}phenyl) oxalate, bis(3,4,6-trichloro-2-{[(3,5-dimethylphenyl)methoxy]carbonyl}phenyl) oxalate, bis(3,4,6-trichloro-2-{[(2,6-dimethylphenyl)methoxy]carbonyl}phenyl) oxalate, bis(3,4,6-trichloro-2-{[(2-ethylphenyl)methoxy]carbonyl}phenyl) oxalate, bis(3,4,6-trichloro-2-{[(3-ethylphenyl)methoxy]carbonyl}phenyl) oxalate, bis(3,4,6-trichloro-2-{[(4-ethylphenyl)methoxy]carbonyl}phenyl) oxalate, bis(3,4,6-trichloro-2-{[2-(2-methylphenyl)ethoxy]carbonyl}phenyl) oxalate, bis(3,4,6-trichloro-2-{[2-(3-methylphenyl)ethoxy]carbonyl}phenyl) oxalate, bis(3,4,6-trichloro-2-{[2-(4-methylphenyl)ethoxy]carbonyl}phenyl) oxalate, bis{3,4,6-trichloro-2-[(2-phenylpropoxy)carbonyl]phenyl} oxalate, bis {3,4,6-trichloro-2-[(3-phenylpropoxy)carbonyl]phenyl} oxalate, bis{3,4,6-trichloro-2-[1-naphthalenylmethoxy)carbonyl]phenyl} oxalate, bis {3,4,6-trichloro-2-[2-naphthalenylmethoxy)carbonyl]phenyl} oxalate, bis{3,4,6-trichloro-2-[(2,2-diphenylethoxy)carbonyl]phenyl} oxalate, bis {3,4,6-trichloro-2-[(9-fluorenylmethoxy)carbonyl]phenyl} oxalate, and bis{3,4,6-trichloro-2-[(9-anthracenylmethoxy)carbonyl]phenyl} oxalate.

2. A chemical light system comprising an activator solution and a branched chain oxalic acid ester in accordance with claim 1.

## Patentansprüche

1. Ein verzweigtkettiger Oxalsäureester ausgewählt aus der Gruppe, bestehend aus bis {3,4,6-Trichloro-2-[(cyclopentylmethoxy)carbonyl]phenyl} oxalat, bis {3,4,6-Trichloro-2-[(cyclohexylmethoxy)carbonyl]phenyl} oxalat, bis{3,4,6-Trichloro-2-[(phenylmethoxy)carbonyl]phenyl}oxalat, bis{3,4,6-Trichloro-2-[(2-phenylethoxy)carbonyl]phenyl}oxalat, bis(3,4,6-Trichloro-2-{[(2-methylphenyl)methoxy]carbonyl}phenyl)oxalat, (3,4,6-Trichloro-2-{[(3-methylphenyl)methoxy]carbonyl}phenyl)oxalat, bis(3,4,6-Trichloro-2-{[(4-methylphenyl)methoxy]carbonyl}phenyl)oxalat, bis(3,4,6-Trichloro-2-{[(2,3-dimethylphenyl)methoxy]carbonyl}phenyl)oxalat, bis(3,4,6-Trichloro-2-{[(2,4-dimethylphenyl)methoxy]carbonyl}phenyl)oxalat, bis(3,4,6-Trichloro-2-{[3,4-dimethylphenyl)methoxy]carbonyl}phenyl)oxalat, bis(3,4,6-Trichloro-2-{[(3,5-dimethylphenyl)methoxy]carbonyl}phenyl)oxalat, bis(3,4,6-Trichloro-2-{[(2,6-dimethylphenyl)methoxy]carbonyl}phenyl)oxalat, bis(3,4,6-Trichloro-2-{[(2-ethylphenyl)methoxy]carbonyl}phenyl)oxalat, bis(3,4,6-Trichloro-2-{[(3-ethylphenyl)methoxy]carbonyl}phenyl)oxalat, bis(3,4,6-Trichloro-2-{[(4-ethylphenyl)methoxy]carbonyl}phenyl)oxalat, bis(3,4,6-Trichloro-2-{[2-(2-methylphenyl)ethoxy]carbonyl}phenyl)oxalat, bis(3,4,6-Trichloro-2-{[2-(3-methylphenyl)ethoxy]carbonyl}phenyl)oxalat, bis(3,4,6-Trichloro-2-{[2-(4-methylphenyl)ethoxy]carbonyl}phenyl)oxalat, bis{3,4,6-Trichloro-2-[(2-phenylpropoxy)carbonyl]phenyl}oxalat, bis {3,4,6-Trichloro-2-[(3-phenylpropoxy)carbonyl]phenyl}oxalat, bis{3,4,6-Trichloro-2-[1-naphthalenylmethoxy)carbonyl]phenyl}oxalat, bis{3,4,6-Trichloro-2-[2-naphthalenylmethoxy)carbonyl]phenyl}oxalat, bis{3,4,6-Trichloro-2-[(2,2-diphenylethoxy)carbonyl]phenyl}oxalat, bis{3,4,6-Trichloro-2-[(9-fluorenylmethoxy)carbonyl]phenyl}oxalat und bis{3,4,6-Trichloro-2-[(9-anthracenylmethoxy)carbonyl]phenyl}oxalat.

2. Ein chemisches Lichtsystem, umfassend eine Aktivatorlösung und einen verzweigtkettigen Oxalsäureester gemäß Anspruch 1.

## Revendications

1. Ester d'acide oxalique à chaîne ramifiée choisi dans le groupe consistant en le bis{3,4,6-trichloro-2-[(cyclopentylméthoxy)carbonyl]phényl} oxalate, le bis{3,4,6-trichloro-2-[(cyclohexylméthoxy)carbonyl]phényl} oxalate, le bis{3,4,6-trichloro-2-[(phénylméthoxy)carbonyl]phényl} oxalate, le bis{3,4,6-trichloro-2-[(2-phényléthoxy)carbonyl]phényl} oxalate, le bis(3,4,6-trichloro-2-{[(2-méthylphényl)méthoxy]carbonyl} phényl) oxalate, le bis(3,4,6-trichloro-2-{[(3-méthylphényl)méthoxy]carbonyl}phényl) oxalate, le bis(3,4,6-trichloro-2-{[(4-méthylphényl)méthoxy]carbonyl} phényl) oxalate, le bis(3,4,6-trichloro-2-{[(2,3-diméthylphényl)méthoxy]carbonyl}phényl) oxalate, le bis(3,4,6-trichloro-2-{[(2,4-diméthylphényl)méthoxy] carbonyl}phényl) oxalate, le bis(3,4,6-trichloro-2-{[3,4-diméthylphényl)méthoxy]carbonyl}phényl) oxalate, le bis(3,4,6-trichloro-2-{[(3,5-diméthylphényl)méthoxy] carbonyl}phényl) oxalate, le bis(3,4,6-trichloro-2-{[(2,6-diméthylphényl)méthoxy]carbonyl}phényl) oxalate, le bis(3,4,6-trichloro-2-{[(2-éthylphényl)méthoxy]carbonyl} phényl) oxalate, le bis(3,4,6-trichloro-2-{[(3-éthylphényl)méthoxy]carbonyl}phényl) oxalate, le bis(3,4,6-trichloro-2-{[(4-éthylphényl)méthoxy]carbonyl}phényl) oxalate, le bis(3,4,6-trichloro-2-{[2-(2-méthylphényl)éthoxy]carbonyl}phényl) oxalate, le bis(3,4,6-trichloro-2-{[2-(3-méthylphényl)éthoxy]carbonyl}phényl) oxalate, le bis(3,4,6-trichloro-2-{[2-(4-méthylphényl)éthoxy]carbonyl}phényl) oxalate, le bis{3,4,6-trichloro-2-[(2-phénylpropoxy)carbonyl]phényl} oxalate, le bis{3,4,6-trichloro-2-[(3-phénylpropoxy)carbonyl]phényl} oxalate, le bis{3,4,6-trichloro-2-[1-naphtalénylméthoxy)carbonyl]phényl} oxalate, le bis{3,4,6-trichloro-2-[2-naphtalénylméthoxy)carbonyl]phényl} oxalate, le bis{3,4,6-trichloro-2-[(2,2-diphényléthoxy)carbonyl]phényl} oxalate, le bis{3,4,6-trichloro-2-[(9-fluorénylméthoxy)carbonyl]phényl} oxalate et le bis{3,4,6-trichloro-2-[(9-anthracénylméthoxy)carbonyl]phényl} oxalate.

2. Système de lumière chimique comprenant une solution activatrice et un ester d'acide oxalique à chaîne ramifiée selon la revendication 1.
